# EUROPEAN PATENT SPECIFICATION

(11) **EP 1 499 620 B1**
(45) Date of publication and mention of the grant of the patent: **27.01.2010**
(21) Application number: 03726543.6
(22) Date of filing: 30.04.2003
(51) Int. Cl.: C07D 499/88, C07D 519/00

(54) **PROCESS FOR PREPARING 6-ALKYLIDENE PENEM DERIVATIVES**
VERFAHREN ZUR HERSTELLUNG VON 6-ALKYLIDEN-PENEMDERIVATEN
PROCEDE DE PREPARATION DE DERIVES DE 6-ALKYLIDENE PENEM

(30) Priority: 01.05.2002 US 377048 P
(43) Date of publication of application: 26.01.2005
(73) Proprietor: Wyeth, Madison, NJ 07940 (US)
(72) Inventor: ABE, Takao, Saitama 350-0227 (JP); MATSUNAGA, Hiroshi, Saitama 350-0064 (JP); MIHIRA, Ado, Saitama 351-0025 (JP); SATO, Chisato, Saitama 336-0931 (JP); USHIROGOCHI, Hideki, Saitama 352-0002 (JP); SATO, Koichi, Saitama 336-0931 (JP); TAKASAKI, Tsuyoshi, Saitama 336-0931 (JP); VENKATESAN, Aranapakam Mudumbai, Rego Park, NY 11374 (US); MANSOUR, Tarek Suhayl, New City, NY 10956 (US)
(74) Representative: Wileman, David Francis
(86) International application number: PCT/US2003/013429
(87) International publication number: WO 2003/093277

(56) References cited:
- EP-A- 0 232 966
- EP-A- 0 321 186
- WO-A-87/00525
- WO-A-94/10178
- OSBORNE N F ET AL: "A NOVEL AND STEREOCONTROLLED SYNTHESIS OF (5R)-(Z)-6-(1-METHYL-1,2,3- TRIAZOL-4-YLMETHYLENE)PENEM-3-CARBOXYLICAC ID, A POTENT BROAD SPECTRUM BETA-LACTAMASE INHIBITOR" JOURNAL OF THE CHEMICAL SOCIETY, CHEMICAL COMMUNICATIONS, CHEMICAL SOCIETY. LETCHWORTH, GB, no. 6, 1989, pages 371-373, XP000985235 ISSN: 0022-4936

## Description

This invention relates to a novel process for the production of 6-alkylidene penem derivatives that can be important as broad spectrum β-lactamase inhibitors and anti-bacterial agents.

β-Lactamases are enzymes produced by the bacteria, that hydrolyze β-lactam antibiotics and as such serve as the primary cause of bacterial resistance. Penicillins and cephalosporins are the most frequently and widely used β-lactam antibiotics in the clinic. However, the development of resistance to β-lactam antibiotics by different pathogens has had a damaging effect on maintaining the effective treatment of bacterial infections. (Coleman, K. Expert Opin. Invest. Drugs 1995, 4, 693; Sutherland, R. Infection 1995, 23 (4) 191; Bush, K, Cur. Pharm. Design 1999, 5, 839-845) The most significant known mechanism related to the development of bacterial resistance to the β-lactam antibiotics is the production of class-A, class-B and class-C serine β-lactamases. These enzymes degrade the β-lactam antibiotics, resulting in the loss of antibacterial activity. Class-A enzymes preferentially hydrolyze penicillins where as Class-C lactamases have a substrate profile favoring cephalosporin hydrolysis. (Bush, K.; Jacoby, G.A.; Medeiros, A.A. Antimicrob. Agents Chemother. 1995, 39, 1211). To date over 250 different β-lactamases have been reported (Payne, D.J,: Du, W and Bateson, J.H. Exp. Opin. Invest. Drugs 2000, 247.) and there is a need for a new generation of broad spectrum β-lactamases inhibitors. Bacterial resistance to these antibiotics could be greatly reduced by administering the β-lactam antibiotics in combination with a compound which inhibits these enzymes.

The commercially available β-lactamase inhibitors such as clavulanic acid, sulbactam and tazobactam are all effective against class-A producing pathogens.

The mechanism of inactivation of class-A β-lactamases (such as PCI and TEM-1) has been elucidated. (Bush, K.; Antimicrob. Agents Chemother. 1993, 37, 851; Yang, Y.; Janota, K.; Tabei, K.; Huang, N.; Seigal, M.M.; Lin, Y.L; Rasmussen, B.A. and Shalaes, D.M. J. Biol. Chem. 2000, 35, 26674-26682) However, these compounds are ineffective against class-C producing organisms. Clavulanic acid is used in combination with amoxicillin and ticarcillin; similarly sulbactam with ampicillin and tazobactam with piperacillin..

EP 0 041 768A, EP 0 120 613 A, EP 0 150 781, EP 0210065, WO 87/00525, EP 0 003 960 B1, GB 2 042 514 A, GB 2 042 515 A, EP 0 087 792 A, EP 0115 308, A, GB 2 1246148, EP 0 150984 A, EP 0087792 , EP 0115308, EP 81 301683.9, EP 84 301255.0, EP 85100520.7, EP 85100521.5, EP 86305585.1, EP 86 305584, EP 88 311786.3, EP 88 311787.1, EP 87 300193.7, WO 93/03042, WO 94/10178, WO 95/28935 and WO 95/17184 disclose 6-(substituted methylene)-penem as β-lactamase inhibitors. EP 0 232 966 B1 discloses a process for preparing 6-(substituted methylene)-penems and their intermediates. According to this patent, the compounds of general formula **I** were prepared by a four step process starting from p-methoxybenzyl(5R,6S) 6-bromopenem-3-carboxylate **A** and an aldehyde in the presence of pyrophoric reagents such as n.butyllithium/diphenyl amine or lithium bis(trimethylsilyl)amide at -78°C (Scheme 1). The third step of this process, namely the reductive elimination step gave E and Z compounds isomers **D** and **F** in the ratio of 5:1 respectively.

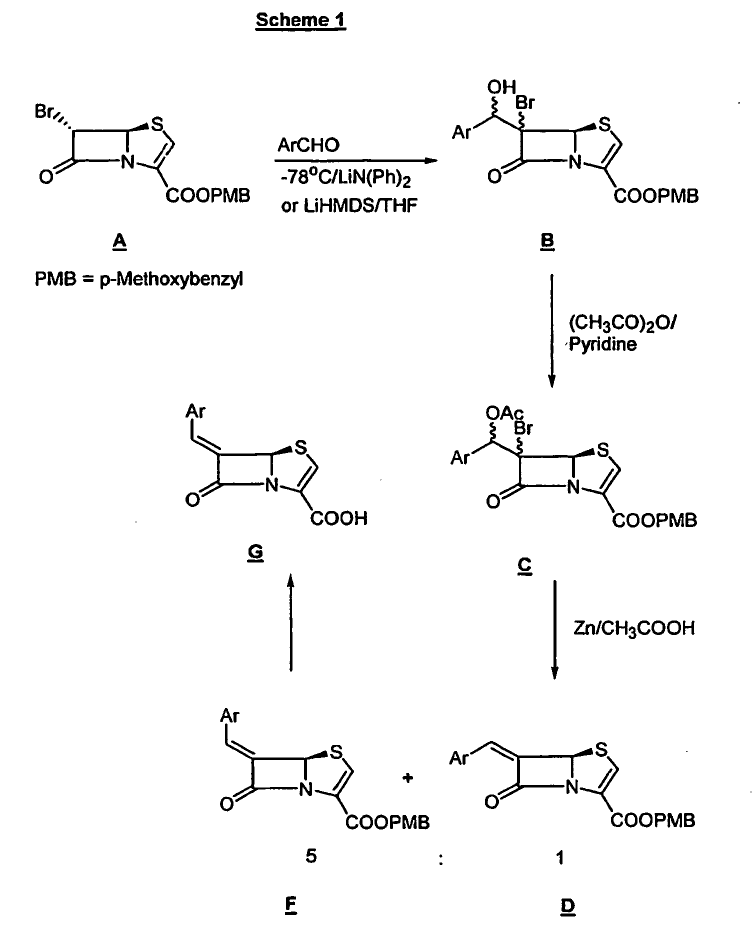

### SUMMARY OF THE INVENTION

The present invention relates to a process for the preparation of compound of formula **I** 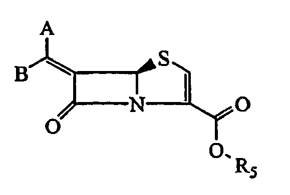 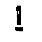 wherein:
one of A and B denotes hydrogen and the other is an aryl optionally substituted with one or two R₂, heteroaryl optionally substituted with one or two R₂, fused bicyclic heteroaryl optionally substituted with one or two R₂, fused tricyclic heteroaryl optionally substituted with one or two R₂, cycloalkyl optionally substituted with one or two R₂, alkyl optionally substituted with one or two R₂, alkenyl optionally substituted with one or two R₂, alkynyl optionally substituted with one or two R₂, saturated or partially saturated heteroaryl optionally substituted with one or two R₂; and wherein any of said heteroaryl moieties containing a NH ring atom may be optionally substituted on said nitrogen by R₁;
R₅ is H, C1 -C6 alkyl, C5 - C6 cycloalkyl, or CHR₃OCOC1-C6alkyl or a salt thereof;
R₁ is H, optionally substituted alkyl, optionally substituted aryl, optionally substituted heteroaryl or mono or bicyclic saturated heterocycles, optionally substituted cycloalkyl, optionally substituted alkenyl, optionally substituted alkynyl with the proviso that neither the double bond nor the triple bond should be present at the carbon atom which is directly linked to N; optionally substituted perfluoroalkyl, -S(O)p optionally substituted alkyl or aryl where p is 0-2, optionally substituted - C=Oheteroaryl, optionally substituted -C=Oaryl, optionally substituted -C=Oalkyl, optionally substituted -C=Ocycloalkyl, optionally substituted -C=O mono or bicyclic saturated heterocycles, optionally substituted C1-C6 alkylaryl, optionally substituted C1-C6 alkylheteroaryl, optionally substituted aryl-C1-C6alkyl, optionally substituted heteroaryl-C1-C6alkyl, optionally substituted C1-C6 alkyl mono or bicyclic saturated heterocycles, optionally substituted arylalkenyl of 8 to 16 carbon atoms, -CONR₆R₇, -SO₂NR₆R₇, optionally substituted arylalkyloxyalkyl, optionally substituted -alkyl-O-alkyl-aryl, optionally substituted -alkyl-O-alkyl-heteroaryl, optionally substituted aryloxyalkyl, optionally substituted heteroaryloxyalkyl, optionally substituted aryloxyaryl, optionally substituted aryloxyheteroaryl, optionally substituted C1-C6alkylaryloxyaryl, optionally substituted C1-C6 alkylaryloxyheteroaryl , optionally substituted alkylaryloxyalkylamines, optionally substituted alkoxycarbonyl, optionally substituted aryloxycarbonyl, or optionally substituted heteroaryloxy carbonyl;
R₂ is hydrogen, optionally substituted C1-C6 alkyl, optionally substituted C2-C6 alkenyl, optionally substituted C2-C6 alkynyl, halogen, cyano, N-R₆R₇, optionally substituted C1-C6 alkoxy, hydroxy; optionally substituted aryl, optionally substituted heteroaryl, COOR₆, optionally substituted alkylaryloxyalkylamines, optionally substituted aryloxy, optionally substituted heteroaryloxy, optionally substituted C3-C6 alkenyloxy, optionally substituted C3-C6 alkynyloxy, C1-C6 alkylamino-C1-C6 alkoxy, alkylenedioxy, optionally substituted aryloxy-C1-C6 alkyl amine, C1-C6 perfluoro alkyl, S(O)_{q}-optionally substituted C1-C6 akyl, S(O)_{q}- optionally substituted aryl where q is 0, 1 or 2, CONR₆R₇, guanidino or cyclic guanidino, optionally substituted alkylaryl, optionally substituted arylalkyl, optionally substituted C1-C6 alkylheteroaryl, optionally substituted heteroaryl-C1-C6 alkyl, optionally substituted C1-C6 alkyl mono or bicyclic saturated heterocycles, optionally substituted arylalkenyl of 8 to 16 carbon atoms, SO₂NR₆R₇, optionally substituted arylalkyloxyalkyl, optionally substituted aryloxyalkyl, optionally substituted heteroaryloxyalkyl, optionally substituted aryloxyaryl, optionally substituted aryloxyheteroaryl, optionally substituted heteroaryloxyaryl, optionally substituted C1-C6alkyl aryloxyaryl, optionally substituted C1-C6 alkylaryloxyheteroaryl , optionally substituted aryloxyalkyl, optionally substituted heteroaryloxyalkyl, or optionally substituted alkylaryloxyalkylamine;
R₃ is hydrogen, C1-C6 alkyl, C5 - C6 cycloalkyl, optionally substituted aryl, optionally substituted heteroaryl;
R₆ and R₇ are independently H, optionally substituted C1-C6 alkyl, optionally substituted aryl, optionally substituted heteroaryl, optionally substituted C1-C6 alkyl aryl, optionally substituted arylalkyl, optionally substituted heteroarylalkyl, optionally substituted C1-C6 alkyl heteroaryl, R₆ and R₇ can be together to form a 3-7 membered saturated ring system optionally having one or two heteroatoms such as N-R₁, O, S=(O)ₙ n = 0-2;
said process comprising:
   (a) condensing an appropriately substituted aldehyde **17**

      **A'-CHO** **17**

      wherein A' is A as defined as above when B is hydrogen, or B as defined above when A is hydrogen)
      with 6-bromo-penem derivative of structure **16** 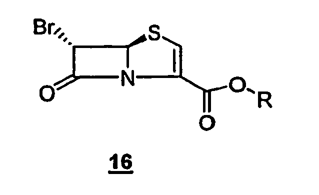 wherein R is p-nitrobenzyl, in the presence of a Lewis acid and a mild base, at low temperature to form an intermediate aldol product **18** 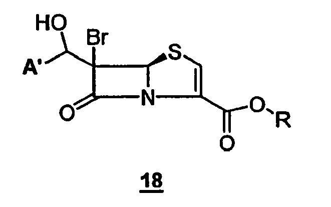 wherein A' and R are as defined above;
   (b) reacting intermediate **18** with an acid chloride or anhydride of formula: (R₈)Cl or
      (R₈)₂O, or with tetrahalomethane of formula: C(X₁)₄, and triphenyl phosphine, wherein R₈ is alkylSO₂, arylSO₂, alkylCO, or arylCO; X₁ is Br, I, or Cl; to form intermediate compound **19** 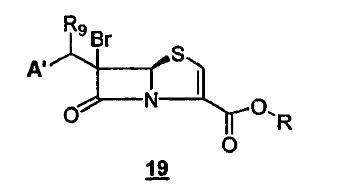 wherein R₉ is X₁ or OR₈ wherein R₈, X₁, A' and R are as defined above; and (c) converting the intermediate compound **19** to the desired formula I compound wherein R₅ is hydrogen by a reductive elimination process; and if desired converting to a pharmaceutically acceptable salt or to an ester wherein R₅ is C1 -C6 alkyl, C5 - C6 cycloalkyl, or CHR₃OCOC1-C6alkyl.

This invention also provides the 6-bromo-penem derivative of structure **16** 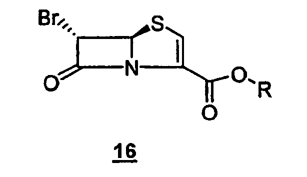 wherein R is p-nitrobenzyl used in step (a) above.

This invention further provides a process for the preparation of the 4-nitrobenzyl (5R,6S)-6-bromopenem-3-carboxylate of formula **16** which comprises the following steps:
(A) (i) reacting 6-aminopenicillanic acid with hydrobromic acid in an organic solvent and water to form the 6-bromo derivative **21** 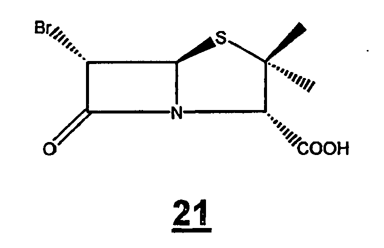 and (ii) converting the 6-bromopenicillanic acid **21** derivative to the p-nitrobenzyl 6-brompenicillanate **22** 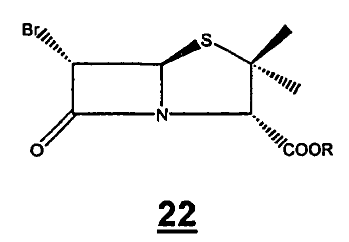 whrein R is p-nitrobenzyl, using 4-nitrobenzylbromide in the presence of base in an organic solvent;
(B) oxidizing the 4-nitrobenzyl 6-bromopenicillanate **22** to form 4-nitrobenzyl 6-bromopenicillanate 1-oxide **23** 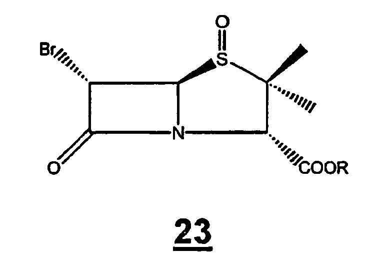
(C) refluxing the 4-nitrobenzyl 6-bromopenicillanate 1-oxide **23** with 2-mercaptobenzothiazole in an aromatic solvent to form 4-nitrobenzyl(2R)-2-[(3S,4R)-4-(benzothiazol-2-yldithio)-3-bromo-2-oxoazetidine-1-yl]-3-methylbut-3-enoate **24 ;**
(D) dissolving the 4-nitrobenzyl(2R)-2-[(3S,4R)-4-(benzothiazol-2-yldithio)-3-bromo-2-oxoazetidine-1-yl]-3-methylbut-3-enoate **24** in an organic solvent and reacting with an organic tertiary base to form 4-nitrobenzyl-2-[(3S,4R)-4-(benzothiazol-2-yldithio)-3-bromo-2-oxoazetidine-1-yl]-3-methylbut-2-enoate **25**
(E) converting the 4-nitrobenzyl-2-[(3S,4R)-4-(benzothiazol-2-yldithio)-3-bromo-2-oxoazetidine-1-yl]-3-methylbut-2-enoate **25** to 4-nitrobenzyl 2-[(3S,4R)-3-bromo-4-formylthio-2-oxoazetidin-1-yl]-3-methylbut-2-enoate **26** by reacting in an aromatic organic solvent in the presence of an organic acid, acetic anhydride/ organic tertiary base and trialkyl or triaryl phosphine at from about -10° C to about -30° C;
(F) said 4-nitrobenzyl 2-[(3S,4R)-3-bromo-4-formylthio-2-oxoazetidin-1-yl]-3-methylbut-2-enoate **26** being taken up in an organic solvent at -70°C to -90° C and ozonized oxygen being passed through it for 3 to 4 hrs followed by intramolecular cyclization using a phosphite reagent to form 4-nitrobenzyl (5R,6S)-6-bromopenem-3-carboxylate **16.**
Conveniently the process may be carried out using the following steps:
**Step 1:** Dissolving 6-aminopenicillanic acid **20** in an organic solvent (preferably methanol or THF) and water to form the 6-bromo derivative in **21** the presence of 48% w/w hydrobromic acid at -10° C to -30°C and sodium or potassium nitrite solution. The 6-bromopenicillanic acid **21** derivative either can be isolated or *insitu* converted to the p-Nitrobenzyl 6-brompenicillanate **22** using 4-nitrobenzylbromide in the presence of organic bases or inorganic bases (preferably sodium or potassium carbonate) in an organic solvent (preferably THF or DMF).
**Step 2:** The product 4-nitrobenzyl 6-bromopenicillanate **22** obtained by the process outlined in step 1 can be isolated or be transformed to 4-nitrobenzyl 6-bromopenicillanate 1-oxide **23** in the same pot (i.e Step 1; sequential formation) by oxidizing **22** to 4-nitrobenzyl 6-bromopenicillanate 1-oxide **23** using any known oxidizing agents such as 3-chloroperoxybenzoic acid (mcpba) or hydrogen peroxide.
**Step 3:** The product from step 2, namely 4-nitrobenzyl 6-bromopenicillanate 1-oxide **23** can be converted to 4-nitrobenzyl(2R)-2-[(3S,4R)-4-(benzothiazol-2-yldithio)-3-bromo-2-oxoazetidine-1-yl]-3-methylbut-3-enoate **24** by refluxing 4-nitrobenzyl 6-bromopenicillanate 1-oxide **23** with 2-mercaptobenzothiazole in an aromatic solvent (preferably toluene or xylene).
**Step 4:** The product from step 3, namely 4-nitrobenzyl(2R)-2-[(3S,4R)-4-(benzothiazol-2-yldithio)-3-bromo-2-oxoazetidine-1-yl]-3-methylbut-3-enoate **24** can be dissolved in an organic solvent (preferably Toluene or Xylene) and upon reaction with an organic tertiary base (preferably triethylamine) at ambient temperature gave 4-nitrobenzyl-2-[(3S,4R)-4-(benzothiazol-2-yldithio)-3-bromo-2-oxoazetidine-1-yl]-3-methylbut-2-enoate **25.**
**Step 5:** The product from step 4, namely 4-nitrobenzyl-2-[(3S,4R)-4-(benzothiazol-2-yldithio)-3-bromo-2-oxoazetidine-1-yl]-3-methylbut-2-enoate **25** can be converted to **26** 4-nitrobenzyl 2-[(3S,4R)-3-bromo-4-formylthio-2-oxoazetidin-1-yl]-3-methylbut-2-enoate by carrying out the reaction in an aromatic organic solvent (preferably toluene) in the presence of an organic acid (preferably formic acid), acetic anhydride/ organic tertiary base (preferably pyridine) and trialkyl or triaryl phosphine (preferably triphenylphosphine) at -10° C to -30° C (preferably about -15 to 20° C).
   An alternative embodiment of the present invention relates to the sequential conversion of compound **23** to **26** without isolating the intermediate:
   Product from Step 2 namely 4-nitrobenzyl 6-bromopenicillanate 1-oxide **23** may be reacted with mercaptobenzothiazole in refluxing aromatic organic solvent (preferably Toluene) preferably for 1 to 3 hrs and treated with triethylamine at 0 to -20° C for 3 to 4 hrs. After this treatment, reaction mixture is charged with an organic acid (preferably formic acid) and an anhydride (acetic anhydride), an organic tertiary base (preferably pyridine) and a trialkyl or triaryl phosphate sequentially at -10° C to -40° C.
**Step 6:** The product 4-nitrobenzyl 2-[(3S,4R)-3-bromo-4-formylthio-2-oxoazetidin-1-yl]-3-methylbut-2-enoate **26** from either step 5 or from the sequential conversion was taken up in an organic solvent (preferentially ethyl acetate) at -70°C to -90° C and ozonized oxygen was passed through it for 3 to 4 hrs followed by intramolecular cyclization using a phosphite reagent (preferably trimethyl phosphite). The product 4-nitrobenzyl (5R,6S)-6-bromopenem-3-carboxylate **16** was crystallized from ethylacetate:hexane.
   The present invention also relates to the compound represented by the following formula (**16**): 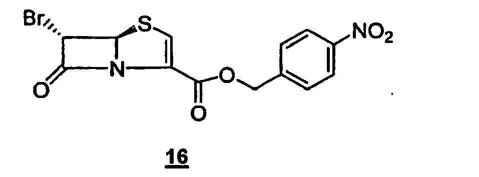
   An above mentioned process (Step 1 to Step 6) for the preparation of the compound represented by the formula **16** is an intermediate useful for the preparation of 6-(substituted methylene)penems of general formula **I**.
   The compound represented by the formula **16** is a crystalline derivative (X-ray powder diffraction parameters are given in the Table 2)
   The crystalline nature of this intermediate imparts stability and thereby increases the shelf-life time. The stability data of the compound of the formula **16** is given in Table 1.
**Step 7:** Reaction of 4-nitrobenzyl (5R,6S)-6-bromopenem-3-carboxylate **16** with the appropriately substituted aldehydes (defind as before) to effect the aldol condensation step can be carried out in the presence of a Lewis acid (preferably anhydrous MgBr₂ or MgBr₂: 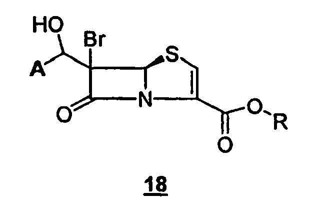 etherate) and a organic tertiary base (preferably Et₃N, pyridine, dimethylamino pyridine (DMAP), or diisopropylethylamine trialkylamine) in an aprotic polar organic solvent(s) (preferably THF and/or acetonitrile) at temperature -10° C to -40°C. The intermediate aldol products of general formula **18** are functionalized to give an ester leaving group, e.g., with an acid chloride or anhydride to give an acetate, triflic anhydride to a triflate or tosylchloride to a tosylate at 0°C to - 10° C in the same pot; or, if formulae **18** is isolated, it can be converted to a halogen derivative by reacting **18** with tetrahalomethane and triphenyl phosphine at room temperature in a suitable organic solvent preferably CH₂Cl₂.
**Step 8:** The final step, namely the reductive elimination step to yield the compound of the general formula **I** can be carried out by dissolving the aldol product mixture in an organic solvent (preferably THF/ acetonitrile) and at a pH range of 6.0 - 8.5, preferably 6.5 to 7, pH phosphate buffer and activated metal such as zinc, tin or aluminum at ambient temperature (preferably at 20° C to 35°C). The product as a alkalie metal salt can be purified by a reverse phase resin column chromatography.
   Alternatively Step 7 and Step 8 can be carried out sequentially in the same pot without isolating the aldol intermediate.
   The final step, namely the reductive elimination step can be carried out by dissolving the aldol product in an organic solvent (such as THF or acetonitrile) and 6.5-7.0 phosphate buffer and hydrogenating over Pd/C at 10 to 100 psi (preferably at 40 psi) pressure.
   A representative example is shown in Scheme A which illustrates the present invention. In Scheme A suitable reaction conditions are shown, however, other reaction conditions may be used without departing from the scope of the invention. For example shorter or longer reaction times may be employed; generally the longer the reaction time the more complete the reaction.

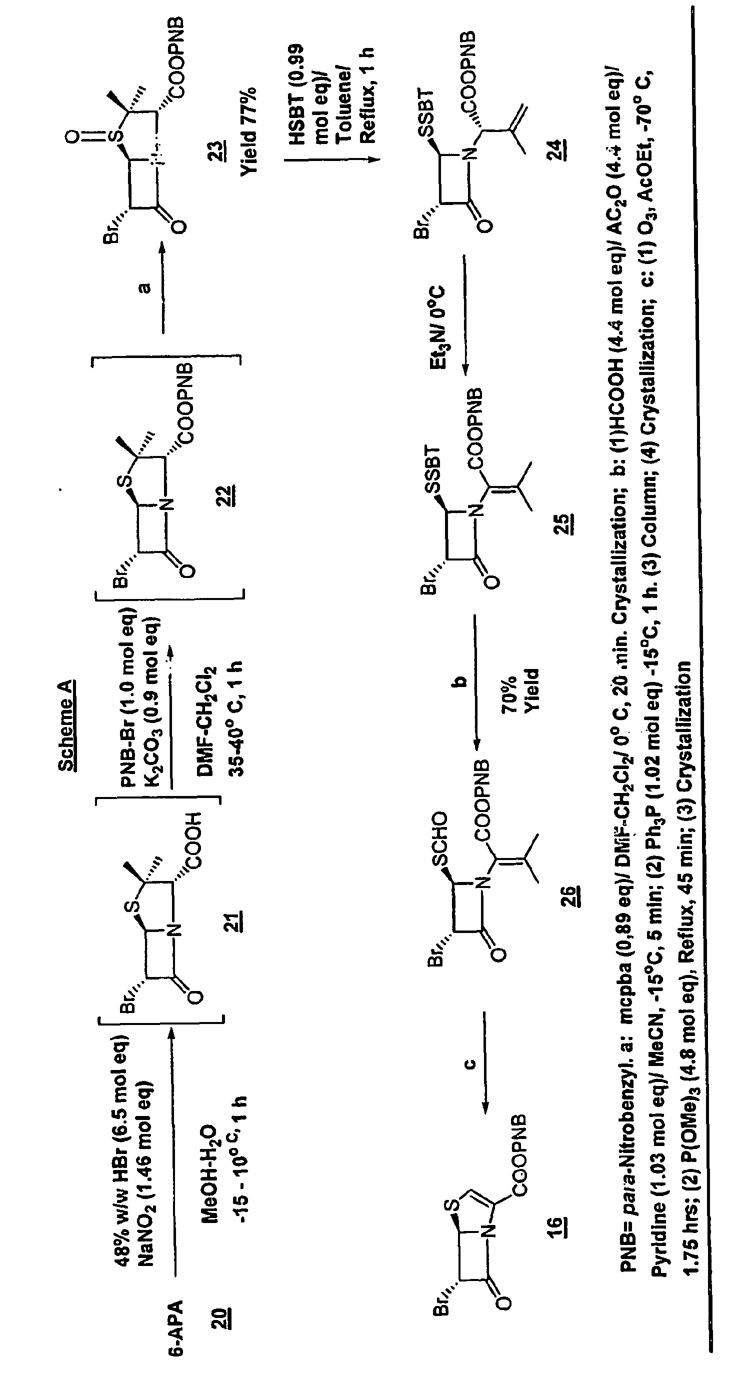

### DESCRIPTION OF THE PREFERRED EMBODIMENTS

The present invention provides a novel process for the preparation of a compound of the formula **I**. 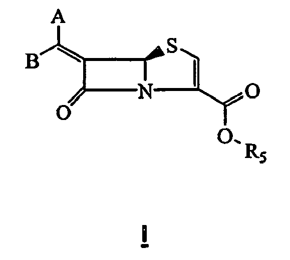 wherein:
One of A and B denotes hydrogen and the other is an aryl optionally substituted with one or two R₂, heteroaryl optionally substituted with one or two R₂, fused tricyclic heteroaryl optionally substituted with one or two R₂, cycloalkyl optionally substituted with one or two R₂, alkyl optionally substituted with one or two R₂, alkenyl optionally substituted with one or two R₂, alkynyl optionally substituted with one or two R₂, saturated or partially saturated heteroaryl optionally substituted with one or two R₂.

R₅ is H, an in vivo hydrolyzable ester such as C1 -C6 alkyl, C5 - C6 cycloalkyl, CHR₃OCOC1-C5 or salts such as Na, K, or Ca.

R₁ is H, optionally substituted -C1-C6 alkyl, optionally substituted -aryl, optionally substituted -heteroaryl or mono or bicyclic saturated heterocycles, optionally substituted -C3-C7 cycloalkyl, optionally substituted -C3-C6 alkenyl, optionally substituted -C3-C6 alkynyl with the proviso that both the double bond and the triple bond should not be present at the carbon atom which is directly linked to N; optionally substituted -C1-C6 per fluoro alkyl, -S(O)p optionally substituted alkyl or aryl where p is 2, optionally substituted -C=Oheteroaryl, optionally substituted - C=Oaryl, optionally substituted -C=O (C1-C6) alkyl, optionally substituted -C=O (C3-C6) cycloalkyl, optionally substituted -C=O mono or bicyclic saturated heterocycles, optionally substituted C1-C6 alkyl aryl, optionally substituted C1-C6 alkyl heteroaryl, optionally substituted aryl-C1-C6 alkyl, optionally substituted heteroaryl-C1-C6 alkyl, optionally substituted C1-C6 alkyl mono or bicyclic saturated heterocycles, optionally substituted arylalkenyl of 8 to 16 carbon atoms, -CONR₆R₇, SO₂NR₆R₇, optionally substituted arylalkyloxyalkyl, optionally substituted -alkyl-O-alkyl-aryl, optionally substituted -alkyl-O-alkyl-heteroaryl, optionally substituted aryloxyalkyl, optionally substituted heteroaryloxyalkyl, optionally substituted aryloxyaryl, optionally substituted aryloxyheteroaryl, optionally substituted C1-C6alkyl aryloxyaryl, optionally substituted C1-C6 alkyl aryloxyheteroaryl , optionally substituted alkyl aryloxy alkylamines, optionally substituted alkoxy carbonyl, optionally substituted aryloxy carbonyl, optionally substituted heteroaryloxy carbonyl. Preferred R₁ groups are H, optionally substituted alkyl, optionally substituted aryl, -C=O(C1-C6)alkyl, C3-C6alkenyl, C3-C6alkynyl, optionally substituted cycloalkyl, SO₂alkyl, SO₂aryl, optionally substituted heterocycles, -CONR₆R₇, and optionally substituted heteroaryl.

R₂ is hydrogen, optionally substituted C1-C6 alkyl, optionally substituted C2-C6 alkenyl having 1 to 2 double bonds, optionally substituted C2-C6 alkynyl having 1 to 2 triple bonds, halogen, cyano, N-R₆R₇, optionally substituted C1-C6 alkoxy, hydroxy; optionally substituted aryl, optionally substituted heteroaryl, COOR₆, optionally substituted alkyl aryloxy alkylamines, optionally substituted aryloxy, optionally substituted heteroaryloxy, optionally substituted C3-C6 alkenyloxy, optionally substituted C3 -C6 alkynyloxy, C1-C6 alkylamino-C1-C6 alkoxy, alkylene dioxy, optionally substituted aryloxy-C1-C6 alkyl amine, C1-C6 perfluoro alkyl, S(O)_{q}- optionally substituted C1-C6 akyl, S(O)_{q}- optionally substituted aryl where q is 0, 1 or 2, CONR₆R₇, guanidino or cyclic guanidino, optionally substituted C1-C6 alkylaryl, optionally substituted arylalkyl, optionally substituted C1-C6 alkylheteroaryl, optionally substituted heteroaryl-C1-C6 alkyl, optionally substituted C1-C6 alkyl mono or bicyclic saturated heterocycles, optionally substituted arylalkenyl of 8 to 16 carbon atoms, SO₂NR₆R₇, optionally substituted arylalkyloxyalkyl, optionally substituted aryloxyalkyl, optionally substituted heteroaryloxyalkyl, optionally substituted aryloxyaryl, optionally substituted aryloxyheteroaryl, substituted heteroaryloxyaryl, optionally substituted C1-C6alkyl aryloxyaryl, optionally substituted C1-C6 alkylaryloxyheteroaryl , optionally substituted aryloxyalkyl, optionally substituted heteroaryloxyalkyl, optionally substituted alkylaryloxyalkylamines. Preferred R₂ groups are H, optionally substituted alkyl, optionally substituted alkoxy, optionally substituted heteroaryl, halogen, CN, hydroxy, optionally substituted heterocycle, -CONR₆R₇, COOR₆, optionally substituted aryl, S(O)_{q}-alkyl, and S(O)_{q}-aryl.

R₃ is hydrogen, C1-C6 alkyl, C5 - C6 cycloalkyl, optionally substituted aryl, optionally substituted heteroaryl.

R₄ is H, optionally substituted C1-C6 alkyl, one of R₄ is OH, C1-C6 alkoxy, -S-C1-C6 alkyl, COOR₆, -NR₆R₇, -CONR₆R₇ ; or R₄R₄ may together be =O or R₄R₄ together with the carbon to which they are attached may form a spiro system of five to eight members with or without the presence of heteroatoms selected N, O, S=(O)n (where n =0 to 2), N-R₁;

R₆ and R₇ are independently H, optionally substituted C1-C6 alkyl, optionally substituted aryl, optionally substituted heteroaryl, optionally substituted C1-C6 alkyl aryl, optionally substituted arylalkyl, optionally substituted heteroarylalkyl, optionally substituted C1-C6 alkyl heteroaryl, R₆ and R₇ can be together to form a 3-7 membered saturated ring system optionally having one or two heteroatoms such as N-R₁, O, S=(O)ₙ n = 0-2.

### Chemical Definitions

The term alkyl means both straight and branched chain alkyl moieties of 1-12 carbons, preferably of 1-6 carbon atoms.

The term alkenyl means both straight and branched alkenyl moieties of 2-8 carbon atoms containing at least one double bond, and no triple bond, preferably the alkenyl moiety has 1 or two double bonds. Such alkenyl moieties may exist in the E or Z conformations; the compounds of this invention include both conformations. In the case of alkenyl, hetero atoms such as O, S or N-R₁ should not be present on the carbon that is bonded to a double bond;

The term alkynyl includes both straight chain and branched alkynyl moieties containing 2-6 carbon atoms containing at least one triple bond, preferably the alkynyl moiety has one or two triple bonds. In the case of alkynyl, hetero atoms such as O, S or N-R₁ should not be present on the carbon that is bonded to a double or triple bond;

The term cycloalkyl refers to a alicyclic hydrocarbon group having 3-7 carbon atoms. The term perfluoroalkyl is used herein to refer to both straight- and branched-chain saturated aliphatic hydrocarbon groups having at least one carbon atom and two or more fluorine atoms. Examples include CF₃, CH₂CF₃, CF₂CF₃ and CH(CF₃)₂.
The term halogen is defined as Cl, Br, F, and I.

If alkyl, alkenyl, alkynyl, or cycloalkyl is "optionally substituted", one or two of the following are possible substituents: nitro, -aryl, -heteroaryl, alkoxycarbonyl-, -alkoxy, -alkoxy-alkyl, alkyl-O-C2-C4alkyl-O-, -cyano, -halogen, -hydroxy, -N-R₆R₇, - trifluoromethyl, -trifluoromethoxy, arylalkyl, alkylaryl, R₆R₇N-alkyl-, HO-C1-C6-alkyl-, alkoxyalkyl-, alkyl-S-, -SO₂N-R₆R₇, -SO₂NHR₆, -CO₂H, CONR₆R₇, aryl-O-, heteroaryl-O-, -S(O)ₛ-aryl (where s = 0 -2), -alkyl-O-alkyl-NR₆R₇, -alkyl-aryl-O-alkylN-R₆R₇, C1-C6alkyl, alkenyl, alkynyl, cycloalkyl, alkoxy-alkyl-O-, R₆R₇N-alkyl-, and -S(O)ₛ-heteroaryl (where s = 0 -2); Preferred substitutents for alkyl, alkenyl, alkynyl, and cycloalkyl include: halogen, nitro, aryl, heteroaryl, alkoxycarbonyl-, alkoxy, -alkoxy-alkyl, -cyano, hydroxy, and -N-R₆R₇.

Aryl is defined as an aromatic hydrocarbon moiety selected from the group: phenyl, α-naphthyl, β-naphthyl, biphenyl, anthryl, tetrahydronaphthyl, fluorenyl, indanyl, biphenylenyl, acenaphthenyl, groups.

Heteroaryl is defined as a aromatic heterocyclic ring system (monocyclic or bicyclic) where the heteroaryl moieties are selected from: (1) furan, thiophene, indole, azaindole, oxazole, thiazole, isoxazole, isothiazole, imidazole, N-methylimidazole, pyridine, pyrimidine, pyrazine, pyrrole, N-methylpyrrole, pyrazole, N-methylpyrazole, 1,3,4-oxadiazole, 1,2,4-triazole, 1-methyl-1,2,4-triazole, 1H-tetrazole, 1-methyltetrazole, benzoxazole, benzothiazole, benzofuran, benzisoxazole, benzimidazole, N-methylbenzimidazole, azabenzimidazole, indazole, quinazoline, quinoline, and isoquinoline; (2) a bicyclic aromatic heterocycle where a phenyl, pyridine, pyrimidine or pyridizine ring is: (a) fused to a 5-membered aromatic (unsaturated) heterocyclic ring having one nitrogen atom; (b) fused to a 5 or 6-membered aromatic (unsaturated) heterocyclic ring having two nitrogen atoms; (c) fused to a 5-membered aromatic (unsaturated) heterocyclic ring having one nitrogen atom together with either one oxygen or one sulfur atom; or (d) fused to a 5-membered aromatic (unsaturated) heterocyclic ring having one heteroatom selected from O, N or S.

If aryl or heteroaryl is 'optionally substituted', one or two of the following are possible substituents: nitro, -aryl, -heteroaryl, alkoxycarbonyl-, -alkoxy, -alkoxy-alkyl, alkyl-O-C2-C4alkyl-O-, -cyano, -halogen, -hydroxy, -N-R₆R₇, -trifluoromethyl, - trifluoromethoxy, arylalkyl, alkylaryl, R₆R₇N-alkyl-, HO-C1-C6-alkyl-, alkoxyalkyl-, alkyl-S-, -SO₂N-R₆R₇, -SO₂NHR₆, -CO₂H, CONR₆R₇, aryl-O-, heteroaryl-O-, -S(O)ₛ-aryl (where s = 0 -2), -alkyl-O-alkyl-NR₆R₇, -alkyl-aryl-O-alkylN-R₆R₇, C1-C6alkyl, alkenyl, alkynyl, cycloalkyl, alkoxy-alkyl-O-, R₆R₇N-alkyl-, and -S(O)ₛ-heteroaryl (where s = 0 -2); Preferred substituents for aryl and heteroaryl include: alkyl, halogen, -N-R₆R₇, trifluoromethyl, -trifluoromethoxy, arylalkyl, and alkylaryl.

Arylalkyl is defined as Aryl-C1-C6alkyl--; Arylalkyl moieties include benzyl, 1-phenylethyl, 2-phenylethyl, 3-phenylpropyl, 2-phenylpropyl and the like. The term 'optionally substituted' refers to unsubstituted or substituted with 1 or 2 substituents on the alkyl or aryl moiety as defined above.

Alkylaryl is defined as C1-C6alkyl-aryl-. The term 'optionally substituted' refers to unsubstituted or substituted with 1 or 2 substituents on the aryl or alkyl moiety as defined above.

Heteroaryl-C1-C6- alkyl is defined as a heteroaryl substituted alkyl moiety wherein the alkyl chain is 1-6 carbon atoms (straight or branched). Alkyl heteroaryl moieties include Heteroaryl-(CH₂)₁₋₆- and the like. The term 'optionally substituted' refers to unsubstituted or substituted with 1 or 2 substituents on the alkyl or heteroaryl moiety as defined above;
C1-C6 alkylheteroaryl is defined an alkyl chain of 1-6 carbon atoms (straight or branched) attached to a heteroaryl moiety, which is bonded to the rest of the molecule. Ex. C1-C6-alkyl-Heteroaryl--. The term 'optionally substituted' refers to unsubstituted or substituted with 1 or 2 substituents on the alkyl or heteroaryl moiety as defined above;
Saturated or partially saturated heterocycles groups are defined as heterocyclic rings selected from the moieties; aziridinyl, azetidinyl, 1,4-dioxanyl, hexahydroazepinyl, piperazinyl, piperidinyl, pyrrolidinyl, morpholinyl, thiomorpholinyl, dihydrobenzimidazolyl, dihydrobenzofuranyl, dihydrobenzothienyl, dihydrobenzoxazolyl, dihydrofuranyl, dihydroimidazolyl, dihydroindolyl, dihydroisooxazolyl, dihydroisothiazolyl, dihydrooxadiazolyl, dihydrooxazolyl, dihydropyrrazinyl, dihydropyrazolyl, dihydropyridinyl, dihydropyrimidinyl, dihydropyrrolyl, dihydroquinolinyl, dihydrotetrazolyl, dihydrothiadiazolyl, dihydrothiazolyl, dihydrothienyl, dihydrotriazolyl, dihydroazetidinyl, dihydro-1,4-dioxanyl, tetrahydrofuranyl, tetrahydrothienyl, tetrahydroquinolinyl, and tetrahydroisoquinolinyl
C1-C6 alkyl mono or bicyclic saturated or partially saturated heterocycles is defined as an alkyl group (straight or branched) of C1-C6 attached to a heterocycles (which is defined before) through a carbon atom or a nitrogen atom and the other end of the alkyl chain attached to the rest of the molecule. The terms 'optionally substituted' refers to unsubstituted or substituted with 1 or 2 substituents present on the alkyl or heterocyclic portion of the molecule, as defined before;
Arylalkyloxyalkyl is defined as Aryl-C1-C6alkyl-O-C1-C6alkyl---. The term 'optionally substituted' refers to unsubstituted or substituted with 1 or 2 substituents present on the alkyl and/or aryl portions as defined before;
Alkyloxyalkyl is defined as C1-C6 alkyl-O-C1-C6alkyl-. The term 'optionally substituted' refers to unsubstituted or substituted with 1 or 2 substituents present at the alkyl moiety as defined before;
Aryloxyalkyl is defined as Aryl-O-C1-C6 alkyl---. The term 'optionally substituted' refers to unsubstituted or substituted with 1 or 2 substituents present at the alkyl or aryl moiety as defined before;
Heteroarylalkyloxyalkyl is defined as Heteroaryl-C1-C6alkyl-O-C1-C6alkyl---. The term 'optionally substituted' refers to unsubstituted or substituted with 1 or 2 substituents present on the alkyl or heteroaryl moiety as defined before;
Aryloxyaryl is defined as Aryl-O-Aryl---. The term 'optionally substituted' refers to unsubstituted or substituted with1 or 2 substituents present on the aryl moiety as defined before;
Aryloxyheteroaryl is defined as Aryl-O-Heteroaryl- or -Aryl-O-Heteroaryl; In this definition either the aryl moiety or the heteroaryl moiety can be attached to the remaining portion of the molecule; The term 'optionally substituted' refers to unsubstituted or substituted with1 or 2 substituents present on the aryl moiety or on the heteroaryl moiety as defined before;
Alkyl aryloxyaryl is defined as Aryl-O-Aryl-C1-C6alkyl----; The term 'optionally substituted' refers to unsubstituted or substituted with1 or 2 substituents present at the aryl moiety as defined before;
Alkylaryloxyheteroaryl is defined as Heteroaryl-O-Aryl-C1-C6alkyl---; The term 'optionally substituted' refers to unsubstituted or substituted with 1 or 2 substituents present on the aryl moiety or on the hetroaryl moiety as defined before;
Alkylaryloxyalkylamine is defined as R₆R₇N-C1-C6alkyl-O-Aryl-C1C6alkyl---; The terms 'optionally substituted' refers to unsubstituted or substituted with 1 or 2 substituents present on the alkyl or aryl moiety as defined before; R₆ and R₇ as defined before;
Alkoxycarbonyl is defined as C1-C6alkyl-O-C=O--; The term 'optionally substituted' refers to unsubstituted or substituted with 1 or 2 substituents present on the alkyl portion of the alkoxy moiety as defined before;
Aryloxycarbonyl is defined as Aryl-O-C=O--; The term 'optionally substituted' refers to unsubstituted or substituted with 1 or 2 substituents present at the aryl moiety as defined before;
Heteroaryloxy carbonyl is defined as Heteroaryl-O-C=O--; The term 'optionally substituted' refers to unsubstituted or substituted with 1 or 2 substituents present at the heteroaryl moiety as defined before;
Alkoxy is defined as C1-C6alkyl-O--; The terms 'optionally substituted' refers to unsubstituted or substituted with 1 or 2 substituents present at the alkyl moiety as defined before;
Aryloxy is defined as Aryl-O--; The term 'optionally substituted' refers to unsubstituted or substituted with 1 or 2 substituents present at the aryl moiety as defined before;
Heteroaryloxy is defined as Heteroaryl-O--; The term 'optionally substituted' refers to unsubstituted or substituted with 1 or 2 substituents present at the heteroaryl moiety as defined before;
Alkenyloxy is defined as C3-C6 alkene-O--; Example allyl-O--, bute-2-ene-O like moieties; The term 'optionally substituted' refers to unsubstituted or substituted with 1 or 2 substituents present at the alkene moiety as defined before, with the proviso that no hetero atom such as O, S or N-R₁ is present on the carbon atom, which is attached to a double bond;
Alkynyloxy is defined as C3-C6alkyne-O--; Example CH triple bond C-CH₂-O-, like moieties; The term 'optionally substituted' refers to unsubstituted or substituted with 1 or 2 substituents present at the alkyne moiety as defined before, with the proviso that no hetero atom such as O, S or N-R₁ is present on a carbon atom which is attached to a double or triple bond; Alkylaminoalkoxy is defined as R₆R₇N-C1-C6-alkyl-O-C1-C6-alkyl---, where the terminal alkyl group attached to the oxygen is connected to the rest of the molecule; The terms R₆ and R₇ are defined above; The term 'optionally substituted' refers to unsubstituted or substituted with 1 or 2 substituents present at the alkyl moiety as defined before; Alkylenedioxy is defined as -O-(CH₂)₂---O---;
Aryloxyalkylamine is defined as R₆R₇N-C1-C6-alkyl-O-Aryl--, where the aryl is attached to the rest of the molecule; The term 'optionally substituted' refers to unsubstituted or substituted with 1 or 2 substituents present at the alkyl or aryl moiety as defined before;
Arylalkenyl is defined as Aryl-C2-C8alkene--, with the proviso that no hetero atom such as O, S or N-R₁ is present on the carbon atom, which is attached to a double bond; The term 'optionally substituted' refers to unsubstituted or substituted with 1 or 2 substituents present on the alkene or aryl moiety as defined before;
Heteroaryloxyalkyl is defined as Heteroaryl-O-C1-C6alkyl--; The term 'optionally substituted' refers to unsubstituted or substituted with 1 or 2 substituents present at the heteroaryl moiety as defined before;
Heteroaryloxyaryl is defined as Heteroaryl-O-aryl--, where the aryl moiety is attached to the rest of the molecule; The term 'optionally substituted' refers to unsubstituted or substituted with 1 or 2 substituents present at the heteroaryl moiety or the aryl moiety as defined before;
Alkoxy, alkoxyalkyl, alkoxyalkyloxy and alkylthioalkyloxy are moieties wherein the alkyl chain is 1-6 carbon atoms (straight or branched). Aryloxy, heteroaryloxy, arylthio and heteroarylthio are moieties wherein the aryl and heteroaryl groups are as herein before defined. Arylalkyloxy, heteroarylalkyloxy, arylalkylthio and heteroarylalkylthio are moieties wherein the aryl and heteroaryl groups are as herein before defined and wherein the alkyl chain is 1-6 carbons (straight or branched).

Aryloxyalkyl, heteroaryloxyalkyl, aryloxyalkyloxy and heteroaryloxyalkyloxy are substituents wherein the alkyl radical is 1-6 carbon atoms. The terms monoalkylamino and dialkylamino refer to moieties with one or two alkyl groups wherein the alkyl chain is 1-6 carbons and the groups may be the same or different. The terms monoalkylaminoalkyl and dialkylaminoalkyl refer to monoalkylamino and dialkylamino moieties with one or two alkyl groups (the same or different) bonded to the nitrogen atom which is attached to an alkyl group of 1-3 carbon atoms.

The expression "Fused tricyclic heteroaryl group" is used in the specification and claims to mean:
a group comprising three fused rings in which at least one ring has aromatic character (i.e meets Huckel's rule (4n+2)). The fused tricyclic heteroaryl group contains 1-6 heteroatoms selected from the group consisting of O, S, N and N-R₁. The fused tricyclic heteroaryl must be bonded through a carbon preferably in one of the at least one aromatic rings to the remainder of the formula **I** molecule. The fused tricyclic heteroaryl group may contain 1-3 aromatic rings and 0-2 non-aromatic rings. Each aromatic ring(s) in the fused tricyclic heteroaryl group may contain 5 to 7 ring atoms (including the bridgehead atoms) selected from CR₂, O, S, N, and N-R₁. Each of the aromatic ring(s) of the fused tricyclic heteroaryl group may contain 0 to 3 heteroatoms selected from O, S, N or N-R₁. The non-aromatic ring(s), if any, of the fused tricyclic heteroaryl group may contain 5-8 ring atoms (including bridgehead atoms) and contain 0-4 heteroatoms selected from N, N-R₁, O or S(O)ₙ, wherein n is 0-2. In each non-aromatic ring of the fused tricyclic heteroaryl group, one or two of the non-bridgehead carbon atoms may each be optionally substituted with one or two R₄, and each R₄ may be independently the same or different.

Examples of fused tricyclic heteroaryl groups include:

### Ring size and arrangements: (5-5-5)

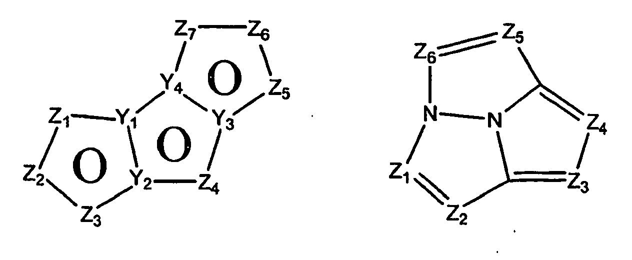

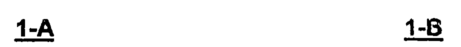

In both formula **1-A** and **1-B** Z₁, Z₂, Z₃, Z₄, Z₅, Z₆ and Z₇ are independently selected from CR₂, N, O, S or N-R₁ and as mentioned above one of Z₁ - Z₇ is a carbon atom to which the penem portion of the molecule is attached. Y₁, Y₂, Y₃ and Y₄ may independently be C or N.

### Ring size and arrangement: (5-5-6)

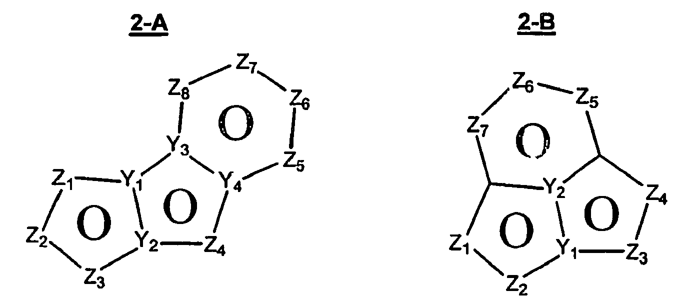

In both formula **2-A** and **2-B** Z₁, Z₂, Z₃, Z₄, Z₅, Z₆, Z₇ and Z₈ are independently selected from CR₂, N, O, S or N-R₁ and as mentioned above one of the Z₁ - Z₈ is a carbon atom to which the penem portion of the molecule is attached. Y₁, Y₂, Y₃ and Y₄ may be independently be C or N.

### Ring size and arrangement: (5-6-5)

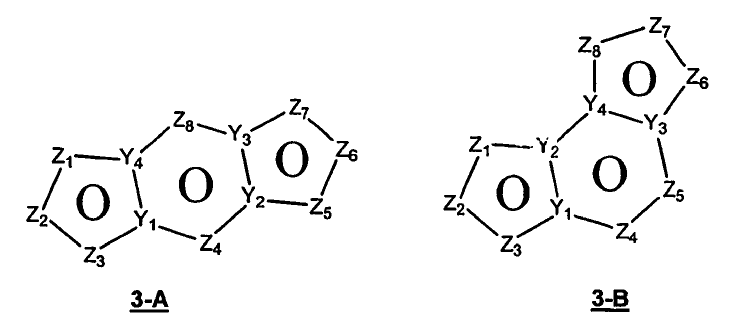

In both formula **3-A** and **3-B** Z₁, Z₂, Z₃, Z₄, Z₅, 2₆, Z₇ and Z₈ are independently selected from CR₂, N, O, S or N-R₁ and as mentioned above one of Z₁ - Z₈ is a carbon atom to which the penem portion of the molecule is attached. Y₁, Y₂, Y₃ and Y₄ may be C or N.

### Ring size and arrangements: (5-6-6)

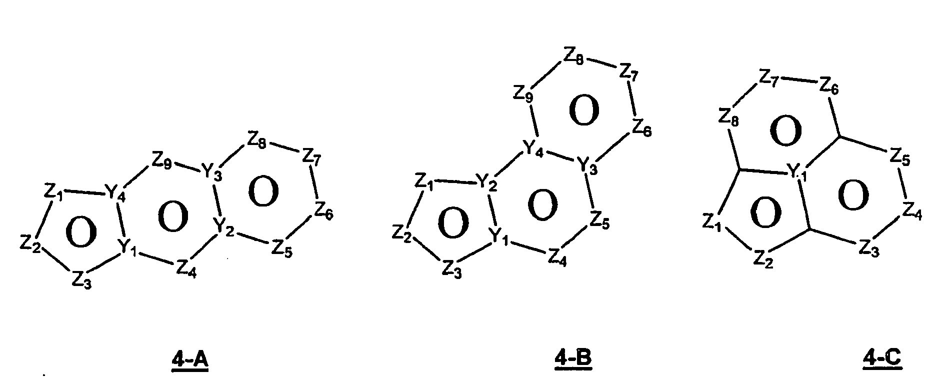

In formula **4-A, 4-B** and **4-C** Z₁, Z₂, Z₃, Z₄, Z₅, Z₆, Z₇ and Z₈ are independently selected from CR₂, N, O, S or N-R₁ and as mentioned above one of the Z₁ - Z₈ is a carbon atom to which the penem portion of the molecule is attached. Y₁, Y₂, Y₃ and Y₄ are independently C or N.

### Ring size and arrangements: [5-5-(non-aromatic)]

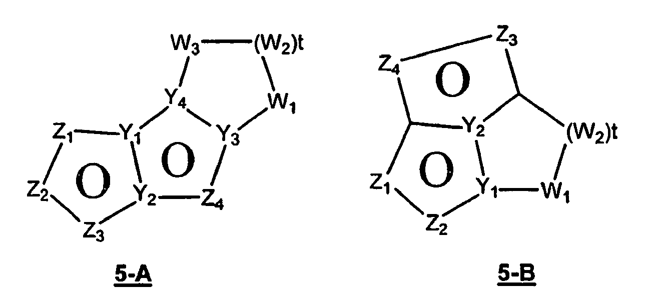

In both formula **5-A** and **5-B** Z₁, Z₂, Z₃ and Z₄ are indpendently selected from CR₂, N, O, S or N-R₁ and as mentioned above one of the Z₁ - Z₄ is a carbon atom to which the penem portion of the molecule is attached; Y₁, Y₂, Y₃ and Y₄ are independently C or N. W₁, W₂ and W₃ are independently selected from CR₄R₄, S(O)r ( r = 0 -2), O, and N-R₁ with the proviso that no S-S, S-O or O-O bond formation can occur to form a saturated ring; and t = 1 to 3.

### Ring size and arrangement: [5-6-(non-aromatic)]

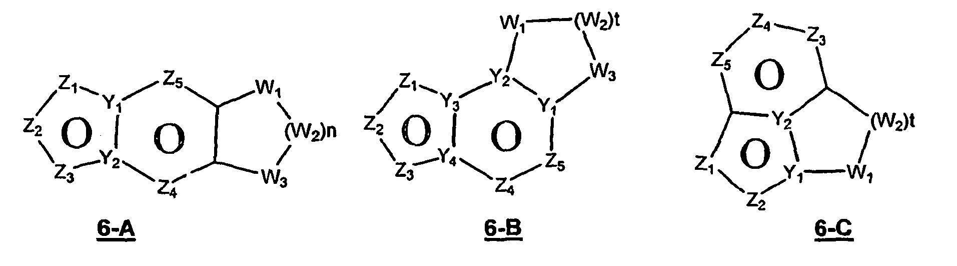

In formulae **6-A, 6-B** and **6-C** Z₁, Z₂, Z₃, Z₄ and Z₅ are independently selected from CR₂, N, O, S or N-R₁ and as mentioned above one of the Z₁ - Z₅ is a carbon atom to which the penem portion of the molecule is attached. Y₁, and Y₂ are independently C or N. W₁, W₂ and W₃ are independently selected from CR₄R₄, S(O)r ( r = 0 -2) , O, and N-R, with the proviso that no S-S, S-O or O-O bond formation can occur to form a saturated ring; and t = 1 to 3.

### Ring size and arrangement: [5-(non-aromatic)-5]

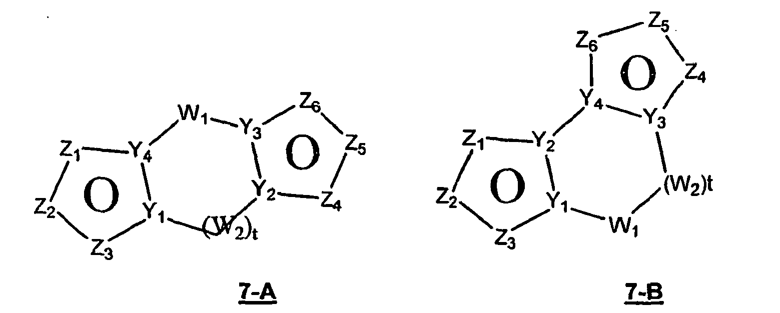

In formulae **7-A** and **7-B** Z₁, Z₂, Z₃, Z₄, Z₅ and Z₆ are independently selected from CR₂, N, O, S or N-R₁ and as mentioned above one of Z₁ - Z₆ is a carbon atom to which the penem portion of the molecule is attached. Y₁, Y₂, Y₃ and Y₄ are independently C or N. W₁ and W₂ are independently selected from CR₄R₄, S(O)r ( r = 0 -2), O, and N-R₁ with the proviso that no S-S, S-O or O-O bond formation can occur to form a saturated ring; and t = 1 to 3.

### Ring size and arrangement: [5-(non-aromatic)-6]

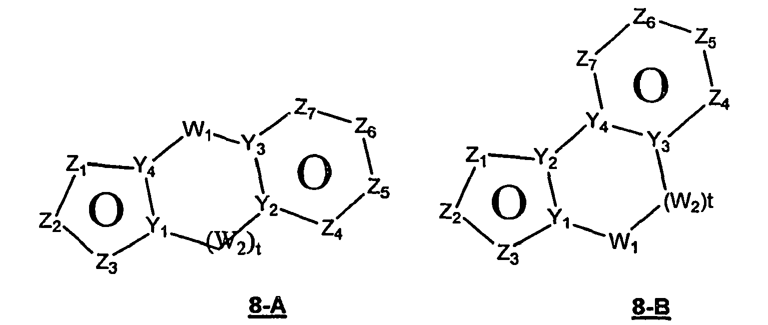

In formulae **8-A** and **8-B** Z₁, Z₂, Z₃, Z₄, Z₅, Z₆ and Z₇ are independently selected from CR₂, N, O, S or N-R₁ and as mentioned above one of the Z₁ - Z₇ is a carbon atom to which the penem portion of the molecule is attached. Y₁, Y₂, Y₃ and Y₄ are independently C or N. W₁ and W₂ are independently CR₄R₄, S(O)r (r = 0 -2), O, or N-R₁ with the proviso that no S-S, S-O or O-O bond formation can occur to form a saturated ring; and t = 0 to 3.

### Ring size and arrangement [5-(non-aromatic)-(non-aromatic)]

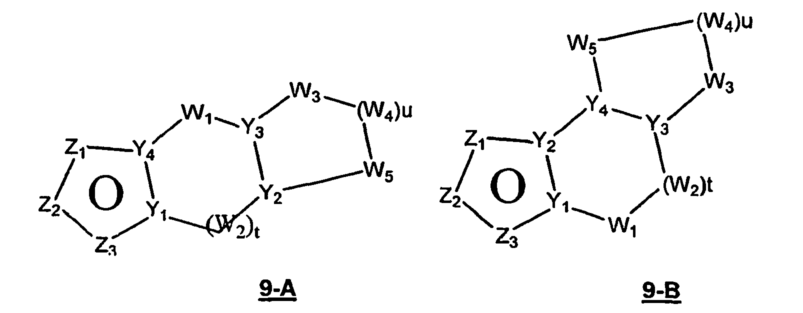

In formulae **9-A** and **9-B** Z₁, Z₂ and Z₃ are independently selected from CR₂ N, O, S and N-R₁ and as mentioned above one of the Z₁ - Z₃ is a carbon atom to which the penem portion of the molecule is attached. Y₁ and Y₄ are independently C or N; Y₂ and Y₃ are independently CH or N; W₁, W₂ W₃, W₄ and W₅ are independently CR₄R₄, S(O)r ( r = 0 -2), O, or N-R₁ with the proviso that no S-S, S-O or O-O bond formation can occur to form a saturated ring; t = 0 to 2 and u = 1 to 3.

### Ring size and arrangement (6-5-6)

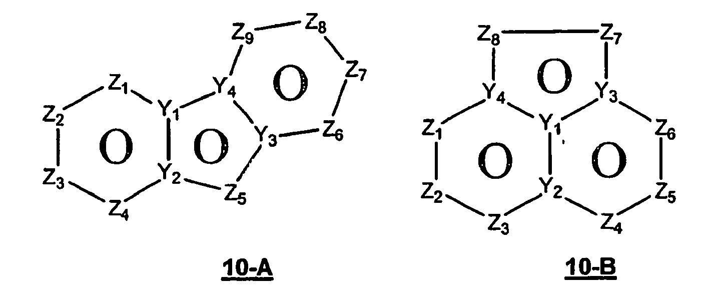

In formula **10-A** and **10-B** Z₁, Z₂, Z₃, Z₄, Z₅, Z₆, Z₇, Z₈ and Z₉ are independently CR₂, N, O, S or N-R₁ and as mentioned above one of the Z₁ - Z₉ is a carbon atom to which the penem portion of the molecule is attached. Y₁, Y₂, Y₃ and Y₄ are independently C or N.

### Ring size and arrangement (6-6-6)

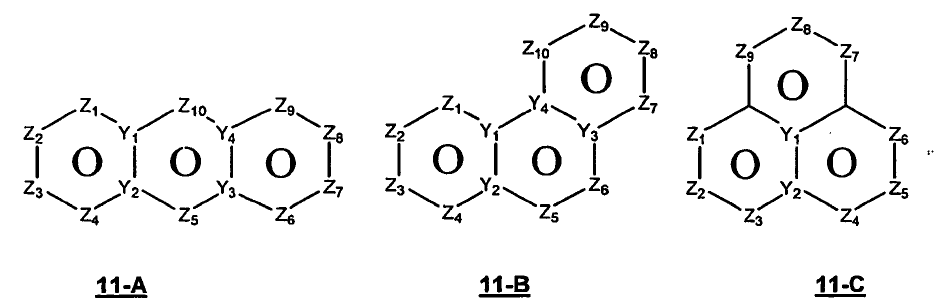

In formula **11-A, 11-B** and **11-C** Z₁, Z₂, Z₃, Z₄, Z₅, Z₆, Z₇, Z₈, Z₉ and Z₁₀ are independently CR₂, N, O, S or N-R₁ and as mentioned above one of Z₁ - Z₁₀ is a carbon atom to which the penem portion of the molecule is attached. Y₁, Y₂, Y₃ and Y₄ are independently C or N.

### Ring size and arrangement [6-5-(non-aromatic)]

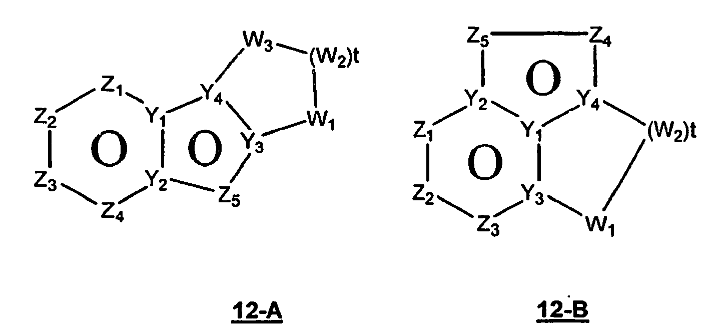

In formula **12-A** and **12-B** Z₁, Z₂, Z₃, Z₄ and Z₅ are independently CR₂, N, O, S or N-R₁ with the proviso that one of Z₁ - Z₅ is a carbon atom to which the penem portion of the molecule is attached. Y₁, Y₂, Y₃ and Y₄ are independently C or N; W₁, W₂, W₃ are indepdently O, N-R₁, or S=(O)ᵣ (r = 0-2) with the proviso that no S-S, S-O or O-O bond formation can occur to form a saturated ring; and t =1-4.

### Ring size and arrangement [6-6-(non-aromatic)]

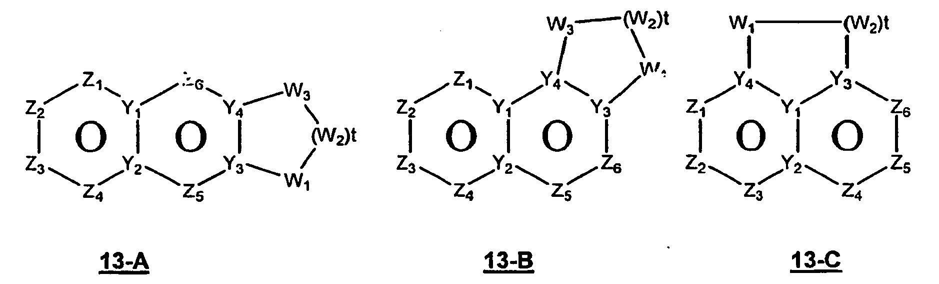

In formula **13-A, 13-B** and **13-C** Z₁, Z₂, Z₃, Z₄, Z₅ and Z₆ are independently CR₂, N, O, S or N-R₁ and as mentioned above one of the Z₁ - Z₆ is a carbon atom to which the penem portion of the molecule is attached. Y₁, Y₂, Y₃ and Y₄ are independently C or N; W₁, W₂ and W₃ are independently CR₄R₄, S(O)r ( r = 0 -2), O, or N-R₁ with the proviso that no S-S, S-O or O-O bond formation can occur to form a saturated ring; and t = 1 to 3.

### Ring size and arrangement [6-(non-aromatic)-6]

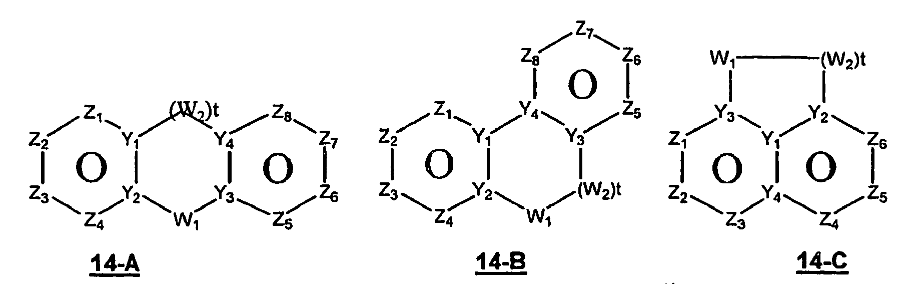

In formula **14-A, 14-B** and **14-C** Z₁ Z₂, Z₃, Z₄, Z₅, Z₆, Z₇ and Z₈ are independently CR₂, N, O, S or N-R₁ and as mentioned above one of Z₁ - Z₈ is a carbon atom to which the penem portion of the molecule is attached. Y₁, Y₂, Y₃ and Y₄ are independently C or N; W₁, W₂ and W₃ are independently CR₄R₄, S(O)r ( r = 0 -2), O, or N-R₁ with the proviso that no S-S, S-O or O-O bond formation can occur to form a saturated ring; and t = 1 to 2.

### Ring size and arrangement [6-(non-aromatic)-(non-aromatic)]

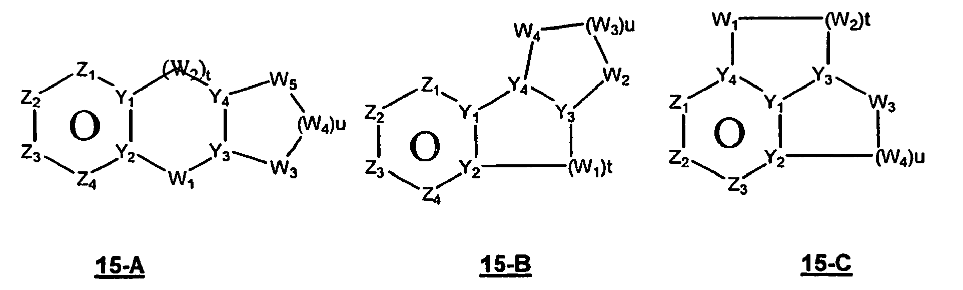

In formula **15-A, 15-B** and **15-C** Z₁, Z₂, Z₃ and Z₄ are independently CR₂, N, O, S or N-R₁ and as mentioned above one of Z₁ - Z₄ is a carbon atom to which the penem portion of the molecule is attached. Y₁, Y₂, Y₃ and Y₄ are independently C or N; W₁, W₂, W₃ and W₄ are independently CR₄R₄, S(O)r ( r = 0 -2), O, or N-R₁ with the proviso that no S-S, S-O or O-O bond formation can occur to form a saturated ring; t = 1 to 3 and u = 1 to 3.

The expression 'fused bicyclic heteroaryl group' is used in the specification and claims to mean:
A group comprising two fused rings in which one has aromatic character [i.e. Huckel's rule (4n+2)] and the other ring is non-aromatic;
the fused bicyclic heteroaryl group contains one to six heteroatoms selected from the group O, S, N and N-R₁;
the fused bicyclic heteroaryl group is bonded to the remainder of the molecule through a carbon atom in the aromatic ring as shown in the formula **I**;
the aromatic ring of the fused bicyclic heteroaryl group contains five or six ring atoms (including bridgehead atoms) selected from CR₂, N, O, S or N-R₁. The aromatic ring of the fused bicyclic heteroaryl group contains 0 to 3 heteroatoms selected from the group O, S, N and N-R₁;
the non-aromatic ring of the fused bicyclic heteroaryl group contains five to eight ring atoms (including bridgehead atoms) selected from CR₄R₄, N, N-R₁, O, S(O)ₙ where n= 0-2. The non-aromatic ring of the fused bicyclic heteroaryl group contains 0 to 4 heteroatoms selected from N, N-R₁, O or S(O)ₙ where n = 0 to 2.

Examples of the fused bicyclic heteroaryl group include: 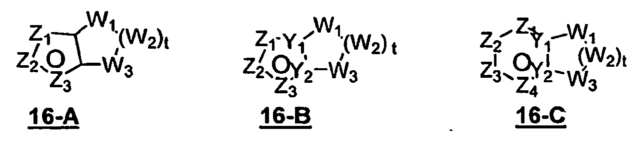
In formula **16-A** Z1, Z2 and Z3 are independently CR₂, N, O, S or
N-R₁ and one of Z1 -Z3 is carbon and is bonded to the remainder of the molecule as shown in formula **I.** When one of Z's is CR₂ the other two Zs can be either two N or one N and O, S, N-R₁ in any combinations with out disrupting the aromaticity; when two Z,s = CR₂ the other Z can be optionally selected from one N, O, S or N-R₁ in any combination with out disrupting the aromaticity;
W₁, W₂ and W₃ are independently CR₄R₄, S, SO, SO₂, O, N-R₁, C=O; with the proviso that no S-S or O-O or S-O bond formation can occur to form the saturated ring system; t= 1 to 4.

In formula **16-B** Z1, Z2 and Z3 are independently CR₂, N, O, S or N-R₁ and one of Z1 -Z3 is carbon and is bonded to the remainder of the molecule as shown in formula **I.**
When one of Z's = CR₂, then the other two Z's can be independently CR₂, N, O, S or N-R₁ in any combinations with out disrupting the aromaticity;
When two Z's =N, then the other carbon in the ring is bonded to the penem portion of the molecule as shown in formula **I**.
W₁, W₂ and W₃ are independently CR₄R₄, S, SO, SO₂, O, N-R ,
t= 1 to 4;
Y₁ and Y₂ = N or C; with the proviso that when the aromatic heterocycle is imidazole, the saturated ring may not contain a S adjacent to the bridgehead carbon.

In formula **16-C** Z1, Z2, Z3 and Z4 are independently CR₂ or N and one of Z1 -Z4 is carbon and is bonded to the remainder of the molecule.
W₁, W₂ and W₃ are independently CR₄R₄, S, SO, SO₂, O, or N-R₁; with the proviso that no S-S or O-O or S-O bond formation can occur to form the saturated ring system; t= 1 to 4.
Y₁ and Y₂ are independently C or N.

### PROCESS OF THE INVENTION

Compounds of the general formula **I** can be prepared in a mild and facile way by condensing an appropriately substituted aldehyde **17** with 6-bromo-penem derivative of structure **16.** (Scheme 2) in the presence of a Lewis acid, preferably anhydrous magnesium halide more preferably anhydrous MgBr₂ or MgBr₂: etherate and a mild base such as triethylamine, DMAP, or diisopropyl ethyl amine, at low temperature preferably at about -20°C to -40°C. The intermediate aldol product **18** can be functionalized with acid chlorides or anhydrides preferably to an acetate, triflate or a tosylate **19;** or, if formulae **18** is isolated, it can be converted to a halogen derivative by reacting **18** with tetrahalomethane and triphenyl phosphine at room temperature in a suitable organic solvent preferably CH₂Cl₂. The compound **19** can be smoothly converted to the desired product by a reductive elimination process using a metal such as activated zinc and phosphate buffer at mild temperatures preferably about 20°C to 35°C at a pH of about 6.5 to 8.0 or hydrogenating over a catalyst preferably palladium on charcoal. It should be noted that the reductive elimination step could be conducted such that deprotection of the carboxyl group occurs. If the protecting group on the carboxylate oxygen is para-nitrobenzyl substituent then the reductive elimination and deprotection can be achieved by a single step. However if the protecting group is other than *para*-nitrobenzyl substituent, a two step procedure can be followed depending up on the nature of the protecting group. The product can be isolated as a free acid or as an alkalie metal salt. The above mentioned two step procedure can be carried out in one step by carrying out the entire process without isolating the intermediate **19**. This is a relatively simple procedure and extremely efficient in terms of yield and economic feasibility which can be used to make a wide variety of compounds. This procedure is amenable to a large scale synthesis and applicable to a variety of aldehydes.

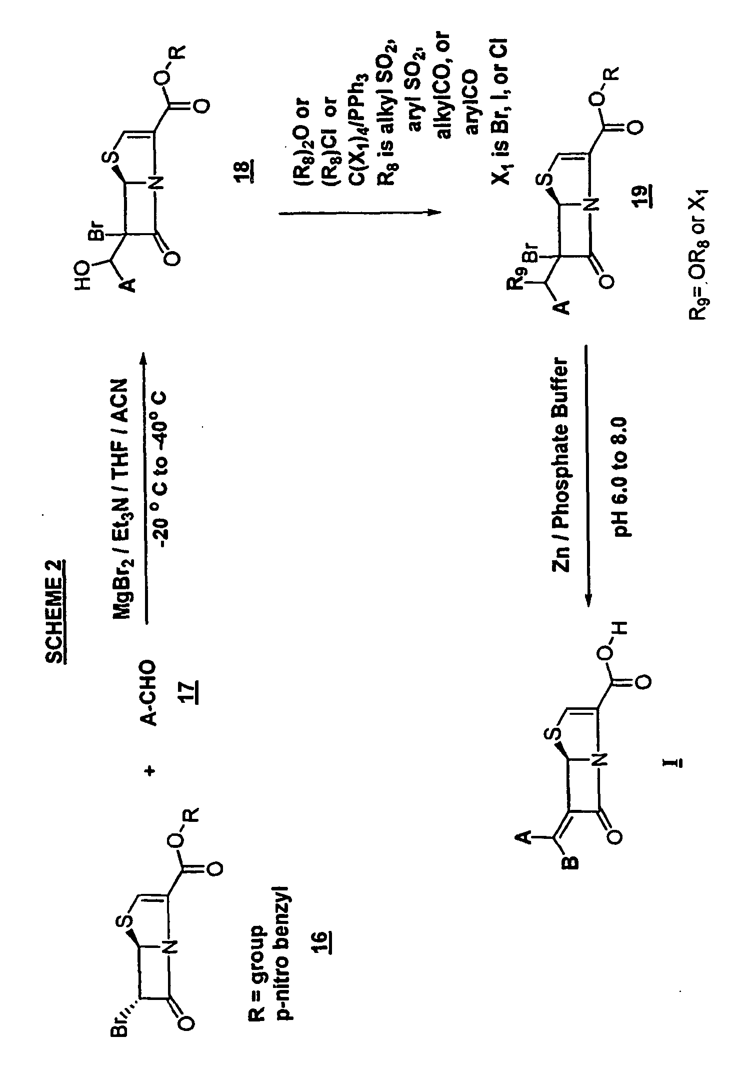

One important aspect of the present invention relates to the use of stable bromopenem intermediate **16.** The stability of the intermediate is relevant to the aldol condensation step where the decomposition is minimized [as well as to the shelf life stability]. In this invention the key intermediate **16,** where R = *para*-Nitrobenzyl group is a stable and a crystalline intermediate. In the present invention, it has been found that the intermediate **16** is more stable than the intermediate **Q.** The comparative thermal stability data for the intermediates **16** and **Q** are given below. (Table 1) This remarkable stability of intermediate **16** enhances the shelf-life time of the compound as well as the scale-up feasibility. As mentioned above, protection of carboxyl group with *para-*Nitrobenzyl group reduces the number of steps in the present process of preparing the final compound of structure **I**. The crystalline nature of the intermediate was established by X-ray diffraction studies. Table 2 shows the X-ray powder parameters of the formula **16.**

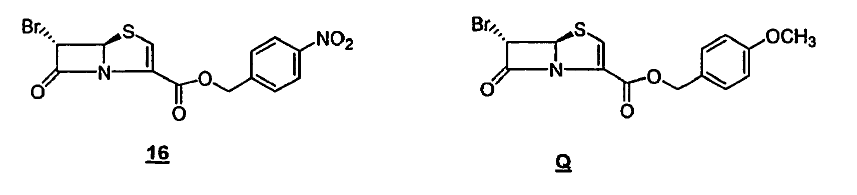

| **Table 1** | | |
|---|---|---|
| **Residual Ratio (%)¹** | | |
| **Day** | **Formula 16** | **Formula Q** |
| **0** | 100.0 | 100.0 |
| **1** | 100.0 | 99.7 |
| **2** | 100.0 | 95.3 |
| **4** | 100.0 | 72.9 |
| **7** | 98.7 | 69.8 |
| **14** | 98.4 | Not tested |

| | | |
|---|---|---|
| **1) Checked by HPLC analysis.** **Temp. 40°C in a solid state.** **HPLC conditions:** **Column: Inertsil SIL 100-5 4.6X150 mm (GL Science Inc.)** **Mobile phase; Ethylacetae: n-hexane (10:35 vv)** **Wave length: 312 nM; Flow rate: 1.0 ml/min @ Room temperature.** | | |

**Table 2**

| **Peak #** | **2 θ** | **d value (Q)** | **intensity (cps)** | **Relative intensity** |
|---|---|---|---|---|
| 1 | 9.540 | 9.2631 | 1575 | 28 |
| 2 | 13.560 | 6.5246 | 1225 | 22 |
| 3 | 15.460 | 5.7268 | 3913 | 70 |
| 4 | 18.680 | 4.7462 | 2509 | 45 |
| 5 | 19.180 | 4.6236 | 820 | 15 |
| 6 | 19.940 | 4.4491 | 1366 | 25 |
| 7 | 21.060 | 4.2149 | 699 | 13 |
| 8 | 22.360 | 3.9727 | 1652 | 30 |
| 9 | 23.740 | 3.7448 | 5560 | 100 |
| 10 | 24.260 | 3.6657 | 3560 | 64 |
| 11 | 24.940 | 3.5673 | 1958 | 35 |
| 12 | 25.180 | 3.5338 | 1799 | 32 |
| 13 | 25.780 | 3.4529 | 878 | 16 |
| 14 | 26.540 | 3.3558 | 1514 | 27 |
| 15 | 26.900 | 3.3117 | 1136 | 20 |
| 16 | 28.900 | 3.0869 | 2206 | 40 |
| 17 | 29.220 | 3.0538 | 1507 | 27 |
| 18. | 29.720 | 3.0035 | 1473 | 26 |
| 19 | 30.560 | 2.9229 | 906 | 16 |
| 20 | 31.200 | 2.8644 | 815 | 15 |
| 21 | 31.480 | 2.8395 | 888 | 16 |
| 22 | 34.700 | 2.5830 | 906 | 16 |
| 23 | 36.700 | 2.4467 | 785 | 14 |
| 24 | 37.780 | 2.3792 | 862 | 16 |
| 25 | 38.980 | 2.3087 | 762 | 14 |
| 26 | 40.180 | 2.2425 | 728 | 13 |
| 27 | 41.220 | 2.1883 | 718 | 13 |

Advantageously, during the reductive elimination step, in the present invention the desired "Z" isomer is formed extremely preferentially (about 50:1) 45-55:1. Formation of the "E" isomer is not observed in the reaction mixture. The reductive elimination step can also be carried out by hydrogenating the intermediate **19** over 10% Pd/C.

The intermediate **16** used in the present invention may be prepared from the commercially available 6-aminopenicillanic acid (6-APA) **20.** (Scheme 3). This may be converted to the bromopenem **16** by the procedure outlined in Scheme 3. 6-aminopenicillanic acid (6-APA) **20** was converted to the bromo-PNB derivative **22** by a one pot procedure. This was oxidized to sulfoxide **23** which under went a ring opening reaction to yield **24.** Compound **24** was converted to **16** by the procedure outlined in Scheme 3. Advantageously, in the present invention, conversion of compound **23** to **26** can be carried out in one pot, without isolating the intermediates **24** and **25.** The bromopenem **16** thus obtained was reacted with the aldehyde **17** (Ref. Scheme 2) in the presence of anhydrous MgBr₂ or commercially available MgBr₂: O(Et)₂. The aldol product was trapped as an acetate and the bromo acetate **19** can be conveniently converted to the final product **I** by reacting it with activated zinc (e.g. freshly activated with 0.1 N HCl) and phosphate buffer (6.5 pH) at room temperature. The product can be conveniently purified by knowns means such as dianion HP-21 column chromatography. Initially the column can be eluted with water to remove any inorganic impurity and latter with 10% MeCN; water. The product obtained through this process is 98% pure and it can be further purified by crystallization. The aldol condensation reaction using Et₃N/and MgBr₂ is extremely efficient and general. The present invention may be extended to a variety of aldehydes to yield the final product of general structure **I**.

The present invention relates to a process for the preparation of compound of formula **I** 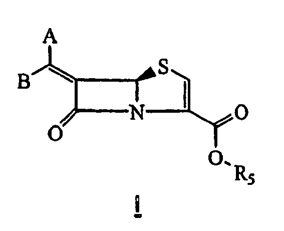 wherein
one of A and B denotes hydrogen and the other is aryl optionally substituted with one or two R₂, heteroaryl optionally substituted with one or two R₂, a fused bicyclic heteroaryl optionally substituted with one or two R₂, fused tricyclic heteroaryl optionally substituted with one or two R₂, cycloalkyl optionally substituted with one or two R₂, alkyl optionally substituted with one or two R₂, alkenyl optionally substituted with one or two R₂, alkynyl optionally substituted with one or two R₂, saturated or partially saturated heteroaryl optionally substituted with one or two R₂;
R₅ is H, an in vivo hydrolyzable ester selected from the group C1 -C6 alkyl, C5 - C6 cycloalkyl, CHR₃OCOC1-C6 or a salt selected from the group consisting of Na, K, and Ca;
R₂ is hydrogen, optionally substituted C1-C6 alkyl, optionally substituted C2-C6 alkenyl having 1 to 2 double bonds, optionally substituted C2-C6 alkynyl having 1 to 2 triple bonds, halogen, cyano, N-R₆R₇, optionally substituted C1-C6 alkoxy, hydroxy; optionally substituted aryl, optionally substituted heteroaryl, COOR₆, optionally substituted alkyl aryloxy alkylamines, optionally substituted aryloxy, optionally substituted heteroaryloxy, optionally substituted C3-C6 alkenyloxy, optionally substituted C3 -C6 alkynyloxy, C1-C6 alkylamino-C1-C6 alkoxy, alkylene dioxy, optionally substituted aryloxy-C1-C6 alkyl amine, C1-C6 perfluoro alkyl, S(O)_{q}-optionally substituted C1-C6 akyl, S(O)_{q}- optionally substituted aryl where q is 0, 1 or 2, CONR₆R₇, guanidino or cyclic guanidino, optionally substituted C1-C6 alkylaryl, optionally substituted arylalkyl, optionally substituted C1-C6 alkylheteroaryl, optionally substituted heteroaryl-C1-C6 alkyl, optionally substituted C1-C6 alkyl mono or bicyclic saturated heterocycles, optionally substituted arylalkenyl of 8 to 16 carbon atoms, SO₂NR₆R₇, optionally substituted arylalkyloxyalkyl, optionally substituted aryloxyalkyl, optionally substituted heteroaryloxyalkyl, optionally substituted aryloxyaryl, optionally substituted aryloxyheteroaryl, substituted heteroaryloxyaryl, optionally substituted C1-C6alkyl aryloxyaryl, optionally substituted C1-C6 alkylaryloxyheteroaryl , optionally substituted aryloxyalkyl, optionally substituted heteroaryloxyalkyl, optionally substituted alkylaryloxyalkylamines. Preferred R₂ groups are H, optionally substituted alkyl, optionally substituted alkoxy, optionally substituted heteroaryl, halogen, CN, hydroxy, optionally substituted heterocycle, -CONR₆R₇, COOR₆, optionally substituted aryl, S(O)_{q}-alkyl, and S(O)_{q}-aryl.
R₃ is hydrogen, C1-C6 alkyl, C3 - C6 cycloalkyl, optionally substituted aryl, optionally substituted heteroaryl;
R₆ and R₇ are independently H, optionally substituted C1-C6 alkyl, optionally substituted aryl, optionally substituted heteroaryl, optionally substituted C1-C6 alkyl aryl, optionally substituted arylalkyl, optionally substituted heteroarylalkyl, optionally substituted C1-C6 alkyl heteroaryl, R₆ and R₇ can be taken together to form a 3-7 membered saturated ring system optionally having one or two heteroatoms such as N-R₁, O, S=(O)ₙ n = 0-2;
the process for the preparation of compound of formula **I** comprises

**Step 1:** 6-aminopenicillanic acid **20** dissolved in an organic solvent (preferably methanol or THF) and water was converted to the 6-bromo derivative in the presence of 48% w/w hydrobromic acid at -10° C to -30°C and sodium or potassium nitrite solution. The 6-bromopenicillanic acid **21** derivative either can be isolated or *insitu* converted to the p-Nitrobenzyl 6-brompenicillanate **22** using 4-nitrobenzylbromide in the presence of organic bases or inorganic bases (preferably sodium or potassium carbonate) in an organic solvent. (Preferably THF or DMF).

**Step 2:** The product 4-nitrobenzyl 6-bromopenicillanate **22** obtained by the process outlined in step 1 can be isolated or be transformed to 4-nitrobenzyl 6-bromopenicillanate 1-oxide **23** in the same pot (i.e Step 1; sequential formation) by oxidizing **22** to 4-nitrobenzyl 6-bromopenicillanate 1-oxide **23** using oxidizing agents such as_3-chloroperoxybenzoic acid (mcpba) or hydrogen peroxide.

**Step 3:** The product from step 2, namely 4-nitrobenzyl 6-bromopenicillanate 1-oxide **23** can be converted to 4-nitrobenzyl(2R)-2-[(3S,4R)-4-(benzothiazol-2-yldithio)-3-bromo-2-oxoazetidine-1-yl]-3-methylbut-3-enoate **24** by refluxing 4-nitrobenzyl 6-bromopenicillanate 1-oxide **23** with 2-mercaptobenzothiazole (HSBT) in an aromatic solvent. (preferably Toluene).

**Step 4:** The product from step 3, namely 4-nitrobenzyl(2R)-2-[(3S,4R)-4-(benzothiazol-2-yldithio)-3-bromo-2-oxoazetidine-1-yl]-3-methylbut-3-enoate **24** can be dissolved in an organic solvent (preferably Toluene) and upon reaction with an organic tertiary base (preferably triethylamine) at ambient temperature gave 4-nitrobenzyl-2-[(3S,4R)-4-(benzothiazol-2-yldithio)-3-bromo-2-oxoazetidine-1-yl]-3-methylbut-2-enoate **25.**

**Step 5:** The product from step 4, namely 4-nitrobenzyl-2-[(3S,4R)-4-(benzothiazol-2-yldithio)-3-bromo-2-oxoazetidine-1-yl]-3-methylbut-2-enoate **25** can be converted to **26** 4-nitrobenzyl 2-[(3S,4R)-3-bromo-4-formylthio-2-oxoazetidin-1-yl]-3-methylbut-2-enoate by carrying out the reaction in an aromatic organic solvent (Preferably Toluene) in the presence of an organic acid (Preferably formic acid), acetic anhydride/ organic tertiary base (preferably Pyridine) and trialkyl or triaryl phosphine (preferably triphenylphosphine) at -10° C to -30° C.

### Sequential conversion of compound 23 to 26 with out isolating the intermediates:

Product from Step 2 namely 4-nitrobenzyl 6-bromopenicillanate 1-oxide **23** was reacted with mercaptobenzothiazole in refluxing aromatic organic solvent (preferably Toluene) for 1 to 3 hrs and treated with triethylamine at 0 to -20° C for 3 to 4 hrs. After this treatment, reaction mixture was charged with organic acid (preferably formic acid) and an anhydride (acetic anhydride), an organic tertiary base (preferably pyridine) and a trialkyl or triaryl phosphate sequentially at -10° C to -40° C.

**Step 6:** The product 4-nitrobenzyl 2-[(3S,4R)-3-bromo-4-formylthio-2-oxoazetidin-1-yl]-3-methylbut-2-enoate **26** from either step 5 or from the sequential conversion was taken up in an organic solvent (preferentially ethyl acetate) at -70°C to -90° C and ozonized oxygen was passed through it for 3 to 4 hrs followed by intramolecular cyclization using a phosphite reagent (preferably trimethyl phosphite). The product 4-nitrobenzyl (5R,6S)-6-bromopenem-3-carboxylate **16** was crystallized from ethylacetae:hexane.

The compound represented by the follwing formula (**16**):

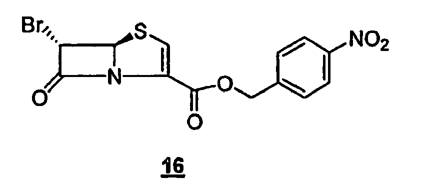

An above mentioned process (Step 1 to Step 6) for the preparation of the compound represented by the formula **16** is an intermediate useful for the preparation of 6-(substituted methylene)penems of general formula **I**.

The compound represented by the formula **16** is a crystalline derivative (X-ray powder diffraction parameters are given in the Table 2)

The crystalline nature of this intermediate impart stability and there by increases the shelf-life time. The stability data of the compound of the formula **16** is given in Table 1.

**Step 7:** Reaction of 4-nitrobenzyl (5R,6S)-6-bromopenem-3-carboxylate **16** with the appropriately substituted aldehydes (defind as before) to effect the aldol condensation step can be carried out in the presence of a Lewis acid (preferably anhydrous MgBr₂ or MgBr₂: 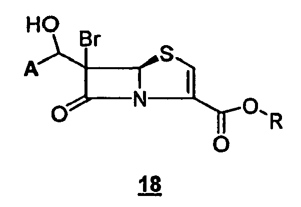 etherate) and a organic tertiary base (preferably triethylamine, DMAP or diisopropyl ethylamine) in an aprotic polar organic solvents (preferably THF and acetonitrile) at temperature -20° C to -40°C. The intermediate aldol products of general formula **18** can be functionalized with an acid chloride or anhydride to an acetate, triflic anhydride to a triflate or tosylchloride to a tosylate at 0°C to -10° C in the same pot; or, if formulae **18** is isolated, it can be converted to a halogen derivative by reacting **18** with tetrahalomethane and triphenyl phosphine at room temperature in a suitable organic solvent preferably CH₂Cl₂.

**Step 8:** The final step, namely the reductive elimination step to yield the compound of the general formula **I** can be carried out by dissolving the aldol product mixture in an organic solvent (preferably THF/ acetonitrile) and 6.5 -7.0 pH phosphate buffer and activated metal such as zinc, tin or aluminum at ambient temperature. (preferably at 20° C to 35°C) The product as a alkalie metal salt can be purified by a reverse phase resin column chromatography.

Alternatively Step 7 and Step 8 can be carried out sequentially in the same pot with out isolating the aldol intermediate.

The final step, namely the reductive elimination step can also be carried out by dissolving the aldol product in an organic solvent and 6.5-7.0 phosphate buffer and hydrogenating over Pd/C at 10 to 100 psi (preferably at 40 psi) pressure.

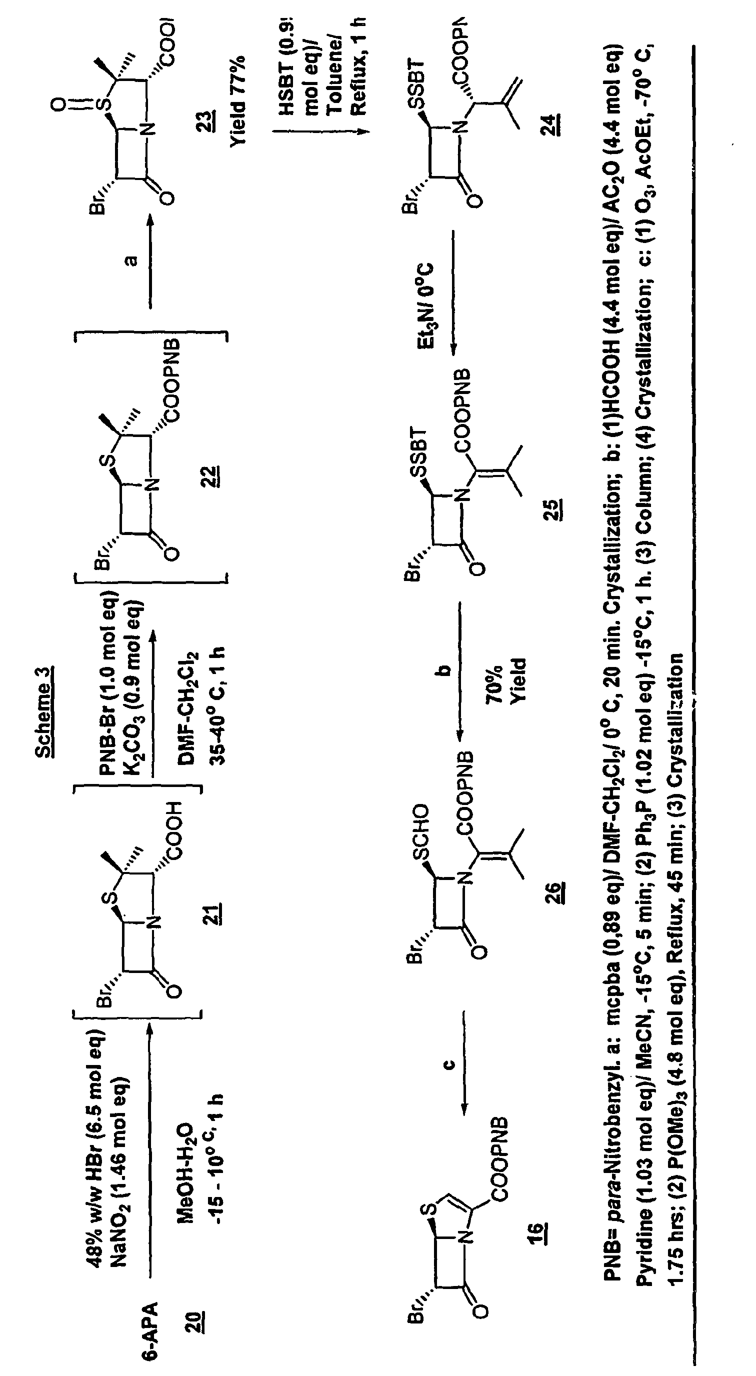

### Experimentals

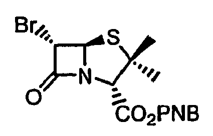

### Example 1: Preparation of Sodium (5R)-(Z)-6-(2,3-Dihydro-imidazo[2,1-b]thiazol-6-ylmethylene)penem-3-carboxylate

### Step 1: p-Nitrobenzyl 6-bromopenicillanate

6-Aminopenicillanic acid (5.0 g) was added to the cooled solution of methanol (44 mL), water (14 mL), and 48% w/w hydrobromic acid (14 mL) at -10 °C. After the addition was complete, the mixture was cooled to -15 °C. Sodium nitrite solution (2.4 g dissolved in 6.6 mL of water) was added over 5 min, and the resulting solution was stirred without cooling for a further 30 min. Sodium chloride (2.4 g) was dissolved in the reaction solution. The reaction mixture was extracted with dichloromethane (2 x 36 mL). The combined organic layers were washed with brine (36 mL), dried (MgSO₄), and concentrated to 20 mL under reduced pressure at 25°C. The residual solution contains 6-bromopenicillanic acid and used to next reaction as it is.
Anhydrous potassium carbonate (2.9 g), dimethylformamide (40 mL), and 4-Nitrobenzyl bromide (5.0 g) were successively added to the residual solution and the mixture was stirred at 35 - 40 °C for 1 h. The reaction solution was poured into a mixture of water (33 mL) and dichloromethane (41 mL) and the organic layer was separated. The organic layer was washed with water (40 mL) and brine (40 mL), dried (MgSO₄), and evaporated. The residue was applied to silica gel column chromatography, eluted with ethyl acetate - hexane (4/1), and the title compound was obtained as a colorless solid (7.2 g, 75%).

¹H-NMR (δ, CDCl3): 1.41 (s, 3H), 1.62 (s, 3H), 4.61 (s, 1H), 4.83 (d, 1H, *J* = 1.5 Hz), 5.25 and 5.34 (AB, 2H, *J* = 13.0 Hz), 5.41 (d, 1H, *J* = 1.5 Hz), 7.56 (d, 2H, *J* = 8.7 Hz), 8.26 (d, 2H, *J* = 8.7 Hz).

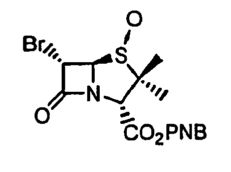

### Step 2: p-Nitrobenzyl 6-bromopenicillanate 1-oxide

p-Nitrobenzyl 6-bromopenicillanate (1.36 g) was dissolved in dichloromethane (20 mL) and cooled to 0 °C. 3-Chloroperbezoic acid (0.56 g) was added to the solution and stirred for 10 min. The reaction solution was washed with saturated sodium hydrogen carbonate, water and brine, dried (MgSO₄), and evaporated under reduced pressure. The residue was applied to silica gel column chromatography, eluted with ethyl acetate - hexane (1/1), and the title compound was obtained as a colorless solid (1.4 g, quantitative).

¹H-NMR (δ, CDCl3): 1.17 (s, 3H), 1.69 (s, 3H), 4.60 (s, 1H), 5.04 (d, 1H, *J* = 1.5 Hz), 5.10 (d, 1H, *J* = 1.5 Hz), 5.30 and 5.37 (AB, 2H, *J* = 12.9 Hz), 7.56 (d, 2H, *J* = 8.7 Hz), 8.27 (d, 2H, *J* = 8.7 Hz).

### Sequential Preparation of p-Nitrobenzyl 6-bromopenicillanate 1-oxide:

6-Aminopenicillanic acid (500 g) was added to the cooled solution of methanol (3.5 L), water (1.36 L), and 48% w/w hydrobromic acid (1.36 L) at -10 °C. After the addition was complete, the mixture was cooled to -15 °C. Sodium nitrite solution (235 g dissolved in 660 mL of water) was added over 30 min, and the resulting solution was stirred without cooling for a further 30 min. Sodium chloride (240 g) was dissolved in the reaction solution. The reaction mixture was extracted with dichloromethane (2 x 3 L). The combined organic layers were washed with brine (3 L), dried (MgSO₄), and concentrated to 1.6 L under reduced pressure at 25°C. The residual solution contains 6-bromopenicillanic acid and used to next reaction as it is.

Anhydrous potassium carbonate (288 g), dimethylformamide (2.8 L), and 4-Nitrobenzyl bromide (500 g) were successively added to the residual solution and the mixture was stirred at 35 - 40 °C for 1 h. The reaction solution was poured into a mixture of water (2.8 L) and dichloromethane (3.4 L) and the organic layer was separated. The organic layer was washed with water (2.8 L) and brine (3 L) and cooled to -2 °C. The organic layer contains p-nitrobenzyl 6-bromopenicillanate and used to next reaction as it is.

3-Chloroperbezoic acid (355 g) was added to the organic layer over 15 min and stirred for 10 min. The reaction mixture was diluted with ethyl acetate (4.8 L) and washed with saturated sodium hydrogen carbonate (7.6 L). The organic layer was separated and the aqueous layer was re-extracted with ethyl acetate (3.9 L). The combined organic layers were washed with water (3.9 L) and brine (3.9 L), dried (MgSO₄), and evaporated under reduced pressure at 30 °C. The residual solid was triturated with ethyl acetate (1.8 L) for 30 min and hexane (5.4 L) was added dropwise to this mixture over 1 h. The solid was filtered off and the filter cake was washed with ethyl acetate - hexane (1/4, 0.8 L). The solid was air-dried for 1 h and dried in vacuo overnight at room temperature. The title compound was obtained as a pale-yellow crystalline solid (768 g, 77%).

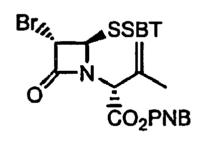

### Step 3: p-Nitrobenzyl (2R)-2-[(3S,4R)-4-(benzothiazol-2-yldithio)-3-bromo-2-oxoazetidin-1-yl]-3-methylbut-3-enoate

p-Nitrobenzyl 6-bromopenicillanate 1-oxide (4.31 g) and mercaptobenzothiazole (1.67 g) were heated in refluxing toluene (14 mL) with provision for the azeotropic removal of water (Dean & Stark apparatus) for 1 h. After cooling to room temperature, the reaction solution was evaporated under reduced pressure. The residue was applied to silica gel column chromatography, eluted with ethyl acetate - hexane (2/5), and the title compound was obtained as a pale yellow solid (5.67 g, 98%).

¹H-NMR (δ, CDCl3): 1.92 (s, 3H), 4.87 (s, 1H), 5.05 (s, 1H), 5.10 (d, 1H, *J* = 1.7 Hz), 5.21 (s, 4H), 7.39 (t, 1H, *J* = 7.1 Hz), 7.41 - 7.50 (m, 3H), 7.81 (d, 1H, *J* = 8.0 Hz), 7.90 (d, 1H, *J* = 8.0 Hz), 8.20 (d, 2H, *J* = 8.8 Hz).

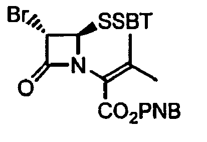

### Step-4: p-Nitrobenzyl 2-[(3S,4R)-4-(benzothiazol-2-yldithio)-3-bromo-2-oxoazetidin-1-yl]-3-methylbut-2-enoate

p-Nitrobenzyl (2R)-2-[(3S,4R)-4-(benzothiazol-2-yldithio)-3-bromo-2-oxoazetidin-1-yl]-3-methylbut-3-enoate (5.1 g) was dissolved in toluene (50 mL), cooled to 0 °C, and treated with triethylamine (0.12 mL). After being stirred at 0 °C for 2 h, the reaction solution was washed with 1M HCl, water, saturated sodium hydrogen carbonate, and brine, dried (MgSO₄), and evaporated under reduced pressure. The residue was applied to silica gel column chromatography, eluted with ethyl acetate - hexane (1/3), and the title compound was obtained as a colorless solid (4.16 g, 81%). The more polar fraction contains the symmetric disulfide as a colorless solid (0.68 g, 19%).
¹H-NMR (δ, CDCl3): 2.01 (s, 3H), 2.24 (s, 3H), 5.01 (d, 1H, *J* = 1.7 Hz), 5.24 and 5.30 (AB, 2H, *J* = 13.1 Hz), 5.35 (d, 1H, *J* = 1.7 Hz), 7.34-7.42 (m, 1H), 7.48 -7.57 (m, 1H), 7.80 (d, 1H, *J* = 8.0 Hz), 7.90 (d, 1H, *J* = 8.0 Hz), 8.14 (d, 3H, *J* = 8.7 Hz).

Symmetric disulfide; ¹H-NMR (δ, CDCl3): 2.01 (s, 6H), 2.29 (s, 6H), 4.73 (d, 2H, *J* = 1.7 Hz), 5.06 (d, 2H, *J* = 1.7 Hz), 5.32 (s, 4H), 7.54 (d, 4H, *J* = 8.6 Hz), 8.24 (d, 4H, *J* = 8.6 Hz).

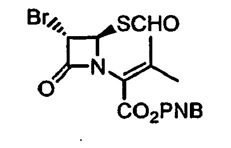

### Step 5:p-Nitrobenzyl 2-[(3S, 4R)-3-bromo-4-formylthio-2-oxoazetidin-1-yl]-3-methylbut-2-enoate

p-Nitrobenzyl 2-[(3S,4R)-4-(benzothiazol-2-yldithio)-3-bromo-2-oxoazetidin-1-yl]-3-methylbut-2-enoate (3.16 g) was suspended in toluene (20 mL) and cooled to -20 °C. Formic acid (1.10 g), acetic anhydride (2.45 g), and pyridine (0.44 g) were added sequentially. The mixture was cooled to -20 °C. Triphenylphosphine (1.45 g) was added in portions over 5 min. After being stirred at -15 ~ -10 °C tor further 1 h, the resulting suspension was cooled to -30 °C then filtered. The residual solid was rinsed with cold (-30 °C) toluene (15 mL). The combined filtrate was washed successively with a mixture of ice-water (8 mL) and ice-cold brine (1 mL), a mixture of ice-water (7 mL) and ice-cold brine (3 mL), cold saturated sodium hydrogen carbonate (2 x 11 mL), and cold brine (11 mL). The organic layer was dried (MgSO₄) and evaporated. The residue was applied to silica gel column chromatography, eluted with ethyl acetate - hexane (1/2), and the title compound was crystallized from a cold isopropanol (10 mL). The solid was filtered off, washed with a cold isopropanol (6 mL), and dried in vacuo at room temperature overnight. The title compound was obtained as a colorless crystalline solid (1.87 g, 77%)

m.p. 104-106 °C; ¹H-NMR (δ, CDCl3): 2.00 (s, 3H), 2.30 (s, 3H), 4.82 (d, 1H, *J* = 1.9 Hz), 5.32 and 5.38 (AB, 2H, *J* = 13.3 Hz), 5.81 (d, 1H, *J* = 1.9 Hz), 7.61 (d, 2H, *J* = 8.6 Hz), 8.25 (d, 2H, *J* = 8.6 Hz), 10.09 (s, 1H).

### Sequential Preparation of p-Nitrobenzyl 2-[(3S, 4R)-3-bromo-4-formylthio-2-oxoazetidin-1-yl]-3-methylbut-2-enoate from p-Nitrobenzyl 6-bromopenicillanate 1-oxide

p-Nitrobenzyl 6-bromopenicillanate 1-oxide (766 g) and mercaptobenzothiazole (294 g) were heated in refluxing toluene (2.4 L) with provision for the azeotropic removal of water (Dean & Stark apparatus) for 1 h. The reaction mixture was cooled to 0 °C and treated with triethylamine (25 mL). After being stirred at 0 °C for 2 h, the resulting suspension was cooled to -20 °C. Formic acid (360 g), acetic anhydride (798 g), and pyridine (145 g) were added sequentially maintaining the temperature below -10 °C. The mixture was cooled to -20 °C. Triphenylphosphine (473 g) was added in portions over 10 min maintaining the temperature between -15 °C and -10 °C. After being stirred at -15 ~ -10 °C for further 1 h, the resulting suspension was cooled to -30 °C then filtered. The residual solid was rinsed with cold (-30 °C) toluene (500 mL). The combined filtrate was washed successively with a mixture of ice-water (2 L) and ice-cold brine (0.26 L), a mixture of ice-water (1.72 L) and ice-cold brine (0.8 L), cold saturated sodium hydrogen carbonate (2 x 2.6 L), and cold brine (2.6 L). The organic layer was dried (MgSO₄) and evaporated. The residue was applied to silica gel (6 Kg) column chromatography, eluted with ethyl acetate - hexane (1/2), and the title compound was crystallized from a cold isopropanol (800 mL). The solid was filtered off, washed with a cold isopropanol (400 mL), air-dried for 1 h, and dried over P₂O₅ in vacuo at room temperature overnight. The title compound was obtained as a reddish yellow crystalline solid (553 g, 70%)

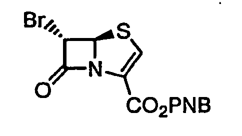

### Step 6: p-Nitrobenzyl (5R, 6S)-6-bromopenem-3-carboxylate

The p-Nitrobenzyl 2-[(3S, 4R)-3-bromo-4-formylthio-2-oxoazetidin-1-yl]-3-methylbut-2-enoate (300 g) was dissolved in ethyl acetate (7.5 L) and cooled to -70 °C. Ozonized oxygen was passed through the vigorously stirred solution for ca. 1.5 h until a blue color persisted. The solution was purged with nitrogen for 2.5 h, and treated with trimethyl phosphite (384 mL) at -70 °C. The mixture was warmed slowly and stirred at ambient temperature for 17 h. The solution was then heated to reflux gently for 45 min. After cooling to room temperature, the mixture was diluted with ethyl acetate (1.25 L) and washed with water (2 x 2.5 L) and brine (2.5 L). The organic layer was dried (MgSO₄) and evaporated under reduced pressure at 25 °C. The residual solid was triturated with ethyl acetate (0.6 L). Hexane (0.45 L) was added dropwise to this mixture over 50 min. The solid was filtered off and the filter cake was washed with ethyl acetate - hexane (1/1, 2 x 200 mL) and with ethyl acetate - hexane (1/2, 300 mL). The solid was air-dried for 1 h and dried in vacuo overnight at room temperature. The title compound (109.6 g, 42%) was obtained as a colorless crystalline solid.

m.p. 148-151°C; ¹H-NMR (□, CDCl3): 5.20 (d, 1H, *J* = 0.9 Hz), 5.29 and 5.43 (AB, 2H, *J* = 13.5 Hz), 5.81 (d, 1H, *J* = 1.5 Hz), 7.37 (d, 1H, *J* = 0.9 Hz), 7.60 (d, 2H, *J* = 8.7 Hz), 8.25 (d, 2H, *J* = 8.7 Hz).

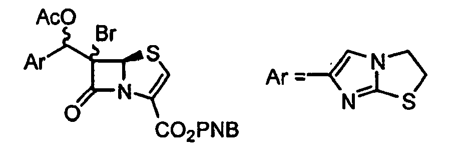

### Step 7: p-Nitrobenzyl (5R)-6-[acetoxy-(2,3-dihydro-imidazo[2,1-b]thiazol-6-yl)-methyl]-6-bromopenem-3-carboxylate by using Ph₂NLi and MgBr₂ etherate

MgBr₂ etherate (335 mg) was added to the dry THF (12 mL) solution of p-Nitrobenzyl (5R, 6S)-6-bromopenem-3-carboxylate (385 mg) under an argon atmosphere at room temperature. The mixture was cooled to -78 °C. The reaction vessel was covered with foil to exclude light. Lithium diphenylamide, prepared by adding n-butyl lithium (0.87 mL, 1.5 mol/L in n-hexane) to a dry THF solution (4 mL) of diphenylamine (220 mg) at -20 °C, was added to the vigorously stirred suspension in one portion. After 15-20 s, the vigorously stirred mixture was treated, in one portion, with a dry acetonitrile solution (5 mL) of 2,3-dihydro-imidazo[2,1-b]thiazole-6-carbaldehyde (170 mg). The reaction mixture was stirred for 1 h at -78 °C and treated with acetic anhydride (133 mg) in one portion. The reaction mixture was warmed to 0 °C and stirred for 1 h at 0 °C. The mixture was diluted with ethyl acetate and washed with water and brine. The organic layer was dried (MgSO₄) and concentrated under reduced pressure. The residue was applied to silica gel column chromatography, eluted with ethyl acetate - hexane (1/2 ~ 1/1), and the title compound was obtained as two diastereo mixture (3/1, a colorless amorphous solid, 447 mg, 77%).

Main diastereomer: ¹H NMR (δ, CDCl₃): 2.00 (s, 3H), 3.76-3.86 (m, 2H), 4.10-4.21 (m, 2H), 5.27 and 5.45 (AB, 2H, *J* = 13.6 Hz), 6.23 (s, 1H), 6.30 (s, 1H), 7.15 (s, 1H), 7.48 (s, 1H), 7.59-7.65 (m, 2H), 8.24 (d, 2H, *J* = 8.6 Hz).

Minor diastereomer: ¹H NMR (δ, CDCl₃): 2.24 (s, 3H), 3.76-3.86 (m, 2H), 4.10-4.21 (m, 2H), 5.27 and 5.45 (AB, 2H, *J* = 13.6 Hz), 6.08 (s, 1H), 6.79 (s, 1H), 6.91 (s, 1H), 7.44 (s, 1H), 7.59-7.65 (m, 2H), 8.24 (d, 2H, *J* = 8.6 Hz).

### Preparation of p-Nitrobenzyl (5R)-6-[acetoxy-(2,3-dihydro-imidazo[2,1-b]thiazol-6-yl)-methyl]-6-bromopenem-3-carboxylate by using by using anhydrous MgBr₂ and Et₃N

The 2,3-dihydro-imidazo[2,1-b]thiazole-6-carbaldehyde (88 mg) was added to the dry acetonitrile (4 mL) solution of anhydrous MgBr₂ (115 mg) under an argon atmosphere at room temperature. Colorless powder was deposited over 30 min. The dry THF solution (4 mL) of p-nitrobenzyl (5R, 6S)-6-bromopenem-3-carboxylate (200 mg) was added, cooled to -20 °C, and triethylamine (0.18 mL) was added in one portion. The reaction vessel was covered with foil to exclude light. The reaction mixture was stirred for 3 h at -20 °C and treated with acetic anhydride (0.99 mL) in one portion. The reaction mixture was warmed to 0 °C and stirred for 16 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, eluted with ethyl acetate - hexane (1/2 ~ 1/1), and the title compound was obtained as two diastereo mixture (10/1, a colorless amorphous solid, 286 mg, 90%).

### Preparation of p-Nitrobenzyl (5R)-6-[acetoxy-(2,3-dihydro-imidazo[2,1-b]thiazol-6-yl)-methyl]-6-bromopenem-3-carboxylate by using MgBr₂ etherate and Et₃N

MgBr₂ etherate was used for the reaction instead of anhydrous MgBr₂ and the product was isolated in 84% yields.

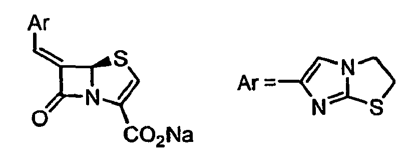

### Step 8:Sodium (5R)-(Z)-6-(2,3-Dihydro-imidazo[2,1-b]thiazol-6-ylmethylene)penem-3-carboxylate

p-Nitrobenzyl (5R)-6-[acetoxy-(2,3-dihydro-imidazo[2,1-b]thiazol-6-yl)-methyl]-6-bromopenem-3-carboxylate (500 mg) was dissolved with THF (7.0 mL) and acetonitrile (3.2 mL). Freshly activated Zn dust (2.0 g) was added rapidly with 0.5 M phosphate buffer (pH 6.5, 10.2 mL). The reaction vessel was covered with foil to exclude light. The reaction mixture was vigorously stirred for 2 h at room temperature. The mixture was diluted with water, cooled to 3 °C, and 1 M NaOH was added to adjust pH to 8. The reaction solution was mixed with ethyl acetate and filtered through a pad of Celite. The pad was washed with water and the aqueous layer was separated. The aqueous layer, contains 224 mg (79%) of the title compound from HPLC assay, was concentrated under high vacuum at 35 °C. The concentrate was applied to Daiaion HP-21 (20 mL, Mitsubishi Kasei Co. Ltd.) resin column chromatography. After absorbing, the column was eluted with water and then with 10% acetonitrile-water to give the purified active fractions of the title compound. The combined fractions was concentrated under high vacuum at 35 °C and lyophilized to give the title compound as a yellow amorphous solid (213 mg, 75%). The amorphous solid of the product was dissolved with water (1.9 mL). Acetone (8 mL) was added to the solution with stirring at room temperature and cooled to 3 °C then stirred for 30 min. Acetone was added to the mixture in 2 mL portions every 30 min over 4 h period at 3 °C (total 16 mL of acetone was added). After stirring for 1 hour at 3 °C, the solid was filtered off and the filter cake was washed with acetone (2 mL). The solid was air-dried under excluding light for 1 h and dried in vacuo overnight at room temperature to give the title compound (200 mg, 71%) as a yellow crystalline solid.

¹H NMR (δ, D₂O) 3.82 (d, 2H, *J* = 7.4 Hz), 4.14-4.20 (m, 2H), 6.42 (s, 1H), 6.82 (s, 1H), 6.96 (s, 1H), 7.42 (s, 1H).

### Sequential Preparation of Sodium (5R)-(Z)-6-(2,3-Dihydro-imidazo[2,1-b]thiazol-6-ylmethylene)penem-3-carboxylate from p-Nitrobenzyl (5R)-6-[acetoxy-(2,3-dihydro-imidazo[2,1-b]thiazol-6-yl)-methyl]-6-bromopenem-3-carboxylate

The p-Nitrobenzyl (5R)-6-[acetoxy-(2,3-dihydro-imidazo[2,1-b]thiazol-6-yl)-methyl]-6-bromopenem-3-carboxylate (110.1 g) was added to the dry acetonitrile (5 L) solution of anhydrous MgBr₂ (143.4 g) under an argon atmosphere at room temperature. Colorless powder was deposited over 30 min. The dry THF solution (5 L) of p-nitrobenzyl 6-bromopenicillanate 1-oxide (250 g) was transferred to the suspension via a PTFE tube under positive argon pressure over 12 minutes. After the reaction mixture was cooled to -20 °C, triethylamine (217 mL) was added in one portion. The reaction vessel was covered with foil to exclude light. The reaction mixture was stirred for 3 h at -20 °C and treated with acetic anhydride (133 g) in one portion. The reaction mixture was warmed to 0°C and stirred for 16 h at 0 °C. The mixture was diluted with ethyl acetate (20 L) and washed with 5% citric acid (10 L) aqueous solution, saturated sodium hydrogen carbonate (10 L), and brine (10 L). The organic layer was dried (MgSO₄) and filtered through a pad of Celite. The pad was washed with ethyl acetate (1.4 L). The filtrate was concentrated under reduced pressure at 30 °C. The residual brown oil contains 327 g (86.7%) of the aldol product from HPLC assay.

The residual brown oil of the product was dissolved with THF (5.4 L) and acetonitrile (2.5 L). Freshly activated Zn dust (1.5 Kg) was added rapidly with 0.5 M phosphate buffer (pH 6.5, 7.9 L). The reaction vessel was covered with foil to exclude light. The reaction mixture was vigorously stirred for 2.5 h maintaining the temperature about 35 °C. The mixture was cooled to 3 °C and an ice cold 1 M NaOH (ca. 1750 mL) was added to adjust pH to 8 at below 3°C. The mixture was diluted with ethyl acetate (4 L) and filtered through a pad of Celite. The pad was washed with water (2.5 L) and the aqueous layer was separated. The aqueous layer, contains 137 g (64%) of from HPLC assay, was concentrated to 8.85 Kg (ca. 8L) under high vacuum at 35 °C.

The concentrate was applied to Sepabeads SP-207 (7 L, Mitsubishi Kasei Co. Ltd.) resin column chromatography. After absorbing, the column was eluted with water (14 L) and then with 20% acetonitrile-water to give the purified active fractions of sodium (5R)-(Z)-6-(2,3-dihydro-imidazo[2,1-b]thiazol-6-ylmethylene)penem-3-carboxylate.

The combined fractions was concentrated to 1.25 Kg (ca. 1 L, contains 130 g (61%) of sodium (5R)-(Z)-6-(2,3-dihydro-imidazo[2,1-b]thiazol-6-ylmethylene)penem-3-carboxylate from HPLC assay) under high vacuum at 35°C. Acetone (2.5 L) was added to the concentrate with stirring at room temperature. The mixture was cooled to 3°C, then stirred for 30 min. Acetone was added to the mixture in 625 mL portions every 30 min over 4 h period at 3 °C (total 5 L of acetone was added). After stirring for 1 hour at 3°C, the solid was filtered off and the filter cake was washed with acetone (800 mL). The solid was air-dried under excluding light for 1 h and dried in vacuo overnight at room temperature to give the title (112 g, 50% from p-Nitrobenzyl (5R)-6-[acetoxy-(2,3-dihydro-imidazo[2,1-*b*]thiazol-6-yl)-methyl]-6-bromopenem-3-carboxylate) as a yellow crystalline solid.

### Sodium (5R)(Z)-6-(2,3-Dihydro-imidazo[2,1-b]thiazol-6-ylmethylene)penem-3-carboxylate from 4-Nitrobenzyl (5R)-6-[acetoxy-(2,3-dihydro-imidazo[2,1-b]thiazol-6-yl)-methyl]-6-bromopenem-3-carboxylate using hydrogenolysis procedure:

4-Nitrobenzyl (5R)-6-[acetoxy-(2,3-dihydro-imidazo[2,1-b]thiazol-6-yl)-methyl]-6-bromopenem-3-carboxylate (116 mg0 and 10% Pd?C (50% wet, 58 mg) were addedto a mixture of THF (4 ml) and 0.5 mol/L phosphate buffer (pH 6.5, 4 ml). The mixture was hydrogenated under H₂ at 40 psi pressure at room temperature for 1 hr. The reaction mixture was filtered, cooled to 3° C and 1 M NaOH was added to adjust the pH to 8.0. The mixture was filtered again and the filtrate was washed with ethyl acetate. The combined aqueous layer was concentrated under reduced pressure at 35° C and applied to Diaion HP-21 resin (7 ml, Mitsubishi Kasei Co. Ltd) column chromatography. After adsorbing the column was eluted with water and then 10% acetonitrile; water. The combined pure fractions were concentrated under reduced pressure at 35°C and lyophilized to give the title compound as a yellow amorphous solid.

### Example 2

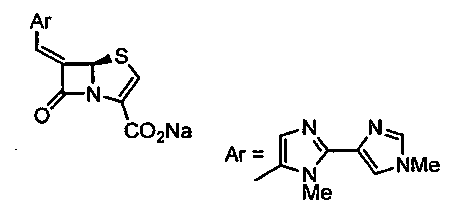

### Step 1: 3,1'-Dimethyl-3H,1'H-[2,4']biimidazolyl-4-carbaldehyde

Potassium tert-butoxide (3.23 g) was added to the mixture of dry DMF (140 mL) solution of 1H,1'H-[2,4']Biimidazolyl-4-carbaldehyde (1.95 g) and 18-crown-6 (634 mg) at 0°C. The reaction mixture was stirred for 10 min and treated with methyl iodide (1.85 mL). After stirring for 30 at °C and then 17 h at room temperature, The reaction mixture was concentrated under reduced pressure. The residue was dissolved with H₂O-CHCl₃ and separated. The aqueous layer was extracted with chloroform. The organic layer was dried (MgSO₄) and filtered. The filtrate was evaporated under reduced pressure. The crude material was purified with silica gel column chromatography (chloroform - methanol =9/1). The title compound (887 mg, 39%) and its positional isomer 1,1'-Dimethyl-1H,1'H-[2,4']biimidazolyl-4-carbaldehyde (296 mg, 13%) were obtained as a pale pink solid.

3,1'-Dimethyl-3H,1'H-[2,4']biimidazolyl-4-carbaldehyde: ¹H NMR (δ, CDCl₃) 3.78 (s, 3H), 4.37 (s, 3H), 7.51 (d, 1H, *J* =1.0 Hz), 7.62 (d, 1H, *J* = 1.0 Hz), 7.76 (s, 1H), 9.71 (s, 1H).

1,1'-Dimethyl-1H,1'H-[2,4']biimidazolyl-4-carbaldehyde : ¹H NMR (δ, CDCl₃) 3.76 (s, 3H), 4.10 (s, 3H), 7.47 (d, 1H, *J* = 0.9 Hz), 7.57 (s, 1H), 7.59 (d, 1H *J* = 0.9 Hz), 9.85 (s, 1H).

### Step 2: (5R, 6RS)-6-[(RS)-Acetoxy-(3,1'-dimethyl-3H,1'H-[2,4']biimidazolyl-4-yl)-methyl]-6-bromo-7-oxo-4-thia-1-aza-bicyclo[3.2.0]hept-2-ene-2-carboxylic acid 4-nitro-benzyl ester

3,1'-Dimethyl-3H,1'H-[2,4']biimidazolyl-4-carbaldehyde (171 mg) was added to the dry acetonitrile (20 mL) solution of anhydrous MgBr₂ (800 mg) under a nitrogen atmosphere at room temperature. The dry THF solution (20 mL) of (5R, 6S)-6-bromo-7-oxo-4-thia-1-aza-bicyclo[3.2.0]hept-2-ene-2-carboxylic acid 4-nitro-benzyl ester (346 mg) was added to the mixture, cooled to -20 °C, and triethylamine (0.768 mL) was added in one portion. The reaction vessel was covered with foil to exclude light. The reaction mixture was stirred for 2 h at -20 °C and treated with 4-dimethylaminopyridine (22 mg) and acetic anhydride (0.17 mL) in one portion. The reaction mixture was warmed to 0 °C and stirred for 17 h at 0°C. The mixture was diluted with ethyl acetate and washed with 5% citric acid aqueous solution, saturated sodium hydrogen carbonate, and brine. The organic layer was dried (MgSO₄) and filtered. The filtrate was concentrated under reduced pressure. The residue was applied to silica gel column chromatography, then eluted with chloroform - methanol (9/1). The title compound was obtained (507.6 mg, 91.4%). ¹H NMR (δ, CDCl₃) 2.19 (s, 3H), 3.75 (s, 3H), 4.03 (s, 3H), 5.29 (d, 1H, *J* = 13.5 Hz), 5.42 (d, 1H, *J* = 13.5 Hz), 6.03 (s, 1H), 6.65 (s, 1H), 7.34 (s, 1H), 7.48 (d, 1H, *J* = 1.2 Hz), 7.55 (d, 1H, *J* = 1.2 Hz), 7.59-7.61 (m, 3H), 8.23-8.26 (m, 2H).

### Step 3: (5R), (6Z)-6-(3,1'-Dimethyl-3H,1'H-[2,4]biimidazolyl-4-ylmethylene)-7-oxo-4-thia-1-aza-bicyclo[3.2.0]hept-2-ene-2-carboxylic acid sodium salt

(5*R*, 6*RS*)-6-[(*RS*)-Acetoxy-(3,1'-dimethyl-3H,1'H-[2,4']biimidazolyl-4-yl)-methyl]-6-bromo-7-oxo-4-thia-1-aza-bicyclo[3.2.0]hept-2-ene-2-carboxylic acid 4-nitro-benzyl ester (596 mg) was dissolved in THF (8.3 mL) and acetonitrile (3.9 mL). Freshly activated Zn dust (2.38 g) and 0.5 M phosphate buffer (pH 6.5, 12.2 mL) were added to the mixture. The reaction vessel was covered with foil to exclude light. The reaction mixture was vigorously stirred for 2 h at room temperature. The reaction solution was mixed with ethyl acetate and filtered through a pad of Celite. The pad was washed with water and the aqueous layer was separated. The aqueous layer was concentrated under high vacuum at 35 °C. The concentrate was applied to Diaion HP-21 (60 mL, Mitsubishi Kasei Co. Ltd.) resin column chromatography. After adsorbing, the column was eluted with water and then with 5% - 10% acetonitrile-water. The combined fractions was concentrated under high vacuum at 35 °C and lyophilized to give the title compound as a yellow amorphous solid (66.3 mg, 19%). Mp 113 °C (dec); ¹H NMR (δ, D₂O) 3.31 (s, 3H), 3.52 (s, 3H), 5.74 (s, 1H), 6.43 (s, 2H), 6.70 (s, 1H), 7.27 (s, 1H), 7.46 (s 1H)

### Example 3

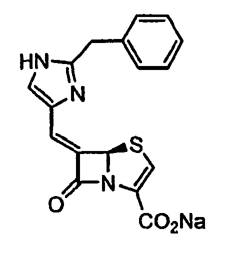

### Step 1: 2-Benzyl-1H-imidazole-4-carbaldehyde

2-Phenyl-acetoamidine (2 g) was added to the solution of 2-bromo-3-isopropoxy-propenal (2.88 g) in CHCl₃ (30 mL). The reaction mixture was stirred for 1 h at room temperature. Then triethylamine (2.09 mL) was added to the mixture and heated to reflux for 6 h. The mixture was cooled to room temperature, diluted with CHCl₃, and washed with 10% potassium hydrogen carbonate. The organic layer was dried (MgSO₄) and concentrated under reduced pressure. The residue was applied to silica gel column chromatography, then eluted with ethyl acetate - hexane (3/1). The crude compound was crystallized from ethyl acetate and n-hexane, to give the title compound (1.10 g, 40%). ¹H NMR (δ, CDCl₃) 4.17 (s, 2H), 7.24-7.36 (m, 5H), 7.72 (brs, 1H), 9.60 (brs, 1H), 10.28 (brs, 1H).

### Step 2: 2-Benzyl-4-formyl-imidazole-1-carboxylic acid 4-nitro-benzyl ester

2-Benzyl-1*H*-imidazole-4-carbaldehyde (1 g) and sodium hydrogen carbonate (1.13 g) were dissolved in dioxane (30 mL) and water (30 mL). The 48.7% solution of p-nitrobenzyl chloroformate (PNZCI) in dioxane (2.62 g) was added to the solution at 0 °C and stirred for 2.5 h. The reaction mixture was diluted with ethyl acetate and washed with saturated sodium hydrogen carbonate and brine. The organic layer was dried (MgSO₄) and concentrated under reduced pressure. The residue was applied to silica gel column chromatography, then eluted with chloroform - acetone (9/1). The title compound was obtained as pale brown oil (1.97 g, 100%). ¹H NMR (δ, CDCl₃) 4.46 (s, 2H), 5.42 (s, 2H), 7.19-7.30 (m, 5H), 7.44-7.47 (m, 2H), 8.06 (s, 1H), 8.21-8.25 (m, 2H), 9.91 (s, 1H).

### Step 3: (5R, 6RS)-6-{(RS)-Acetoxy-[2-benzyl-1-(4-nitro-benzyloxycarbonyl)-1H-imidazol-4-yl]-methyl}-6-bromo-7-oxo-4-thia-1-aza-bicyclo[3.2.0]hept-2-ene-2-carboxylic acid 4-nitro-benzyl ester

The solution of 2-benzyl-4-formyl-imidazole-1-carboxylic acid 4-nitro-benzyl ester (1.9 g) in dry acetonitrile (10 mL) was added to the dry acetonitrile (50 mL) solution of anhydrous MgBr₂ (2.41 g) under a nitrogen atmosphere at room temperature. The dry THF solution (60 mL) of (5*R*, 6*S*)-6-bromo-7-oxo-4-thia-1-aza-bicyclo[3.2.0]hept-2-ene-2-carboxylic acid 4-nitro-benzyl ester (2 g) was added to the mixture, cooled to - 20 °C, and triethylamine (1.8 mL) was added in one portion. The reaction vessel was covered with foil to exclude light. The reaction mixture was stirred for 2 h at -20 °C and treated with 4-dimethylaminopyridine (127 mg) and acetic anhydride (0.98 mL) in one portion. The reaction mixture was warmed to 0 °C and stirred for 16 h at 0 °C. The mixture was diluted with ethyl acetate and washed with H₂O, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then eluted with ethyl acetate - hexane (2/3 ~ 1/1). The title compound was obtained as two diastereo mixture (3/2, a pale yellow amorphous solid, 2.8 g, 70%). ¹H NMR (δ, CDCl₃) 2.00 (s, 0.6 x 3H), 2.26 (s, 0.4 x 3H), 4.34-4.37 (m, 2H), 5.25-5.47 (m, 4H), 6.02 (s, 0.4 x 1H), 6.22 (s, 0.6 x 1H), 6.25 (s, 0.6 x 1H), 6.85 (d, 0.4 x 1H, *J* = 1.5 Hz), 7.14-7.62 (m, 11H), 8.21-8.25 (m, 4H).

### Step 4: (5R), (6Z)-6-(2-Benzyl-1H-imidazol-4-ylmethylene)-7-oxo-4-thia-1-aza-bicyclo[3.2.0]hept-2-ene-2-carboxylic acid sodium salt

(5*R*, 6*RS*)-6-{(*RS*)-Acetoxy-[2-benzyl-1-(4-nitro-benzyloxycarbonyl)-1*H-*imidazol-4-yl]-methyl}-6-bromo-7-oxo-4-thia-1-aza-bicyclo[3.2.0]hept-2-ene-2-carboxylic acid 4-nitro-benzyl ester (2.5 g) was dissolved in THF (35 mL) and acetonitrile (16.3 mL). Freshly activated Zn dust (10 g) and 0.5 *M* phosphate buffer (pH 6.4, 51.3 mL) were added to the mixture. The reaction vessel was covered with foil to exclude light. The reaction mixture was vigorously stirred for 2 h at room temperature. The mixture was cooled to 9°C, and 1 M NaOH aqueous solution was added to adjust pH to 7.5. The reaction solution was mixed with ethyl acetate and filtered through a pad of Celite. The pad was washed with water and the aqueous layer was separated. The aqueous layer was concentrated under high vacuum at 35 °C. The concentrate was applied to Diaion HP-21 (250 mL, Mitsubishi Kasei Co. Ltd.) resin column chromatography. After adsorbing, the column was eluted with water and then with 5-10% acetonitrile-water. The combined fractions was concentrated under high vacuum at 35 °C and lyophilized to give the title compound as a yellow amorphous solid (780 mg, 67%). Mp 146 °C (dec); ¹H NMR (δ, D₂O) 3.92 (s, 2H), 6.39 (d, 1H, *J* = 0.8 Hz), 6.74 (s, 1H), 6.89 (s, 1H), 7.13-7.16 (m, 3H), 7.21-7.25 (m, 3H).

### Example 4

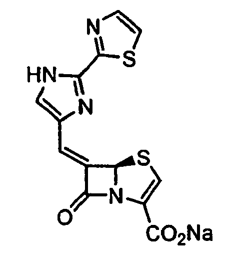

### Step 1: 4-Formyl-2-thiazol-2-yl-imidazol-1-carboxylic acid 4-nitro-benzyl ester

2-Thiazol-2-yl-1*H*-imidazol-4-carbaldehyde (570 mg) was dissolved in dry CH₂Cl₂ (35 mL). *N,N'*-Diisopropylethylamine and 48.7% solution of *p*-nitrobenzyl chloroformate (PNZCl) in dioxane (1.69 mL) were added successively to the solution at 0 °C and stirred for 2 h. The reaction mixture was diluted with CHCl₃ and washed with saturated sodium hydrogen carbonate and brine. The organic layer was dried (MgSO₄) and concentrated under reduced pressure. The residue was crystallized from ethyl acetate and n-hexane to give the title compound (1.05 g, 92%). ¹H NMR (δ, CDCl₃) 5.49 (s, 2H), 7.50 (d, 2H, *J* = 8.8 Hz), 7.55 (d, 1H, *J* = 3.4 Hz), 7.89 (d, 1H, *J* = 3.1 Hz), 8.19 (s, 1H), 8.24 (dq, 2H, *J* = 8.8, 2.0 Hz), 9.97 (s, 1H).

### Step 2: (5R), (6Z)-6-(2-thiazol-2-yl-1H-imidazol-4-ylmethylene)-7-oxo-4-thia-1-aza-bicyclo[3.2.0]hept-2-ene-2-carboxylic acid sodium salt

4-Formyl-2-thiazol-2-yl-imidazol-1-carboxylic acid 4-nitro-benzyl ester (940 mg) was added to the dry acetonitrile (35 mL) solution of anhydrous MgBr₂ (1.26 g) under a nitrogen atmosphere at room temperature. The dry THF solution (28 mL) of (5R, 6S)-6-bromo-7-oxo-4-thia-1-aza-bicyclo[3.2.0]hept-2-ene-2-carboxylic acid 4-nitro-benzyl ester (1.01 g) was added to the mixture, cooled to -20 °C, and triethylamine (0.942 mL) was added in one portion. The reaction vessel was covered with foil to exclude light. The reaction mixture was stirred for 2.8 h at -20 °C and treated with 4-dimethylaminopyridine (64 mg) and acetic anhydride (0.495 mL) in one portion. The reaction mixture was warmed to 0 °C and stirred for 20 h at 0 °C. The mixture was diluted with ethyl acetate and washed with 0.1 M phosphate buffer (pH 7), saturated sodium hydrogen carbonate, and brine. The organic layer was dried (MgSO₄) and filtered through a pad of Celite. The pad was washed with ethyl acetate. The filtrate was concentrated under reduced pressure. The residue was roughly purified by silica gel column chromatography (ethyl acetate - hexane = 1/1 ~ ethyl acetate only) and the crude (5*R*, 6*RS*)-6-{(*RS*)-acetoxy-[1-(4-nitro-benzyloxycarbonyl)-2-thiazol-2-yl-1*H*-imidazol-4-yl]-methyl}-6-bromo-7-oxo-4-thia-1-aza-bicyclo[3.2.0]hept-2-ene-2-carboxylic acid 4-nitro-benzyl ester (860 mg) was obtained.

### Step 3:

The crude (5*R*, 6*RS*)-6-{(*RS*)-acetoxy-[1-(4-nitro-benzyloxycarbonyl)-2-thiazol-2-yl-1*H*-imidazol-4-yl]-methyl}-6-bromo-7-oxo-4-thia-1-aza-bicyclo[3.2.0]hept-2-ene-2-carboxylic acid 4-nitro-benzyl ester (860 mg) was dissolved in THF (12 mL) and acetonitrile (5.6 mL). Freshly activated Zn dust (3.44 g) and 0.5 *M* phosphate buffer (pH 6.4, 17.6 mL) were added to the solution. The reaction vessel was covered with foil to exclude light. The reaction mixture was vigorously stirred for 2 h at room temperature. The mixture was mixed with ethyl acetate and filtered through a pad of Celite. The pad was washed with water and the aqueous layer was separated. The aqueous layer was concentrated under high vacuum at 35 °C. The concentrate was applied to Diaion HP-21 (90 mL, Mitsubishi Kasei Co. Ltd.) resin column chromatography. After adsorbing, the column was eluted with water and then with 2.5-5% acetonitrile-water. The combined fractions was concentrated under high vacuum at 35 °C and lyophilized to give the title compound as a yellow amorphous solid (126 mg, 11 % from 7). Mp 145 °C (dec); ¹H NMR (δ, D₂O) 6.40 (d, 1H, *J* = 0.7 Hz), 6.81 (s, 1H), 6.89 (s, 1H), 7.38 (s, 1H), 7.48 (d, 1H, *J* = 3.1 Hz), 7.71 (d, 1H, *J* = 3.3 Hz).

### Example 5

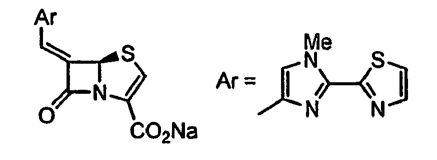

### Step 1: 1-Methyl-2-thiazol-2-yl-1H-imidazol-4-carbaldehyde

Potassium tert-butoxide (494 mg) was added to the dry THF (30 mL) and dry DMF (10 mL) mixed solution of 2-thiazol-2-yl-1*H*-imidazol-4-carbaldehyde (717 mg) and 18-crown-6 (106 mg) at room temperature. The reaction mixture was stirred for 10 min and treated with methyl iodide (0.274 mL). After stirring for 17.5 h at room temperature, it was concentrated under reduced pressure. The residue was dissolved with ethyl acetate and filtered. The filtrate was evaporated under reduced pressure. The crude material was purified with silica gel column chromatography (ethyl acetate - hexane = 1 /1). The title compound (410 mg, 53%) and its positional isomer 1-methyl-2-thiazol-2-yl-1*H*-imidazol-5-carbaldehyde (240 mg, 31%) were obtained as a white solid.

1-Methyl-2-thiazol-2-yl-1*H*-imidazol-4-carbaldehyde: ¹H NMR (δ, CDCl₃) 4.21 (s, 3H), 7.44 (d, 1H, *J* = 3.2 Hz), 7.68 (s, 1H), 7.89 (d, 1H, *J* = 3.2 Hz), 9.91 (s, 1H).

1-methyl-2-thiazol-2-yl-1*H*-imidazol-5-carbaldehyde: ¹H NMR (δ, CDCl₃) 4.48 (s, 3H), 7.51 (d, 1H, *J* = 3.1 Hz), 7.82 (s, 1H), 7.97 (d, 1H, *J* = 3.1 Hz), 9.82 (s, 1H).

### Step 2: (5R, 6RS)-6-[(RS)-Acetoxy-(1-methyl-2-thiazol-2-yl-1H-imidazol-4-yl)-methyl]-6-bromo-7-oxo-4-thia-1-aza-bicyclo[3.2.0]hept-2-ene-2-carboxylic acid 4-nitro-benzyl ester

1-Methyl-2-thiazol-2-yl-1*H*-imidazol-4-carbaldehyde (380 mg) was added to the dry acetonitrile (28 mL) solution of anhydrous MgBr₂ (1.03 g) under a nitrogen atmosphere at room temperature. Colorless powder was deposited over 10 min. The dry THF solution (28 mL) of (5R, 6S)-6-bromo-7-oxo-4-thia-1-aza-bicyclo[3.2.0]hept-2-ene-2-carboxylic acid 4-nitro-benzyl ester (760 mg) was added to the mixture, cooled to -20 °C, and triethylamine (0.768 mL) was added in one portion. The reaction vessel was covered with foil to exclude light. The reaction mixture was stirred for 2 h at -20 °C and treated with 4-dimethylaminopyridine (48 mg) and acetic anhydride (0.371 mL) in one portion. The reaction mixture was warmed to 0 °C and stirred for 22 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then eluted with ethyl acetate - hexane (3/2). The title compound was obtained as two diastereo mixture (1/1, a pale yellow amorphous solid, 673.4 mg, 52%). ¹H NMR (δ, CDCl₃) 2.00 (s, 0.5 x 3H), 2.28 (s, 0.5 x 3H), 4.12 (s, 3H), 5.28 (d, 0.5 x 1H, *J* = 13.5 Hz), 5.29 (d, 0.5 x 1H, *J* = 13.5 Hz), 5.44 (d, 0.5 x 1H, *J* = 13.5 Hz), 5.47 (d, 0.5 x 1H, *J* = 13.5 Hz), 6.16 (s, 0.5 x 1H), 6.30 (s, 0.5 x 1H), 6.42 (s, 0.5 x 1H), 6.87 (d, 0.5 x 1H, *J* = 0.8 Hz), 6.92 (d, 0.5 x 1H, *J* = 0.8 Hz), 7.19 (s, 0.5 x 1H), 7.34-7.36 (m, 1H), 7.47 (s, 0.5 x 1H), 7.50 (s, 0.5 x 1H), 7.60-7.64 (m, 2H), 7.83-7.85 (m, 1H), 8.23-8.26 (m, 1H).

### Step 3: (5R), (6Z)-6-(1-Methyl-2-thiazol-2-yl-1H-imidazol-4-ylmethylene)-7-oxo-4-thia-1-aza-bicyclo[3.2.0]hept-2-ene-2-carboxylic acid sodium salt

(5*R*, 6*RS*)-6-[(*RS*)-Acetoxy-(1-methyl-2-thiazol-2-yl-1*H*-imidazol-4-yl)-methyl]-6-bromo-7-oxo-4-thia-1-aza-bicyclo[3.2.0]hept-2-ene-2-carboxylic acid 4-nitro-benzyl ester (604 mg) was dissolved in THF (8.5 mL) and acetonitrile (3.9 mL). Freshly activated Zn dust (2.41 g) and 0.5 *M* phosphate buffer (pH 6.4, 23.6 mL) were added to the mixture. The reaction vessel was covered with foil to exclude light. The reaction mixture was vigorously stirred for 2 h at room temperature. The reaction solution was mixed with ethyl acetate and filtered through a pad of Celite. The pad was washed with water and the aqueous layer was separated. The aqueous layer was concentrated under high vacuum at 35 °C. The concentrate was applied to Diaion HP-21 (60 mL, Mitsubishi Kasei Co. Ltd.) resin column chromatography. After adsorbing, the column was eluted with water and then with 5% acetonitrile-water and 10% acetonitrile-water. The combined fractions was concentrated under high vacuum at 35 °C and lyophilized to give the title compound as a yellow amorphous solid (231 mg, 64%). Mp 214 °C (dec); ¹H NMR (δ, D₂O) 3.91 (s, 3H), 6.44 (s, 1H), 6.84 (s, 1H), 6.91 (s, 1H), 7.43 (s, 1H), 7.60 (d, 1H, *J* = 3.3 Hz), 7.84 (d, 1H, *J* = 3.3 Hz).

### Example 6

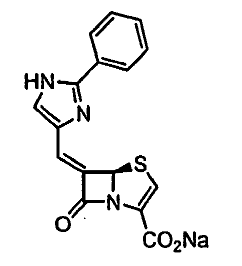

### Step 1: 4-Formyl-2-phenyl-imidazole-1-carboxylic acid 4-nitro-benzyl ester

4-Formyl-2-phenylimidazole (624 mg) and sodium hydrogen carbonate (791 mg) were dissolved in dioxane (3.6 mL), THF (3.6 mL) and water (7.2 mL). The 48.7% solution of p-nitrobenzyl chloroformate (PNZCl) in dioxane (2.08 mL) was added to the mixture at room temperature and stirred for 2.5 h. The mixture was diluted with ethyl acetate and washed with brine. The organic layer was dried (MgSO₄) and concentrated under reduced pressure. The residue was crystallized from ethyl acetate and n-hexane to give the title compound (956 mg, 75%). ¹H NMR (δ, CDCl₃) 5.41 (s, 2H), 7.32 (d, 2H, *J* = 8.6 Hz), 7.40-7.51 (m, 3H), 7.56-7.58 (m, 2H), 8.17-8.20 (m, 2H), 8.22 (s, 1H), 9.97 (s, 1H).

### Step 2: (5R, 6RS)-6-{(RS)-Acetoxy-[1-(4-nitro-benzyloxycarbonyl)-2-phenyl-1H-imidazol-4-yl]-methyl}-6-bromo-7-oxo-4-thia-1-aza-bicyclo[3.2.0]hept-2-ene-2-carboxylic acid 4-nitro-benzyl ester

4-Formyl-2-phenyl-imidazole-1-carboxylic acid 4-nitro-benzyl ester (568 mg) and the dry THF solution (15 mL) of (5R, 6S)-6-bromo-7-oxo-4-thia-1-aza-bicyclo[3.2.0]hept-2-ene-2-carboxylic acid 4-nitro-benzyl ester (587 mg) were added successively to the dry acetonitrile (15 mL) solution of anhydrous MgBr₂ (622.4 mg) under a nitrogen atmosphere at room temperature. After cooling the mixture to -20 °C, triethylamine (0.494 mL) was added in one portion. The reaction vessel was covered with foil to exclude light. The reaction mixture was stirred for 1.5 h at -20 °C and treated with acetic anhydride (0.277 mL) in one portion. The reaction mixture was warmed to 0 °C and stirred for 27 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then eluted with ethyl acetate - hexane (2/3 ~ 1/1). The title compound was obtained as three diastereo mixture (6/4/1.5, a pale yellow amorphous solid, 986 mg, 86%). ¹H NMR (δ, CDCl₃) 2.04 (s, 0.35 x 3H), 2.10 (s, 0.13 x 3H), 2.30 (s, 0.52 x 3H), 5.25-5.43 (m, 4H), 6.09 (s, 0.52 x 1H), 6.22 (s, 0.13 x 1H), 6.31 (s, 0.35 x 1H), 6.33 (s, 0.35 x 1H), 6.87 (s, 0.13 x 1H), 6.92 (d, 0.52 x 1H, *J* = 1.4 Hz), 7.31-7.76 (m, 11H), 8.17-8.25 (m, 4H).

### Step 3:(5R), (6Z)-6-(2-phenyl-1H-imidazol-4-ylmethylene)-7-oxo-4-thia-1-aza-bicyclo[3.2.0]hept-2-ene-2-carboxylic acid

(5*R*, 6*RS*)-6-{(*RS*)-Acetoxy-[1-(4-nitro-benzyloxycarbonyl)-2-phenyl-1*H-*imidazol-4-yl]-methyl}-6-bromo-7-oxo-4-thia-1-aza-bicyclo[3.2.0]hept-2-ene-2-carboxylic acid 4-nitro-benzyl ester (4) (1.15 g) was dissolved in THF (16.1 mL) and acetonitrile (7.5 mL). Freshly activated Zn dust (4.6 g) and 0.5 M phosphate buffer (pH 6.4, 23.6 mL) were added to the mixture. The reaction vessel was covered with foil to exclude light. The reaction mixture was vigorously stirred for 2 h at room temperature. The reaction solution was mixed with ethyl acetate and filtered through a pad of Celite. The pad was washed with water and the aqueous layer was separated. The aqueous layer was concentrated under high vacuum at 35 °C. The concentrate was applied to Diaion HP-21 (100 mL, Mitsubishi Kasei Co. Ltd.) resin column chromatography. After adsorbing, the column was eluted with water and then with 5-10% acetonitrile-water. The combined fractions was concentrated under high vacuum at 35 °C and lyophilized to give the title compound as a yellow amorphous solid (322 mg, 63%). Mp 281 °C (dec); ¹H NMR (δ, D₂O) 6.32 (s, 1H), 6.76 (s, 1H), 6.79 (s, 1H), 7.22 (s, 1H), 7.24-7.33 (m, 3H), 7.60-7.63 (m, 2H).

### Example 7

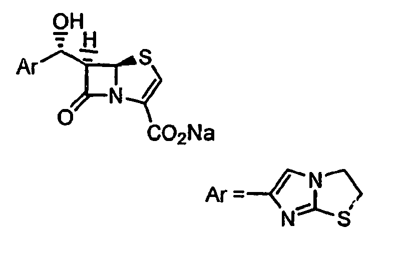

### Step 1: (5R, 6S)-6-Bromo-6-[(S)-(2,3-dihydro-imidazo[2,1-b]thiazol-6-yl)-hydroxy-methyl]-7-oxo-4-thia-1-aza-bicyclo[3.2.0]hept-2-ene-2-carboxylic acid 4-nitrobenzyl ester

2,3-Dihydro-imidazo[2,1-b]thiazole-6-carbaldehyde (2.63 g) was added to the dry acetonitrile (124 mL) solution of anhydrous MgBr₂ (3.8 g) under a nitrogen atmosphere at room temperature. Colorless powder was deposited over 30 min. The dry THF solution (124 mL) of (5R, 6S)-6-bromo-7-oxo-4-thia-1-aza-bicyclo[3.2.0]hept-2-ene-2-carboxylic acid 4-nitro-benzyl este (6.2 g) was added to the mixture. After cooling the mixture to -20 °C, triethylamine (5.21 mL) was added in one portion. The reaction vessel was covered with foil to exclude light. The reaction mixture was stirred for 2.5 h at -20 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then eluted with ethyl acetate - hexane (3/1 ~ ethyl acetate only). The mixture of the title compound 3 and its diastereo isomer was obtained (10/1, a pale brown amorphous solid, 6.63 g, 79%) and the title compound (3.20 g, 38%) was separated from the mixture by silica gel column chromatography. ¹H NMR (δ, CDCl₃) 3.80-3.89 (m, 2H), 4.15-4.25 (m, 2H), 5.22 (s, 1H), 5.31 (d, 1H, *J* = 13.5 Hz), 5.45 (d, 1H, *J* = 13.5 Hz), 6.12 (s, 1H), 7.12 (s, 1H), 7.42 (s, 1H), 7.62 (d, 2H, *J* = 8.6 Hz), 8.22-8.25 (m, 2H).

### Step 2: (5R, 6S)-6-[(S)-(2,3-Dihydro-imidazo[2,1-b]thiazol-6-yl)-hydroxy-methyl]-7-oxo-4-thia-1-aza-bicyclo[3.2.0]hept-2-ene-2-carboxylic acid

(5*R*, 6*S*)-6-Bromo-6-[(*S*)-(2,3-dihydro-imidazo[2,1-b]thiazol-6-yl)-hydroxymethyl]-7-oxo-4-thia-1-aza-bicyclo[3.2.0]hept-2-ene-2-carboxylic acid 4-nitro-benzyl ester (2.0 g) was dissolved in THF (28 mL) and acetonitrile (13 mL). Freshly activated Zn dust (8.0 g) and 0.5 M phosphate buffer (pH 6.4, 41 mL) were added to the mixture. The reaction vessel was covered with foil to exclude light. The reaction mixture was vigorously stirred for 2 h at room temperature. The mixture was cooled to 3 °C, and 1 M NaOH aqueous solution was added to adjust pH to 7.5. The reaction solution was mixed with ethyl acetate and filtered through a pad of Celite. The pad was washed with water and the aqueous layer was separated. The aqueous layer was concentrated under high vacuum at 35 °C. The concentrate was applied to Diaion HP-21 (200 mL, Mitsubishi Kasei Co. Ltd.) resin column chromatography. After adsorbing, the column was eluted with water and then with 2.5% acetonitrile-water. The combined fractions were concentrated under high vacuum at 35 °C and lyophilized to give the title compound as a pale yellow amorphous solid (171 mg, 13%). Mp 152 °C; ¹H NMR (δ, D₂O) 3.91 (t, 2H, *J* = 7.4 Hz), 4.23 (t, 2H, *J* = 7.4 Hz), 4.33-4.35 (m, 1H), 5.07 (d, 1H, *J* = 5.5 Hz), 5.70 (d, 1H, *J* = 1.6 Hz), 7.05 (d, 1H, *J* = 1.0 Hz), 7.24 (s, 1H).

### Example 8

### Preparation of (5R,6Z)-6-[(5-benzyl-4,5,6,7-tetrahydrothieno[3,2-c]pyridin-2-yl)methylene]-7oxo-4-thia-1-azabicyclo[3.2.0]hept-2-ene-2-carboxylic acid

### Step 1: Ethyl-5-benzoyl-4,5,6,7-tetrahydrothieno[3,2-c]pyridine-2-carboxylate:

To a stirred dry DMF (7.3 g, 100 mmol), POCl₃ (12.25 g, 80 mmol) was slowly added between 0°C to 5° C. After the addition the solidified mass was dissolved in CH₂Cl₂ (20 ml) and stirred at room temperature for 2 hrs. Again the temperature was cooled to 0°C and 1-benzoyl-4-piperidone in CH₂Cl₂ was added slowly. After the addition the reaction mixture was stirred at room temperature for 2 hrs and poured over crushed ice and sodium acetate. It was stirred for 30 minutes at room temperature. Extracted with CH₂Cl₂; washed well with water; dired over anhydrous MgSO₄ and concentrated. The crude product was dissolved in CH₂Cl₂ and ethylmercaptoacetae ( 9.6 g, 80 mmol) / Et₃N (10.1 g, 100 mmol) was added slowly at room temperature. The reaction mixture was refluxed for 2 hrs and quenched with water. CH₂Cl₂ layer was washed well with water; dried over anhydrous MgSO₄; filtered and concentrated. The product was purified by SiO₂ column chromatography by elting it with 50% ethylacetae; hexane. Yellow oil; Yield: 6.4 gms (25%); M+H 316.

### Step: 2 (5-benzyl-4,5,6,7-tetrahydrothieno[3,2-c]pyridin-2-yl)methanol:

To stirred suspension of LAH (2.0 gms) a solution of ethyl 5-benzoyl-4,5,6,7-tetrahydrothieno[3,2-c]pyridine-2-carboxylate (6.0 g, 19 mmol) in THF was added slowly at 0° C. After addition reaction mixture was stirred for 30 minutes and quenched with saturated NH₄Cl. It was diluted with CHCl₃ and filtered. The fitrate was washed with saturated brine solution and dried over anhydrous MgSO₄. It was filtered and taken to next step with out purifications. Yield: 4.5 g 91%. Yellow liquid.

### Step 3: 2-Formyl (5-benzyl-4,5,6,7-tetrahydrothieno[3,2-c]pyridine:

To a stirred solution of (5-benzyl-4,5,6,7-tetrahydrothieno[3,2-c]pyridin-2-yl)methanol (4.0 g, 15.4 mmol) in CH₂Cl₂ (300 ml) active MnO₂ (20 g, excess, was added and stirred at room temperature for 18 hrs. At the end, the reaction mixture was filtered through celite and washed with CHCl₃. Reaction mixture was washed well with water; dried and concentrated. The product was found to be pure and taken to next step with oiut purifications. Yield: 3.0 g (76%); (M+H: 257).

### Step 4: 4-Nitrobenzy-6-[(acetyloxy)(5-benzyl-4,5,6,7-tetrahydrothieno[3,2-c]pyridin-2-yl)methyl]-6-bromo-7-oxo-4-thia-1-azabicyclo[3.2.0]hept-2-ene-2-carboxylate:

2-Formyl (5-benzyl-4,5,6,7-tetrahydrothieno[3,2-c]pyridine (565 mg, 2.2 mmol) and the dry THF solution (20 mL) of (5R, 6S)-6-bromo-7-oxo-4-thia-1-aza-bicyclo[3.2.0]hept-2-ene-2-carboxylic acid 4-nitro-benzyl ester (772 mg, 2.0 mmol) were added successively to the dry acetonitrile (15 mL) solution of anhydrous MgBr₂: O(Et)₂ (390 mg, 1.5 mmol)under an argon atmosphere at room temperature. After cooling to -20 °C, Et₃N (2.0 mL) was added in one portion. The reaction vessel was covered with foil to exclude light. The reaction mixture was stirred for 2 h at -20 °C and treated with acetic anhydride (1.04 mL) in one portion. The reaction mixture was warmed to 0 °C and stirred for 15 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then the column was eluted with ethyl acetate: hexane (1:1). Collected fractions were concentrated under reduced pressure and the mixture of diastereo isomers were taken to next step. Pale yellow amorphous solid; Yield: 550 mg, 40%; M+H 687.

### Step-5: (5R,6Z)-6-[(5-benzyl-4,5,6,7-tetrahydrothieno[3,2-c]pyridin-2-yl)methylene]-7-oxo-4-thia-1-azabicyclo[3.2.0]hept-2-ene-2-carboxylic acid:

4-Nitrobenzy-6-[(acetyloxy)(5-benzyl-4,5,6,7-tetrahydrothieno[3,2-c]pyridin-2-yl)methyl]-6-bromo-7-oxo-4-thia-1-azabicyclo[3.2.0]hept-2-ene-2-carboxylate( 450 mg, 0.65 mmol) was dissolved in THF (20 mL) and acetonitrile (10 mL). Freshly activated Zn dust (5.2 g) was added rapidly with 0.5 M phosphate buffer (pH 6.5, 28 mL). The reaction vessel was covered with foil to exclude light. The reaction mixture was vigorously stirred for 2 h at room temperature. The reaction mixture was filtered, cooled to 3 °C, and 0.1 M NaOH was added to adjust pH to 8.5. The filtrate was washed with ethyl acetate and the aqueous layer was separated. The aqueous layer was concentrated under high vacuum at 35 °C to give yellow precipitate. The product was purified by HP21 resin reverse phase column chromatography. Initially the column was eluted with deionized water (2 lits) and latter with 10% CAN: Water. The fractions containing the product were collected and concentrated at reduced pressure at room temperature. The yellow solid was washed with acetone and filtered. Dried. Yield: 50 mg, 18%; as yellow crystals; mp. 198°C; (M+H) 411.
¹H NMR (DMSO-d₆) d 2.7 (m, 2H), 2.8 (bm, 2H), 3.4 (m, 2H), 3.8 (s, 2H), 6.3 (s, 1H), 6.5 (s, 1H), 7.1 (s, 1H), 7.28(s, 1H), 7.4 (s, 5H).

### Example 9

### Preparation of (5R),(6Z)-6-(7-Methyl-5,6,7,8-tetrahydroimidazo[1,2-a]pyrazin-2-ylmethylene)-7-oxo-4-thia-1-aza-bicyclo[3.2.0]hept-2-ene-2-carboxylic acid, sodium salt

### Step 1: Imidazo[1,2-a]pyrazine-2-carboxylic acid ethyl ester:

Ethyl bromopyruvate (62.9 g) was added to the DME (258 mL) solution of 2-aminopyrazine (24.8 g) at room temperature and stirred for 2.5 h. The reaction mixture was cooled to 0 °C and stirred for 30 min to afford a pale brown precipitate. The precipitate was filtered and washed with Et₂O to give pale brown crystals. The suspension of the precipitate (66.1 g) in EtOH (1.29 L) was heated at reflux temperature to tum to clear solution. After refluxing for 2h, the reaction mixture was concentrated under reduced pressure, then mixed with CHCl₃ and saturated NaHCO₃aq. The mixture was filtered through a pad of Celite and the separated organic layer was dried (MgSO₄) and filtered. The filtrate was concentrated under reduced pressure. The residue was applied to silica gel column chromatography, then the column was eluted with CHCl₃ - MeOH (99/1 ~ 97/3), and collected fractions were concentrated under reduced pressure followed by recrystallization from CHCl₃ - Et₂O. The titled compound was obtained as pale pink crystals. Yield: 10.9 g, 22%).

¹H NMR(CDCl₃) d 1.46(t, 3H, *J* = 7.2 Hz), 4.49(q, 2H, *J* = 7.2 Hz), 7.96(d, 1H, *J* = 4.7 Hz), 8.08(dd, 1H, *J* = 1.2, 4.7 Hz), 8.26(s, 1H), 9.21 (d, 1H, *J* = 1.2 Hz).

### Step 2: 5,6,7,8-Tetrahydroimidazo[1,2-a]pyrazine-2-carboxylic acid ethyl ester, Hydrochloride:

0.46 *M* HCl EtOH (169 mL) and 10% Pd-C (50% wet) (1.37 g) were added to the EtOH (546 mL) solution of imidazo[1,2-a]pyrazine-2-carboxylic acid ethyl ester (13.7 g). The mixture was hydrogenated under H₂ at 40 psi at room temperature for 15 h. The reaction mixture was filtered and Pd-C was washed with EtOH. The filtrate was concentrated under reduced pressure. The residue was applied to silica gel column chromatography, then the column was eluted with CHCl₃ - MeOH (9/1 ~ 2/1). The titled compound was obtained as brown crystals Yield: 10.4 g, 63%.

¹H NMR(CDCl₃) d 1.38(t, 3H, *J* = 7.1 Hz), 3.90(t, 2H, *J* = 5.7 Hz), 4.40(q, 2H, *J* = 7.1 Hz), 4.59(t, 2H, *J* = 5.7 Hz), 4.80(s, 2H), 8.20(s, 1H).

### Step 3: 7-Methyl-5,6,7,8-tetrahydroimidazo[1,2-a]pyrazine-2-carboxylic acid ethyl ester:

Et₃N (3.44 mL), 37% HCHO aq. (2.02 mL) and NaBH₃CN (1.78 g) were added successively to the MeOH (75 mL) solution of 5,6,7,8-tetrahydroimidazo[1,2-a]pyrazine-2-carboxylic acid ethyl ester, hydrochloride (5.2 g) at room temperature and stirred for 3.5 h under a nitrogen atmosphere. The mixture was diluted with CH₂Cl₂ and washed with 50% K₂CO₃ aq. The organic layer was dried (K₂CO₃) and filtered. The filtrate was concentrated under reduced pressure. The residue was applied to silica gel column chromatography, then the column was eluted with CHCl₃ - acetone (1/1 ~ 1/2). The titled compound was obtained as orange oil. Yield: 2.68 g, 57%).

¹H NMR(CDCl₃) d 1.37(t, 3H, *J* = 7.1 Hz), 2.50(s, 3H), 2.85(t, 2H, *J* = 5.5 Hz), 3.69(s, 2H), 4.06(t, 2H, *J* = 5.5 Hz), 4.36(t, 2H, *J* = 7.1 Hz), 7.52(s, 1H).

### Step 4: 7-Methyl-5,6,7,8-tetrahydroimidazo[1,2-a]pyrazine-2-carbaldehyde:

1.01 M solution of DIBAL in toluene (13.6 mL) was added to the dry CH₂Cl₂ (86 mL) solution of 7-methyl-5,6,7,8-tetrahydroimidazo[1,2-a]pyrazine-2-carboxylic acid ethyl ester (1.8 g) under a nitrogen atmosphere at -78 °C and stirred for 2 h. The mixture was quenched with 1*M* HCl. The reaction mixture was filtered through a pad of Celite. The filtrate was washed with 50% K₂CO₃ aq. and the aqueous layer was extracted with CH₂Cl₂ three times. The combined organic layer was dried (K₂CO₃) and filtered. The filtrate was concentrated under reduced pressure. The residue was applied to silica gel column chromatography, then the column was eluted with CHCl₃ - MeOH (19/1 ~ 9/1). The titled compound 5 was obtained as colorless crystals. Yield: 591 mg, 42%).

¹H NMR(CDCl₃) d 2.51 (s, 3H), 2.87(t, 2H, *J* = 5.5 Hz), 3.70(s, 2H), 4.10(t, 2H, *J* = 5.5 Hz), 7.53(s, 1H), 9.82(d, 1H, *J* = 1.4 Hz).

### Step 5: (5R, 6RS)-6-[(RS)-Acetoxy(7-methyl-5,6,7,8-tetrahydroimidazo[1,2-a]pyrazin-2-yl)methyl]-6-bromo-7-oxo-4-thia-1-azabicyclo[3.2.0]hept-2-ene-2-carboxylic acid 4-nitrobenzyl ester (diastereo mixture):

7-Methyl-5,6,7,8-tetrahydroimidazo[1,2-*a*]pyrazine-2-carbaldehyde (1.19 g) was added to the dry acetonitrile (97 mL) solution of anhydrous MgBr₂ (4.05 g) under a nitrogen atmosphere at room temperature. The dry THF solution (97 mL) of (5R, 6S)-6-bromo-7-oxo-4-thia-1-aza-bicyclo[3.2.0]hept-2-ene-2-carboxylic acid 4-nitro-benzyl ester (3.32 g) was added to the mixture, cooled to -20 °C, and Et₃N (3.0 mL) was added in one portion. The reaction vessel was covered with foil to exclude light. The reaction mixture was stirred for 4.5 h at -20 °C and treated with acetic anhydride (1.36 mL) in one portion. The reaction mixture was warmed to 0 °C and stirred for 17 h at 0 °C. The mixture was diluted with ethyl acetate and washed with 5% citric acid aqueous solution, saturated sodium hydrogen carbonate, and brine. The organic layer was dried (MgSO₄) and filtered. The filtrate was concentrated under reduced pressure. The residue was applied to silica gel column chromatography, then the column was eluted with CHCl₃ - acetone (9/1 ∼ 2/1) The titled compound was obtained as two diastereo mixture. Red oil, Yield: 1.13 g.

¹H NMR(CDCl₃) d 1.20(s, 0.81x3H), 2.24(s, 0.19x3H), 2.48(s, 3H), 2.80 ∼ 2.84(m, 2H), 3.57 ∼ 3.67(m, 2H), 3.97 ∼ 4.02(m, 2H), 5.27(d, 1H, *J* = 13.6 Hz), 5.42(d, 0.19x1H, *J* = 13.6 Hz), 5.45(d, 0.81x1H, *J* = 13.6 Hz), 6.07(s, 0.19x1H), 6.30(s. 0.81x2H), 6.79(s, 0.19x1H), 6.80(s, 0.19x1H), 7.02(s, 0.81x1H), 7.44(s, 0.19x1H), 7.47(s, 0.81x1H), 7.60(d, 0.19x2H, *J* = 8.2 Hz), 7.62(d, 0.81x2H, *J* = 8.6 Hz), 8.22 ∼ 8.26(m, 2H).

### Step 6: (5R),(6Z)-6-(7-Methyl-5,6,7,8-tetrahydroimidazo[1,2-a]pyrazin-2-ylmethylene)-7-oxo-4-thia-1-aza-bicyclo[3.2.0]hept-2-ene-2-carboxylic acid, sodium salt.

(5*R*, 6*RS*)-6-[(*RS*)-Acetoxy(7-methyl-5,6,7,8-tetrahydroimidazo[1,2-a]pyrazin-2-yl)methyl]-6-bromo-7-oxo-4-thia-1-azabicyclo[3.2.0]hept-2-ene-2-carboxylic acid 4-nitrobenzyl ester (1.11 g) was dissolved in THF (32 mL) and acetonitrile (32 mL). Freshly activated Zn dust (4.46 g) was added rapidly with 0.5 M phosphate buffer (pH 6.5, 48 mL). The reaction vessel was covered with foil to exclude light. The reaction mixture was vigorously stirred for 2 h at room temperature. The reaction mixture was filtered through a pad of Celite, cooled to 3 °C, and 1 M NaOH was added to adjust pH to 7.5. The filtrate was washed with ethyl acetate and the aqueous layer was separated. The aqueous layer was concentrated under high vacuum at 35 °C. The concentrate was applied to Diaion HP-21 (20 mL, Mitsubishi Kasei Co. Ltd.) resin column chromatography. After adsorbing, the column was eluted with H₂O - MeCN(1/0 ∼ 95/5). The combined fractions were concentrated under high vacuum at 35°C and lyophilized to give the title compound as a yellow amorphous solid. Yield: 417 mg, 65%: mp 200 °C (dec); ¹H NMR(D₂O) d 2.32(s, 3H), 2.79 ∼ 2.81(m, 2H), 3.54(s, 2H), 3.95(t, 2H, *J* = 5.6 Hz), 6.39(s, 1H), 6.85(s, 1H), 6.87(s, 1H), 7.26(s, 1H).

### Example 10

### Preparation of (5R), (6Z)-7-Oxo-6-(5,6,7,8-tetrahydroimidazo[1,2-a]pyrazin-2-ylmethylene)-4-thia-1-azabicyclo[3.2.0]hept-2-ene-2-carboxylic acid, sodium salt

### 2-Ketopiperazine

2-Ketopiperazine was prepared in the method of Merck group (USP 5,629,322).

### Step 1: 4-p-Nitrobenzyloxycarbonyl-2-ketopiperazine

The 48.7% solution of p-nitrobenzyloxycarbonyl chloride in 1,4-Dioxane (10.7 mL) was added to the dichloromethane (110 mL) solution of 2-Ketopiperazine (2.21 g) and diisopropylethylamine (4.6 mL) at 0°C and stirred for 0.5 h at 0 °C. Water (300 mL) was added to the reaction mixture, and extracted with dichloromethane (3 x 100 mL). The organic layer was dried (MgSO₄) and filtered. The filtrate was concentrated under reduce pressure. The residue was applied to silica gel column chromatography, eluted with CHCl₃ - methanol (30 : 1), and the title compound was obtained as white solid (7.1 g, quant.).
¹H NMR (d, CDCl₃) 3.42-3.45 (m, 2H), 3.74 (t, 2H, *J* = 5.4 Hz), 4.19 (s, 2H), 5.26 (s, 2H), 6.39 (brs, 1H), 7.52 (d, 2H, *J* = 8.6 Hz), 8.24 (d, 2H, *J* = 8.6 Hz).

### Step 2: 5-Methoxy-4-p-nitrobenzyloxycarbonyl-1,2,3,6-tetrahydropyrazine:

Trimethyloxonium tetrafluoroborate (97%, 3.7 g) was added to the dry dichloromethane (120 mL) solution of 4*-p*-nitrobenzyloxycarbonyl-2-ketopiperazine (6.7 g) at room temperature and stirred for 17 h. The reaction mixture was treated with saturated sodium hydrogen carbonate aqueous solution, and the organic layer was separated. The aqueous layer was extracted with ethyl acetate (3 x 100 mL), then the combined organic layer was washed with saturated sodium hydrogen carbonate aqueous solution and brine. The organic layer was dried (MgSO₄) and filtered. The filtrate was concentrated under reduce pressure and the title compound was obtained as a pale brown solid. Yield; 5.7 g, 80.6.
¹H NMR (d, CDCl₃) 3.48 (m, 2H), 3.57 (m, 2H), 3.70 (s, 3H), 3.97 (s, 2H), 5.26 (s, 2H), 7.52 (d, 2H, *J* = 8.7 Hz), 8.23 (d, 2H, *J* = 8.7 Hz).

### Step 3: 2-Imino-4-p-nitrobenzyloxycarbonyl piperazine:

The mixture of 5-methoxy-4*-p*-nitrobenzyloxycarbonyl-1,2,3,6-tetranydropyrazine (5.7 g) and ammonium chloride (1.6 g) in dry ethanol (100 mL) was heated to reflux for 4 h. The reaction mixture was then concentrated under reduce pressure. Dichloromethane (100 mL) was added to the residue and extracted with water (3 x 50 mL) then the combined aqueous layer was washed with dichloromethane. The aqueous layer was neutralized with 10% potassium carbonate aqueous solution and then extracted with dichloromethane (8 x 50 mL). The combined organic layer was dried (MgSO₄) and filtered. The filtrate was concentrated under reduce pressure and the title compound was obtained as a white solid. Yield: 4.9 g, 91.2%.
¹H NMR (d, CDCl₃) 3.49 (brs, 4H), 3.98 (brs, 2H), 5.26 (s, 2H), 7,52 (d, 2H, J = 8.6 Hz), 8.23 (d, 2H, *J* = 8.6 Hz).

### Step 4: 7-p-Nitrobenzyloxycarbonyl-5,6,7,8-tetrahydroimidazo[1,2-a]pyrazine-2-carbaldehyde (9) and 7-p-nitrobenzyloxycarbonyl-5,6,7,8 tetrahydroimidazo[1,2-a]pyrazine-3-carbaldehyde:

The mixture of 2-bromo-3-hydroxypropenal (2.8 g), p-toluenesulfonic acid monohydrate (33 mg) and 2-propanol (3.5 mL) in cyclohexane (28 mL) was azeotroped until the vaper temperature rose to 80°C. The reaction mixture was concentrated under reduce pressure. The residue was dissolved in dry acetonitrile (30 mL). The dry acetonitrile (310 mL) solution of 2-imino-4*-p*-nitrobenzyloxycarbonyl piperazine (4.7 g) was added at room temperature. The reaction mixture was stirred at room temperature for 3 h, and then the reaction solution was removed in vacuo. The residue was dissolved in ethyl acetate (170 mL) and triethylamin (2.4 mL) was added, then the reaction mixture was heated to reflux for 1.5 h. The reaction mixture was cooled to room temperature, and then water (170 mL) was added to the reaction mixture and separated. The aqueous layer was extracted with dichloromethane (2 x 100 mL). The combined organic layer was dried (MgSO₄) and filtered. The filtrate was concentrated under reduce pressure. The residue was applied to silica gel column chromatography, eluted with CHCl₃ - methanol (50 : 1), and the title compound was obtained as a brown solid, (Yield: 2.9 g, 51.6%) and its regio isomer (orange amorphous solid, Yield; 0.8 g, 14.9%) were obtained.
¹H NMR (d, CDCl₃) 3.99 (t, 2H, *J* = 5.4 Hz), 4.14 (t, 2H, *J* = 5.4 Hz), 4.85 (s, 2H), 5.29 (s, 2H), 7.54 (d, 2H, *J* = 8.6 Hz), 7.57 (s, 1H), 8.24 (d, 2H, *J* = 8.6 Hz), 9.85 (s, 1H).
Regio isomer ¹H NMR (d, CDCl₃) 3.95 (t, 2H, *J* = 5.4 Hz), 4.44 (t, 2H, *J* = 5.4 Hz), 4.87 (s, 2H), 5.29 (s 2H), 7.54 (d, 2H, *J* = 8.7 Hz), 7.78 (s, 1H), 8.24 (d, 2H, *J* = 8.7 Hz), 9.71 (s, 1H).

### Step 5: (5R)-6-[Acetoxy-(7-p-nitrobenzyloxycarbonyl-5,6,7,8-tetrahydroimidazo[1,2-a]pyrazin-2-yl)-methyl]-6-bromo-7-oxo-4-thia-1azabicyclo[3.2.0]hept-2-ene-2-carboxylic acid p-nitrobenzyl ester:

The dry acetonitrile (25 mL) solution of 7*-p*-nitrobenzyloxycarbonyl-5,6,7,8-tetrahydroimidazo[1,2-a]pyrazine-2-carbaldehyde (1.6 g) was added to the dry acetonitrile (55 mL) solution of MgBr₂ (2.2 g) under an nitrogen atmosphere at room temperature then the mixture was stirred for 10 min. The dry THF (80 mL) solution of (5R, 6S)-6-bromo-7-oxo-4-thia-1-aza-bicyclo[3.2.0]hept-2-ene-2-carboxylic acid 4-nitro-benzyl ester (1.8 g) was added, the mixture was cooled to -20 °C then triethylamine (1.6 mL) was added in one portion. The reaction vessel was covered with foil to exclude light. The reaction mixture was stirred for 3 h at -20 °C and treated with 4,4-dimethylamino pyridine (58.3 mg) and acetic anhydride (0.89 mL) in one portion. The reaction mixture was warmed to 0 °C and stirred for 15 h at 0 °C. 10% Citric acid aqueous solution (320 mL) was added to the reaction mixture and the aqueous layer was extracted with ethyl acetate (3 x 160 mL). The organic layer was washed with water, saturated sodium hydrogen carbonate and brine, dried (MgSO₄) and filtered. The filtrate was concentrated under reduced pressure. The residue was applied to silica gel column chromatography, eluted with CH₂Cl₂ - acetone (20 : 1), and the title compound was obtained as two diastereo mixture (81 : 19, brown foamy amorphous solid. Yield: 2.1 g, 59.9%.
¹H NMR (d, CDCl₃) 2.01 (s, 2.43H), 2.24 (s, 0.57H), 3.93-,3.96 (m, 2H), 4.02-4.05 (m, 2H), 4.74-4.76 (m, 2H), 5.28 (d, 1H, *J* = 13.5 Hz), 5.28 (s, 2H), 5.45 (d, 1H, *J* = 13.5 Hz), 6.07 (s, 0.19H), 6.29 (s, 0.81H), 6.31 (s, 0.81H), 6.80 (s, 0.19H), 6.83 (s, 0.19H), 7.08 (s, 0.81 H), 7.43 (s, 0.19H), 7.46 (s, 0.81 H), 7.54 (d, 2H, *J* = 8.6 Hz), 7.61 (d, 2H, *J* = 8.8 Hz), 8.24 (d, 4H, *J* = 8.3 Hz).

### Step 6: (5R), (6Z)-7-Oxo-6-(5,6,7,8-tetrahydroimidazo[1,2-a]pyrazin-2-ylmethylene)-4-thia-1-azabicyclo[3.2.0]hept-2-ene-2-carboxylic acid, sodium salt:

(5R)-6-[Acetoxy-(7-p-nitrobenzyloxycarbonyl-5,6,7,8-tetrahydroimidazo[1,2-a]pyrazin-2-yl)-methyl]-6-bromo-7-oxo-4-thia-1azabicyclo[3.2.0]hept-2-ene-2-carboxylic acid p-nitrobenzyl ester (2.0 g) was dissolved in THF (63 mL). Freshly activated Zn dust (7.9 g) was added rapidly with 0.5 mol/L phosphate buffer (pH 6.5, 63 mL). The reaction vessel was covered with foil to exclude light. The reaction mixture was vigorously stirred for 2 h at room temperature. The reaction solution was filterd through a pad of Celite and the pad was washed with water (150 mL) and n-butanol (150 mL). The aqueous layer was separated and then the organic layer was extracted with water (2 x 50 mL). The combined aqueous layer was concentrated to 61 g and applied to Diaion HP-21 resin (80 mL, Mitsubishi Kasei Co. Ltd.) column chromatography. After adsorbing, the column was eluted with water and then 5% acetonitrile aqueous solution. The combined fractions were concentrated under high vacuum at 35°C and lyophilized to give the title compound as a yellow amorphous solid. Yield: 172 mg, 20.1%: mp 150 °C (dec); ¹H NMR (d, D₂O) 3.02 (t, 2H, *J* = 5.6 Hz), 3.82 (s, 2H), 3.89 (d, 2H, *J* = 5.6 Hz), 6.38 (s, 1H), 6.84 (s, 1H), 6.87 (s, 1H), 7.24 (s, 1H); IR (KBr)

### Example 11

### Preparation of (5R,6Z)-6-{[5-(4-methoxybenzyl)-4,5,6,7-tetrahydrothieno[3,2-c]pyridin-2-yl]methylene}-7oxo-4-thia-1-azabicyclo[3.2.0]hept-2-ene-2-carboxylic acid

### Step 1: 5-tert-butyl 2-ethyl 6,7-dihydrothieno[3,2-c]pyridine-2,5(4H)-dicarboxylate:

5-tert-butyl 2-ethyl 6,7-dihydrothieno[3,2-c]pyridine-2,5(4H)-dicarboxylate was prepared according to the procedure as outlined in Example 5, (Step 1). Starting from tert-butyl-1-piperidinecarboxylate (9.9 g, 50 mmol), POCl₃ (6.3 g, 40 mmol) and DMF (3.8 g, 50 mmol). The chloro formyl intermediate was reacted with ethyl mercaptoacetate (6.0 g, 50 mmol) and Et₃N. The product was purified by SiO₂ column chromatography by eluting it with 3:1 hexane; ethylacetae. Yeld: 8.7 g, 56%; White liquid. (M+H) 312.

### Step 2: tert-butyl 2-(hydroxymethyl)-6,7-dihydrothieno[3,2-c]pyridine-5(4H)-carboxylate:

tert-butyl 2-(hydroxymethyl)-6,7-dihydrothieno[3,2-c]pyridine-5(4H)-carboxylate was prepared according to the procedure outlined in Example 5, (Step 2). Starting from 5-tert-butyl 2-ethyl 6,7-dihydrothieno[3,2-c]pyridine-2,5(4H)-dicarboxylate (1.0 g, 3.21 mmol) and LiAIH₄ (500 mg , excess), 807 mg (92% yield) of the alcohol derivative was isolated as white liquid. (M+H) 270.

### Step 3: tert-butyl 2-(formyl)-6,7-dihydrothieno[3,2-c]pyridine-5(4H)-carboxylate:

tert-butyl 2-(formyl)-6,7-dihydrothieno[3,2-c]pyridine-5(4H)-carboxylate was prepared according to the procedure outlined in Example 5, (Step 3). Starting from tert-butyl 2-(hydroxymethyl)-6,7-dihydrothieno[3,2-c]pyridine-5(4H)-carboxylate (1.0 g 3.7 mmol) in methylene chloride (100 ml) and active MnO₂ (5 g, excess), 800 g (81% Yield) of the aldehyde derivative was isolated as brown solid. (M+H) 268.

### Step 4: 2-(formyl)-6,7-dihydrothieno[3,2-c]-5(4H)-pyridine:

2-(formyl)-6,7-dihydrothieno[3,2-c]-5(4H)-pyridine was prepared starting from tert-butyl 2-(formyl)-6,7-dihydrothieno[3,2-c]pyridine-5(4H)-carboxylate (1.0 g 3.7 mmol) was dissolved in CH₂Cl₂ (20 ml), MeOH (90% 20 ml) and 1N. HCl in dioxane (10 ml). The reaction mixture was stirred at room temperature for 48 hrs. At the end reaction mixture was concentrated to dryness and taken to next step with out purification. Yield: 750 mg (HCl salt, Quantitative); M+H 168.

### Step 5: 2-Formyl [5-(4-methoxybenzyl)-4,5,6,7-tetrahydrothieno][3,2-c]pyridine:

To a stirred solution of 2-(formyl)-6,7-dihydrothieno[3,2-c]-5(4H)-pyridine (1.4 g, 5.2 mmol) in DMF ( 20 ml) , 4-methoxybenzyl chloride (0.94 g, 6.2 mmol) and N,N-diisopropylethylamine (10 ml, excess) was added at room temperature. The reaction mixture was stirred for 24 hrs and quenched with water. The reaction mixture was extracted with chloroform; washed well with water and dried over anhydrous MgSO₄. It was filtered and concentrated. The product was purified by SiO₂ column chromatography by eluting it with ethylacetate. Pale yellow oil. Yield: 470 mg , 35%; M+H 288.

### Step 6: .4-Nitrobenzy-6-[(acetyloxy)[5(4-methoxybenzyl)-4,5,6,7-tetrahydrothieno[3,2-c]pyridin-2-yl)methyl]-6-bromo-7-oxo-4-thia-1-azabicyclo[3.2.0]hept-2-ene-2-carboxylate:

2-Formyl [5-(4-methoxybenzyl)-4,5,6,7-tetrahydrothieno][3,2-c]pyridine (574 mg, 2.0 mmol) and the dry THF solution (20 mL) of (5R, 6S)-6-bromo-7-oxo-4-thia-1-aza-bicyclo[3.2.0]hept-2-ene-2-carboxylic acid 4-nitro-benzyl ester (772 mg, 2.0 mmol) were added successively to the dry acetonitrile (15 mL) solution of anhydrous MgBr₂: O(Et)₂ (390 mg, 1.5 mmol)under an argon atmosphere at room temperature. After cooling to - 20 °C, Et₃N (2.0 mL) was added in one portion. The reaction vessel was covered with foil to exclude light. The reaction mixture was stirred for 2 h at -20 °C and treated with acetic anhydride (1.04 mL) in one portion. The reaction mixture was warmed to 0 °C and stirred for 15 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then the column was eluted with ethyl acetate: hexane (1:1). Collected fractions were concentrated under reduced pressure and the mixture of diastereo isomers were taken to next step. Pale yellow amorphous solid; Yield: 550 mg, 40%; M+H 714 and 716.

### Step-7: (5R,6Z)-6-{[5-(4-methoxybenzyl)-4,5,6,7-tetrahydrothieno[3,2-c]pyridin-2-yl)]methylene}-7oxo-4-thia-1-azabicyclo[3.2.0]hept-2-ene-2-carboxylic acid:

4-Nitrobenzy-6-[(acetyloxy)[5(4-methoxybenzyl)-4,5,6,7-tetrahydrothieno[3,2-c]pyridin-2-yl)methyl]-6-bromo-7-oxo-4-thia-1-azabicyclo[3.2.0]hept-2-ene-2-carboxylate ( 300 mg, 0.42 mmol) was dissolved in THF (20 mL) and acetonitrile (10 mL). Freshly activated Zn dust (5.2 g) was added rapidly with 0.5 M phosphate buffer (pH 65, 28 mL). The reaction vessel was covered with foil to exclude light. The reaction mixture was vigorously stirred for 2 h at room temperature. The reaction mixture was filtered, cooled to 3 °C, and 0.1 M NaOH was added to adjust pH to 8.5. The filtrate was washed with ethyl acetate and the aqueous layer was separated. The aqueous layer was concentrated under high vacuum at 35 °C to give yellow precipitate. The product was purified by HP21 resin reverse phase column chromatography. Initially the column was eluted with deionized water (2 lits) and latter with 10% CAN: Water. The fractions containing the product were collected and concentrated at reduced pressure at room temperature. The yellow solid was washed with acetone and filtered. Dried. Yield: 50 mg, 18%; as yellow crystals; mp. 127°C; (M+H) 441 .
¹H NMR (DMSO-d₆) d 2.7 (m, 2H), 2.8 (bm, 2H), 3.4 (m, 2H), 3.74 (s, 3H) 3.8 (s, 2H), 6.6 (s, 1H), 6.88 (dd, 2H), 7.14(s, 1H), 7.24(dd, 2H), 7.4 (s, 1H), 7.59 (s, 1H).

### Example 12

### Preparation of (5R), (6Z)-6-(5,6-dihydro-8H-imidazo[2,1-c][1,4]thiazin-2-ylmethylene)-7-oxo-4-thia-1-azabicyclo[3.2.0]hept-2-ene-2-carboxylic acid, sodium salt

### Step 1: 5-Methylthio-3,6-dihydro-2H-[1,4]thiazine hydroiodide

5-Methylthio-3,6-dihydro-2*H*-[1,4]thiazine hydroiodide was prepared by the method as outlined in USP 5,629,322.

### Step 2: 3-Iminothiomorpholin hydrochloride

5-Methylthio-3,6-dihydro-2*H*-[1,4]thiazine hydroiodide (7.1 g) was dissolved with 10% K₂CO₃ aqueous solution (150 mL) and the aqueous layer was extracted with CH₂Cl₂ (5 x 70 mL). The combined organic layer was dried (MgSO₄), filtered and concentrated under reduce pressure. Ammonium chloride (1.7 g) was added to the obtained residue in dry ethanol (128 mL) and heated to reflux for 1 h. The reaction mixture was cooled to room temperature. The reaction solution was removed in vacuo and the Iminothiomorpholin hydrochloride was obtained as brown solid (4.3 g, quant.).

¹H NMR (d, DMSO-d⁶) 3.15 (t, 2H, *J* = 5.9 Hz), 3.74 (t, 2H, *J* = 5.9 Hz), 3.83 (s, 2H), 8.97 (brs, 1H), 9.38 (brs, 1H), 9.99 (brs, 1H).

### Step 3: 5,6-Dihydro-8H-imidazo[2,1-c][1,4]thiazine-2-carbardehyde and 5,6-Dihydro-8H-imidazo[2,1-c][1,4]thiazine-3-carbardehyde

The mixture of 2-bromo-3-hydroxypropenal (7, 4.3 g), p-toluenesulfonic acid monohydrate (52 mg) and 2-propanol (5.3 mL) in cyclohexane (43 mL) was azeotroped until the vaper temperature rose to 80°C. The reaction mixture was concentrated under reduce pressure. The residue was dissolved in dry ethanol (28 mL). The mixture of the dry ethanol (143 mL) solution of 3-iminothiomorpholin hydrochloride (4.3 g) and 28% methanol solution of sodium methylate (5.0 mL) were added at room temperature. The reaction mixture was stirred at room temperature for 1 h, and then the reaction solution was removed in vacuo. The residue was dissolved in chloroform (128 mL) and triethylamine (3.6 mL) was added, then the reaction mixture was heated to reflux for 2.5 h. The reaction mixture was cooled to room temperature and then concentrated under reduce pressure. The residue was dissolved with dichloromethane (300 mL) and washed with 50% K₂CO₃ aqueous solution (2 x 100 mL). The organic layer was dried (MgSO₄) and filtered. The filtrate was concentrated under reduce pressure. The residue was applied to silica gel column chromatography, eluted with CHCl₃ - acetone (10 : 1), and 5,6-Dihydro-8*H*-imidazo[2,1-c][1,4]thiazine-2-carbardehyde (brown solid, 445 mg, 10.3%) and 5,6-Dihydro-8*H*-imidazo[2,1-c][1,4]thiazine-3-carbardehyde (brown solid, 872 mg, 20.2%) were obtained.
5,6-Dihydro-8*H*-imidazo[2,1-c][1,4]thiazine-2-carbardehyde: ¹H NMR (d, CDCl₃) 3.07 (t, 2H, *J* = 5.7 Hz), 3.95 (s, 2H), 4.33 (t, 2H, *J* = 5.7 Hz), 7.55 (s, 1H), 9.83 (s, 1H). 5,6-Dihydro-8*H*-imidazo[2,1-c][1,4]thiazine-3-carbardehyde: ¹H NMR (d, CDCl₃) 3.05 (t, 2H, *J* = 5.7 Hz), 3.98 (s, 2H), 4.61 (t, 2H, *J* = 5.7 Hz), 7.73 (s, 1H), 9.69 (s, 1H).

### Step 4: (5R), (6Z)-6-(5,6-dihydro-8H-imidazo[2,1-c][1,4]thiazin-2-ylmethylene)-7-oxo-4-thia-1-azabicyclo[3.2.0]hept-2-ene-2-carboxylic acid, sodium salt:

The dry acetonitrile (20 mL) solution of 5,6-dihydro-8*H*-imidazo[2,1-c][1,4]thiazine-2-carbardehyde (9, 392 mg) was added to the dry acetinitrile (20 mL) solution of MgBr₂ (1.1 g) under a nitrogen atmosphere at room temperature then the mixture was stirred for 10 min. The dry THF (40 mL) solution of (5R, 6S)-6-bromo-7-oxo-4-thia-1-aza-bicyclo[3.2.0]hept-2-ene-2-carboxylic acid 4-nitro-benzyl ester (1.0 g) was added and the mixture was cooled to -20 °C then triethylamine (0.8 mL) was added in one portion. The reaction vessel was covered with foil to exclude light. The reaction mixture was stirred for 3.5 h at -20 °C and treated with 4-dimethylamino pyridine (30 mg) and acetic anhydride (0.44 mL) in one portion. The reaction mixture was warmed to 0 °C and stirred for 14 h at 0 °C. 10% Citric acid aqueous solution (240 mL) was added to the reaction mixture and the aqueous layer was extracted with ethyl acetate (3 x 100 mL). The combined organic layer was washed with water, saturated sodium hydrogen carbonate and brine, dried (MgSO₄) and filtered. The filtrate was concentrated under reduced pressure. The residue was roughly purified by silica gel column chromatography, eluted with CH₂Cl₂ - acetone (50 : 1), and crude (5*R*)-6-[acetoxy-(5,6-dihydro-8*H*-imidazo[2,1-c][1,4]thiazin-2-yl)methyl]-6-bromo-7-oxo-4-thia-1-azabicyclo[3.2.0]hept-2-ene-2-carboxylic acid p-nitrobenzyl ester was obtained as solid.

The solid obtained above was purified by SiO₂ column chromatography by eluting it with 505 ethylacetae:hexane. The pale yellow solid obtained was dissolved in THF (17 mL). Freshly activated Zn dust (2.2 g) was added rapidly with 0.5 mol/L phosphate buffer (pH 6.5, 17 mL). The reaction vessel was covered with foil to exclude light. The reaction mixture was vigorously stirred for 2 h at room temperature. The reaction solution was filterd through a pad of Celite and the pad was washed with water (40 mL) and n-butanol (30 mL). The aqueous layer was separated and then the organic layer was extracted with 0.5 mol/L phosphate buffer (pH 6.5, 2 x 10 mL). The combined aqueous layer was concentrated to 23 g, 1 mol/L NaOH was added to adjust pH to 7.25 and applied to Diaion HP-21 resin (30 mL, Mitsubishi Kasei Co. Ltd.) column chromatography. After adsorbing, the column was eluted with water and then 10% acetonitrile aqueous solution. The combined active fractions were concentrated under high vacuum at 35°C and lyophilized to give (5*R*), (62)-6-(5,6-dihydro-8*H*-imidazo[2,1-c][1,4]thiazin-2-ylmethylene)-7-oxo-4-thia-1-azabicyclo[3.2.0]hept-2-ene-2-carboxylic acid, sodium salt as a yellow amorphous solid (168 mg, 20.9%).

mp 135 °C (dec); ¹H NMR (d, D₂O) 3.00 (t, 2H, *J* = 5.7 Hz), 3.80 (AB, 2H, *J* = 16.7, 18.1 Hz), 4.19 (t, 2H, *J* = 5.7 Hz), 6.44 (d, 1H, *J* = 0.8 Hz), 6.89 (s, 1H), 6.93 (s, 1H), 7.29 (s, 1H); M+H = 322.

### Example 13

### Preparation of (5R), (6Z)-6-(6,7-Dihydro-5H-pyrrolo[1,2-a]imidazol-2-ylmethylene)-7-oxo-4-thia-1-aza-bicyclo[3.2.0]hept-2-ene-2-carboxylic acid, sodium salt

### Step 1: 6,7-Dihydro-5H-pyrrolo[1,2-a]imidazole-2-carbaldehyde

28% Sodium methoxide (5.26g) was added to the EtOH (250 mL) solution of 4,5-dihydro-3H-pyrrol-2-ylamine hydrochloride ( 3.27g) at room temperature. After stirring for 5 min at room temperature, 2-bromo-3-propoxy-propenal (5.79g) was added to the mixture at room temperature, then the reaction mixture was stirred for 1 h at room temperature. The reaction mixture was taken to dryness in vacuo. The residue was dissolved in CHCl₃ (300 mL) and triethylamine (3.8 mL) was added. The mixture was heated to reflux for 3 hours. The reaction mixture was cooled to room temperature, washed with 50% K2CO3, dried over anhydrous K2CO3, filtered, and evaporated under reduced pressure. The residue was applied with silicagel column chromatography, eluted with CHCl3-acetone (2:1), and 6,7-Dihydro-5H-pyrrolo[1,2-a]imidazole-2-carbaldehyde (41%, 1.51g) was obtained as a pale yellow solid.
¹H NMR (d, CDCl₃): 2.62-2.7 (m, 2H), 2.90-2.94 (m, 2H), 4.07 (t, 2H, *J* = 7.2 Hz), 7.59 (s, 1H), 9.80 (s, 1H).

### Step 2: (5R), (6Z)-6-(6,7-Dihydro-5H-pyrrolo[1,2-a]imidazol-2-ylmethylene)-7-oxo-4-thia-1-aza-bicycto[3.2.0]hept-2-ene-2-carboxylic acid, sodium salt

6,7-Dihydro-5H-pyrrolo[1,2-a]imidazole-2-carbaldehyde (1.36 g) was added to the dry acetonitrile (155 mL) solution of anhydrous MgBr₂ (5.64 g) under an argon atmosphere at room temperature. The dry THF solution (155 mL) of (5R, 6S)-6-bromo-7-oxo-4-thia-1-aza-bicyclo[3.2.0]hept-2-ene-2-carboxylic acid 4-nitro-benzyl ester ( 3.86 g) was added to the mixture, cooled to -20 °C, and Et₃N (4.18 mL) was added in one portion. The reaction vessel was covered with foil to exclude light. The reaction mixture was stirred for 3 h at -20 °C and treated with acetic anhydride (1.89 mL) and DMAP (370 mg) in one portion. The reaction mixture was warmed to 0 °C and stirred for 14.5 h at 0 °C. The mixture was diluted with ethyl acetate and washed with 1 M citric acid aqueous solution, saturated sodium hydrogen carbonate, and brine. The organic layer was dried (MgSO₄) and filtered. The pad was washed with ethyl acetate. The filtrate was concentrated under reduced pressure. The residue was dissolved in THF (166 mL) and acetonitrile (77 mL). Freshly activated Zn dust (23.2 g) was added rapidly with 0.5 M phosphate buffer (pH 6.5, 243 mL). The reaction vessel was covered with foil to exclude light. The reaction mixture was vigorously stirred for 2 h at room temperature. The reaction mixture was filtered, cooled to 3 °C, and 1 M NaOH was added to adjust pH to 8. The filtrate was washed with ethyl acetate and the aqueous layer was separated. 1 M NaOH was added to the aqueous layer again to adjust pH to 8. The resultant mixture was concentrated under high vacuum at 35 °C. The concentrate was applied to Diaion HP-21 (20 mL, Mitsubishi Kasei Co. Ltd.) resin column chromatography. After adsorbing, the column was eluted with H₂O - MeCN(1/0 ∼ 9/1) to give the purified active fractions of (5*R*), (6Z)-6-(6,7-Dihydro-5*H*-pyrrolo[1,2-a]imidazol-2-ylmethylene)-7-oxo-4-thia-1-aza-bicyclo[3.2.0]hept-2-ene-2-carboxylic acid, sodium salt. The combined fractions were concentrated under high vacuum at 35 °C and lyophilized to give the titled as a yellow amorphous solid (681 mg, 24%, pH 7.8).
mp 190 °C (dec); ¹H NMR(d, D₂O): 2.48-2.56 (m, 2H), 2.74-2.79 (m, 2H), 3.94-3.99 (m, 2H), 6.47 (d, 1H, *J* = 0.7 Hz), 6.94 (s, 1H), 6.95 (s, 1H), 7.36 (s, 1H); (M+H) 291.

### Example 14

### Preparation of (5R,6Z)-6-(Imidazo[2,1-b][1,3]benzothiazol-2-ylmethylene)-7-oxo-4-thia-1-azabicyclo[3.2.0]hept-2-ene-2-carboxylic acid.

### Step 1: Ethyl imidazo[2,1-b]-benzthiazole-2-carboxylate:

Ethyl bromopyruvate (9.8 g, 50 mmol) was added dropwise to a stirred solution of 2-aminobenzothiazole (7.5 g, 50 mmol) in DMF (100 ml) at room temperature. After the addition, the reaction mixture was heated to reflux for 6 h. The reaction mixture was cooled to room temperature and quenched with ice cold water. The aqueous layer was neutralized with NH₄OH and the separated solid was filtered. It was washed well with water and dried. The crude product obtained was taken to next step without purification. Brown solid; Yield: 10 g, 81%; M+H 248. mp 97°C

### Step 2: Imidazo[2,1-b]-benzthiazole-2-methanol:

To a stirred slurry of LiAlH₄ (2.0 g, excess) in dry THF, ethyl imidazo[2,1-b]-benzthiazole-2-carboxylate (4.9 g, 20 mmol) was slowly added in THF (100 ml) at 0° C. After the addition, the reaction mixture was stirred at room temperature for 1 h and quenched with saturated NH₄Cl/ NH₄OH. The separated solid was diluted with Chloroform/ MeOH (3:1). and filtered through a pad of celite. The organic layer was washed once with saturated NaCl and dried over anhydrous MgSO₄. It was filtered and concentrated. The brown solid obtained was taken to next step with out purification. Yield: 3.8 g, 93%; M+H 205; mp 131°C.

### Step 3: 2-Formyl-Imidazo[2,1-b]-benzthiazole:

To a stirred solution of imidazo[2,1-b]-benzthiazole-2-methanol (2.04 g, 10 mmol) in methylene chloride (200 ml), activated MnO₂ ( 15 g, excess) was added. The reaction mixture was stirred at room temperature for 24 h and filtered through a pad of celite. The reaction mixture was concentrated and the product was purified by silica gel column chromatography by eluting it with 75% ethyl acetate; hexane. Brown solid; Yield: 800 mg, 40%; M+H 203.

### Step 4: 4-Nitrobenzyl-6-[(acetyloxy) (imidazo[2,1-b][1,3]benzothiazol-2-yl)methyl]-6-bromo-7-oxo-4-thia-1-azabicyclo[3.2.0]hept-2-ene-2-carboxylate:

2-Formyl-Imidazo[2,1-b]-benzthiazole (444 mg, 2.2 mmol) and a dry THF solution (20 mL) of (5R, 6S)-6-bromo-7-oxo-4-thia-1-aza-bicyclo[3.2.0]hept-2-ene-2-carboxylic acid 4-nitro-benzyl ester (772 mg, 2 mmol) were added successively to a dry acetonitrile (15 mL) solution of anhydrous MgBr₂:etherate (619 mg 2.4 mmol) under an argon atmosphere at room temperature. After cooling to -20 °C, Et₃N (2.0 mL) was added in one portion. The reaction vessel was covered with foil to exclude light. The reaction mixture was stirred for 2 h at -20 °C and treated with acetic anhydride (1.04 mL) in one portion. The reaction mixture was warmed to 0 °C and stirred for 15 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to a silica gel column, then the column was eluted with ethyl acetate: hexane (1·1). Collected fractions were concentrated under reduced pressure and the mixture of diastereo isomers were taken to the next step. Pale yellow amorphous solid; Yield: 850 mg, 67%; mp 69°C; M+H 630

### Step-5: (5R),(62)-6-(Imidazo[1,2-b][1,3]benzothiazol-2-ylmethylene)-7-oxo-4-thia-1-azabicyclo [3.2.0]hept-2-ene-2-carboxylic acid:

4-Nitrobenzyl-6-[(acetyloxy) (imidazo[2,1-b][1,3]benzothiazol-2-yl)methyl]-6-bromo-7-oxo-4-thia-1-azabicyclo[3.2.0]hept-2-ene-2-carboxylate ( 500 mg, 0.79 mmol) was dissolved in THF (17 mL) and acetonitrile (36 mL). Freshly activated Zn dust (5.2 g) was added rapidly with 0.5 M phosphate buffer (pH 6.5, 28 mL). The reaction vessel was covered with foil to exclude light. The reaction mixture was vigorously stirred for 2 h at room temperature. The reaction mixture was filtered, cooled to 3 °C, and 1 N HCl was added to adjust the pH to 7.5. The filtrate was washed with ethyl acetate and the aqueous layer was separated. The aqueous layer was concentrated under high vacuum at 35 °C to give a yellow precipitate. The precipitate was dissolved in acetonitrile and loaded on a HP-21 reverse phase column. It was eluted with deionized water (2 L) and latter eluted with 10% acetonitrile:water. Yield: 105 mg, 35%; as yellow crystals; mp 233°C; M+H 356.
¹H NMR (DMSO-d₆) □ 6.51 (s, 1H), 6.53(s, 1H), 7.09(s, 1H), 7.47(t, 1H, *J* = 7.5 Hz), 7.54(t, 1H, *J* = 7.5 Hz), 8,06(t, 1H), 8.62(s, 1H).

### Example 15

### Preparation of (5R,62)-6-[(7-methoxyimidazo[2,1-b][1,3]benzothiazol-2-ylmethylene)-7-oxo-4-thia-1-azabicyclo[3.2.0]hept-2-ene-2-carboxylic acid.

### Step 1: Ethyl 7-methoxyimidazo[2,1-b]-benzthiazole-2-carboxylate:

Ethyl 7-methoxyimidazo[2,1-b]-benzthiazole-2-carboxylate was prepared according to the procedure as outlined in Example 1, (Step 1). Starting from 6-methoxy-2-amino benzothiazole (27 g, 0.15 mol) and ethyl bromopyruvate (39.9 g, 0.2 mol), 24 g (43% Yield) of ethyl 7-methoxyimidazo[2,1-b]-benzthiazole-2-carboxylate was isolated as a brown solid. (M+H) 277.

### Step 2: 7-methoxy imidazo[2,1-b]-benzthiazole-2-methanol:

7-methoxy imidazo[2,1-b]-benzthiazole-2-methanol was prepared according to the procedure outlined in Example 1, (Step 2). Starting from ethyl 7-methoxyimidazo[2,1-b]-benzthiazole-2-carboxylate (12.5 g, 43.5 mmol) and LiAIH₄ solution (43.5 ml, 0.5 M solution in THF), 4.0 g (40% yield) of the alcohol derivative was isolated as a brown solid. (M+H) 235.

### Step 3: 2-Formyl-7-methoxyimidazo[2,1-b]-benzthiazole:

2-Formyl-7-methoxyimidazo[2,1-b]-benzthiazole was prepared according to the procedure outlined in Example 1, (Step 3). Starting from 7-methoxy imidazo[2,1-b]-benzthiazole-2-methanol (4.0 g 17 mmol) in methylene chloride/ DMF(300 mL: 50 mL) and active MnO₂ (12 g, excess), 822 mg (21% Yield) of the aldehyde derivative was isolated as brown solid. (M+H) 233.

### Step 4: 4-Nitrobenzyl-6-[(acetyloxy) (7-methoxyimidazo[2,1-b][1,3]benzothiazol-2-yl)methyl]-6-bromo-7-oxo-4-thia-1-azabicyclo[3.2.0]hept-2-ene-2-carboxylate:

2-Formyl-7-methoxyimidazo[2,1-b]-benzthiazole (822 mg, 3.5 mmol) and the dry THF solution (40 mL) of (5R, 6S)-6-bromo-7-oxo-4-thia-1-aza-bicyclo[3.2.0]hept-2-ene-2-carboxylic acid 4-nitro-benzyl ester (1.364, 3.54 mmol) were added successively to the dry acetonitrile (15 mL) solution of anhydrous MgBr₂:etherate (1.3 g, 5mmol) under an argon atmosphere at room temperature. After cooling to -20 °C, Et₃N (2.0 mL) was added in one portion. The reaction vessel was covered with foil to exclude light. The reaction mixture was stirred for 2 h at -20 °C and treated with acetic anhydride (1.04 mL) in one portion. The reaction mixture was warmed to 0 °C and stirred for 15 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to a silica gel column, then the column was eluted with ethyl acetate: hexane (1:1). Collected fractions were concentrated under reduced pressure and the mixture of diastereo isomers were taken to next step. Pale yellow amorphous solid; Yield: 2.24 g, 95%; M+H 660.

### Step-5: (5R),(6Z)-6-[(7-methoxyimidazo[1,2-b][1,3]benzothiazol-2-ylmethylene)]-7-oxo-4-thia-1-azabicyclo [3.2.0]hept-2-ene-2-carboxylic acid:

4-Nitrobenzyl-6-[(acetyloxy) (7-methoxyimidazo[2,1-b][1,3]benzothiazol-2-yl)methyl]-6-bromo-7-oxo-4-thia-1-azabicyclo[3.2.0]hept-2-ene-2-carboxylate ( 659 mg, 1.0 mmol) was dissolved in THF (17 mL) and acetonitrile (36 mL). Freshly activated Zn dust (5.2 g) was added rapidly with 0.5 M phosphate buffer (pH 6.5, 28 mL). The reaction vessel was covered with foil to exclude light. The reaction mixture was vigorously stirred for 2 h at room temperature. The reaction mixture was filtered, cooled to 3 °C, and 1 N HCl was added to adjust pH to 7.5. The filtrate was washed with ethyl acetate and the aqueous layer was separated. The aqueous layer was concentrated under high vacuum at 35 °C to give yellow precipitate. The precipitate was filtered and washed with H₂O, MeCN, acetone to give the title compound. Yield: 68 mg, 23%; as yellow crystals; mp 284; M+H 386.
¹H NMR (DMSO-d₆) □ 3.89 (s, 3H), 6.58(s, 1H), 6.64(s, 1H), 7.14(s, 1H), 7.2(dd, 1H, *J* = 6.0 Hz), 7.75(d, 1H, *J* = 3.0 Hz), 8,03(d, *J*= 6.0 Hz 1H), 8.62(s, 1H).

### Example 16

### Preparation of (5R,6Z)-6-[(7-chloroimidazo[2,1-b][1,3]benzothiazol-2-ylmethylene)-7-oxo-4-thia-1-azabicyclo[3.2.0]hept-2-ene-2-carboxylic acid

### Step 1: Ethyl 7-chloroimidazo[2,1-b]-benzthiazole-2-carboxylate:

Ethyl 7-chloroimidazo[2,1-b]-benzthiazole-2-carboxylate was prepared according to the procedure as outlined in Example 1, (Step 1). Starting from 6-chloro-2-amino benzothiazole (9.2 g, 50 mmol) and ethyl bromopyruvate (11.6 g, 60 mmol), 8.5 g (60% Yield) of ethyl 7-chloroimidazo[2,1-b]-benzthiazole-2-carboxylate was isolated as brown solid. (M+H) 281.

### Step 2: 7-chloroimidazo[2,1-b]-benzthiazole-2-methanol:

7-chloro imidazo[2,1-b]-benzthiazole-2-methanol was prepared according to the procedure outlined in Example 1, (Step 2). Starting from ethyl 7-chloroimidazo[2,1-b]-benzthiazole-2-carboxylate (9.0 g, 32.1 mmol) and LiAlH₄ (4.0 g, excess), 5.5 g (72% yield) of the alcohol derivative was isolated as brown solid. mp 166°C(M+H) 239.

### Step 3: 2-Formyl-7-chloroimidazo[2,1-b]-benzthiazole:

2-Formyl-7-chloroimidazo[2,1-b]-benzthiazole was prepared according to the procedure outlined in Example 1, (Step 3). Starting from 7-chloroimidazo[2,1-b]-benzthiazole-2-methanol (4.0 g 16.8mmol) in methylene chloride/ MeOH (300 mL: 50 mL) and active MnO₂ (20 g, excess), 2.2 g (55% yield) of the aldehyde derivative was isolated as brown solid. (M+H) 236.

### Step 4: 4-Nitrobenzyl-6-[(acetyloxy) (7-chloroimidazo[2,1-b][1,3]benzothiazol-2-yl)methyl]-6-bromo-7-oxo-4-thia-1-azabicyclo[3.2.0]hept-2-ene-2-carboxylate:

2-Formyl-7-chloroimidazo[2,1-b]-benzthiazole (270 mg, 1.14 mmol) and the dry THF solution (20 mL) of (5R, 6S)-6-bromo-7-oxo-4-thia-l-aza-bicyclo[3.2.0]hept-2-ene-2-carboxylic acid 4-nitro-benzyl ester (500 mg, 1.14 mmol) were added successively to the dry acetonitrile (15 mL) solution of anhydrous MgBr₂: O(Et)₂ (390 mg, 1.5 mmol)under an argon atmosphere at room temperature. After cooling to -20 °C, Et₃N (2.0 mL) was added in one portion. The reaction vessel was covered with foil to exclude light. The reaction mixture was stirred for 2 h at -20 °C and treated with acetic anhydride (1.04 mL) in one portion. The reaction mixture was warmed to 0 °C and stirred for 15 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then the column was eluted with ethyl acetate: hexane (1:1). Collected fractions were concentrated under reduced pressure and the mixture of diastereomers were taken to the next step. Pale yellow amorphous solid; Yield: 495 mg, 65%; M+H 665.

### Step-5: (5R),(6Z)-6-[(7-chloroimidazo[1,2-b][1,3]benzothiazol-2-ylmethylene)]-7-oxo-4-thia-1-azabicyclo [3.2.0]hept-2-ene-2-carboxylic acid:

4-Nitrobenzyl-6-[(acetyloxy)(7-chloroimidazo[2,1-b][1,3]benzothiazol-2-yl)methyl]-6-bromo-7-oxo-4-thia-1-azabicyclo[3.2.0]hept-2-ene-2-carboxylate ( 450 mg, 0.67 mmol) was dissolved in THF (20 mL) and acetonitrile (10 mL). Freshly activated Zn dust (5.2 g) was added rapidly with 0.5 M phosphate buffer (pH 6.5, 28 mL). The reaction vessel was covered with foil to exclude light. The reaction mixture was vigorously stirred for 2 h at room temperature. The reaction mixture was filtered, cooled to 3 °C, and 0.1 N NaOH was added to adjust the pH to 8.5. The filtrate was washed with ethyl acetate and the aqueous layer was separated. The aqueous layer was concentrated under high vacuum at 35 °C to give a yellow precipitate. The product was purified by HP21 resin reverse phase column chromatography. Initially the column was eluted with deionized water (2 L) and latter with 10% acetonitrile:water. The fractions containing the product were collected and concentrated under reduced pressure at room temperature. The yellow solid was washed with acetone, filtered and dried. Yield: 80 mg, 18%; as yellow crystals; mp 240°C; (M+H+Na) 412.
¹H NMR (DMSO-d₆) 0 6.6 (s, 2H), 7.1 (s, 1H), 7.62 (dd, 1H), 8.11 (d, 1H), 8.2 (s, 1H), 8.6 (s, 1H).

### Example 17

### Preparation of (5R),(6Z)-6-Imidazo[1,2-a]quinolin-2-ylmethylene-7-oxo-4-thia-1-azabicyclo[3.2.0]hept-2-ene-2-carboxylic acid Imidazo[1,2-a]quinoline-2-carbaldehyde

Imidazo[1,2-*a*]quinoline-2-carbaldehyde(**4**) was prepared by the method of Westwood and co-workers (J. Med. Chem. 1988, 31, 1098-1115).

### Step 1: (5R, 6RS)-6-[(RS)-Acetoxyimidazo[1,2-a]quinolin-2-ylmethyl]-6-bromo-7-oxo-4-thia-1-azabicyclo[3.2.0]hept-2-ene-2-carboxylic acid 4-nitrobenzyl ester:

Imidazo[1,2-*a*]quinoline-2-carbaldehyde (1.09 g) and a dry THF solution (75.5 mL) of (5R,6S)-6-bromo-7-oxo-4-thia-1-aza-bicyclo[3.2.0]hept-2-ene-2-carboxylic acid 4-nitrobenzyl ester (2.22 g) were added successively to a dry acetonitrile (75.5 mL) solution of anhydrous MgBr₂ (2.5 g) under an argon atmosphere at room temperature. After cooling to -20 °C, Et₃N (1.85 mL) was added in one portion. The reaction vessel was covered with foil to exclude light. The reaction mixture was stirred for 2 h at -20 °C and treated with acetic anhydride (1.04 mL) in one portion. The reaction mixture was warmed to 0 °C and stirred for 15 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to a silica gel column, then the column was eluted with CHCl₃ -acetone(1/0 ∼ 95/5). Collected fractions were concentrated under reduced pressure followed by recrystallization from CHCl₃-Et₂O to give the title compound as one isomer. (pale yellow crystals, yield: 1.3 g, 38%).
¹H NMR (CDCl₃) □ 2.37(s, 3H), 5.29(d, 1H, *J* = 13.5 Hz), 5.45(d, 1H, *J* = 13.5 Hz), 6.22(s, 1H), 7.14(s, 1H), 7.46 ∼ 7.52(m, 3H), 7.56(d, 1H, *J* = 9.6 Hz), 7.62(d, 2H, *J* = 8.6 Hz), 7.64 ∼ 7.69(m, 1H), 7.83(dd, 1H, *J* = 1.1, 7.9 Hz), 7.93(d, 1H, *J* = 8.3 Hz), 7.99(s, 1H), 8.25(d, 2H, *J* = 8.6 Hz).

### Step 2: (5R),(6Z)-6-Imidazo[1,2-a]quinolin-2-ylmethylene-7-oxo-4-thia-1-azabicyclo[3.2.0]hept-2-ene-2-carboxylic acid:

(5*R*,6*RS*)-6-[(*RS*)-Acetoxyimidazo[1,2*-a*]quinolin-2-ylmethyl]-6-bromo-7-oxo-4-thia-1-azabicyclo[3.2.0]hept-2-ene-2-carboxylic acid 4-nitrobenzyl ester (1.3 g) was dissolved in THF (17 mL) and acetonitrile (36 mL). Freshly activated Zn dust (5.2 g) was added rapidly with 0.5 M phosphate buffer (pH 6.5, 28 mL). The reaction vessel was covered with foil to exclude light. The reaction mixture was vigorously stirred for 2 h at room temperature. The reaction mixture was filtered, cooled to 3 °C, and 1 N HCl was added to adjust the pH to 7.5. The filtrate was washed with ethyl acetate and the aqueous layer was separated. The aqueous layer was concentrated under high vacuum at 35 °C to give a yellow precipitate. The precipitate was filtered and washed with H₂O, acetonitrile, and acetone to give the title compound, yield 297 mg, 38%, as yellow crystals mp 205°C.
¹H NMR (D₂O) d 6.19(s, 1H), 6.36 (s, 1H), 6.87 (s, 1H), 6.96 (d, 1H, *J* = 9.5 Hz), 7.32 (d, 1H, *J* = 9.5 Hz), 7.33 (s, 1H), 7.44 ∼ 7.57 m, 4H).

### Example 18

### Preparation of (5R),(6Z)-6-(6,7-dihydro-5H-cyclopenta[d]imidazo[2,1-b][1,3]thiazol-2-ylmethylene)-7-oxo-4-thia-1-azabicyclo[3.2.0]hept-2-ene-2-carboxylic acid

### Step 1: Preparation of ethyl 6,7-dihydro-5H-cyclopenta[d]imidazo[2,1-b][1,3]thiazole-2-carboxylate;

A mixture of 2-chlorocyclopentanone ( 11.8 g, 100 mmol) and thiourea (8.0 g 101 mmol) was refluxed in ethanol: THF (1:2) for 16 hrs. The reaction mixture was cooled to room temperature and the separated white solid was filtered. ( 9.0 g separated) This was dissolved in anhydrous ethanol (100 ml) and sodium methoxide (1.9 g 2.7 g, 51 mmol). To this ethyl bromopyruvate (10 .0 g) was added and stirred at room temperature for 2 hrs. Then it was refluxed for 48 hrs. At the end reaction mixture was cooled to room temperature and concentrated. The residue was extracted with chloroform and washed well with water. The product was purified by silica-gel column chromatography by eluting it with 50% ethyl acetae: hexane. Red semi-solid; Yield: 3.0 g; M+H 237.

The ester was reduced with LiAlH₄ and the resultant alcohol was oxidized with active MnO₂. The aldehyde obtained was taken to next step.

### Step:3 Preparation of 4-nitrobenzyl (5R)-6-[(acetyloxy)(6,7-dihydro-5H-cyclopenta[d]imidazo[2,1-b][1,3]thiazol-2-yl)-6-bromo-7-oxo-4-thia-1-azabicyclo[3.2.0]hept-2-ene-2-carboxylate:

2-Formyl-6,7-dihydro-5H-cyclopenta[d]imidazo[2,1-b][1,3]thiazole (600 mg, 3.1 mmol) and the dry THF solution (20 mL) of (5R, 6S)-6-bromo-7-oxo-4-thia-1-azabicyclo[3.2.0]hept-2-ene-2-carboxylic acid 4-nitro-benzyl ester (1.2 g, 3 mmol) were added successively to the dry acetonitrile (15 mL) solution of anhydrous MgBr₂: O(Et)₂ (1.2 g, 3.0 mmol)under an argon atmosphere at room temperature. After cooling to -20 °C, Et₃N (2.0 mL) was added in one portion. The reaction vessel was covered with foil to exclude light. The reaction mixture was stirred for 2 h at -20 °C and treated with acetic anhydride (1.04 mL) in one portion. The reaction mixture was warmed to 0 °C and stirred for 15 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then the column was eluted with ethyl acetate: hexane (1:1). Collected fractions were concentrated under reduced pressure and the mixture of diastereomers were taken to the next step. Pale yellow amorphous solid; Yield: 850 mg, 45%; M+H 620.

### Step 4: Preparation of (5R),(6Z)-6-(6,7-dihydro-5H-cyclopenta[d]imidazo[2,1b][1,3]thiazol-2-ylmethylene)-7-oxo-4-thia-1-azabicyclo[3.2.0]hept-2-ene-2-carboxylic acid

4-nitrobenzyl (5R)-6-[(acetyloxy)(6,7-dihydro-5H-cyclopenta[d]imidazo[2,1-b][1,3]thiazol-2-yl)-6-bromo-7-oxo-4-thia-1-azabicyclo[3.2.0]hept-2-ene-2-carboxylate (850 mg, 1.37 mmol) was dissolved in THF (20 mL) and acetonitrile (10 mL). Freshly activated Zn dust (5.2 g) was added rapidly with 0.5 M phosphate buffer (pH 6.5, 28 mL). The reaction vessel was covered with foil to exclude light. The reaction mixture was vigorously stirred for 2 h at room temperature. The reaction mixture was filtered, cooled to 3 °C, and 0.1 N NaOH was added to adjust the pH to 8.5. The filtrate was washed with ethyl acetate and the aqueous layer was separated. The aqueous layer was concentrated under high vacuum at 35 °C to give a yellow precipitate. The product was purified by HP21 resin reverse phase column chromatography. Initially the column was eluted with deionized water (2 L) and latter with 10% acetonitrile:water. The fractions containing the product were collected and concentrated under reduced pressure at room temperature. The yellow solid was washed with acetone, filtered and dried. Yield: 138 mg, 29%; as yellow crystals; mp 192°C; (M+H+Na) 346 ¹H NMR (DMSO-d₆) □ 8.2 (s, 1H), 7.1 (s, 1H), 6.55 (s, 1H), 6.4 (s, 1H), 3.01 (m, 2H), 2.51 (m, 4H).

### Example 19

### Preparation of (5R),(6Z)-6-(Imidazo[1.2-a]aquinoxaline-2-ylmethylene)-7-oxo-4-thia-1-azabicyclo[3.2.0]hepto-2-ene-2-carboxylic acid, sodium salt

### Imidazo[9,2-a]quinoxaline-2-carboxaldehyde

Imidazo[1,2-a]quinoxaline-2-carboxaldehyde was prepared by the method of Westwood and co-workers (J. Med. Chem. 1998, 31, 1098-1115).

### Step 1: (5R, 6RS)-6-((RS)-Acetoxy imidazo[1,2-a]quinoxalin-2-ylmethyl)-6-bromo-7-oxo-4-thia-1-azabicyclo [3.2.0]hept-2-ene-2-carboxylic acid p-nitrobenzyl ester:

A dry acetonitrile (33 mL) solution of imidazo[1,2-a]quinoxaline-2-carboxaldehyde (505 mg) was added to a dry acetonitrile (20 mL) solution of MgBr₂ (1.1 g) under an nitrogen atmosphere at room temperature, and the mixture was stirred for 10 min. After addition of the dry THF (25 mL) solution of (5R, 6S)-6-bromo-7-oxo-4-thia-1-azabicyclo[3.2.0]hept-2-ene-2-carboxylic acid 4-nitro-benzyl ester (931 mg), the mixture was cooled to -20 °C then triethylamine (0.8 mL) was added in one portion. The reaction vessel was covered with foil to exclude light. The reaction mixture was stirred for 4 h at -20 °C and treated with 4,4-dimethylamino pyridine (58 mg) and acetic anhydride (0.44 mL) in one portion. The reaction mixture was warmed to 0 °C and stirred for 16 h at 0 °C. 10% Citric acid aqueous solution (200 mL) was added to the reaction mixture and the aqueous layer was extracted with ethyl acetate (3 x 100 mL). The organic layer was washed with water, saturated sodium hydrogen carbonate and brine, dried (MgSO₄) and filtered. The filtrate was concentrated under reduced pressure. The residue was purified by silica gel column chromatography, eluted with CH₂Cl₂ - acetone (50:1), and the title compound was obtained as a diastereomeric mixture (78 · 22, pale brown foamy amorphous, 1.0 g, 68.9%).
¹H NMR (CDCl₃) □ 2.07 (s, 0.66H), 2.38 (s, 2.34H), 5.30 (d, 1H, *J* = 13.5 Hz), 5.45 (d, 0.78H, *J* = 13.5 Hz), 5.48 (d, 0.22H, *J* = 13.5 Hz), 6.24 (s, 0.78H), 6.46 (s, 0.22H), 6.63 (s, 0.22H), 7.18 (s, 0.78H), 7.50 (s, 0.78H), 7.52 (s, 0.22H), 7.61 (d, 1.56H, *J* = 8.7 Hz), 7.63 (d 0.44H, *J* = 8.8 Hz), 7.64-7.67 (m, 1H), 7.68-7.73 (m, 1H), 7.92-7.95 (m, 1H), 8.08 (s, 0.78H), 8.13-8.16 (m, 1H), 8.24 (d, 1.56H, *J* = 8.7 Hz), 8.25 (d, 0.44H, *J* = 8.8 Hz), 8.33 (s, 0.22H), 9.05 (s, 0.78H), 9.09 (s, 0.22H).

### Step 2: (5R),(6Z)-6-(Imidazo[1.2-a]quinoxaline-2-ylmethylene)-7-oxo-4-thia-1-azabicyclo[3.2.0] hepto-2-ene-2-carboxylic acid, sodium salt:

(5R, 6RS)-6-((RS)-Acetoxy imidazo[1,2-a]quinoxalin-2-yimethyl)-6-bromo-7-oxo-4-thia-1-azabicyclo [3.2.0]hept-2-ene-2-carboxylic acid p-nitrobenzyl ester (951 mg) and 10% Pd-C (50% wet, 477 mg) were added to a mixture of THF (48 mL) and 0.5 mol/L phosphate buffer (pH 6.5, 48 mL). The mixture was hydrogenated at 400 kPa at room temperature for 4 h. The reaction solution was filtered and Pd-C was washed with water and *n*-butanol. The aqueous layer was separated and then the organic layer was extracted with water. The combined aqueous layer was concentrated to 57 g and applied to Diaion HP-21 resin (60 mL, Mitsubishi Kasei Co. Ltd.) column chromatography. After adsorbing, the column was eluted with water and then 5, 10, 15 and 20% acetonitrile:water solution (each 60 mL). The combined fractions were concentrated under high vacuum at 35 °C and lyophilized to give the title compound as a yellow amorphous solid, yield 148 mg (26.1%), mp 300 °C (dec).
¹H NMR (D₂O) □ 5.92 (s, 1H), 6.23 (s, 1H), 6.66 (s, 1H), 7.11-7.22 (m, 3H), 7.25 (d, 1H, *J* = 7.9 Hz), 7.50 (s, 1H), 8.03 (s, 1H); IR (KBr) 3413, 1748, 1592, 1553 cm⁻¹; I^{max} (H₂O) 340, 293, 237, 218 nm.

### Example 20

### Preparation of (5R), (6Z)-6-(5,6-Dihydro-8H-imidazo[2,1-c][1,4]oxazin-2-ylmethylene)-7-oxo-4-thia-1-azabicyclo[3.2.0]hept-2-ene-2-carboxylic acid, sodium salt

### Step 1: Morpholin-3-one

Morpholin-3-one may be prepared in the method of USP 5,349,045.

### Step 2: Morpholin-3-thione

A mixture of morpholin-3-one (4.7 g) and Lawesson's reagent (10.3 g) in dry THF (94 mL) was heated to reflux for 1.5 h. The reaction mixture was cooled to room temperature and the reaction solvent was removed in vacuo. The residue was applied to silica gel column chromatography and eluted with CHCl₃ - methanol (50 : 1) to obtain a yellow solid. Recrystallization of the crude product from hexane-ethyl acetate gave the title (4.0 g, 72.2%) as yellow powder.

¹H NMR (CDCl₃) δ 3.45 (t, 2H, *J* = 5.1 Hz), 3.91 (t, 2H, *J* = 5.1 Hz), 4.55 (s, 2H).

### Step 3: 5-Methylthio-3,6-dihydro-2H-[1,4]oxazine

A mixture of morpholin-3-thione (4.7 g) and methyl iodide (13 mL) in dry CH₂Cl₂ (140 mL) was stirred at room temperature for 15 h. The reaction mixture was filtered and the solid was washed with CH₂Cl₂. The obtained solid was dissolved with 50% K₂CO₃ aqueous solution (150 mL) and the aqueous layer was extracted with CH₂Cl₂ (8 x 100 mL). The combined CH₂Cl₂ layer was dried (MgSO₄) and filtered. The filtrate was concentrated under reduce pressure and the title was obtained as pale yellow oil (3.6 g, 67.8%).

¹H NMR (CDCl₃) δ 2.32 (s, 3H), 3.71-3.74 (m, 4H), 4.14-4.15 (m, 2H).

### Step 4: 3-Iminomorpholin hydrochloride

A mixture of 5-methylthio-3,6-dihydro-2*H*-[1,4]oxazine ( 3.6 g) and ammonium chloride (1.5 g) in dry ethanol (136 mL) was heated to reflux for 1 h. The reaction mixture was cooled to room temperature. The reaction solvent was removed in vacuo and the title was obtained as a pale brown solid (3.6 g, 97.7%).

¹H NMR (DMSO-d⁶) δ 3.34 (m, 2H), 3.86 (t, 2H, *J* = 5.2 Hz), 4.47 (s, 2H).

### Step 5: 5,6-Dihydro-8H-imidazo[2,1-c][1,4]oxazine-2-carbaldehyde (9) and 5,6-dihvdro-8H-imidazo[2,1-c][1,4]oxazine-3-carbaldehyde

The mixture of 2-bromo-3-hydroxypropenal (4.1 g), *p*-toluenesulfonic acid monohydrate (52 mg) and 2-propanol (5.2 mL) in cyclohexane (42 mL) was azeotroped until the vapor temperature rose to 80°C. The reaction mixture was concentrated under reduce pressure. The residue was dissolved in dry ethanol (50 mL). A mixture of the dry ethanol (200 mL) solution of 3-iminomorpholin hydrochloride (3.4 g) and 28% methanol solution of sodium methylate (4.8 g) was added at room temperature. The reaction mixture was stirred at room temperature for 2 h, and then the reaction solvent was removed in vacuo. The residue was dissolved in chloroform (125 mL) and triethylamine (3.5 mL) was added, then the reaction mixture was heated to reflux for 2 h. The reaction mixture was cooled to room temperature and then concentrated under reduce pressure. The residue was dissolved in dichloromethane (300 mL) and washed with 50% K₂CO₃ aqueous solution (2 x 100 mL). The organic layer was dried (MgSO₄) and filtered. The filtrate was concentrated under reduce pressure. The residue was applied to silica gel column chromatography and eluted with CHCl₃ - acetone (4 : 1) to obtain the title (pale orange solid, 1.4 g, 36.3%) and the other regio isomer. (pale orange solid, 609 mg, 16.1%).

Desired product: ¹H NMR (CDCl₃) δ 4.08-4.15 (m, 4H), 4.88 (s, 2H), 7.58 (s, 1H), 9.85 (s, 1H).

The unwanted regio isomer: ¹H NMR (CDCl₃) δ 4.06 (t, 2H, *J* = 5.2 Hz), 4.40 (t, 2H, *J* = 5.2 Hz), 4.90 (s, 2H), 7.75 (s, 1H), 9.72 (s, 1H).

### Step 6: 5R), (6Z)-6-(5,6-Dihydro-8H-imidazo[2,1-c][1,4]oxazin-2-ylmethylene)-7-oxo-4-thia-1-azabicyclo[3.2.0]hept-2-ene-2-carboxylic acid, sodium salt

The dry acetonitrile (66 mL) solution of 5,6-dihydro-8*H*-imidazo[2,1-c][1,4]oxazine-2-carbaldehyde (1.2 g) was added to the dry acetonitrile (66 mL) solution of MgBr₂ (3.6 g) under a nitrogen atmosphere at room temperature then the mixture was stirred for 10 min. The dry THF (132 mL) solution of p-nitrobenzyl (5R, 6S)-6-bromopenem-3-carboxylate (3.4 g) was added and the mixture was cooled to -20 °C then triethylamine (2.8 mL) was added in one portion. The reaction vessel was covered with foil to exclude light. The reaction mixture was stirred for 4 h at -20 °C and treated with 4-dimethylamino pyridine (100 mg) and acetic anhydride (1.5 mL) in one portion. The reaction mixture was warmed to 0 °C and stirred for 18 h at 0 °C. 10% Citric acid aqueous solution (1 L) was added to the reaction mixture and the aqueous layer was extracted with ethyl acetate (3 x 500 mL). The combined organic layer was washed with water, saturated sodium hydrogen carbonate and brine, dried (MgSO₄) and filtered. The filtrate was concentrated under reduced pressure and crude (5*R*)-6-[acetoxy-(5,6-dihydro-8*H*-imidazo[2,1-c][1,4]oxazin-2-yl)methyl]-6-bromo-7-oxo-4-thia-1-azabicyclo[3.2.0]hept-2-ene-2-carboxylic acid p-nitrobenzyl ester was obtained as brown amorphous solid.

Freshly activated Zn dust (14 g) was added rapidly with 0.5 mol/L phosphate buffer (pH 6.5, 72 mL) to the THF (72 mL) solution of (5*R*)-6-[acetoxy-(5,6-dihydro-8*H-*imidazo[2,1-c][1,4]oxazin-2-yl)methyl]-6-bromo-7-oxo-4-thia-1-azabicyclo[3.2.0]hept-2-ene-2-carboxylic acid p-nitrobenzyl ester. The reaction vessel was covered with foil to exclude light. The reaction mixture was vigorously stirred for 2.5 h at room temperature. The reaction solution was filtered through a pad of Celite and the pad was washed with water (170 mL) and *n*-butanol (170 mL). The aqueous layer was separated and then the organic layer was extracted with 0.5 mol/L phosphate buffer (pH 6.5, 2 x 50 mL). The combined aqueous layer was concentrated to 90 g, 1 mol/L NaOH was added to adjust pH to 7.5 and applied to Diaion HP-21 resin (120 mL, Mitsubishi Kasei Co. Ltd.) column chromatography. After adsorbing, the column was eluted with water and then 5% acetonitrile aqueous solution. The combined active fractions was concentrated under high vacuum at 35°C and lyophilized to give the title as a yellow amorphous solid (756 mg, 29.1%).

Mp 130 °C (dec); ¹H NMR (DMSO-d₆) δ 3.98-4.01 (m, 2H), 4.04-4.07 (m, 2H), 4.74 (AB, 2H, *J* = 15.3, 22.9 Hz), 6.40 (d, 1H, *J* = 0.8 Hz), 6.55 (s, 1H), 6.95 (d, 1H, *J* = 0.6 Hz), 7.54 (s, 1H); IR (KBr) 3412, 1741, 1672, 1592, 1549 cm⁻¹; λ^{max} (H₂O) 304 nm.

### Example 21

### Preparation of (5R),(6Z)-6-(5,6-Dihydro-4H-pyrrolo[1,2-b]pyrazol-2-ylmethylene)-7-oxo-4-thia-1-azabicyclo[3.2.0]hept-2-ene-2-carboxylic acid, sodium salt

### Step 1: 5,6-Dihydro-4H-pyrrolo[1,2-b]pyrazole-2-carboxylic acid ethyl ester

The titled compound was prepared in the same way of Ranganathan and co-workers (Indian J. Chem. 1991, 30 B, 169-175).

### Step 2: (5,6-Dihydro-4H-pyrrolo[1,2-b]pyrazol-2-yl)methanol

MeOH (2.73 mL) was added to the THF (180 mL) solution of LiBH₄ (1.63 g) under a nitrogen atmosphere at room temperature, and then 5,6-dihydro-4*H*-pyrrolo[1,2-b]pyrazole-2-carboxylic acid ethyl ester (8.11 g) was added to the suspension and stirred for 2 h at 40 °C. The mixture was quenched with 1 mol/L HCl at pH 1 and stirred for 1 h at room temperature. Solid K₂CO₃ was added to the solution to adjust pH to 8 and the mixture was extracted with AcOEt. The organic layer was dried (MgSO₄) and filtered. The filtrate was concentrated under reduced pressure to afford the title compound as brown crystals (4.87 g, 78%).

¹H NMR (CDCl₃) δ 2.44 (t, 1H, *J* = 5.8 Hz), 2.54 - 2.62 (m, 2H), 2.87 (t, 2H, *J* = 7.4 Hz), 4.10 (t, 2H, *J* = 7.2 Hz), 4.63 (d, 2H, *J* = 5.8 Hz), 5.96 (s, 1H).

### Step 3: 5.6-Dihydro-4H-pyrrolo[1,2-b]pyrazole-2-carbaldehyde

MnO₂ (activated) (24.4 g) was added to the CHCl₃ (350 mL) solution of (5,6-dihydro-4*H*-pyrrolo[1,2-*b*]pyrazol-2-yl)methanyl (4.87 g) and refluxed for 1 h under a nitrogen atmosphere. The reaction mixture was filtered through a pad of Celite. The filtrate was reduced under reduced pressure. The residue was applied to silica gel column chromatography, then the column was eluted with n-hexane -AcOEt (1/1 - 1/2). The title compound was obtained as yellow oil (4.35 g, 91%).

¹H NMR (CDCl₃) δ 2.63 - 2.71 (m, 2H), 2.95 (t, 2H, *J* = 7.4 Hz), 4.22 (t, 2H, *J* = 7.4 Hz), 6.52 (s, 1H), 9.89 (s, 1H).

### Step 4: (5R),(6Z)-6-(5,6-Dihydro-4H-pyrrolo[1,2-b]pyrazol-2-ylmethylene)-7-oxo-4-thia-1-azabicyclo[3.2.0]hept-2-ene-2-carboxylic acid, sodium salt

5,6-Dihydro-4*H*-pyrrolo[1,2-*b*]pyrazole-2-carbaldehyde (1.36 g) was added to the dry acetonitrile (148 mL) solution of anhydrous MgBr₂ (5.52 g) under a nitrogen atmosphere at room temperature. The dry THF solution (148 mL) of (5*R*, 6*S*)-6-bromo-7-oxo-4-thia-1-azabicyclo[3.2.0]hept-2-ene-2-carboxylic acid 4-nitrobenzyl ester (cont. 97%) (3.97 g) was added to the mixture, cooled to -20 °C, and Et₃N (4.18 mL) was added in one portion. The reaction vessel was covered with foil to exclude light. The reaction mixture was stirred for 4 h at -20 °C and treated with acetic anhydride (1.89 mL) and DMAP (123 mg) in one portion. The reaction mixture was warmed to 0 °C and stirred for 14 h at 0 °C. The mixture was diluted with ethyl acetate and washed with 5% citric acid aqueous solution, saturated sodium hydrogen carbonate, water and brine. The organic layer was dried (MgSO₄) and filtered. The filtrate was concentrated under reduced pressure.

The residue was dissolved in THF (106 mL) and acetonitrile (49 mL). Freshly activated Zn dust (22.5 g) was added rapidly with 0.5 M phosphate buffer (pH 6.5, 155 mL). The reaction vessel was covered with foil to exclude light. The reaction mixture was vigorously stirred for 1.5 h at room temperature. The reaction mixture was filtered through a pad of Celite. The filtrate was washed with ethyl acetate and the aqueous layer was separated. The aqueous layer was cooled to 3 °C and 1 M NaOH was added to adjust pH to 8.0. The mixture was concentrated under high vacuum at 35 °C. The concentrate was applied to Diaion HP-21 (79 mL, Mitsubishi Kasei Co. Ltd.) resin column chromatography. After adsorbing, the column was eluted with H₂O - MeCN (1/0 - 9/1). The combined fractions were concentrated under high vacuum at 35 °C and lyophilized to give the title compound as a yellow amorphous solid (848 mg, 29%, pH 7.1).

Mp 190 °C (dec); ¹H NMR (D₂O) δ 2.49 (m, 2H), 2.78 (t, 2H, *J* = 7.4 Hz), 4.02 (t, 2H, *J* = 7.4 Hz), 6.01 (s, 1H), 6.29 (s, 1H), 6.90 (s, 2H).

### Example 22

### Preparation of (5R)(6Z)-7-Oxo-6-(4,5,6,7-tetrahydropyrazolo[1,5-a]pyridin-2-ylmethylene)-4-thia-1-azabicyclo[3.2.0]hept-2-ene-2-carboxylic acid, sodium salt

### Step 1: Tetrahydropyridino[1,2-c][1,2,3]oxadiazolone

Conc. HCl (1.96 mL) and NaNO₂ (2.2 g) were added to the H₂O (21 mL) solution of *DL-*pipecolic acid (3.04 g) under a nitrogen atmosphere at 0 °C and stirred for 1 h. The solution was extracted with CH₂Cl₂ and organic layer was washed with brine. The mixture was dried over Na₂SO₄ and concentrated under reduced pressure to afford crude (2R*S*)-1-nitrosopiperidine-2-carboxylic acid as pale yellow crystals.

Trifluoroacetic anhydride (1.93 g) was added to the THF (92 mL) solution of crude (2*RS*)-1-nitrosopiperidine-2-carboxylic acid under a nitrogen atmosphere at 0 °C and stirred for 5 h at 0 °C and for 2 h at room temperature. The solution was concentrated under a reduced pressure. The residue was applied to silica gel column chromatography, then the column was eluted with n-hexane - AcOEt (1/1 - 0/1). The titled compound was obtained as colorless crystals (1.10 g, 33%).

¹H NMR (CDCl₃) δ 1.93 - 1.99 (m, 2H), 2.08 - 2.15 (m, 2H), 2.65 (t, 2H, *J* = 6.5 Hz), 4.26 (t, 2H, *J* = 6.1 Hz).

### Step 2: 4,5,6,7-Tetrahydropyrazolo[1,5-a]pyridine-2-carboxylic acid ethylester:

Ethyl propiolate (804 mg) was added to the o-xylene (15 mL) solution of tetrahydropyridino[1,2-c][1,2,3]oxadiazolone (1.04 g) under a nitrogen atmosphere and refluxed for 16 h. The solution was concentrated under a reduced pressure. The residue was applied to silica gel column chromatography, then the column was eluted with n-hexane - AcOEt (2/1 - 1/1). The titled compound was obtained as yellow oil (871 mg, 65%), and 4,5,6,7-tetrahydropyrazolo[1,5-*a*]pyridine-3-carboxylic acid ethyl ester was obtained as yellow oil (345 mg, 26%).

¹H NMR (CDCl₃) δ 1.39 (t, 3H, *J* = 7.1 Hz), 1.84 - 1.91 (m, 2H), 2.02 - 2.09 (m, 2H), 2.82 (t, 2H, *J* = 6.4 Hz), 4.22 (t, 2H, *J* = 6.2 Hz), 4.39 (q, 2H, *J* = 7.1 Hz), 6.53 (s, 1H).

### Step 3: (4,5,6,7-Tetrahydropyrazolo[1,5-a]pyridin-2-yl)methanol

MeOH (0.29 mL) was added to the THF (19 mL) solution of LiBH₄ (cont. 90%) (174 mg) under a nitrogen atmosphere at room temperature, then 4,5,6,7-tetrahydropyrazolo[1,5-a]pyridine-2-carboxylic acid ethyl ester (862 mg) was added to the suspension and stirred for 1 h at room temperature and 1.5 h at 40 °C. The mixture was quenched with 1 mol/L HCl at pH 1 and stirred for 1 h at room temperature. Solid K₂CO₃ was added to the solution to adjust pH to 8 and the mixture was extracted with AcOEt. The organic layer was dried (MgSO₄) and filtered. The filtrate was concentrated under reduced pressure to afford titled compound as pale yellow oil (691 mg, 95%).

¹H NMR (CDCl₃) δ 1.80 - 1.87 (m, 2H), 1.98 - 2.05 (m, 2H), 2.77 (t, 2H, *J* = 6.4 Hz), 2.81 - 2.84 (br, 1H), 4.09 (t, 2H, *J* = 6.1 Hz), 4.62 (d, 2H, J = 5.3 Hz), 5.96 (s, 1H).

### Step 4: 4,5,6,7-Tetrahydropyrazolo[1,5-a]pyridine-2-carbaldehyde

MnO₂ (activated) (3.36 g) was added to the CHCl₃ (44 mL) solution of (4,5,6,7-tetrahydropyrazolo[1,5-a]pyridin-2-yl)methanol (673 mg) and refluxed for 1 h under a nitrogen atmosphere. The reaction mixture was filtered through a pad of Celite. The filtrate was reduced under reduced pressure. The residue was applied to silica gel column chromatography, then the column was eluted with *n*-hexane - AcOEt (2/1 - 1/2). The titled compound was obtained as pale yellow oil (510 mg, 77%).

¹H NMR (CDCl₃) δ 1.90 (m, 2H), 2.10 (m, 2H), 2.84 (t, 2H, *J* = 6.4 Hz), 4.23 (t, 2H, *J* = 6.2 Hz), 6.52 (s, 1H). 9.92 (s, 1H).

### Step 5: (5R)(6Z)-7-Oxo-6-(4,5,6,7-tetrahydropyrazolo[1,5-a]pyridin-2-ylmethylene)-4-thia-1-azabicyclo[3.2.0]hept-2-ene-2-carboxylic acid, sodium salt

4,5,6,7-Tetrahydropyrazolo[1,5-a]pyridine-2-carbaldehyde (483 mg) was added to the dry acetonitrile (48 mL) solution of anhydrous MgBr₂ (1.81 g) under a nitrogen atmosphere at room temperature. The dry THF solution (48 mL) of (5*R*, 6*S*)-6-bromo-7-oxo-4-thia-1-azabicyclo[3.2.0]hept-2-ene-2-carboxylic acid 4-nitrobenzyl ester (cont. 97%) (1.28 g) was added to the mixture, cooled to -20 °C, and Et₃N (1.35 mL) was added in one portion. The reaction vessel was covered with foil to exclude light. The reaction mixture was stirred for 2 h at -20°C and treated with acetic anhydride (0.61 mL) and DMAP (40 mg) in one portion. The reaction mixture was warmed to 0 °C and stirred for 16 h at 0 °C. The mixture was diluted with ethyl acetate and washed with 5% citric acid aqueous solution, saturated sodium hydrogen carbonate, water and brine. The organic layer was dried (MgSO₄) and filtered. The filtrate was concentrated under reduced pressure.

The residue was dissolved in THF (35 mL) and acetonitrile (16 mL). Freshly activated Zn dust (7.43 g) was added rapidly with 0.5 *M* phosphate buffer (pH 6.5, 51 mL). The reaction vessel was covered with foil to exclude light. The reaction mixture was vigorously stirred for 1.5 h at room temperature. The reaction mixture was filtered through a pad of Celite. The filtrate was washed with ethyl acetate and the aqueous layer was separated. The aqueous layer was cooled to 3 °C and 1 *M* NaOH was added to adjust pH to 8.0. The mixture was concentrated under high vacuum at 35°C. The concentrate was applied to Diaion HP-21 (105 mL, Mitsubishi Kasei Co. Ltd.) resin column chromatography. After adsorbing, the column was eluted with H₂O - MeCN (1/0 - 85/15). The combined fractions were concentrated under high vacuum at 35 °C and lyophilized to give the title compound as a yellow amorphous solid (427 mg, 41%, pH 7.7).

Mp 190 °C (dec); ¹H NMR (D₂O) δ 1.67-1.71 (m, 2H), 1.85-1.89 (m, 2H), 2.64 (t, 2H, *J* = 6.3 Hz), 3.97 (t, 2H, *J* = 6.1 Hz), 5.97 (s, 1H), 6.25 (s, 1H). 6.85 (s, 1H). 6.88 (s, 1H).

### Example 23

### Preparation of (5R),(6Z)-6-(7-Methyl-6-oxo-5,6,7,8-tetrahydro-imidazo[1,2-a]pyrazin-2-ylmethylene)-7-oxo-4-thia-1-aza-bicyclol[3.2.0]hept-2-ene-2-carboxylic acid sodium salt

### Step 1: 5-Methoxy-1-methyl-3,6-dihydro-1H-pyrazin-2-one

The titled compound was prepared in the same way of S.Rajappa and B.G.Advani (Tetrahedron. 1973, 29, 1299-1302).

### Step 2: 5-Amino-1-methyl-3,6-dihydro-1H-pyrazin-2-one

A mixture of 5-methoxy-1-methyl-3,6-dihydro-1H-pyrazin-2-one (2.3 g) and ammonium chloride (936 mg) in dry ethanol (32 mL) was stirred at room temperature for 1 h and then refluxed for 2 h. The reaction mixture was cooled to room temperature and evaporated under reduced pressure. The residue was triturated with chloroform at room temperature for 30 min. The precipitate was filtered off and dried in vacuo. The 5-amino-1-methyl-3,6-dihydro-1H-pyrazin-2-one hydrochloride was obtained as a pale brown powder (1.7 g, 66%).
A solution of 5-amino-1-methyl-3,6-dihydro-1H-pyrazin-2-one hydrochloride (662mg) in methanol (10 mL) was added 10% potassium carbonate aqueous solution at 0□ and then stirred for 40 min at 0□. The mixture was concentrated under reduced pressure. The residue was triturated with chloroform (18mL) and methanol (2 mL) at room temperature for 30 min. The precipitate was filtered off and dried in vacuo. The compound was obtained as a pale brown powder (515 mg, quantitative).
¹H NMR (DMSO-d⁶) δ 2.88 (s, 3H), 3.94 (s, 2H), 4.42 (s, 2H).

### Step 3: 7-Methyl-6-oxo-5,6,7,8-tetrahydro-imidazo [1,2-a]pyrazine-2-carbaldehyde and 7-Methyl-6-oxo-5,6,7,8-tetrahydro-imidazo[1,2-a]pyrazine-3-carbaldehyde

The solution of 2-bromo-3-isopropoxy-propenal (1.3 g) in dry acetonitrile (60 mL) was added to the solution of 5-amino-1-methyl-3,6-dihydro-1H-pyrazin-2-one (782 mg) in dry acetonitrile (60 mL) at room temperature. The reaction mixture was stirred at room temperature for 20 h, added triethylamine (0.95 mL) and then refluxed for 2 h. The reaction mixture was cooled to room temperature and then evaporated under reduce pressure. The residue was dissolved in chloroform (10mL) and washed with 50% K₂CO₃ aqueous solution (10mL). The aqueous layer was extracted with chloroform. The organic layer was dried (MgSO₄) and filtered. The filtrate was evaporated under reduce pressure. The residue was applied to silica gel column chromatography and eluted with CHCl₃ - MeOH (95 : 5) to obtain the title compound 7-Methyl-6-oxo-5,6,7,8-tetrahydro-imidazo [1,2-a]pyrazine-2-carbaldehyde as a pale yellow solid (541 mg, 49.1%) and its regio isomer 7-Methyl-6-oxo-5,6,7,8-tetrahydro-imidazo[1,2-a]pyrazine-3-carbaldehyde as a pale yellow solid (128 mg, 11.6%).
7-Methyl-6-oxo-5,6,7,8-tetrahydro-imidazo [1,2-a]pyrazine-2-carbaldehyde: ¹H NMR (CDCl₃) δ 3.17 (s, 3H), 4.68 (s, 2H), 4.78 (s, 2H), 7.66 (s, 1H), 9.83 (s, 1H). 7-Methyl-6-oxo-5,6,7,8-tetrahydro-imidazo[1,2-a]pyrazine-3-carbaldehyde: ¹H NMR (CDCl₃) δ 3.16 (s, 3H), 4.70 (s, 2H), 5.03 (s, 2H), 7.82 (s, 1H), 9.73 (s, 1H).

### Step 4: (5R, 6RS)-6-[Acetoxy-(7-methyl-6-oxo-5,6,7,8-tetrahydro-imidazo[1,2-a]pyrazin-2-yl)-methyl]-6-bromo-7-oxo-4-thia-1-aza-bicyclo[3,2,0]hept-2-ene-2-carboxylic acid 4-nitro-benzyl ester

7-Methyl-6-oxo-5,6,7,8-tetrahydro-imidazo [1,2-a]pyrazine-2-carbaldehyde (319 mg) was added to the dry acetonitrile (32 mL) solution of anhydrous MgBr₂ (786 mg) under a nitrogen atmosphere at room temperature. The dry THF solution (32 mL) of (5R, 6S)-6-bromo-7-oxo-4-thia-1-aza-bicyclo[3.2.0]hept-2-ene-2-carboxylic acid 4-nitrobenzyl ester (687 mg) was added to the mixture, cooled to -20 °C, and triethylamine (0.60 mL) was added in one portion. The reaction vessel was covered with foil to exclude light. The reaction mixture was stirred for 3 h at -20°C and treated with 4-dimethylaminopyridine (44 mg) and acetic anhydride (0.35 mL) in one portion. The reaction mixture was warmed to 0 °C and stirred for 20 h at 0°C. The mixture was diluted with ethyl acetate and H₂O. After separating organic layer, the aqueous layer was extracted with ethyl acetate. The organic layers were combined and washed with 5% citric acid aqueous solution and brine. The organic layer was dried (MgSO₄) and filtered. The filtrate was concentrated under reduced pressure. The residue was applied to silica gel column chromatography, then eluted with chloroform. The title compound was obtained as diastereo mixture (yellow amorphous solid ; 410 mg, 38%).
¹H NMR (δ, CDCl₃) 2.03 (s, 0.7 x 3 H), 2.09 (s, 0.3 x 3H), 3.15 (s, 3H), 4.59-4.62 (m, 2H), 4.66 (s, 0.3 x 2H), 4.67 (s, 0.7 x 2H), 5.28 (d, 1H, *J* = 13.5 Hz), 5.43 (d, 0.3 x 1H, *J* = 13.5 Hz), 5.45 (d, 0.7 x 1H, *J* = 13.5 Hz), 6.07 (s, 0.3 x 1H), 6.28 (s, 0.7 x 1H), 6.32 (s, 0.7 x 1H), 6.83 (s, 0.3 x 1H), 6.86 (s, 0.3 x 1H), 7.10 (s, 0.7 x 1H), 7.44 (s, 0.3 x 1H), 7.47 (s, 0.7 x 1H), 7.60 (d, 0.7 x 2H, *J* = 8.6 Hz), 7.61 (d, 0.3 x 2H, *J* = 8.6 Hz), 8.24 (d, 2H, *J* = 8.6 Hz),

### Step 5: (5R),(6Z)-6-(7-Methyl-6-oxo-5,6,7,8-tetrahydro-imidazo[1,2-a]pyrazin-2-ylmethylene)-7-oxo-4-thia-1-aza-bicyclo[3.2.0]heat-2-ene-2-carboxylic acid sodium salt and (5R),(6E)-6-(7-Methyl-6-oxo-5,6,7,8-tetrahydro-imidazo[1,2-a]pyrazin-2-ylmethylene(-7-oxo-4-thia-1-aza-bicyclo[3.2.0]hept-2-ene-2-carboxylic acid sodium salt

(5*R*, 6*RS*)- 6- [Acetoxy-(7-methyl-6-oxo-5,6,7,8-tetrahydro-imidazo[1,2-a]pyrazin-2-yl)-methyl]-6-bromo-7-oxo-4-thia-1-aza-bicyclo[3.2.0]hept-2-ene-2-carboxylic acid 4-nitrobenzyl ester (481 mg) was dissolved in THF (6.7 mL) and acetonitrile (3.1 mL). Freshly activated Zn dust (1.92 g) and 0.5 *M* phosphate buffer (pH 6.5, 9.9 mL) were added to the mixture. The reaction vessel was covered with foil to exclude light. The reaction mixture was vigorously stirred for 2 h at room temperature. The reaction solution was mixed with ethyl acetate and filtered through a pad of Celite. The pad was washed with water and the aqueous layer was separated. The aqueous layer was cooled to 3 °C and 1 *M* NaOH was added to adjust pH to 8.0. The mixture was concentrated under high vacuum at 35 °C and lyophilized. The residue was separated by the preparative HPLC (Inertsil ODS-2, GL Science Inc., 10 x 250 mm, 0.05 mol/L phosphate buffer (pH 7.1) : CH₃CN = 93 : 7, 4.0 mUmin.). The separated fractions of (5*R*),(6*Z*)-6-(7-Methyl-6-oxo-5,6,7,8-tetrahydro-imidazo[1,2-a]pyrazin-2-ylmethylene)-7-oxo-4-thia-1-aza-bicyclo[3.2.0]hept-2-ene-2-carboxylic acid sodium salt and (5*R*),(6*E*)-6-(7-Methyl-6-oxo-5,6,7,8-tetrahydro-imidazo[1,2-a]pyrazin-2-ylmethylene)-7-oxo-4-thia-1-aza-bicyclo[3.2.0]hept-2-ene-2-carboxylic acid sodium salt were cooled to 3 °C and 1 *M* NaOH was added to adjust pH to 8.0 respectively. Each solution was concentrated under high vacuum at 35 °C. Each concentrate was applied to Diaion HP-21 (60 mL, Mitsubishi Kasei Co. Ltd.) resin column chromatography. After adsorbing, the column was eluted with water and then with 5% acetonitrile-water. The combined fractions were concentrated under high vacuum at 35 °C and lyophilized to give the title compound(5*R*),(6*Z*)-6-(7-Methyl-6-oxo-5,6,7,8-tetrahydro-imidazo[1,2-a]pyrazin-2-ylmethylene)-7-oxo-4-thia-1-aza-bicyclo[3.2.0]hept-2-ene-2-carboxylic acid sodium salt as a yellow amorphous solid (125 mg, 44.4 %, Mp 115-117°C (dec)) and compound (5*R*),(6*E*)-6-(7-Methyl-6-oxo-5,6,7,8-tetrahydro-imidazo[1,2-a]pyrazin-2-ylmethylene)-7-oxo-4-thia-1-aza-bicyclo[3.2.0]hept-2-ene-2-carboxylic acid sodium salt as yellow amorphous solid (19 mg, 6.7 %) respectively.
Compound (5*R*),(6*Z*)-6-(7-Methyl-6-oxo-5,6,7,8-tetrahydro-imidazo[1,2-a]pyrazin-2-ylmethylene)-7-oxo-4-thia-1-aza-bicyclo[3.2.0]hept-2-ene-2-carboxylic acid sodium salt ¹H NMR (δ, D₂O) 2.99 (s, 3H), 4.54 (s, 2H), 4.66 (s, 2H), 6.38 (s, 1H), 6.85 (s, 1H), 6.90 (s 1H), 7.30 (s, 1H).
Compound (5*R*),(6*E*)-6-(7-Methyl-6-oxo-5,6,7,8-tetrahydro-imidazo[1,2-a]pyrazin-2-ylmethylene)-7-oxo-4-thia-1-aza-bicyclo[3.2.0]hept-2-ene-2-carboxylic acid sodium salt ¹H NMR (δ, D₂O) 2.94 (s, 3H), 4.45 (s, 2H), 4.56 (s, 2H), 6.22 (s, 1H), 6.48 (s, 1H), 6.94 (s, 1H), 7.69 (s 1H).

### Examples 24

### Preparation of (5R)(6Z)-6-(6,7-Dihydro-4H-pyrazolo[5,1-c][1,4]thiazin-2-ylmethylene)-7-oxo-4-thia-1-azabicyclo[3.2.0]hept-2-ene-2-carboxylic acid, sodium salt:

### Step 1: (3R)-Thiomorpholine-3-carboxylic acid

The titled compound was prepared in the same way of Shiraiwa and co-workers (Biosci. Biotechnol. Biochem. 1998, 62, 2382-2387).

### Step 2: 3-Oxo-3a,4,6,7-tetrahydro-3H-2-oxa-5-thia-1-aza-7a-azonioindenide

NaNO₂ (3.14 g) was added to the 1 mol/L HCl (33.7 mL) solution of (3*R*)-thiomorpholine-3-carboxylic acid (4.96 g) under a nitrogen atmosphere at 0 °C and stirred for 0.5 h. The solution was extracted with CHCl₃ (5 times) and the organic layer was washed with brine. The mixture was dried over MgSO₄ and concentrated under reduced pressure to afford crude (3*R*)-4-nitrosothiomorpholine-3-carboxylic acid as pale yellow crystals.

Trifluoroacetic anhydride (7.07 g) was added to the THF (169 mL) solution of crude (3*R*)-4-nitrosothiomorpholine-3-carboxylic acid under a nitrogen atmosphere at 0 °C and stirred for 3 h at 0 °C and for 17 h at room temperature. The solution was concentrated under a reduced pressure. The residue was applied to silica gel column chromatography, then the column was eluted with n-hexane - AcOEt (1/1 - 0/1). The titled compound was obtained as pale brown crystals (3.41 g, 64%).

¹H NMR (CDCl₃) δ 3.15 (t, 2H, *J* = 5.5 Hz), 3.71 (s, 2H), 4.54 (t, 2H, *J* = 5.5 Hz).

### Step 3:6,7-Dihydro-4H-pyrazolo[5,1-c][1,4]thiazine-2-carboxylic acid ethyl ester

Ethyl propiolate (2.33 g) was added to the o-xylene (72 mL) solution of 3-oxo-3a,4,6,7-tetrahydro-3H-2-oxa-5-thia-1-aza-7a-azonioindenide (3.41 g) under a nitrogen atmosphere and refluxed for 15 h. The solution was concentrated under a reduced pressure. The residue was applied to silica gel column chromatography, then the column was eluted with n-hexane - AcOEt (2/1 - 1/1). The titled compound was obtained as yellow oil (3.13 g, 68%), and the other unwanted regio isomer 6,7-dihydro-4H-pyrazolo[5,1-*c*][1,4]thiazine-3-carboxylic acid ethyl ester was obtained as yellow oil (556 mg, 12%).

¹H NMR (CDCl₃) δ1.31 (t, 3H, *J* = 7.1 Hz), 3.04 (t, 2H *J* = 5.7 Hz), 3.81 (s, 2H), 4.32 (q, 2H, *J* = 7.1 Hz), 4.40 (t, 2H, *J* = 5.7 Hz), 6.54 (s, 1H).

### Step 4: 16,7-Dihydro-4H-pyrazolo[5,1-c][1,4]thiazin-2-yl)methanol

LiBH₄ (cont. 90%) (536 mg) and MeOH (0.9 mL) was added to the THF (59 mL) solution of 6,7-dihydro-4*H-*pyrazolo[5,1-*c*][1,4]thiazine-2-carboxylic acid ethyl ester (3.13 g) under a nitrogen atmosphere at room temperature and stirred for 3 h at 40°C. The mixture was quenched with 1 mol/L HCl at pH 1 and stirred for 1 h at room temperature. Solid K₂CO₃ was added to the solution to adjust pH to 8 and the mixture was extracted with AcOEt. The organic layer was dried (K₂CO₃) and filtered. The filtrate was concentrated under reduced pressure to afford titled compound as pale yellow oil (2.51 g, quant.).

¹H NMR (CDCl₃) δ 2.58 (br, 1H), 3.07 (t, 2H, *J* = 5.7 Hz), 3.84 (s, 2H), 4.33 (t, 2H, *J* = 5.7 Hz), 4.63 (d, 2H, *J* = 3.9 Hz), 6.05 (s, 1H).

### Step 5: 6.7-Dihydro-4H-pyrazolo[5,1-c][1,4]thiazine-2-carbaldehyde

MnO₂ (activated) (11.46 g) was added to the CHCl₃ (135 mL) solution of (6,7-dihydro-4H-pyrazolo[5,1-*c*][1,4]thiazin-2-yl)methanol (2.31 g) and refluxed for 1 h under a nitrogen atmosphere. The reaction mixture was filtered through a pad of Celite. The filtrate was concentrated under a reduced pressure. The residue was applied to silica gel column chromatography, then the column was eluted with n-hexane - AcOEt (1/1). The titled compound was obtained as pale yellow crystals (1.78 g, 78%).

¹H NMR (CDCl₃) δ 3.15 (t, 2H, *J* = 5.8 Hz), 3.90 (s, 2H), 4.48 (t, 2H, *J* =5.8 Hz), 6.58 (s, 1H), 9.92 (s, 1H).

### Step 6: (5R)(6Z)-6-(6,7-Dihydro-4H-pyrazolo[5,1-c][1,4]thiazin-2-ylmethylene)-7-oxo-4-thia-1-azabicyclo[3.2.0]hept-2-ene-2-carboxylic acid, sodium salt

6,7-Dihydro-4*H*-pyrazolo[5,1-*c*][1,4]thiazine-2-carbaldehyde (841 mg) was added to the dry acetonitrile (39 mL) solution of anhydrous MgBr₂ (1.88 g) under a nitrogen atmosphere at room temperature. The dry THF solution (39 mL) of (5R, 6S)-6-bromo-7-oxo-4-thia-1-azabicyclo[3.2.0]hept-2-ene-2-carboxylic acid 4-nitrobenzyl ester (cont. 99.7%) (1.93 g) was added to the mixture, cooled to -20 °C, and Et₃N (2.79 mL) was added in one portion. The reaction vessel was covered with foil to exclude light. The reaction mixture was stirred for 3 h at -20°C and treated with acetic anhydride (0.94 mL) and DMAP (61 mg) in one portion. The reaction mixture was warmed to 0 °C and stirred for 17 h at 0 °C. The mixture was diluted with ethyl acetate and washed with 5% citric acid aqueous solution, saturated sodium hydrogen carbonate, water and brine. The organic layer was dried (MgSO₄) and filtered. The filtrate was concentrated under reduced pressure.

The residue was dissolved in THF (83 mL) and acetonitrile (39 mL). Freshly activated Zn dust (7.72 g) was added rapidly with 0.5 *M* phosphate buffer (pH 6.5, 122 mL). The reaction vessel was covered with foil to exclude light. The reaction mixture was vigorously stirred for 1.5 h at room temperature. The reaction mixture was filtered through a pad of Celite. The filtrate was washed with ethyl acetate and the aqueous layer was separated. The aqueous layer was cooled to 3 °C and 1 *M* NaOH was added to adjust pH to 8.0. The mixture was concentrated under high vacuum at 35°C. The concentrate was applied to Diaion HP-21 (150 mL, Mitsubishi Kasei Co. Ltd.) resin column chromatography. After adsorbing, the column was eluted with H₂O - MeCN (1/0 - 85/15). The combined fractions were concentrated under high vacuum at 35 °C and lyophilized to give the title compound as a yellow amorphous solid (371 mg, 22%, pH 8.0).

Mp 190°C (dec); ¹H NMR (D₂O) δ 3.03 (t, 2H, *J* = 5.7 Hz), 3.75 (s, 2H), 4.22 (t, 2H, *J* = 5.7 Hz), 6.07 (s, 1H). 6.27 (s, 1H), 6.86 (s, 1H), 6.89 (s, 1H).

### Example 25

### Preparation of (5R)(6Z)-7-Oxo-6-(4H-5-thia-1,6a-diazapentalen-2-ylmethylene)-4-thia-1-azabicyclo[3,2,0]hept-2-ene-2-carboxylic acid, sodium salt

### Step 1: 3-Oxo-3a, 4-dihydro-3H, 6H-2-oxa-5-thia-1-aza-6a-azanio-3a-pentalenide

Conc. HCl (15 mL) and NaNO₂ (16.6 g) were added to the H₂O (166 mL) solution of L-thioproline (24.3 g) under a nitrogen atmosphere at 0 °C and stirred for 2 h. The solution was extracted with CH₂Cl₂, organic layer was dried over MgSO₄ and concentrated under reduced pressure to afford the crude N-nitroso derivative as a yellow solid.

Trifluoroacetic anhydride (5.0 mL) was added to the THF (350 mL) solution of crude N-nitrosothioproline under a nitrogen atmosphere at 0 °C and stirred for 5 h at 0 °C. The solution was concentrated under reduced pressure. The residue was applied to silica gel column chromatography, then the column was eluted with n-hexane - AcOEt (1 : 1). The titled compound was obtained as a pale brown solid (4.0 g, 15.1%).

¹H NMR (CDCl₃): δ4.04 (t, 2H, *J* = 1.7 Hz), 5.40 (t, 2H, *J* = 1.7 Hz).

### Step 2: 4H-5-Thia-1,6a-diazapentalen-2-carboxylic acid ethylester

Ethyl propiolate (3.1 mL) was added to the o-xylene (130 mL) solution of 3-oxo-3a, 4-dihydro-3*H*, 6*H*-2-oxa-5-thia-1-aza-6a-azonio-3a-pentalenide (4.0 g) under a nitrogen atmosphere and refluxed for 19 h. The solution was cooled to room temperature and concentrated under reduced pressure. The residue was applied to silica gel column chromatography, then the column was eluted with *n*-hexane - AcOEt (4 : 1). The titled compound was obtained as a yellow solid (2.7 g, 49.3%), and 4*H*-5-thia-1,6a-diazapentalen-3-carboxylic acid ethylester was obtained as pale yellow crystals (1.2 g, 21.7%).

¹H NMR (CDCl₃) δ 1.40 (t, 3H, *J* = 7.1 Hz), 4.11 (d, 2H, *J* = 2.1 Hz), 4.40 (q, 2H, *J* = 7.1 Hz), 5.24 (t, 2H, *J* = 1.6 Hz), 6.61 (s, 1H).

### Step 3: (4H-5-Thia-1,6a-diazapentalen-2-yl)methanol

LiBH₄ (cont. 90%) (459 mg) was added to the ether (126 mL) solution of 4*H*-5-thia-1,6a-diazapentalen-2-carboxylic acid ethylester (2.5 g) and MeOH (0.77 mL) under a nitrogen atmosphere at room temperature, then refluxed for 1.5 h. The mixture was quenched with 1 mol/L HCl (25 mL) and stirred for 1 h at room temperature. The mixture was neutralized by saturated sodium hydrogen carbonate solution and separated. The aqueous layer was extracted with dichloromethane (10 x 25 mL). The organic layer was dried (MgSO₄) and filtered. The filtrate was concentrated under reduced pressure. The residue was applied to silica gel column chromatography, then the column was eluted with AcOEt. The titled compound was obtained as a pale yellow solid (1.7 g, 87.9%).

¹H NMR (CDCl₃) δ 2.95 (t, 1H, *J* = 5.6 Hz), 4.07 (s, 2H), 4.62 (d, 2H, *J* = 5.1 Hz), 5.13 (t, 1H, *J* = 1.6 Hz), 6.04 (s, 1H).

### Step 4: 4H-5-Thia-1,6a-diazapentalen-2-carbaldehyde

The dry dichloromethane (8 mL) solution of dimethylsulfoxide (2.2 mL) was added dropwise to the dry dichloromethane (110 mL) solution of oxalyl chloride (2.0 mL) at -78°C. The reaction mixture was stirred for 15 min at the same temperature. The dry dichloromethane (40 mL) solution of (4*H*-5-thia-1,6a-diazapentalen-2-yl)methanol, (1.7 g) was added dropwise to the reaction mixture at -78°C, and stirring was continued for an additional 15 min. The reaction mixture was allowed to warm to -45°C and stirred for 1 h. Triethylamine (11.3 mL) was added dropwise and the reaction mixture was allowed to warm to 0°C. After 20min, saturated ammonium chloride solution (50 mL) and water (100 mL) were added and separated. The aqueous layer was extracted with AcOEt (3 x 150 mL). The combined organic layers were washed with water (200 mL) and brine (200 mL), dried (MgSO₄) and filtered. The filtrate was concentrated under reduced pressure. The residue was applied to silica gel column chromatography, then the column was eluted with hexane-AcOEt (1 : 1). The titled compound was obtained as a yellow solid (1.7 g, quant.).

¹H NMR (CDCl₃) δ 4.13 (s, 2H), 5.26 (d, 2H, *J* = 1.4 Hz), 6.59 (s, 1H), 9.90 (s, 1H).

### Step 5; (5R)(6Z)-7-Oxo-6-(4H-5-thia-1,6a-diazapentalen-2-ylmethylene)-4-thia-1-azabicyclo[3.2.0]hept-2-ene-2-carboxylic acid, sodium salt

The dry acetonitrile (92 mL) solution of 4H-5-thia-1,6a-diazapentalen-2-carbaldehyde (1.7 g) was added to the dry acetinitrile (92 mL)) solution of MgBr₂ (5.0 g) under a nitrogen atmosphere at room temperature then the mixture was stirred for 10 min. The dry THF (184 mL) solution of p-nitrobenzyl (5R, 6S)-6-bromopenem-3-carboxylate (4.3 g) was added and the mixture was cooled to -20°C then triethylamine (7.4 mL) was added in one portion. The reaction vessel was covered with foil to exclude light. The reaction mixture was stirred for 3 h at -20°C and treated with 4-dimethylamino pyridine (138 mg) and acetic anhydride (2.1 mL) in one portion. The reaction mixture was warmed to 0 °C and stirred for 15 h at 0 °C. The 1mol/L Citric acid aqueous solution (1000 mL) was added to the reaction mixture and the aqueous layer was extracted with ethyl acetate (3 x 400 mL). The combined organic layers were washed with water, saturated sodium hydrogen carbonate and brine, dried (MgSO₄) and filtered. The filtrate was concentrated under reduced pressure and crude (5R)-6-[acetoxy-(4*H*-5-thia-1,6a-diazapentalen-2-yl)methyl]-6-bromo-7-oxo-4-thia-1-azabicyclo [3.2.0]hept-2-ene-2-carboxylic acid p-nitrobenzyl ester was obtained as a brown amorphous solid.

Freshly activated Zn dust (19.3 g) was added rapidly with 0.5 mol/L phosphate buffer (pH 6.5, 100 mL) to the THF (100 mL) solution of crude (5*R*)-6-[acetoxy-(4*H*-5-thia-1,6a-diazapentalen-2-yl)methyl]-6-bromo-7-oxo-4-thia-1-azabicyclo [3.2.0]hept-2-ene-2-carboxylic acid *p*-nitrobenzyl ester. The reaction vessel was covered with foil to exclude light. The reaction mixture was vigorously stirred for 2.5 h at room temperature. The reaction solution was filtered through a pad of Celite and the pad was washed with water (200 mL) and n-butanol (200 mL). The aqueous layer was separated and then the organic layer was extracted with 0.5 mol/L phosphate buffer (pH 6.5, 2 x 50 mL). The combined aqueous layers were concentrated to 90 g, 1mol/L NaOH was added to adjust pH to 8.0 and applied to Diaion HP-21 resin (180 mL, Mitsubishi Kasei Co. Ltd.) column chromatography. After adsorbing, the column was eluted with water and then 15% acetonitrile aqueous solution. The combined active fractions were concentrated under high vacuum at 35°C and lyophilized to give the title compound as a yellow amorphous solid (634 mg, 17.4%, pH 7.25).

Mp 150°C (dec); ¹H NMR (D₂O) δ4.00 (s, 2H), 5.09 (s, 2H), 6.14 (s, 1H), 6.36 (s, 1H), 6.91 (s, 1H), 6.92 (s, 1H); IR (KBr) 3381, 1752, 1683, 1600, 1558 cm⁻¹; λ^{max}(H₂O) 292, 196 nm.

### Example 26

### Preparation of (5R)(6Z)-6-(7H-Imidazo[1,2-c]thiazol-2-ylmethylene)-7-oxo-4-thia-1-azabicyclo[3.2.0]hept-2-ene-2-carboxylic acid, sodium salt

### Step 1: Thiazolidin-4-one

The titled compound was prepared in the same way of Marvin M. and Allen R. Harkness. (Tetrahedron Letters. 1994, 35, 6971-6974).

### Step 2: Thiazolidine-4-thione

Lawesson's reagents (33.5 g) added to the solution of thiazolidin-4-one (14.2 g) in dry THF (690 mL) and the reaction mixture was refluxed for 2 h. The mixture was cooled to room temperature and evaporated under reduced pressure. The residue was triturated with CHCl₃:MeOH=7:3 solution (65 mL) at room temperature for 30 min. The precipitate was filtered off, washed with CHCl₃:*n*-hexane=7:3 solution (15 mL) and dried in vacuo. The thiazolidine-4-thione was obtained as a pale yellow powder (10.7 g, 65%).

¹H NMR (CDCl₃) δ 4.08 (s, 2H), 4.70 (s, 2H).

### Step 3: 4-Methylthio-2,5-dihydro-thiazole

Methyl iodide (28.4 g) was added to the boiling solution of thiazolidine-4-thione (9.5 g) in chloroform (400 mL), and the reaction mixture was refluxed for 1.5 h. To the reaction mixture, an additional methyl iodide (56.8 g) was added in 5 portions at 30-60 min intervals. After refluxing for additional 1 h, the reaction mixture was cooled to room temperature. Then 10% potassium carbonate aqueous solution (200 mL) was added and stirred for 15min at room temperature. After separating organic layer, the aqueous layer was extracted with CHCl₃ (100 mL x 3). Organic layers were combined, dried (MgSO₄) and filtered. The filtrate was concentrated under reduced pressure and dried in vacuo. After drying, the title compound was obtained as brown oil (11.0 g, quant.).

¹H NMR (CDCl₃) δ 2.51 (s, 3H), 3.91 (t, 2H, J = 3.5 Hz), 5.21 (t, 2H, J = 3.5 Hz).

### Step 4: Thiazolidin-4-ylideneamine

A mixture of 4-methylthio-2,5-dihydrothiazole (10.7 g) and ammonium chloride (6.4 g) in dry ethanol (400 mL) was refluxed for 27.5 h. The reaction mixture was cooled to room temperature and evaporated under reduced pressure. The residue was dissolved in chloroform (300 mL) and 10% potassium carbonate aqueous solution (200 mL), then stirred for 20 min at room temperature. After separating organic layer, the aqueous layer was extracted with chloroform (100 mL x 5). Organic layers were combined, dried (MgSO₄) and filtered. The filtrate was concentrated under reduced pressure and dried in vacuo to obtain crude thiazolidin-4-ylideneamine (5.5g) as a brown solid that included by product, which is an ethoxy derivative and 4-methylthio-2,5-dihydrothiazole, which is the starting material. The ratio of these three compounds was determined to be 61:34:5 respectively by ¹H-NMR.

¹H NMR (CDCl₃) δ 3.75 (t, 2H, *J* = 2.8 Hz), 4.97 (t, 2H, *J* = 2.9 Hz).

### Step 5: 7H-Imidazo[1,2-c]thiazole-2-carbaldehyde

The solution of 2-bromo-3-isopropoxypropenal (6.9 g) in dry acetonitrile (326 mL) was added to the solution of crude thiazolidin-4-ylideneamine (3.3 g) in dry acetonitrile (326 mL) at room temperature. The reaction mixture was stirred at room temperature for 19.5 h, added triethylamine (4.9 mL) and then refluxed for 2 h. The reaction mixture was cooled to room temperature and then evaporated under reduce pressure. The residue was dissolved in dichloromethane (300 mL) and washed with 50% potassium carbonate aqueous solution (20 g). After filtration and separation, the aqueous layer was extracted with dichloromethane (50 mL x 4). The organic layers were combined, dried (MgSO₄) and filtered. The filtrate was evaporated under reduced pressure. The residue was applied to silica gel column chromatography and eluted with CHCl₃ - MeOH (100 : 3) to obtain crude 7*H*-Imidazo[1,2-c]thiazole-2-carbaldehyde as a brown solid. The crude product was re-crystallized twice from CHCl₃ - n-hexane (1^{st}: 30:5, 2^{nd}: 30:60) at 0 °C to give the required aldehyde as pale brown crystals (Yield: 1.84 g, 15 %).

¹H NMR (CDCl₃) δ 4.09 (t, 2H, *J* = 1.3 Hz), 5.08 (t, 2H, *J* = 1.2 Hz), 7.63 (s, 1H), 9.81 (s, 1H).

### Step 6: (5R)(6Z)-6-(7H-Imidazo[1,2-c]thiazol-2-ylmethylene)-7-oxo-4-thia-1-azabicyclo [3.2.0]hept-2-ene-2-carboxylic acid, sodium salt

7*H*-Imidazo[1,2-c]thiazole-2-carbaldehyde (841 mg) was added to the dry acetonitrile (116 mL) solution of anhydrous MgBr₂ (2.93 g) under a nitrogen atmosphere at room temperature. The dry THF solution (116 mL) of (5*R*, 6*S*)-6-bromo-7-oxo-4-thia-1-azabicyclo[3.2.0]hept-2-ene-2-carboxylic acid 4-nitrobenzyl ester (cont. 99.7%) (2.51 g) was added to the mixture, cooled to -20 °C, and Et₃N (2.20 mL) was added in one portion. The reaction vessel was covered with foil to exclude light. The reaction mixture was stirred for 4 h at -20 °C and treated with acetic anhydride, (1.26 mL) and DMAP (160 mg) in one portion. The reaction mixture was warmed to 0 °C and stirred for 15 h at 0 °C. The mixture was diluted with ethyl acetate and washed with 5% citric acid aqueous solution, saturated sodium hydrogen carbonate, water and brine. The organic layer was dried (MgSO₄) and filtered. The filtrate was concentrated under reduced pressure.

The residue was dissolved in THF (53 mL) and acetonitrile (25 mL). Freshly activated Zn dust (15.1 g) and 0.5 M phosphate buffer (pH 6.5, 78 mL) were added to the mixture. The reaction vessel was covered with foil to exclude light. The reaction mixture was vigorously stirred for 1.5 h at room temperature. The reaction mixture was filtered through a pad of Celite. The filtrate was washed with ethyl acetate and the aqueous layer was separated. The aqueous layer was cooled to 3 °C and 1 M NaOH was added to adjust pH to 8.0. The mixture was concentrated under high vacuum at 35 °C. The concentrate was applied to Diaion HP-21 (321 mL, Mitsubishi Kasei Co. Ltd.) resin column chromatography. After adsorbing, the column was eluted with H₂O - MeCN (1/0 - 9/1). The combined fractions were concentrated under high vacuum at 35 °C and lyophilized to give the title compound as a yellow amorphous solid (1.1 g, 51%, pH 7.5).

Mp 145 °C (dec); ¹H NMR (D₂O) δ 3.85 (s, 2H), 4.88 (s, 2H), 6.32 (s, 1H), 6.78 (s, 1H), 6.85 (s, 1H), 7.27 (s, 1H).

### Example 27

### Preparation of (5R,6Z)-7-oxo-6-[(4-oxo-6,7-dihydro-4H-pyrazolo[5,1-c][1,4]oxazin-2-yl)methylene]-4-thia-1-azabicyclo[3.2.0]hept-2-ene-2-carboxylic Acid.

### Step 1: Diethyl 1-(2-{[tert-butyl(dimethyl)silyl]oxy}ethyl)-1H-pyrazole-3,5-dicarboxylate

To a solution of diethyl 3,5-pyrazoledicarboxylate (2.17 g, 10 mmol) in acetonitrile (10 ml), under nitrogen, was added potassium carbonate (2.07 g, 15 mmol), and 2-bromoethoxy-t-butyldimethylsilane (2.90 g, 12 mmol). The mixture was stirred at reflux for 18 hr. It was then cooled to room temperature, diluted with ethyl acetate (20 ml), and filtered through Magnesol. The filter pad was eluted wtih 2 x 10 ml of ethyl acetate, and the combined filtrate was evaporated. The residue was dissolved in hexanes and passed through a column of silica gel (70 g). After eluting with hexanes (100 ml), the column was eluted with ethyl acetate. The ethyl acetate eluent was evaporated to give 3.71 g of a colorless oil; MS m/e 371 (MH⁺).

### Step 2: 1-(2-{[tert-butyl(dimethyl)silyl]oxy}ethyl)-1H-pyrazole-3,5-dimethanol

To a solution of diethyl 1-(2-{[tert-butyl(dimethyl)silyl]oxy}ethyl)-1H-pyrazole-3,5-dicarboxylate (0.74 g, 2 mmol) in methylene chloride (8 ml), under nitrogen, was added 12 ml of a 1.0 M solution of diisobutylaluminum hydride in methylene chloride at 0 °C. After stirring at 0 °C for 0.5 hr, the mixture waas warmed to room temperature for 0.5 hr. It was then quenched with 15 ml of saturated ammonium chloride solution and extracted with ethyl acetate. The organic extract was washed with brine, dried over anhydrous sodium sulfate, and evaporated to give 0.44 g of a white solid; mp 82-83 °C; MS m/e 287 (MH⁺).

### Step 3: 1-(2-{[tert-butyl(dimethyl)silyl]oxy}ethyl)-1H-pyrazole-3,5-dicarbaldehyde

To a stirred solution of 1-(2-{[tert-butyl(dimethyl)silyl]oxy}ethyl)-1H-pyrazole-3,5-dimethanol (1.18 g, 4 mmol) in methylene chloride (20 ml), was added 4-methylmorpholine-N-oxide (2.89 g, 24 mmol) and molecular sieve 4A (4 g). The reaction mixture was stirred at room temperature for 10 min. and then treated with tetrapropylammonium peruthenate (0.15 g, 0.4 mmol). Stirring was continued for 2 hr. The methylene chloride solution was concentrated and diluted with ether (40 ml). The mixture was filtered through a pad of silica gel (40 g) and the filter pad was eluted with 2 x 20 ml ether. The combined eluent was washed with 1N HCl and brine, dried over anhydrous sodium sulfate, and evaporated to give 0.79 g of a white solid; mp 63-64 °C; MS m/e 283 (MH⁺).

### Step 4: 4-oxo-6,7-dihydro-4H-pyrazolo[5,1-c][1,4]oxazine-2-carbaldehyde

To a solution of 1-(2-{[tert-butyl(dimethyl)silyl]oxy}ethyl)-1H-pyrazole-3,5-dicarbaldehyde (1.02 g, 6.07 mmol) in THF (30 ml) was added 6.68 ml of a 1.0 M solution of tetrabutylammonium fluoride in THF at 0 °C. After stirring for 1 hr, the mixture was treated with 10 ml of saturated ammonium chloride solution and extracted with ethyl acetate. The organic solution was washed with brine, dried over anhydrous sodium sulfate, filtered through Magnesol and evaporated. The crude gum was washed with hexanes, dried in vacuo, and then dissolved in methylene chloride (20 ml). To this solution was added 4-methylmorpholine-N-oxide (2.89 g, 24 mmol) and molecular sieve 4A (6 g). The mixture was stirred at room temperature for 10 min and then treated with tetrapropylammonium peruthenate (0.11 g, 0.3 mmol). Stirring was continued for 2 hr. The methylene chloride solution was concentrated and diluted with ethyl acetate (40 ml). The mixture was filtered through a pad of silica gel (40 g) and the filter pad was eluted with 2 x 20 ml ethyl acetate. The combined eluent was washed with 1 N HCl and brine, dried over anhydrous sodium sulfate, and evaporated to give 0.30 g of a white solid; mp 135-136 °C; MS m/e 167 (MH⁺).

### Step 5: 4-nitrobenzyl (5R)-6-[(acetyloxy)(4-oxo-6,7-dihydro-4H-pyrazolo[5,1-c][1,4]oxazin-2-yl)methyl]-6-bromo-7-oxo-4-thia-1-azabicyclo[3.2.0]hept-2-ene-2-carboxylate

To a solution of MgBr₂ (0.46 g, 2.52 mmol) in acetonitrile (13 ml) under nitrogen was added 4-oxo-6,7-dihydro-4H-pyrazolo[5,1-c][1,4]oxazine-2-carbaldehyde (0.14 g, 0.84 mmol) at room temperature with stirring. A solution of (5R,6S)-6-bromo-7-oxo-4-thia-1-azabicyclo[3.2.0]hept-2-ene-2-carboxylic acid 4-nitrobenzyl ester (0.32 g, 0.84 mmole) in THF (13 ml) was then added, and the mixture was cooled to -20 °C. Triethylamine (0.35 ml, 2.52 mmol) was introduced, and the mixture was stirred at -20°C in the dark for 4 hr. It was then treated with acetic anhydride (0.2 ml, 2.0 mmol), and 4-N,N-dimethylaminopyridine (12 mg, 0.1 mmol), and kept at 0 °C for 18 hr. The mixture was concentrated and the residue was dissolved in ethyl acetate. The ethyl acetate solution was washed with 5% citric acid, saturated sodium bicarbonate solution, and brine, dried over anhydrous sodium sulfate, and evaporated. The crude material was chromatographed with silica gel (EtOAc-CH2Cl2/1:5) to give 0.27 g of an off-white solid; mp 107-110 °C; MS m/e 595 (MH⁺).

### Step 6: (5R,6Z)-7-oxo-6-[(4-oxo-6,7-dihydro-4H-pyrazolo[5,1-c][1,4]oxazin-2-yl)methylene]-4-thia-1-azabicyclo[3.2.0]hept-2-ene-2-carboxylic Acid

To a solution of 4-nitrobenzyl (5R)-6-[(acetyloxy)(4-oxo-6,7-dihydro-4H-pyrazolo[5,1-c][1,4]oxazin-2-yl)methyl]-6-bromo-7-oxo-4-thia-1-azabicyclo[3.2.0]hept-2-ene-2-carboxylate (0.22 g, 0.37 mmol) in THF (15 ml), under nitrogen, was added 15 ml of a phosphate buffer solution (0.5M, pH 6.5), and 80 mg of 10% Pd/C. The mixture was hydrogenated at 40-50 psi for3 hr, and then filtered through Celite. The filter pad was washed with THF, and the filtrate was extracted with ethyl acetate. The organic extract was dried over anhydrous magnesium sulfate and evaporated. The residue was washed with ether to give 0.07 g of a yellow solid; MS m/e 320 (MH⁺); ¹H NMR (DMSO-d₆) δ 4.55-4.57 (m, 2H), 4.76-4.80 (m, 2H), 6.50 (s, 1H), 6.63 (s, 1H). 7 58 (s, 1H), 7.76 (s, 1H).

### Example 28

### Preparation of 6-(6,7-Dihydro-4H-thieno[3,2-c]pyran-2-ylmethylene)-7-oxo-4-thia-1-aza-bicyclo[3.2.0]hept-2-ene-2-carboxylic acid

### Step 1: Preparation of 6,7-Dihydro-4H-thieno[3,2-c]pyran-2-carbaldehyde

POCl3 (3.83ml, 50mmol) was added dropwise to ice cooled DMF (3.85ml, 50mmol) within 3 minutes. DCM (20ml) was added and the bath was removed when the reaction media appeared to be pasty. The reaction was kept at 23oC for 2 hrs. Then it was cooled to 0oC again. 4H-pyran-4-one (5 gram, 50mmol) in 10ml DCM was then added dropwise within 3 minutes. The reaction was kept at 0oC for 2 hrs. Pour the mixture onto ice and sodium acetate solution and extract with DCM (2x200). The combined organic layers were dried over magnesium sulfate. Filter off the drying agent and concentrate gave 5.0 gram of product. The compound was dissolved in DCM (200ml) and was added 6.0 gram of ethyl 2-6,7-Dihydro-4H-thieno[3,2-c]thiopyran-2-carbaldehyde-acetate and 10 ml TEA. The mixture was refluxed for 18 hrs. Then it was washed with water and dried over magnesium sulfate. It was then filtered, concentrated and flash chromatographed with 20 ethyl acetate in hexane. The collected material was dissolved in 100ml THF and LAH (150ml, 0.5M in THF) was injected and left at 23oC for 10 minutes. Then it was refluxed for 18 hrs. Quenched at 23oC by adding water and eventually 1 N HCl to clear up the mixture. Extract with ethyl acetate (2x200ml) and combined organic layers dried over magnesium sulfate. Filter and concentrate gave 2.3gram product. The crude material was dissolved in DCM (300ml) and manganese dioxide (15 gram was added). The reaction was carried on at 23oC for 0.5 hr. Then 2x15 gram of oxidant was added each half an hour later. The material was then filtered through a pad of celite concentrated. Flash column chromatography gave 1.206gram (14% yield) oil product.
H-NMR:δ 9.84(s, 1H), 7.41 (s, 1H), 4.74 (s, 2H), 4.00 (t, 2H, J=5.6 Hz), 2.96 (t, 2H, J=5.6Hz); MS: 169.1(M+H)

### Step 2: Preparation of 6-(6,7-Dihydro-4H-thieno[3,2-c]pyran-2-ylmethylene)-7-oxo-4-thia-1-aza-bicyclo[3.2.0]hept-2-ene-2-carboxylic acid

6,7-Dihydro-4H-thieno[3,2-c]pyran-2-carbaldehyde (336mg, 2mmol) was dissolved in 20ml acetonitrile and magnesium bromide (516 mg, 2mmol) was then added under N2 atmosphere. The mixture was stirred at 23oC for half an hour. 6-Bromo-7-oxo-4-thia-1-aza-bicyclo[3.2.0]hept-2-ene-2-carboxylic acid 4-nitro-benzyl ester (770mg, 2mmol) in 20ml THF was then injected all at once and the mixture was immediately cooled to - 20oC/ Triethylamine (1ml) was then injected and the mixture stirred at -20oC for three hrs. Then acetic anhydride (0.4ml) was injected and the mixture was stirred at 0oC for 18 hrs. The reaction media was then diluted with 400ml ethyl acetate and washed with 100 ml 5% citric acid, 100 ml saturated sodium bicarbonate, and 100ml brine. The organic layer was then dried over magnesium sulfate, filtered and concentrated. Flash column chromatography using 20% ethyl acetate in hexane gave 491 mg (41%) product. This product was then dissolved in 15ml THF and 15ml aqueous phosphate buffer (pH=6.5). The mixture was then subjected to 45psi hydrogen for one hour with 0.5gram 10% palladium on carbon. Then it was filtered through a pad of celite and concentrated in vacuo to remove most of the THF. The solution was then cooled to zero degree and basified to pH=8 with 1 N sodium hydroxide. Then it was purified via reverse phase HPLC using 2 liter of water followed by 5% acetonitrile in water. Water was then removed through concentrate in vacuo and 100 mg (38%) of product was collected; MP: >250° C;
H-NMR: δ 7.36 (s, 1H), 7.15(s, 1H), 6.55(s, 1H), 6.44(s, 1H), 4.61 (s, 2H), 3.88(m, 2H), 2.86 (m, 2H), 2.27 (m, 2H), 1.43 (t, 3H)
MS: 320.3(M-H)

### Example 29

### Preparation of 6-(6,7-Dihydro-4H-thieno[3,2-c]thiopyran-2-ylmethylene)-7-oxo-4-thia-1-aza-bicyclo[3.2.0]hept-2-ene-2-carboxylic acid

### Step 1: Preparation of 6,7-Dihydro-4H-thieno[3,2-c]thiopyran-2-carbaldehyde

POCl3 (4.02ml, 43mmol) was added dropwise to ice cooled DMF (3.34ml, 43mmol) within 3 minutes. DCM (20ml) was added and the bath was removed when the reaction media appeared to be pasty. The reaction was kept at 23oC for 2 hrs. Then it was cooled to 0oC again. Tetrahydro-thiopyran-4-one (5 gram, 43mmol) in 10ml DCM was then added dropwise within 3 minutes. The reaction was kept at 0oC for 2 hrs. Dilute with DCM (250 ml) and then wash with ice cold 200ml saturated sodium acetate aqueous solution. The organic layer was dried over sodium sulfate. Filter off the drying agent, concentrate and flash column chromatography using 10% ethyl acetate in hexane gave 1.3 gram (8mmol) of product. The compound was dissolved in DCM (100ml) and was added 1.2ml (11 mmol) of ethyl 2-mercapto-acetate and 1ml TEA. The mixture was refluxed for 18 hrs. Then it was washed with water and dried over magnesium sulfate. Filter, concentrate and flash chromatograph with 20 ethyl acetate in hexane produced 1.1 gram (11 % yield) of product
H-NMR:δ 6.68(s, 1H), 4.73 (s, 2H), 3.68(s, 2H), 3.04 (t, 2H, J=7.6 Hz), 2.91 (t, 2H, J=7.6Hz).; MS (EI): 185.99 (M+)

The 1.1 gram (4.8mmol) 6,7-Dihydro-4H-thieno[3,2-c]thiopyran-2-carboxylic acid ethyl ester was dissolved in 100ml THF and LAH (40ml, 0.5M in DMG) was injected and the reaction was left at 23oC for 10 minutes. Then it was refluxed for 18 hrs. Quenched at 23oC with water (10ml). The organic layer decanted and the remaining was washed with 20ml DCM. The combined organic layers dried over sodium sulfate. Filter, concentrate and flash column chromatograph with 10-20% ethyl acetate produced 940mg crude product. This crude material was dissolved in DCM (40ml) and manganese dioxide (2 gram was added). The reaction was carried on at 23oC for half an hour. The material was then filtered through a pad of celite concentrated. Flash column chromatography gave 320mg (36%) product.
H-NMR:δ 9.82(s, 1H), 7.46 (s, 1H), 3.56 (s, 2H), 3.15 (t, 2H, J=7.2 Hz), 2.95 (t, 2H, J=7.2 Hz).; MS (EI): 228.02 (M+)

### Step 2: Preparation of 6-(6,7-Dihydro-4H-thieno[3,2-c]thiopyran-2-ylmethylene)-7-oxo-4-thia-1-aza-bicyclo[3.2.0]hept-2-ene-2-carboxylic acid

6,7-Dihydro-4H-thieno[3,2-c]thiopyran-2-carbaldehyde (320mg, 1.72mmol) was dissolved in 17ml acetonitrile and magnesium bromide etherate (450 mg, 1.74mmol) was then added under N2 atmosphere. The mixture was stirred at 23oC for half an hour. 6-Bromo-7-oxo-4-thia-1-aza-bicyclo[3.2.0]hept-2-ene-2-carboxylic acid 4-nitrobenzyl ester (660mg, 1.72mmol) in 17ml THF was then injected all at once and the mixture was immediately cooled to -20oC/ Triethylamine (1ml) was then injected and the mixture stirred at -20oC for three hrs. Then acetic anhydride (0.4ml) was injected and the mixture was stirred at 0oC for 18 hrs. The reaction media was then diluted with 400ml ethyl acetate and washed with 100 ml 5% citric acid, 100 ml saturated sodium bicarbonate, and 100ml brine. The organic layer was then dried over magnesium sulfate, filtered and concentrated. Flash column chromatography using 20% ethyl acetate in hexane gave 461 mg (44%) product. This product was then dissolved in 20ml THF and 20ml aqueous phosphate buffer (pH=6.5). The mixture was then subjected to 40psi hydrogen for one hour and half with 0.5gram 10% palladium on carbon. Then it was filtered through a pad of celite and concentrated in vacuo to remove most of the THF. The solution was then cooled to zero degree and basified to pH=8 with 1 N sodium hydroxide. Then it was purified via reverse phase HPLC using 2 liter of water followed by 5% acetonitrile in water. Water was then removed through concentrate in vacuo and 21 mg (8.6%) of product was collected.
MP: >250° C
H-NMR: 7.34 (s, 1H), 7.18(s, 1H), 6.59(s, 1H), 6.44(s, 1H), 3.71 (s, 2H), 2.93(s, 2H), 2.50 (s, 2H).; MS: 338.0(M+H)

### Example 30

### Preparation of 6-(5-Methyl-4,5,6,7-tetrahydro-thieno[3,2-c]pyridin-2-ylmethylene)-7-oxo-4-thia-1-aza-bicyclo[3.2.0]hept-2-ene-2-carboxylic acid

### Step 1: Preparation of (5-Methyl-4,5,6,7-tetrahydro-thieno[3,2-c]pyridin-2-yl)-methanol

6,7-Dihydro-4H-thieno[3,2-c]pyridine-2,5-dicarboxylic acid diethyl ester (46 gram, 163mmol) was dissolved in 200ml THF. The solution was injected LAH (1 M, THF) 300ml at 23oC. Then it was stirred at 23oC for 18 hrs. The reaction was quenched with 10ml water and dried directly over sodium sulfate. Filter and concentrate yielded 29.3 gram (160mmol, 98%) crude product.
H-NMR: 6.55(s, 1H), 4.70 (s, 2H), 3.41 (s, 2H), 2.86 (t, 2H, J=5.6 Hz), 2.73 (t, 2H, J=5.6 Hz), 2.38 (s, 3H); MS: 184.0(M+H)

### Step 2: Preparation of 5-Methyl-4,5,6,7-tetrahydro-thieno[3,2-c]pyridine-2-carbaldehyde

DMSO (1.7ml, 24mmol) in 5ml CH₂Cl₂ was cooled to -50-60oC. Oxalyl chloride (1ml, 11mmol) in 20ml DCM was then added within 5 minutes at 50oC. The mixture was kept at -50oC for 5 minutes and then 1.67 gram (9mmol) of (5-Methyl-4,5,6,7-tetrahydro-thieno[3,2-c]pyridin-2-yl)-methanol in 20ml DCM was added at 50oC and the mixture was stirred for another 15minutes at 50oC. Triethylamine (7ml) was then added at - 50oC and after 5 minutes the bath was removed and the mixture is naturally warmed up to 23oC. It was washed with 100ml water and extracted with 100ml ethyl acetate. The combined organic layers were dried over magnesium sulfate. Filter. Concentrate and flash column chromatograph using 0-15% methanol in ethyl acetate yielded 736mg (45% yield) product.
H-NMR: 9.81 (s, 1H), 7.42 (s, 1H), 3.56 (s, 2H), 3.00 (t, 2H, J=5.6 Hz), 2.91 (t, 2H, J=5.6 Hz), 2.51 (s, 3H); MS: 182.1(M+H)

### Step 3: Preparation of 6-(5-Methyl-4,5,6,7-tetrahydro-thieno[3,2-c]pyridin-2-ylmethylene)-7-oxo-4-thia-1-aza-bicyclo[3.2.0]hept-2-ene-2-carboxylic acid

2-formyl-6,7-dihydro-4H-thieno[3,2-c]pyridine-5-carboxylic acid ethyl ester (724mg, 4mmol) was dissolved in 40ml acetonitrile and magnesium bromide etherate (1.2 gram, 4.65mmol) was then added under N2 atmosphere. The mixture was stirred at 23oC for half an hour. 6-Bromo-7-oxo-4-thia-1-aza-bicyclo[3.2.0]hept-2-ene-2-carboxylic acid 4-nitro-benzyl ester (1.54gram, 4mmol) in 40ml THF was then injected all at once and the mixture was immediately cooled to -20oC. Triethylamine (2ml) was then injected and the mixture stirred at -20oC for 3 hrs. Then acetic anhydride (0.66ml) was injected and the mixture was stirred at 0oC for 48 hrs. The reaction media was then diluted with 500ml ethyl acetate and washed with 50 ml 5% citric acid, 50 ml saturated sodium bicarbonate, and 50ml brine. Another 300ml ethyl acetate was used to wash each aqueous solution. The combined organic layers were then dried over sodium sulfate. Filter, concentrate, and flash column chromatograph using 20% ethyl acetate in hexane gave 1.56 gram (64% yield) product. This product was then dissolved in 20ml THF and 20ml aqueous phosphate buffer (pH=6.5). The mixture was then subjected to 40psi hydrogen for two hrs with 0.5gram 10% palladium on carbon. Then it was filtered through a pad of celite and concentrated in vacuo to remove most of the THF. The solution was then cooled to zero degree and basified to pH=8 with 1 N sodium hydroxide. Then it was purified via reverse phase HPLC using 2 liter of water followed by 5% acetonitrile in water. Water was then removed through concentrate in vacuo and 112 mg (13%) of product was collected.
MP: >250oC
H-NMR: δ 7.48 (s, 1H), 7.37(s, 1H), 7.21 (s, 1H), 7.10(s, 1H), 3.41 (s, 2H), 2.88 (s, 2H), 2.68(s, 2H), 2.37(s, 3H); MS: 335.0(M+H)

### Example 31

### Preparation of 2-(2-Carboxy-7-oxo-4-thia-1-aza-bicyclo[3.2.0]hept-2-en-6-ylidenemethyl)-6,7-dihydro-4H-thieno[3,2-c]pyridine-5-carboxylic acid ethyl ester

2-Formyl-6,7-dihydro-4H-thieno[3,2-c]pyridine-5-carboxylic acid ethyl ester (480mg, 2mmol) was dissolved in 20ml acetonitrile and magnesium bromide etherate (516mg, 2mmol) was then added under N2 atmosphere. The mixture was stirred at 23oC for half an hour. 6-Bromo-7-oxo-4-thia-1-aza-bicyclo[3.2.0]hept-2-ene-2-carboxylic acid 4-nitrobenzyl ester (770mg, 2mmol) in 20ml THF was then injected all at once and the mixture was immediately cooled to -20oC. Triethylamine (1ml) was then injected and the mixture stirred at -20oC for 3 hrs. Then acetic anhydride (0.4ml) was injected and the mixture was stirred at 0oC for 48 hrs. The reaction media was then diluted with 200ml ethyl acetate and washed sequentially with 50 ml 5% citric acid, 50 ml saturated sodium bicarbonate, and 50ml brine. The organic layer was then dried over sodium sulfate. Filter, concentrate, and flash column chromatograph using 20% ethyl acetate in hexane gave 690mg (50%, yield) product. A fraction of this product (456mg, 0.69mmol) was then dissolved in 15ml THF and 15ml aqueous phosphate buffer (pH=6.5). The mixture was then subjected to 40psi hydrogen for two hrs with 0.5gram 10% palladium on carbon. Then it was filtered through a pad of celite and concentrated in vacuo to remove most of the THF. The solution was then cooled to zero degree and basified to pH=8 with 1 N sodium hydroxide. Then it was purified via reverse phase HPLC using 2 liter of water followed by 5% acetonitrile in water. Water was then removed through concentrate in vacuo and 18 mg (5%) of product was collected.
MP: >250oC
H-NMR: 7.35 (s, 1H), 7.24 (s, 1H), 6.61 (s, 1H), 6.45(s, 1H), 4.48 (s, 2H), 4.08 (quartet, 2H, J=7.2Hz), 3.68 (m, 2H), 2.87(m, 2H), 1.20 (t, 3H, J=7.2Hz); MS: 393.0(M+H)

### Example 32

### Preparation of 7-Oxo-6-(6,7,8,9-tetrahydro-5H-imidazo[1,2-a]azepin-2-ylmethylene)-4-thia-1-aza-bicyclo[3.2.0]hept-2-ene-2-carboxylic acid

### Step 1: Preparation of 6,7,8,9-Tetrahydro-5H-imidazo[1,2-a]azepine-2-carbaledehyde

Thiocaprolactam (6.45 gram, 50mmol) was dissolved in 400ml CH₂Cl₂ and methyl iodide (16ml, 5eq) was next added. The mixture was stirred under nitrogen for 18 hrs. Then it was treated with 100ml potassium carbonate (50%, aq.). The organic layer was then dried over magnesium sulfate. After filtration and concentration 7.3 gram of material was obtained. This material was dissolved 300ml ethanol and 2.83 gram of ammonium chloride was added. The mixture was refluxed for 1 hr. Then the solvent was removed in vacuo. Half of the material was added 200ml ethanol and then followed by addition of 1.35gram (25mmol) sodium methoxide and 4.8gram (25mmol) 2-Bromo-3-isopropoxy-propenal and the mixture was stirred at 23oC for 2 hrs. Then the solvent was removed and 200ml chloroform was added along with 10ml triethyl amine. The mixture was refluxed for 2 hrs and then cooled to 23oC. The reaction media was partitioned between 300ml DCM and 2x150 potassium carbonate (50%). The organic layer was dried over magnesium sulfate. After filtration and concentration 2.1 gram of oil product was obtained.
H-NMR: 9.62 (s, 1H), 7.60 (s, 1H), 6.61 (s, 1H), 6.45(s, 1H), 4.58 (s, 2H), 2.96 (2m, H), 1.90(m, 2H), 1.72 (m, 2H); MS: 164.9(M+H)

### Step 2: Preparation of 7-Oxo-6-(6,7,8,9-tetrahydro-5H-imidazo[1,2-a]azepin-2-ylmethylene)-4-thia-1-aza-bicyclo[3.2.0]hept-2-ene-2-carboxylic acid

6,7,8,9-Tetrahydro-5H-imidazo[1,2-a]azepine-2-carbaldehyde (1.312gram, 8mmol) was dissolved in 80ml acetonitrile and magnesium bromide etherate (2.94gram, 8mmol) was then added under N2 atmosphere. The mixture was stirred at 23oC for half an hour. 6-Bromo-7-oxo-4-thia-1-aza-bicyclo[3.2.0]hept-2-ene-2-carboxylic acid 4-nitro-benzyl ester (1.155gram, 3mmol) in 60ml THF was then injected all at once and the mixture was immediately cooled to -20oC. Triethylamine (4ml) was then injected and the mixture stirred at -20oC for 4 hrs. Then acetic anhydride (1ml) was injected and the mixture was stirred at 0oC for 20 hrs. The reaction media was then diluted with 500ml ethyl acetate and washed with 100m 5% citric acid, 100 ml saturated sodium bicarbonate, and 100ml brine. The organic layer was then dried over sodium sulfate. Filter, concentrate, and flash column chromatograph using 20% ethyl acetate in hexane gave 800mg product. This product was then dissolved in 20ml THF and 20ml aqueous phosphate buffer (pH=6.5). The mixture was then subjected to 40psi hydrogen for 1 hr with 0.5gram 10% palladium on carbon. Then it was filtered through a pad of celite and concentrated in vacuo to remove most of the THF. The solution was then cooled to zero degree and basified to pH=8 with 1 N sodium hydroxide. Then it was purified via reverse phase HPLC using 2 liter of water followed by 5% acetonitrile in water. Water was then removed through concentrate in vacuo and 131 mg (31%) of product was collected.
MP: >250° C
H-NMR: δ 7.78 (s, 1H), 7.02 (s, 1H), 6.94 (s, 1H), 6.36 (s, 1H), 3.92(m, 2H), 2.80 (m, 2H), 1.78 (m, 2H), 1.61(m, 2H), 1.54(m, 2H); MS: 318.2(M+H).

### Example 33

### (SR),(6Z)-6-(7-Benzyl-5,6,7,8-tetrahydroimidazo[1,2-a]pyrazin-2-ylmethylene)-7-oxo-4-thia-1-azabicyclo[3.2.0]hept-2-ene-2-carboxylic acid, sodium salt

### Step 1: 7-Benzyl-5,6,7,8-tetrahydroimidazo[1,2-a]pyrazine-2-carboxylic acid ethyl ester

Et₃N (6.27 mL), PhCHO (4.92 mL) were added successively to the EtOH (81 mL) solution of 5,6,7,8-tetrahydroimidazo[1,2-a]pyrazine-2-carboxylic acid ethyl ester, hydrochloride (9.47 g) at room temperature and stirred for 3 h under a nitrogen atmosphere. Then NaBH₃CN (2.97 g) was added to the reaction mixture and stirred for 19 h. The mixture was filtered through a pad of Celite and diluted with CH₂Cl₂ and washed with 50% K₂CO₃ aq. The organic layer was dried (K₂CO₃) and filtered. The filtrate was concentrated under reduced pressure. The residue was applied to silica gel column chromatography, then the column was eluted with CHCl₃ - acetone (1/0 ∼ 9/1 ) and CHCl₃ - MeOH (19/1 ∼ 9/1). The titled compound was obtained as pale yellow crystals (4.16 g, 36%).

¹H NMR(CDCl₃) δ 1.36(t, 3H, *J* = 7.1 Hz), 2.87(t, 2H, *J* = 5.2 Hz), 3.71 (s, 2H), 3.75(s, 2H), 4.01(m, 2H), 4.34(q, 2H, *J* = 7.1 Hz), 7.25-7.34(m, 5H), 7.51(s, 1H).

### Step 2: 7-Benzyl-5,6,7,8-tetrahydroimidazo[1,2-a]pyrazine-2-carbaldehyde

1.01 *M* solution of DIBAL in toluene (1 mL + 0.2 mL + 0.3 mL) was added to the dry CH₂Cl₂ (5 mL) solution of 7-benzyl-5,6,7,8-tetrahydroimidazo[1,2-a]pyrazine-2-carboxylic acid ethyl ester (283 mg) under a nitrogen atmosphere at -78°C and stirred for 1.5 h. The mixture was quenched with 1 M HCl (5 mL). The reaction mixture was filtered through a pad of Celite. The filtrate was washed with 50% K₂CO₃ aq. and the aqueous layer was extracted with CH₂Cl₂. The combined organic layer was dried (K₂CO₃) and filtered. The filtrate was concentrated under reduced pressure. The residue was applied to silica gel column chromatography, then the column was eluted with CHCl₃ - acetone (9/1 ∼ 4/1) and CHCl₃ - MeOH (19/1). The titled compound was obtained as colorless crystals (148 mg, 61 %).

¹H NMR(CDCl₃) δ 2.90(t, 2H, *J* = 5.5 Hz), 3.74(s, 2H), 3.76(s, 2H), 4.06(t, 2H, *J* = 5.5 Hz), 7.28 ∼ 7.35(m, 5H), 7.53(s, 1H), 9.80(s, 1H).

### Step 3: (5R, 6RS)-6-[(RS)-Acetoxy(7-benzyl-5,6,7,8-tetrahydroimidazo[1,2-a]pyrazin-2-yl)methyl]-6-bromo-7-oxo-4-thia-1-azabicyclo[3.2.0]hept-2-ene-2-carboxylic acid 4-nitrobenzyl ester (diastereo mixture)

7-Benzyl-5,6,7,8-tetrahydroimidazo[1,2-a]pyrazine-2-carbaldehyde (139 mg) was added to the dry acetonitrile (8.7 mL) solution of anhydrous MgBr₂ (325 mg) under a nitrogen atmosphere at room temperature. The dry THF solution (8.7 mL) of (5R, 6S)-6-bromo-7-oxo-4-thia-1-azabicyclo[3.2.0]hept-2-ene-2-carboxylic acid 4-nitro-benzyl ester (223 mg) was added to the mixture, cooled to -20°C, and Et₃N (0.24 mL) was added in one portion. The reaction vessel was covered with foil to exclude light. The reaction mixture was stirred for 5 h at -20°C and treated with acetic anhydride (0.11 mL) and DMAP (7 mg) in one portion. The reaction mixture was warmed to 0 °C and stirred for 15 h at 0 °C. The mixture was diluted with ethyl acetate and washed with 5% citric acid aqueous solution, saturated sodium hydrogen carbonate, water and brine. The organic layer was dried (MgSO₄) and filtered. The filtrate was concentrated under reduced pressure. The residue was applied to silica gel column chromatography, then the column was eluted with n-hexane - AcOEt (3/1 ∼ 1/1). The titled compound was obtained as two diastereo mixture (80/20, purple amorphous solid, 233 mg, 61%).

¹H NMR(CDCl₃) δ1.99(s, 0.8x3H), 2.23(s, 0.2x3H), 2.83 - 2.89(m, 2H), 3.68(d, 2H, *J* = 4.9 Hz), 3.71 (s, 2H), 3.94 - 4.13(m, 2H), 5.27(d, 1H, *J* = 13.6 Hz), 5.41 (d, 0.2x1H, *J* = 13.6 Hz), 5.45(d, 0.8x1H, *J* = 13.6 Hz), 6.05(s, 0.2x1H), 6.28(s. 0.8x1H), 6.31(s, 0.8x1H), 6.790(s, 0.2x1H), 6.793(s, 0.2x1H), 7.01(s, 0.8x1H), 7.27 - 7.36(m, 5H), 7.42(s, 0.2x1H), 7.46(s, 0.8x1H), 7.61(d, 2H, *J* = 8.6 Hz), 8.22(d, 2H, *J* = 8.6 Hz).

### Step 4: (5R),(6Z)-6-(7-Benzyl-5,6,7,8-tetrahydroimidazo[1,2-a]pyrazin-2-ylmethylene)-7-oxo-4-thia-1-azabicyclo[3.2.0]hept-2-ene-2-carboxylic acid, sodium salt

### (5R, 6RS)-6-[(RS)-Acetoxy(7-benzyl-5,6,7,8-tetrahydroimidazo[1,2-a]pyrazin-2-yl) methyl]-6-bromo-7-oxo-4-thia-1-azabicyclo[3.2.0]hept-2-ene-2-carboxylic acid 4-nitrobenzyl

ester (1.27 g) was dissolved in THF (55 mL) and acetonitrile (25 mL). Freshly activated Zn dust (5.08 g) was added rapidly with 0.5 *M* phosphate buffer (pH 6.5, 80 mL). The reaction vessel was covered with foil to exclude light. The reaction mixture was vigorously stirred for 2 h at room temperature. The reaction mixture was filtered through a pad of Celite. The filtrate was washed with ethyl acetate and the aqueous layer was separated. The aqueous layer was cooled to 3 °C and 1 *M* NaOH was added to adjust pH to 8.0. The mixture was concentrated under high vacuum at 35°C. The concentrate was applied to Diaion HP-21 (79 mL, Mitsubishi Kasei Co. Ltd.) resin column chromatography. After adsorbing, the column was eluted with H₂O - MeCN(1/0 - 4/1). The combined fractions were concentrated under high vacuum at 35°C and lyophilized to give the title compound as a yellow amorphous solid (390 mg, 49%, pH 7.7).

Mp 180 °C (dec); ¹H NMR(D₂O) δ 2.84 - 2.95(m, 2H), 3.61 (d, 2H, *J* = 7.2 Hz), 3.67(s, 2H), 3.96(t, 2H, *J* = 5.7 Hz), 6.43(s, 1H), 6.89(s, 1H), 6.93(s, 1H), 7.28 ∼7.37(m, 6H).

### Example 34

### Preparation of (5R,6Z)-7-oxo-6-{[5-(pyridin-3-ylmethyl)-4,5,6,7-tetrahydrothieno[3,2-c]pyridin-2-yl)]methylene}-7oxo-4-thia-1-azabicyclo[3.2.0]hept-2-ene-2-carboxylic acid:

### Step 1: 2-Formyl [5-(pyridin-3-ylmethyl)-4,5,6,7-tetrahydrothieno][3,2-c]pyridine:

To a stirred solution of 2-(formyl)-6,7-dihydrothieno[3,2-c]-5(4H)-pyridine (1.05 g, 5.2 mmol) in DMF (20 ml), 3-picolyl chloride hydrochloride (0.852 g, 5.2 mmol) and N,N-diisopropylethylamine (10 ml, excess) was added at room temperature. The reaction mixture was stirred for 24 hrs and quenched with water. The reaction mixture was extracted with chloroform; washed well with water and dried over anhydrous MgSO₄. It was filtered and concentrated. The product was purified by SiO₂ column chromatography by eluting it with ethylacetate. Pale yellow semi-solid. Yield: 800 mg , 59%; M+H 259.

### Step 2: 4-Nitrobenzy-6-[(acetyloxy)[5(pyridin-3-ylmethyl)-4,5,6,7-tetrahydrothieno[3,2-c]pyridin-2-yl)methyl]-6-bromo-7-oxo-4-thia-1-azabicyclo[3.2.0]hept-2-ene-2-carboxylate:

2-Formyl [5-(pyridin-3-ylmethyl)-4,5,6,7-tetrahydrothieno][3,2-c]pyridine (516 mg, 2.0 mmol) and the dry THF solution (20 mL) of (5R, 6S)-6-bromo-7-oxo-4-thia-1-aza-bicyclo[3.2.0]hept-2-ene-2-carboxylic acid 4-nitro-benzyl ester (772 mg, 2.0 mmol) were added successively to the dry acetonitrile (15 mL) solution of anhydrous MgBr₂: O(Et)₂ (390 mg, 1.5 mmol)under an argon atmosphere at room temperature. After cooling to-20°C, Et₃N (2.0 mL) was added in one portion. The reaction vessel was covered with foil to exclude light. The reaction mixture was stirred for 2 h at -20°C and treated with acetic anhydride (1.04 mL) in one portion. The reaction mixture was warmed to 0 °C and stirred for 15 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then the column was eluted with ethyl acetate: hexane (1:1). Collected fractions were concentrated under reduced pressure and the mixture of diastereo isomers were taken to next step. Pale yellow amorphous solid; Yield: 700 mg, 51%; M+H 685 and 687.

### Step-3: (5R,6Z)-6-{[5-(pyridin-3-ylmethyl)-4,5,6,7-tetrahydrothieno[3,2-c]pyridin-2-yl)]methylene}-7oxo-4-thia-1-azabicyclo[3.2.0]hept-2-ene-2-carboxylic acid:

4-Nitrobenzy-6-[(acetyloxy)[5(pyridin-3-ylmethyl)-4,5,6,7-tetrahydrothieno[3,2-c]pyridin-2-yl)methyl]-6-bromo-7-oxo-4-thia-1-azabicyclo[3.2.0]hept-2-ene-2-carboxylate (686 mg, 1.0 mmol) was dissolved in THF (20 mL) and acetonitrile (10 mL). Freshly activated Zn dust (5.2 g) was added rapidly with 0.5 *M* phosphate buffer (pH 6.5, 28 mL). The reaction vessel was covered with foil to exclude light. The reaction mixture was vigorously stirred for 2 h at room temperature. The reaction mixture was filtered, cooled to 3°C, and 0.1 *M* NaOH was added to adjust pH to 8.5. The filtrate was washed with ethyl acetate and the aqueous layer was separated. The aqueous layer was concentrated under high vacuum at 35°C to give yellow precipitate. The product was purified by HP21 resin reverse phase column chromatography. Initially the column was eluted with deionized water (2 lits) and latter with 10% CAN: Water. The fractions containing the product were collected and concentrated at reduced pressure at room temperature. The yellow solid was washed with acetone and filtered. Dried. Yield: 50 mg, 12%; as yellow crystals; mp. 134-136°C; (M+H) 412.
¹H NMR (DMSO-d₆)δ d 2.8 (m, 2H), 2.92 (bm, 2H), 3.6 (m, 2H), 3.86 (s, 2H), 6.3 (s, 1H), 6.41 (s, 1H), 7.17 (s, 1H), 7.29 (s, 1H), 7.35 (m, 1H), 7.7 (m, 1H), 8.48 (d,1H), 8.54 (s, 1H).

### Example 35

### Preparation of (5R,6Z)-7-oxo-6-{[5-(pyridin-3-ylcarbonyl)-4,5,6 7-tetrahydrothieno[3,2-c]pyridin-2-yl)]methylenele}-7oxo-4-thia-1-azabicyclo[3.2.0]hept-2-ene-2-carboxylic acid:

### Step 1: 2-Formyl[5-(pyrridin-3-ylcarbonyl)-4,5,6,7-tetrahydrothieno][3,2-c]pyridine:

To a stirred solution of 2-(formyl)-6,7-dihydrothieno[3,2-c]-5(4H)-pyridine (606 mg, 3.0 mmol) in DMF (20 ml), nicotinoyl chloride hydrochloride (531 mg, 3.0 mmol) and N,N-diisopropylethylamine (10 ml, excess) was added at room temperature. The reaction mixture was stirred for 24 hrs and quenched with waster. The reaction mixture was extracted with chloroform; washed well with water and dried over anhydrous MgSO₄. It was filtered and concentrated. The product was purified by SiO₂ column chromatography by eluting it with ethylacetate. Pale yellow semi-solid. Yield: 600mg , 73%; M+H 273.

### Step 2: 4-Nitrobenzy-6-[(acetyloxy)[5(pyridin-3-ylcarbonyl)-4,5,6,7-tetrahydrothieno[3,2-c]pyridin-2-yl)carbonyl]-6-bromo-7-oxo-4-thia-1-azabicyclo[3.2.0]hept-2-ene-2-carboxylate:

2-Formyl [5-(pyridin-3-ylcarbonyl)-4,5,6,7-tetrahydrothieno][3,2-c]pyridine (400 mg, 1.4 mmol) and the dry THF solution (20 mL) of (5R, 6S)-6-bromo-7-oxo-4-thia-1-aza-bicyclo[3.2.0]hept-2-ene-2-carboxylic acid 4-nitro-benzyl ester (772 mg, 2.0 mmol) were added successively to the dry acetonitrile (15 mL) solution of anhydrous MgBr₂: O(Et)₂ (619 mg, 2.4 mmol)under an argon atmosphere at room temperature. After cooling to - 20°C, Et₃N (2.0 mL) was added in one portion. The reaction vessel was covered with foil to exclude light. The reaction mixture was stirred for 2 h at -20°C and treated with acetic anhydride (1.04 mL) in one portion. The reaction mixture was warmed to 0 °C and stirred for 15 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then the column was eluted with ethyl acetate: hexane (1:1). Collected fractions were concentrated under reduced pressure and the mixture of diastereo isomers were taken to next step. Pale yellow amorphous solid; Yield: 300 mg, 30%; M.pt. 71°C; M+H 701.

### Step-3: (5R,6Z)-6-{[5-(pyridin-3-ylcarbonyl)-4,5,6,7-tetrahydrothieno[3,2-c]pyridin-2-yl)]methylene}-7oxo-4-thia-1-azabicyclo[3.2.0]hept-2-ene-2-carboxylic acid sodium salt:

4-Nitrobenzy-6-[(acetyloxy)[5(pyridin-3-ylcarbonyl)-4,5,6,7-tetrahydrothieno[3,2-c]pyridin-2-yl)methyl]-6-bromo-7-oxo-4-thia-1-azabicyclo[3.2.0]hept-2-ene-2-carboxylate (800 mg, 1.14 mmol) was dissolved in THF (20 mL) and acetonitrile (10 mL). Freshly activated Zn dust (5.2 g) was added rapidly with 0.5 *M* phosphate buffer (pH 6.5, 28 mL). The reaction vessel was covered with foil to exclude light. The reaction mixture was vigorously stirred for 2 h at room temperature. The reaction mixture was filtered, cooled to 3°C, and 0.1 *M* NaOH was added to adjust pH to 8.5. The filtrate was washed with ethyl acetate and the aqueous layer was separated. The aqueous layer was concentrated under high vacuum at 35 °C to give yellow precipitate. The product was purified by HP21 resin reverse phase column chromatography. Initially the column was eluted with deionized water (2 lits) and latter with 10% CAN: Water. The fractions containing the product were collected and concentrated at reduced pressure at room temperature. The yellow solid was washed with acetone and filtered. Dried. Yield: 50 mg, 12%; as yellow crystals; mp. 195 °C; (M+H) 426.

### Example 36

### Preparation of (5R,6Z)-7-oxo-6-{[5-(phenylacetyl)-4,5,6,7-tetrahydrothieno[3,2-c]pyridin-2-yl)]methylene}-7oxo-4-thia-1-azabicyclo[3.2.0]hept-2-ene-2-carboxylic acid:

### Step 1: 2-Formyl [5-(phenylacetyl)-4,5,6,7-tetrahydrothieno][3,2-c]pyridine:

To a stirred solution of 2-(formyl)-6,7-dihydrothieno[3,2-c]-5(4H)-pyridine (0.41 mg, 2 mmol) in DMF (20 ml), phenyl acetyl chloride (0.35 mg, 2.2 mmol) and N,N-diisopropylethylamine (10 ml, excess) was added at room temperature. The reaction mixture was stirred for 24 hrs and quenched with water. The reaction mixture was extracted with chloroform; washed well with water and dried over anhydrous MgSO₄. It was filtered and concentrated. The product was purified by SiO₂ column chromatography by eluting it with ethylacetate. White solid. Yield: 510 mg , 89%; M+H 286.

### Step 2: 4-Nitrobenzy-6-[(acetyloxy)[5(phenylacetyl)-4,5,6,7-tetrahydrothieno[3,2-c]pyridin-2-yl)methyl]-6-bromo-7-oxo-4-thia-1-azabicyclo[3.2.0]hept-2-ene-2-carboxylate:

2-Formyl [5-(phenylacetyl)-4,5,6,7-tetrahydrothieno][3,2-c]pyridine (340 mg, 1.2 mmol) and the dry THF solution (20 mL) of (5R, 6S)-6-bromo-7-oxo-4-thia-1-azabicyclo[3.2.0]hept-2-ene-2-carboxylic acid 4-nitro-benzyl ester (390 mg, 1.0 mmol) were added successively to the dry acetonitrile (15 mL) solution of anhydrous MgBr₂: O(Et)₂ (310 mg, 1.2 mmol)under an argon atmosphere at room temperature. After cooling to - 20°C, Et₃N (2.0 mL) was added in one portion. The reaction vessel was covered with foil to exclude light. The reaction mixture was stirred for 2 h at -20 °C and treated with acetic anhydride (1.04 mL) in one portion. The reaction mixture was warmed to 0 °C and stirred for 15 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then the column was eluted with ethyl acetate: hexane (1:1). Collected fractions were concentrated under reduced pressure and the mixture of diastereo isomers were taken to next step. Pale yellow amorphous solid; Yield: 360 mg, 50%; M+H 713.

### Step-3: (5R,6Z)-6-{[5-(phenylacetyl)-4,5,6,7-tetrahydrothieno[3,2-c]pyridin-2-yl)]methylene}-7oxo-4-thia-1-azabicyclo[3.2.0]hept-2-ene-2-carboxylic acid:

4-Nitrobenzy-6-[(acetyloxy)[5(phenylacetyl)-4,5,6,7-tetrahydrothieno[3,2-c]pyridin-2-yl)methyl]-6-bromo-7-oxo-4-thia-1-azabicyclo[3.2.0]hept-2-ene-2-carboxylate (300 mg, 0.4 mmol) was dissolved in THF (50 mL) and 0.5 *M* phosphate buffer (pH 6.5, 28 mL). This was hydrogenated at 40 psi pressure, in the presence of 10% Pd/C (80 mg) for 2 hrs, at the end, reaction mixture was filtered through a pad of celite and concentrated. The separated yellow solid was dissolved in ethyl acetate and washed well with water. The organic layer was dried and concentrated. The separated yellow solid was triturated with diethyl ether and filtered. The yellow solid was washed well with diethyl ether and it was found to be 95% pure compound. Yield: 160 mg, 91%; Yellow solid; mp. 166-169°C; (M+H) 439.

### Example 37

### Preparation of (5R,6Z)-6-[(7-methylimidazo[2,1-b][1,3]benzothiazol-2-ylmethylene)-7-oxo-4-thia-1-azabicyclo[3.2.0]hept-2-ene-2-carboxylic acid

### Step 1: Ethyl 7-methylimidazo[2,1-b]-benzthiazole-2-carboxylate:

Ethyl 7-methylimidazo[2,1-b]-benzthiazole-2-carboxylate was prepared according to the procedure as outlined in Example 1, (Step 1). Starting from 6-methyl-2-amino benzothiazole (3.2 g, 20 mmol) and ethyl bromopyruvate (4.0 g, 20.4 mmol), 3.0 g (57% Yield) of ethyl 7-methylimidazo[2,1-b]-benzthiazole-2-carboxylate was isolated as brown solid. (M+H) 261.

### Step 2: 2-Formyl-7-methylimidazo[2,1-b]-benzthiazole:

To a stirred solution of Ethyl 7-methylimidazo[2,1-b]-benzthiazole-2-carboxylate (4.0 g, 15.38 mmol) in dry THF at -78°C, DIBAL (1M. solution in toluene) (16.0 ml, 16 mmol) was added. The reaction mixture was stirred at -78°C and slowly elevated to room temperature. The reaction mixture was stirred at room temperature for 30 minutes and quenched with saturated NH₄Cl. The reaction mixture was extracted with chloroform and washed well with water. The organic layer was dried over anhydrous MgSO₄; filtered and concentrated. The residue was purified bt SiO₂ column chromatography by eluting it with chloroform: metrhanol (20:1). Brown solid; (M+H) 217; Yield: 800 mg (24%)

### Step 3: 4-Nitrobenzyl-6-[(acetyloxy) (7-methylimidazo[2,1-b][1,3]benzothiazol-2-yl)methyl]-6-bromo-7-oxo-4-thia-1-azabicyclo[3.2.0]hept-2-ene-2-carboxylate:

2-Formyl-7-methylimidazo[2,1-b]-benzthiazole (432 mg, 2.0 mmol) and the dry THF solution (20 mL) of (5R, -6S)-6-bromo-7-ox0-4-thia-1-aza-bicyclo[3.2.0]hept-2-ene-2 carboxylic acid 4-nitro-benzyl ester (772 mg, 2.0 mmol) were added successively to the dry acetonitrile (15 mL) solution of anhydrous MgBr₂: O(Et)₂ (566 mg, 2.0 mmol) under an argon atmosphere at room temperature. After cooling to -20 °C, Et₃N (2.0 mL) was added in one portion. The reaction vessel was covered with foil to exclude light. The reaction mixture was stirred for 2 h at -20 °C and treated with acetic anhydride (1.04 mL) in one portion. The reaction mixture was warmed to 0 °C and stirred for 15 h at 0 °C. The mixture was diluted with ethyl acetate and washed with 5% citric acid aqueous solution, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then the column was eluted with ethyl acetate: hexane (1:1). Collected fractions were concentrated under reduced pressure and the mixture of diastereomers were taken to the next step. Pale yellow amorphous solid; Yield: 400 mg, 31%; M+H 645.

### Step-4: (5R),(6Z)-6-[(7-methylimidazo[1,2-b][1,3]benzothiazol-2-ylmethylene)]-7-oxo-4-thia-1-azabicyclo [3.2.0]hept-2-ene-2-carboxylic acid:

4-Nitrobenzyl-6-[(acetyloxy)(7-methylimidazo[2,1-b][1,3]benzothiazol-2-yl)methyl]-6-bromo-7-oxo-4-thia-1-azabicyclo[3.2.0]hept-2-ene-2-carboxylate (350 mg, 0.54 mmol) was dissolved in THF (20 mL) and acetonitrile (10 mL). Freshly activated Zn dust (5.2 g) was added rapidly with 0.5 *M* phosphate buffer (pH 6.5, 28 mL). The reaction vessel was covered with foil to exclude light. The reaction mixture was vigorously stirred for 2 h at room temperature. The reaction mixture was filtered, cooled to 3°C, and 0.1 N NaOH was added to adjust the pH to 8.5. The filtrate was washed with ethyl acetate and the aqueous layer was separated. The aqueous layer was concentrated under high vacuum at 35°C to give a yellow precipitate. The product was purified by HP21 resin reverse phase column chromatography. Initially the column was eluted with deionized water (2 L) and latter with 10% acetonitrile:water. The fractions containing the product were collected and concentrated under reduced pressure at room temperature. The yellow solid was washed with acetone, filtered and dried. Yield: 110 mg, 55%; as yellow crystals; mp 178°C (Dec); (M+H+Na) 392.
¹H NMR (DMSO-d₆) δ 8.56 (s, 1H), 7.93 (d, 1H), 7.83 (s, 1H), 7.38 (d, 1H), 7.07 (s, 1H), 6.51 (s, 2H), 2.42 (s, 3H).

### Step-4: (5R),(6Z)-6-[(7-methylimidazo[1,2-b][1,3]benzothiazol-2-ylmethylene)]-7-oxo-4-thia-1-azabicyclo [3.2.0]hept-2-ene-2-carboxylic acid: (Procedure B)

4-Nitrobenzyl-6-[(acetyloxy)(7-methylimidazo[2,1-b][1,3]benzothiazol-2-yl)methyl]-6-bromo-7-oxo-4-thia-1-azabicyclo[3.2.0]hept-2-ene-2-carboxylate (350 mg, 0.54 mmol) was dissolved in THF (40 mL) and 6.5 pH phosphate buffer (40 ml) and hydrogenated over Pd/C (10% , 200 mg) at 40 psi pressure for 3 hrs at room temperature. At the end , reaction mixture was filtered through a pad of celite and washen with acetonitrile. The reaction mixture was concentrated to 40 ml and cooled to 0° C and pH was adjusted to 8.5 by adding 1N NaOH. The product was directly loaded over HP21 resin reverse phase column chromatography. Initially the column was eluted with deionized water (2 L) and latter with 10% acetonitrile:water. The fractions were concentarated and the yellow solid was washed with acetone, filtered and dried. Yield: 110 mg, 55% as yellow solid.

### Example 38

### Preparation of (5R), (6Z)-6-(4,5,6,7-tetrahydro-1,3a,3b,8-tetraaza-cyclopenta[a]indene-2-ylmethylene)-7-oxo-4-thia-1-aza-bicyclo[3.2.0]hept-2-ene-2-carboxylic acid sodium salt

### Step 1: 5,6,7,8-Tetrahydro-[1,2,4]triazolo[1,5-a]pyridin-2-ylamine

The 12.7% solution of HCl in ethanol (5.35 mL) and 10% Pd-C (50% wet) (2.5 g) were added to the mixture of [1,2,4]triazolo[1,5-a]pyridin-2-ylamine (2.5 g) in ethanol (72 mL). The reaction mixture was hydrogenated at 400 KPa of H₂ for 3 days at room temperature. The mixture was filtered and concentrated under reduced pressure. The residue was treated with saturated potassium carbonate solution and extracted with chloroform. The organic layer was dried (Na₂SO₄) and concentrated under reduced pressure. The title compound was obtained as a pale yellow solid (2.31 g, 90%). ¹H-NMR (400 MHz, CDCl₃) δ 1.88-1.94 (m, 2H), 1.98-2.05 (m, 2H), 2.77 (t, 2H, *J* = 6.2 Hz), 3.95 (t, 2H, *J* = 6.2 Hz), 4.09 (brs, 2H).

### Step 2: 4,5,6,7-Tetrahydro-1,3a,3b,8-tetraaza-cyclopenta[a]indene-2-carboxylic acid ethyl ester

Ethyl bromopyruvate (10.23 g) was added to the mixture of 5,6,7,8-tetrahydro-[1,2,4]triazolo[1,5-a]pyridin-2-ylamine (5.8 g) in 1,2-dimethoxyethane (320 mL). The reaction mixture was stirred for 5 hours at room temperature and concentrated to 100 mL under reduced pressure. The precipitate was obtained by an addition of diethyl ether (200 mL), followed by filtration. The precipitate was dissolved in ethanol (175 mL) and stirred for 20 hours at 110 °C in shield tube. The reaction mixture was cooled to room temperature and concentrated under reduced pressure. The residue was treated with saturated potassium carbonate solution and extracted with chloroform. The organic layer was dried (Na₂SO₄) and concentrated under reduced pressure. The residue was applied to silica gel column chromatography, then eluted with ethyl acetate - methanol (1/1). The title compound was obtained as a pale yellow solid (7.56 g, 77%). ¹H-NMR (400 MHz, CDCl₃) δ 1.42 (t, 3H, *J* = 7.1 Hz), 2.14-2.25 (m, 4H), 3.11 (t, 2H, *J* = 6.1 Hz), 4.37 (t, 2H, *J* = 5.7 Hz), 4.41 (q, 2H, *J* = 7.1 Hz), 7.57 (s, 1H).

### Step 3: 4,5,6,7-Tetrahydro-1,3a,3b,8-tetraaza-cyclopenta[a]indene-2-carbaldehyde

1.01 M Diisobutylalminium hydride in toluene (1.06 mL) was added dropwise to the solution of 4,5,6,7-tetrahydro-1,3a,3b,8-tetraaza-cyclopenta[a]indene-2-carboxylic acid ethyl ester (100 mg) in dry THF (5 mL) at -78 °C under a nitrogen atmosphere. The reaction mixture was stirred for 30 minutes at -78 °C and treated with ethanol (ca. 1 mL). The mixture was warmed to 0 °C and stirred for 1 h at 0 °C. The reaction solution was diluted with ethyl acetate (20 mL), treated with 0.5 mL saturated ammonium chloride solution, and sonicated for ca. 5 minutes (until a precipitate was deposited enough). The mixture was dried (Na₂SO₄) and filtered through a pad of Celite. The filtrate was concentrated under reduced pressure. The residue was crystallized from dichloromethane and diethyl ether to give the title compound (47.4 mg, 58%). ¹H-NMR (400 MHz, CDCl₃) δ 2.16-2.27 (m, 4H), 3.14 (t, 2H, *J* = 6.1 Hz), 4.39 (t, 2H, *J* = 5.7 Hz), 7.53 (s, 1H), 10.01 (s, 1H).

### Step 4: (5R, 6RS)-6-{(RS)-Acetoxy-[4,5,6,7-tetrahydro-1,3a,3b,8-tetraaza-cyclopenta[a]indene-2-yl]-methyl}-6-bromo-7-oxo-4-thia-1-aza-bicyclo[3.2.0]hept-2-ene-2-carboxylic acid 4-nitro-benzyl ester

4,5,6,7-Tetrahydro-1,3a,3b,8-tetraaza-cyclopenta[a]indene-2-carbaldehyde (2.97 g) was added to the dry acetonitrile (110 mL) solution of anhydrous MgBr₂ (4.45 g) under a nitrogen atmosphere at room temperature. The dry THF solution (110 mL) of (5*R*, 6*S*)-6-bromo-7-oxo-4-thia-1-aza-bicyclo[3.2.0]hept-2-ene-2-carboxylic acid 4-nitrobenzyl ester (2.97 g) was added to the reaction mixture, cooled to -20 °C, and triethylamine (6.45 mL) was added in one portion. The reaction vessel was covered with foil to exclude light. After the mixture was stirred for 1.2 h at -20 °C, acetic anhydride (2.9 mL) was added in one portion. The reaction mixture was warmed to 0 °C and stirred for 16.5 h at 0 °C. The mixture was diluted with ethyl acetate and washed with H₂O and brine. The organic layer was dried (MgSO₄) and filtered through a pad of Celite. The pad was washed with ethyl acetate. The filtrate was concentrated under reduced pressure. The residue was applied to silica gel column chromatography, eluted with ethyl acetate - *n*-hexane (3/1) and then with ethyl acetate - methanol (5/1). The title compound was obtained as a brown amorphous solid (651.6 mg, 13%). ¹H-NMR (400 MHz, CDCl₃) δ 2.10-2.24 (m, 4H), 2.29 (s, 3H), 3.04-3.07 (m, 2H), 4.28-4.32 (m, 2H), 5.27 (d, 1H, *J* = 13.7 Hz), 5.43 (d, 1H, *J*=13.7 Hz), 6.19 (s, 1H), 6.91 (s, 1H), 7.01 (s, 1H), 7.49 (s, 1H), 7.59-7.62 (m, 2H), 8.23-8.25 (m, 2H).

### Step 5: (5R), (6Z)-6-(4,5,6,7-tetrahydro-1,3a,3b,8-tetraaza-cyclopenta[a]indene-2-ylmethylene)-7-oxo-4-thia-1-aza-bicyclo[3.2.0]hept-2-ene-2-carboxylic acid sodium salt

(5*R*, 6*RS*)-6-{(*RS*)-Acetoxy-[4,5,6,7-tetrahydro-1,3a,3b,8-tetraaza-cyclopenta[*a*] indene-2-yl]-methyl}-6-bromo-7-oxo-4-thia-1-aza-bicyclo[3.2.0]hept-2-ene-2-carboxylic acid 4-nitro-benzyl ester (643.6 mg) was dissolved in THF (9 mL) and acetonitrile (4.2 mL). Freshly activated Zn dust (2.57 g) and 0.5 *M* phosphate buffer (pH 6.4, 13.2 mL) were added to the reaction mixture. The reaction vessel was covered with foil to exclude light. The mixture was vigorously stirred for 2 h at room temperature. The mixture was cooled to 9 °C, and 1 *M* NaOH aqueous solution was added to adjust pH to 7.5. The reaction solution was mixed with ethyl acetate and filtered through a pad of Celite. The pad was washed with water. The aqueous layer was concentrated to 20 mL under high vacuum at 35 °C. The concentrate was applied to Diaion HP-21 (60 mL, Mitsubishi Kasei Co. Ltd.) resin column chromatography. After adsorbing, the column was eluted with water and then with 2.5-10% acetonitrile-water. The combined fractions was concentrated under high vacuum at 35 °C and lyophilized to give the title compound as a yellow amorphous solid (68 mg, 18%, pH 7.4). Mp 175 °C (dec); ¹H-NMR (400 MHz, D₂O) δ 1.85-2.03 (m, 4H), 2.85-2.99 (m, 2H), 4.07-4.14 (m, 2H), 6.34 (s, 1H), 6.74 (s, 1H), 6.76 (s, 1H), 7.28 (s, 1H).

### Example 39

### Preparation of (5R,6E)-6-[(10-benzyl-11-oxo-10,11-dihydrodibenzo[b,f][1,4]oxazepin-8-yl)methylene]-7-oxo-4-thia-1-azabicyclo[3.2.0]hept-2-ene-2-carboxylic Acid

### Step 1: Preparation of 8-(hydroxymethyl)dibenzo[b,f][1,4]oxazepin-11(10H)-one;

Lithium aluminum hydride (11 mL, 11 mmole) was slowly added to the solution of 11-Oxo-10,11-dihydro-dibenzo[*b,f*][1,4]oxazepine-8-carboxylic acid methyl ester(1.346 g, 5 mmole) in THF under N₂ at room temperature. The reaction mixture was stirred for 1 hour and 45 minutes then quenched with 2N of HCl until the pH value reaches 2-3. Removed all the THF by rotary evaporation, and extracted the reaction mixture with ethyl acetate for five times, dried the organic layer with sodium sulfate and filtered and concentrated. Obtained the desired compound (white solid) in 46% yield.

### Step 2: Preparation of 11-oxo-10,11-dihydrodibenzo[b,f][1,4]oxazepine-8-carbaldehyde;

8-(hydroxymethyl)dibenzo[b,f][1,4]oxazepin-11(10H)-one (0.241 g, 1 mmole) in acetonitrile was added to the molecular sieves (1 g) under N₂ at room temperature then 4-methylmorpholine N-oxide (0.175 g, 1.5 mmole) was also added into the reaction mixture. After stirring the mixture for 10 minutes, tetrapropylammonium perruthenate (0.0176 g, 0.05 mmole) was added and the reaction followed by t.l.c. until complete. Dilute the reaction mixture with 10ml of ethyl acetate and flashed it through a small silica gel column. Collected all the ethyl acetate that contains desired material, extracted the organic layer with 1 N HCl and also washed it with brine. Dried the organic layer over sodium sulfate and filtered and concentrated. Obtained the desired compound (white solid) in 83% yield.

### Step 3: Preparation of 10-benzyl-11-oxo-10,11-dihydro-dibenzo[b,f][1,4]oxazepine-8-carbaldehyde;

Potassium carbonate anhydrous (0.207g, 1.5 mmole) and benzyl bromide (0.205 g, 1.2 mmole) were added to a solution of the 11-oxo-10,11dihydrodibenzo[b,f][1,4]oxazepine-8-carbaldehyde (0.240 g, 1 mmole) in acetonitrile under N₂ at room temperature. The reaction mixture then was refluxed for 4 hours, and cooled to room temperature. Diluted the reaction mixture with ethyl acetate and filtered through a magnesol pad and concentrated. Purified with silica gel column and 50% ethyl acetate in hexane. Obtained the desired compound (light yellow oil) in 63% yield.

### Step 4: Preparation of 6-[acetoxy-(10-benzyl-11-oxo-10,11-dihydro-dibenzo[b,f][1,4]oxazepin-8-yl)-methyl]-6-bromo-7oxo-4-thia-1-aza-bicyclo[3.2.0]hept-2-ene-2-carboxylic acid 4-nitro-benzyl ester;

10-benzyl-11-oxo-10,11-dihydro-dibenzo[b,f][1,4]oxazepine-8-carbaldehyde (0.250 g, 0.759 mmole) in acetonitrile was added to magnesium bromide (0.419 g, 2.28mmole) under N₂ at room temperature. The dry THF solution of (5R,6S)-6-bromo-7-oxo-4-thia-1-azabicyclo[3.2.0]hept-2-ene-2-carboxylic acid 4-nitrobenzyl ester (0.292 g, 0.758 mmole) then was added to the mixture. After 15 minutes the reaction mixture was cooled to - 20°C, and triethylamine (0.317 mL, 2.27 mmole) was added. The reaction flask was covered with foil to exclude light. After 4 hours at -20°C, treated with acetic anhydride (0.358 mL, 3.795 mmole) and DMAP (0.00927 g, 0.0759 mmole). Warmed up the reaction mixture to 0°C and placed it in freezer overnight. Reaction solution was concentrated and dissolved with ethyl acetate and washed with 5% of citric acid aqueous solution, saturated NaHCO₃. water and brine. Organic layer was dried in sodium sulfate and filtered and concentrated. Purified with silica gel column and 1:15 ethyl acetate/CH₂Cl₂, Obtained the desired compound (light yellow oil) in 41% yield.

### Step 5: Preparation of 6-(10-benzyl-11-oxo-10,11-dihydro-dibenzo[b,f][1,4]oxazepin-8-ylmethylene)-7-oxo-4-thia-1-aza-bicyclo[3.2.0]hept-2-ene-2-carboxylic acid, sodium salt;

A 0.5M phosphate buffer solution (pH 6.5) was added to a solution of 6-[acetoxy-(10-benzyl-11-oxo-10,11-dihydro-dibenzo[b,f][1,4]oxazepin-8-yl)-methyl]-6-bromo-7oxo-4-thia-1-aza-bicyclo[3.2.0]hept-2-ene-2-carboxylic acid 4-nitro-benzyl ester (0.210 g, 0.273 mmole) in THF, followed by 10% Pd-C (0.0546 g). The reaction mixture then was hydrogenated at 40psi for three hours. Filtered through a celite pad and removed the THF by rotary evaporation, extracted the mixture with ethyl acetate and washed with water and brine. Dried the organic layer with sodium sulfate and filtered and concentrated. Dissolved the NaHCO₃ with minimal amount of distal water and added it to the reaction mixture along with a small amount of ethyl acetate until the pH value reaches 7-8, evaporated the ethyl acetate. Purified with reverse phase column (MCl Gel CHP20P) with varying amounts of acetonitrile (0%-20%) in water. Removed the acetonitrile and water by rotary evaporation, and freeze-dried the compound. Obtained the desired material (yellow solid) in 24% yield.Mp: 179°C. ¹H NMR (DMSO) δ 1.755-1.825 (s, 1H), 2.497-2.506 (d, 2H), 5.243-5.434 (m, 2H), 6.516-6.770 (m, 1H), 7.039-7.792 (m, 11H).

### Example 40

### Preparation of 6-(6-ethoxy-7,8-dihydro-6H-3,4,8b-triaza-as-indacen-2-ylmethylene)-7-oxo-4-thia-1-aza-bicyclo[3.2.0]hept-2-ene-2-carboxylic acid

### Step 1: Preparation of 4-ethoxy-6,7-dihydro-5H-cyclopentapyrimidin-2-ylamine (SM:Ross, L. O.; Goodman, L.; Baker, B. R. J. Am. Chem. Soc. 1959, 81, 3108)

5.1 grams of 4-chloro-6,7-dihydro-5H-cyclopentapyrimidin-2-ylamine was dissolved in 200ml xylene and 30 ml absolute ethanol. Then 6.8 gram for sodium ethoxide was added and the mixture was refluxed for 3 hours. Then the solvent was removed in vacuo and 100ml water was added to the residue. Filter and wash the cake with water (50ml). The solid was further vacuumed to dry for several hours. The desired product weighed 5.3 gram (98% yield). Mp: 133.8∼134.9 oC.
H-NMR: (300 MHz, CDCl₃) δ. 6.23(s, NH2), 4.28(quartet, 2H, J= 6.9 Hz), 2.6 (m, 2H), 1.93 (m, 2H), 1.27 (t, CH3, J=6.9 Hz); MS: 180.0 (M+H)

### Step 2: Preparation of 5-Ethoxy-7,8-dihydro-6H-3,4,8b-triaza-as-indacene-2-carboxylic acid ethyl ester

5.2 gram (29mmol) 4-ethoxy-6,7-dihydro-5H-cyclopentapyrimidin-2-ylamine was dissolved in 100 ml dry THF. Bromopyruvate (5.4ml,) was then added dropwise with in five minutes. The mixture was stirred at 23oC for one hour. It was then filtered and washed with ether to give 8.7 gram of solid. This solid was then dissolved in 50ml ethanol and refluxed for two hours. The reaction mixture was cooled to room temperature and partitioned between 350ml chloroform and 200 ml saturated sodium bicarbonate. The organic layer was separated and dried over magnesium sulfate. Filter off the drying agent and concentrate to give 6.5 gram of product.
MP: 168.6-168.7 oC.
H-NMR: (300 MHz, CDCl₃) δ. 7.69(s, 1H), 4.50 (qartet, 2H, J=7.2 Hz), 4.40 (qartet, 2H, J=7.2 Hz), 3.11 (t, 2H, J=9.6 Hz), 2.88 (t, 2H, J=9.6 Hz), 2.88 (m, 2H), 1.43 (t, 2H, J=7.2 Hz).; MS: 276.2(M+H)

### Step 3: Preparation of 5-ethoxy-7,8-dihydro-6H-3,4,8b-triaza-as-indacene-2-carbaldehyde

1.925 grams 5-ethoxy-7, 8-dihydro-6H-3,4,8b-triaza-as-indacene-2-carboxylic acid ethyl ester was dissolved in 40 ml dichloromethane and then cooled to -78oC. DIBAL (1 M, 21 ml, 3 eq.) was then added within five minutes. The reaction media was then quenched with 2ml ethanol and partitioned between 350ml dichloromethane and 100 ml 1 N sodium hydroxide. The aqueous layer was washed with another 150ml chloroform and the combined organic layer was dried over magnesium sulfate and filtered and concentrated to give the corresponding alcohol. The alcohol is then dissolved in 150ml dichloromethane and 10 grams of manganese dioxide is then added. The mixture was stirred at 23 oC for two hours. The reaction mixture was then filtered through a pad of celite and concentrated to give 1.1 gram (68%) of the desired aldehyde.
MP: 237.2∼237.3° C
H-NMR: (300 MHz, CDCl₃) δ. 9.94(s, 1H, CHO), 8.39 (s, 1H), 4.46 (quartet, 2H, J= 7.2Hz), 3.2 (m, 2H, CH2), 2.85 (m, 2H, CH2), 2.24 (m, 2H, CH2), 1.38 (t, 3H, CH3, J=7.2Hz); MS: 232.1 (M+H)

### Step 4: Preparation of 6-[acetoxy-(5-ethoxy-7,8-dihydro-6H-3,4,8b-triaza-as-indacen-2-yl)-methyl]-6-bromo-7-oxo-4-thia-1-aza-bicyclo[3.2.0]hept-2-ene-2-carboxylic acid 4-nitro-benzyl ester

A 30 ml acetonitrile solution of 5-ethoxy-7,8-dihydro-6H-3,4,8b-triaza-as-indacene-2-carbaldehyde (693 mg, 3mmol) was added 1.03 gram of magnesium bromide etherate. The mixture was stirred at 23oC for half an hour. Then a 30ml dry THF solution of the 6-Bromo-7-oxo-4-thia-1-aza-bicyclo[3.2.0]hept-2-ene-2-carboxylic acid 4-nitro-benzyl ester (1.155 gram, 1 eq.) was injected within a minute and the reaction mixture was then cooled to -20oC. Triethylamine (0.7 ml, eq.) was then injected and the reaction mixture was stirred for five hours at -20oC. Then acetic anhydride (0.377 ml, eq.) was injected and the reaction mixture was left at zero degree for 18 hours. The reaction media was then diluted with 400ml ethyl acetate and washed with 100 ml 5% citric acid, 100 ml saturated sodium bicarbonate, and 100ml brine. The organic layer was then dried over magnesium sulfate, filtered and concentrated. Flash column chromatography using 20% ethyl acetate in hexane gave 1.1 gram product.
MP: 118.7∼119.1 °C
H-NMR: (300 MHz, CDCl₃) δ. 8.35(d, 2H, J=11Hz), 7.63 (m, 2H), 741 (d, 1H, J=6.9Hz), 7.08 (d, 1H, J=11 Hz), 6.47(s, 1H), 5.55 (4H, CH2), 4.54 (m, 2H), 3.09 (m, 2H), 2.93 (m, 2H), 2.32 (m, 2H), 1.41 (t, J=9.6Hz); MS: 660.1 (M+H)

### Step 5: Preparation of 6-(5-ethoxy-7,8-dihydro-6H-3,4,8b-triaza-as-indacen-2-ylmethylene)-7-oxo-4-thia-1-aza-bicyclo[3.2.0]hept-2-ene-2-carboxylic acid

6-[acetoxy-(5-ethoxy-7,8-dihydro-6H-3,4,8b-triaza-as-indacen-2-yl)-methyl]-6-bromo-7-oxo-4-thia-1-aza-bicyclo[3.2.0]hept-2-ene-2-carboxylic acid 4-nitro-benzyl ester (1.03 gram, 1.565 mmol) was suspended in 20 ml THF and 20 ml pH=6.5 aqueous phosphate buffer. The mixture was then subjected to 45psi hydrogen for two hours. Then it was filtered through a pad of celite and concentrated in vacuo to remove most of the THF. The solution was then cooled to zero degree and basified to pH=8 with 1 N sodium hydroxide. Then it was purified via reverse phase HPLC using 1 liter of water followed by 5% ∼25% acetonitrile and water. Water was then removed through concentrate in vacuo and 100 mg of product was collected.
MP: >250° C
H-NMR: (300 MHz, CDCl₃) δ. 7.52 (s, 1H), 6.95(s, 1H), 6.54(s, 1H), 4.73 (m, 2H), 3.06(m, 2H), 2.84 (m, 2H), 2.27 (m, 2H), 1.43 (t, 3H); MS: 383.2 (M+H).

### Example 41

### (5R,6E&Z)-7-oxo-6-(4H,10H-pyrazolo[5,1-c][1,4]benzoxazepin-2-ylmethylene)-4-thia-1-azabicyclo[3.2.0]hept-2-ene-2-carboxylic acid, sodium salt

### Step 1: Preparation of 1-(2-fluorobenzyl)-1H-pyrazole-3,5-dicarboxylate

2-fluorobenzyl bromide (2.0 ml, 16.58 mmol) was added to a mixture of diethyl 3,5-pyrazoledicarboxylate (3.01 g, 14.18 mmol), Cs₂CO₃ (5.57 g, 17.1 mmol), and acetonitrile (140 ml) under N₂. Heated to 60°C for two hours and then cooled to room temperature. Filtered and concentrated the reaction solution. Added water (-200mL) to the resulting residue and extracted with EtOAc. Washed organics with water and brine. Dried organics over sodium sulfate and filtered and concentrated. Obtained diethyl 1-(2-fluorobenzyl)-1H-pyrazole-3,5-dicarboxylate (light-yellow oil) in quantitative yield.

### Step 2: Preparation of 1-(2-fluorobenzyl)-1H-pyrazole-3,5-methanediol

A 1M solution of DIBAL-H in THF (90 ml, 90 mmol) was added to a solution of diethyl 1-(2-fluorobenzyl)-1H-pyrazole-3,5-dicarboxylate (4.80 g, 14.99 mmol) in CH₂Cl₂ (90 ml) at 0°C under N₂. After two hours quenched with NH₄Cl_{(aq)} and suspension was formed. Filtered and extracted with EtOAc and washed with brine. Dried organics over sodium sulfate and filtered and concentrated. Purified with silica gel column and 5% MeOH in CH₂Cl₂. Obtained 3.4 g of the diol compound (clear oil) in 96% yield.

### Step 3: Preparation of 4H,10H-pyrazolo[5,1-c][1,4]benzoxazepine-2-carbaldehyde

The diol compound (3.83 g, 16.21 mmol) in HMPA (24 ml) was added to a suspension of NaH (60%, 1.34 g, 33.5 mmol) in toluene (330 ml) under N₂. Rapidly heated to 95°C for three hours and cooled to room temperature. Quenched with water and extracted with EtOAc. Washed organics with water and brine. Dried organics over sodium sulfate and filtered and concentrated. Purified with silica gel column and 2% MeOH in CH₂Cl₂. Obtained 4H,10H-pyrazolo[5,1-c][1,4]benzoxazepin-2-ylmethanol (white solid). Yield: 0.71 g 20%.
4H,10H-pyrazolo[5,1-c][1,4]benzoxazepin-2-ylmethanol (0.71 g, 3.28 mmol), 4-methylmorpholine N-oxide (1/198g, 10.23 mmol), molecular sieves (powder, 4 angstroms) (3.32 g), and acetonitrile (0.07M) were placed together under N₂. Tetrapropylammoniumperruthenate (0.113 g, 0.322 mmol) was added and after three hours the reaction solution was filtered through celite and concentrated. Purified with silica gel column and 1:1 EtOAc/Hexane. Obtained 4H,10H-pyrazolo[5,1-c][1,4]benzoxazepine-2-carbaldehyde (white solid). Yield: 0.31 g 44%.

### Step 4: Preparation of Preparation of 6-[acetoxy-(4H,10H-pyrazolo[5,1-c][1,4]benzoxazepine-8-yl)-methyl]-6-bromo-7oxo-4-thia-1-aza-bicyclo[3.2.0]hept-2-ene-2-carboxylic acid 4-nitro-benzyl ester;

4H,10H-pyrazolo[5,1-c][1,4]benzoxazepine-2-carbaldehyde (0.19 g, 0.887 mmol) in acetonitrile (14 ml) was added to MgBr₂(0.49g,2.66 mmol) under N₂. After 25 minutes 6-Bromo-7-oxo-4-thia-1-aza-bicyclo[3.2.0]hept-2-ene-2-carboxylic acid 4-nitro-benzyl ester (0.342g, 0.888 mmol.) in THF (14 ml) was added. After 15 minutes the reaction was cooled to -20°C. Ten minutes later added Et₃N (3eq) and placed reaction in the dark. After 6.5 hours added Ac₂O (0.42 ml, 4.45 mmol) and DMAP (0.011 g, 0.0900 mmol). Warmed to 0°C and placed in freezer overnight. Reaction solution was concentrated and resulting residue was taken up in EtOAc. Washed with 5% citric acid (aq) and saturated NaHCO₃ (aq). Further washed with water and brine. Dried organics over sodium sulfate and filtered and concentrated. Purified with silica gel prep plates and 1:2 EtOAc/Hexane. Obtained the condensation product (yellow gum/solid). Yield: 0.31 g, 54% yield.

### Step 5: (5R,6E&Z)-7-oxo-6-(4H,10H-pyrazolo[5,1-c][1,4]benzoxazepin-2-ylmethylene)-4-thia-1-azabicyclo[3.2.0]hept-2-ene-2-carboxylic acid, sodium salt:

Step 6: A 0.5M phosphate buffer solution (pH 6.5) (18mL) was added to a solution of the condensation product (5) (0.300g, 0.468mmol) in THF (18mL). The Pd on Carbon (0. 1 02g) was added and the reaction mixture was hydrogenated at 40psi for two hours. Filtered through celite and removed THF by rotary evaporation. Extracted with EtOAc. Dried organics over sodium sulfate and filtered and concentrated. NaHCO₃ (0.08g, 0.952mmol) was dissolved in a minimal amount of water and added to the concentrated organics along with a small amount of EtOAc. Filtered and removed EtOAc by rotary evaporation. Purified with reverse phase column (MCI Gel CHP20P) and varying amounts of acetonitrile (0% to 15%) in water. Removed the acetonitrile and most of the water from the collected fractions by rotary evaporation. Freeze-dried the rest to obtain 41mg of (5R,6E)-7-oxo-6-(4H,10H-pyrazolo[5,1-c][1,4]benzoxazepin-2-ylmethylene)-4-thia-1-azabicyclo[3.2.0]hept-2-ene-2-carboxylic acid, sodium salt (6) (yellow solid) in 22% yield. HPLC found the purity to be 77% and the E/Z isomer ratio to be 3:2. ¹H-NMR (δ, DMSO-d₆) 5.366 (m, 4H), 5.649 (m, 4H), 6.326 (t, 2H), 6.444 (s, 2H), 6.551 (s, 2H), 6.640 (s, 2H), 6.810 (s, 2H), 6.974 (m, 2H), 7.249 (m, 2H), 7.355 (m, 2H). m/z (M+H)390.0

### Example 42

### (5R),(6Z)-6-(5H-Imidazo[2,1-a]isoindol-2-ylmethylene)-7-oxo-4-thia-1-aza-bicyclo[3.2.0]hept-2-ene-2-carboxylic acid sodium salt

### Step 1: Preparation of 5H-Imidazo[2,1-a]isoindole-2-carbaldehyde

The solution of 2-bromo-3-isopropoxy-propenal (4.97 g) in dry acetonitrile (3 mL) was added to the mixture of 3-amino-1*H*-isoindole (3.4 g) in dry acetonitrile (100 mL). The reaction mixture was stirred for 3.25 h at room temperature. Then triethylamine (3.6 mL) was added to the mixture and heated to reflux for 2 h. The mixture was cooled to room temperature, diluted with ethyl acetate, and washed with 20% potassium hydrogen carbonate. After filtration through a pad of Celite, the organic layer was dried (MgSO₄) and concentrated under reduced pressure. The residue was applied to silica gel column chromatography, then eluted with ethyl acetate - hexane (3/1 - 4/1). The crude compound was crystallized from ethyl acetate and n-hexane to give the title compound (1.04 g, 22%). ¹H NMR (400 MHz, CDCl₃) δ 5.01 (s, 2H), 7.28-7.52 (m, 3H), 7.90 (s, 1H), 7.91-7.93 (m, 1H), 9.92 (s, 1H).

### Step 2: Preparation of (5R,6RS)-6-[(RS)-Acetoxy-(5H imidazol[2,1-a]isoindol-2-yl)-methyl]-6-bromo-7-oxo-4-thia-1-aza-bicyclo[3.2.0]hept-2-ene-2-carboxylic acid 4-nitro-benzyl ester :

5*H*-Imidazo[2,1-*a*]isoindole-2-carbaldehyde (736.8 mg) was added to the dry acetonitrile (50 mL) solution of anhydrous MgBr₂ (1.8 g) under a nitrogen atmosphere at room temperature. The dry THF solution (50 mL) of (5*R*, 6*S*)-6-bromo-7-oxo-4-thia-1-aza-bicyclo[3.2.0]hept-2-ene-2-carboxylic acid 4-nitro-benzyl ester (1.55 g) was added to the reaction mixture, cooled to -20 °C, and triethylamine (1.34 mL) was added in one portion. The reaction vessel was covered with foil to exclude light. The mixture was stirred for 2 h at -20 °C and treated with acetic anhydride (0.76 mL) in one portion. The reaction mixture was warmed to 0 °C and stirred for 18 h at 0 °C. The mixture was diluted with ethyl acetate and washed with H₂O, saturated sodium hydrogen carbonate, and brine. The organic layer was dried (MgSO₄) and filtered through a pad of Celite. The pad was washed with ethyl acetate. The filtrate was concentrated under reduced pressure. The residue was applied to silica gel column chromatography, then eluted with ethyl acetate - hexane (2/3 ~ 1/1). The title compound was obtained as two diastereo mixture (5/1, a pale yellow amorphous solid, 1.8 g, 73%). ¹H NMR (400 MHz, CDCl₃) δ 2.02 (s, 0.84 x 3H), 2.27 (s, 0.16 x 3H), 4.89-4.94 (m, 2H), 5.29 (d, 1H, *J* = 13.6 Hz), 5.47 (d, 1H, *J* = 13.6 Hz), 6.18 (s, 0.16 x 1H), 6.40 (s, 0.84 x 1H), 6.42 (s, 0.84 x 1H), 6.94 (d, 0.16 x 1H, *J* = 0.9 Hz), 7.18 (d, 0.16 x 1H, *J* = 0.7 Hz), 7.35-7.48 (m, 3H), 7.51 (s, 0.84 x 1H), 7.60-7.64 (m, 2H), 7.79-7.83 (m, 1H), 8.23-8.27 (m, 2H).

### Step 3: (5R), (6Z)-6-(5H-Imidazo[2,1-a]isoindol-2-ylmethylene)-7-oxo-4-thia-1-aza-bicyclo[3.2.0]hept-2-ene-2-carboxylic acid sodium salt

(5*R*, 6*RS*)-6-[(*RS*)-Acetoxy-(5*H*-imidazo[2,1-*a*]isoindol-2-yl)-methyl]-6-bromo-7-oxo-4-thia-1-aza-bicyclo[3.2.0]hept-2-ene-2-carboxylic acid 4-nitro-benzyl ester (1.5 g) was dissolved in THF (21 mL) and acetonitrile (9.8 mL). Freshly activated Zn dust (6 g) and 0.5 *M* phosphate buffer (pH 6.4, 30.8 mL) were added to the reaction mixture. The reaction vessel was covered with foil to exclude light. The mixture was vigorously stirred for 2 h at room temperature. The mixture was cooled to 9°C, and 1 *M* NaOH aqueous solution was added to adjust pH to 7.5. The reaction solution was mixed with ethyl acetate and filtered through a pad of Celite. The pad was washed with water and the aqueous layer was separated. The aqueous layer was concentrated to 25 mL under high vacuum at 35°C. The concentrate was applied to Diaion HP-21 (100 mL, Mitsubishi Kasei Co. Ltd.) resin column chromatography. After adsorbing, the column was eluted with water and then with 5-15% acetonitrile-water. The combined fractions was concentrated under high vacuum at 35°C and lyophilized to give the title compound as a yellow amorphous solid (527 mg, 58%). Mp 170°C (dec); ¹H NMR (400 MHz, D₂O) δ 4.62 (s, 2H), 6.27 (s, 1H), 6.56 (s, 1H), 6.78 (s, 1H), 7.22-7.31 (m, 4H), 7.52 (d, 1H, *J* = 6.7 Hz).

## Claims

1. A process for the preparation of compounds of the formula I 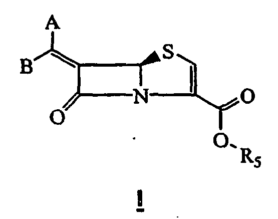 wherein:
one of A and B denotes hydrogen and the other is an aryl optionally substituted with one or two R₂, heteroaryl optionally substituted with one or two R₂, fused bicyclic heteroaryl optionally substituted with one or two R₂, fused tricyclic heteroaryl optionally substituted with one or two R₂, cycloalkyl optionally substituted with one or two R₂, alkyl optionally substituted with one or two R₂, alkenyl optionally substituted with one or two R₂, alkynyl optionally substituted with one or two R₂, saturated or partially saturated heteroaryl optionally substituted with one or two R₂; and wherein any of said heteroaryl moieties containing a NH ring atom may be optionally substituted on said nitrogen by R₁;
R₅ is H, C1 -C6 alkyl, C5 - C6 cycloalkyl, or CHR₃OCOC1-C6alkyl or a salt thereof;
R₁ is H, optionally substituted alkyl, optionally substituted aryl, optionally substituted heteroaryl or mono or bicyclic saturated heterocycles, optionally substituted cycloalkyl, optionally substituted alkenyl, optionally substituted alkynyl with the proviso that neither the double bond nor the triple bond should be present at the carbon atom which is directly linked to N; optionally substituted perfluoroalkyl, -S(O)ₚ optionally substituted alkyl or aryl where p is 0-2, optionally substituted -C=Oheteroaryl, optionally substituted -C=Oaryl, optionally substituted -C=Oalkyl, optionally substituted -C=Ocycloalkyl, optionally substituted -C=O mono or bicyclic saturated heterocycles, optionally substituted C1-C6 alkylaryl, optionally substituted C1-C6 alkylheteroaryl, optionally substituted aryl-C-C6alkyl, optionally substituted heteroaryl-C1-C6alkyl, optionally substituted C1-C6 alkyl mono or bicyclic saturated heterocycles, optionally substituted arylalkenyl of 8 to 16 carbon atoms, -CONR₆R₇, -SO₂NR₆R₇, optionally substituted arylalkyloxyalkyl, optionally substituted -alkyl-O-alkyl-aryl, optionally substituted -alkyl-O-alkyl-heteroaryl, optionally substituted aryloxyalkyl, optionally substituted heteroaryloxyalkyl, optionally substituted aryloxyaryl, optionally substituted aryloxyheteroaryl, optionally substituted C1-C6alkylaryloxyaryl, optionally substituted C1-C6 alkylaryloxyheteroaryl, optionally substituted alkylaryloxyalkylamines, optionally substituted alkoxycarbonyl, optionally substituted aryloxycarbonyl, or optionally substituted heteroaryloxy carbonyl;
R₂ is hydrogen, optionally substituted C1-C6 alkyl, optionally substituted C2-C6 alkenyl, optionally substituted C2-C6 alkynyl, halogen, cyano, N-R₆R₇, optionally substituted C1-C6 alkoxy, hydroxy; optionally substituted aryl, optionally substituted heteroaryl, COOR₆, optionally substituted alkylaryloxyalkylamines, optionally substituted aryloxy, optionally substituted heteroaryloxy, optionally substituted C3-C6 alkenyloxy, optionally substituted C3-C6 alkynyloxy, C1-C6 alkylamino-C1-C6 alkoxy, alkylenedioxy, optionally substituted aryloxy-C1-C6 alkyl amine, C1-C6 perfluoro alkyl, S(O)_{q}-optionally substituted C1-C6 akyl, S(O)q- optionally substituted aryl where q is 0, 1 or 2, CONR₆R₇, guanidino or cyclic guanidino, optionally substituted alkylaryl, optionally substituted arylalkyl, optionally substituted C1-C6 alkylheteroaryl, optionally substituted heteroaryl-C1-C6 alkyl, optionally substituted C1-C6 alkyl mono or bicyclic saturated heterocycles, optionally substituted arylalkenyl of 8 to 16 carbon atoms, SO₂NR₆R₇, optionally substituted arylalkyloxyalkyl, optionally substituted aryloxyalkyl, optionally substituted heteroaryloxyalkyl, optionally substituted aryloxyaryl, optionally substituted aryloxyheteroaryl, optionally substituted heteroaryloxyaryl, optionally substituted C1-C6alkyl aryloxyaryl, optionally substituted C1-C6 alkylaryloxyheteroaryl , optionally substituted aryloxyalkyl, optionally substituted heteroaryloxyalkyl, or optionally substituted alkylaryloxyalkylamine;
R₃ is hydrogen, C1-C6 alkyl, C5- C6 cycloalkyl, optionally substituted aryl, optionally substituted heteroaryl;
R₆ and R₇ are independently H, optionally substituted C1-C6 alkyl, optionally substituted aryl, optionally substituted heteroaryl, optionally substituted C1-C6 alkyl aryl, optionally substituted arylalkyl, optionally substituted heteroarylalkyl, optionally substituted C1-C6 alkyl heteroaryl, R₆ and R₇ can be together to form a 3-7 membered saturated ring system optionally having one or two heteroatoms such as N-R₁, O,S=(O)ₙ n = 0-2;
said process comprising:
(a) condensing an appropriately substituted aldehyde **17**
**A'-CHO** **17**
wherein A' is A as defined as above when B is hydrogen, or B as defined above when A is hydrogen,
with 6-bromo-penem derivative of structure **16** 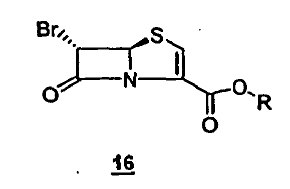 wherein R is p-nitrobenzyl,
in the presence of a Lewis acid and a mild base, at low temperature to form an intermediate aldol product **18** 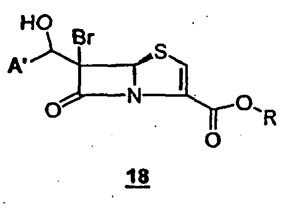 wherein A' and R are as defined above;
(b) reacting intermediate **18** with an acid chloride or anhydride of formula: (R₈)Cl or (R₈)₂O, or with tetrahalomethane of formula: C(X₁)₄, and triphenyl phosphine, wherein R₈ is alkylSO₂, arylSO₂, alkylCO, or arylCO; X₁ is Br, I, or Cl; to form intermediate compound **19** 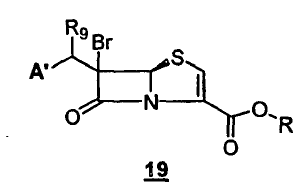 wherein R₉ is X₁ or OR₈ wherein R₈, X₁, A' and R are as defined above; and
(c) converting the intermediate compound **19** to the desired formula I compound wherein R₅ is hydrogen by a reductive elimination process; and if desired converting to a pharmaceutically acceptable salt or to an ester wherein R₅ is C1 -C6 alkyl, C5 - C6 cycloalkyl, or CHR₃OCOC1-C6alkyl.

2. A process according to claim 1 wherein the Lewis acid is anhydrous magnesium halide.

3. A process according to claim 2 wherein the Lewis acid is anhydrous MgBr₂.

4. A process according to any one of claims 1 to 3 wherein the mild base is triethylamine, DMAP or diisopropyl ethyl amine.

5. A process according to any one of claims 1 to 4 wherein the low temperature is from about -20°C to about -40°C.

6. A process according to any one of claims 1 to 5 wherein intermediate compound **19** is an acetate, triflate or a tosylate.

7. A process according to any one of claims 1 to 6 wherein the intermediate compound **19** is not isolated.

8. A process according to any one of claims 1 to 7 wherein step (c) is carried out at a mild temperature.

9. A process according to claim 8 wherein the mild temperature is about 20°C to 35°C.

10. A process according to any one of claims 1 to 9 wherein the reductive elimination process is carried out using activated zinc and a phosphate buffer at a pH of about 6.5 to 8.0 or hydrogenating over a catalyst.

11. A process according to claim 10 wherein the catalyst is palladium on charcoal.

12. A process according to any one of claims 1 to 11 wherein A or B is a fused tricyclic heteroaryl group.

13. A process according to any one of claims 1 to 11 wherein A or B is a fused bicyclic heteroaryl group.

14. A process according to claim 13 wherein the fused bicyclic heteroaryl group has the structural formula 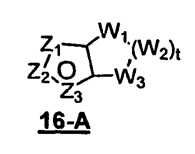
wherein Z₁, Z₂, and Z₃ are independently CR₂, N, O, S or N-R₁ provided one of Z₁, Z₂, or Z₃ is carbon and is bonded to the remainder of the molecule as shown in formula I;
W₁, W₂ and W₃ are independently CR₄R₄, S, SO, SO₂, O, N-R₁, C=O; with the proviso that no S-S or O-O or S-O bond formation can occur to form the saturated ring system;
t= 1 to 4;
R₁, R₂, R₆ and R₇ are as defined in claim 1; and
R₄ is H, optionally substituted C1-C6 alkyl, one of R₄ is OH, C1-C6 alkoxy, -S-C1-C6 alkyl, COOR₆, -NR₆R₇, -CONR₆R₇ ; or R₄R₄ may together be =O or R₄R₄ together with the carbon to which they are attached may form a spiro system of five to eight members with or without the presence of heteroatoms selected N, O, S=(O)ₙ (where n =0 to 2), N-R₁.

15. A process according to claim 13 wherein the fused bicyclic heteroaryl group has the structural formula 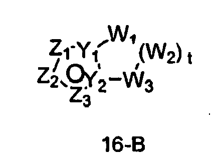 wherein
Z₁, Z₂ and Z₃ are independently CR₂, N, O, S or N-R₁ provided one of Z₁ -Z₃ is carbon and is bonded to the remainder of the molecule;
W₁, W₂ and W₃ are independently CR₄R₄, S, SO, SO₂, O, or N-R₁;
t= 1 to 4;
Y₁ and Y₂ are independently N or C; with the proviso that if the aromatic ring portion of the bicyclic heteroaryl group is imidazole, the nonaromatic ring portion may not contain a S adjacent to the bridgehead carbon;
R₁ and R₂ are as defined in claim 1;
R₄ is H, optionally substituted C1-C6 alkyl, one of R₄ is OH, C1-C6 alkoxy, -S-C1-C6 alkyl, COOR₆, -NR₆R₇, -CONR₆R₇; or R₄R₄ may together be =O or R₄R₄ together with the carbon to which they are attached may form a spiro system of five to eight members with or without the presence of heteroatoms selected from N, O, S=(O)n (where n =0 to 2), and N-R₁; and
R₆ and R₇ are independently H, optionally substituted C1-C6 alkyl, optionally substituted aryl, optionally substituted heteroaryl, optionally substituted C1-C6 alkylaryl, optionally substituted arylalkyl, optionally substituted heteroarylalkyl, optionally substituted C1-C6 alkylheteroaryl, or R₆ and R₇ can be together to form a 3-7 membered saturated ring system optionally having one or two heteroatoms selected from N, O, or S.

16. A process according to claim 13 wherein the fused bicyclic neteroaryl group is 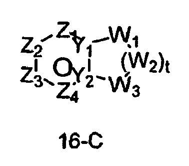 wherein
Z1, Z2, Z3 and Z4 are independently CR₂ or N provided one of Z1 -Z4 is carbon and is bonded to the remainder of the molecule;
W₁, W₂ and W₃ are independently CR₄R₄, S, SO, SO₂, O, or N-R₁; with the proviso that no S-S or O-O or S-O bond formation can occur to form the saturated ring system;
t= 1 to 4;
Y₁ and Y₂ are independently C or N;
R₁, R₂, R₄, R₆, and R₇ are as defined in claim 13.

17. The process according to claim 12 wherein the fused tricyclic heteroaryl group has the formula 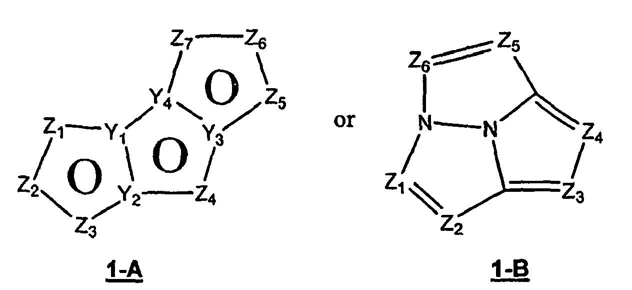
wherein Z₁, Z₂, Z₃, Z₄, Z₅ , Z₆ and Z₇ are independently CR₂ , N, O, S or N-R₁ provided one of Z₁- Z₇ is a carbon atom to which the remainder of the molecule is attached;
R₁, R₂, R₆ and R₇ are as defined in claim 1;
and Y₁, Y₂, Y₃ and Y₄ may independently be C or N.

18. A process according to claim 12 wherein the tricyclic heteroaryl group is 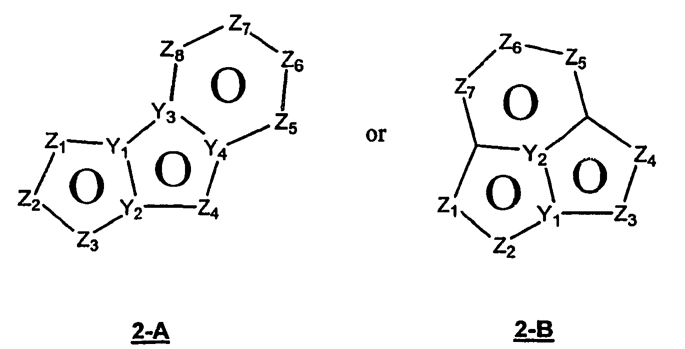
wherein Z₁, Z₂, Z₃, Z₄,Z₅,Z₆,Z₇ and Z₈ are independently CR₂, N, O, S or N-R₁ provided one of the Z₁- Z₈ is a carbon atom to which the remainder of the molecule is attached;
R₁, R₂, R₆ and R₇ are as defined in claim 1;
and Y₁, Y₂,Y₃ and Y₄ are independently C or N.

19. A process according to claim 12 wherein the tricyclic heteroaryl group is 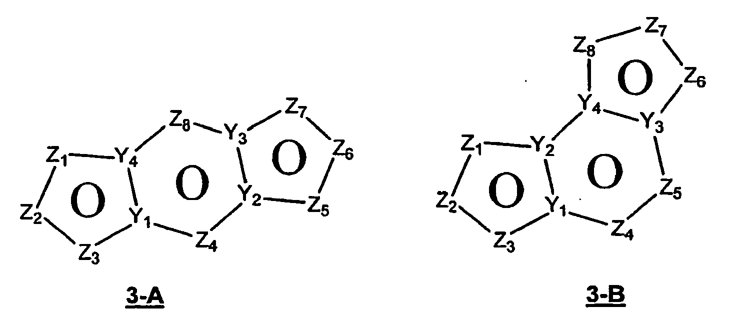
wherein Z₁, Z₂, Z₃, Z₄, Z₅, Z₆, Z₇ and Z₈ are independently CR₂, N, O, S or N-R₁ provided one of Z₁- Z₈ is a carbon atom to which the remainder of the molecule is attached;
R₁, R₂, R₆ and R₇ are as defined in claim 1;
and Y₁, Y₂, Y₃ and Y₄ may be C or N.

20. A process according to claim 12 wherein the tricyclic heteroaryl group is 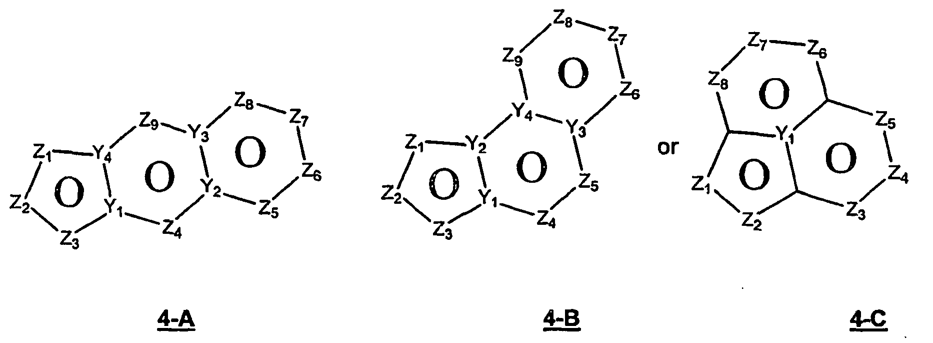
wherein Z₁, Z_{2,} Z₃, Z₄, Z₅ , Z₆ , Z₇, Z₈ and Z₉ are independently CR₂, N, O. S or N-R₁ provided one of the Z₁ - Z₉ is a carbon atom to which the remainder of the molecule is attached;
R₁. R₂, R₆ and R₇ are as defined in claim 1; and
Y₁, Y₂, Y₃ and Y₄ are independently C or N.

21. A process according to claim 12 wherein the tricyclic heteroaryl group is 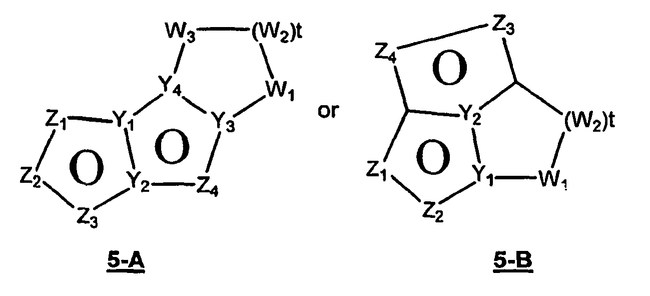
wherein Z₁, Z_{2,} Z₃ and Z₄ are independently CR₂, N, O, S or N-R₁ provided one of Z₁ - Z₄ is a carbon atom to which the remainder of the molecule is attached;
Y₁, Y₂, Y₃ and Y₄ are independently C or N;
W₁, W₂ and W₃ are independently CR₄R₄, S(O)r ( r = 0 -2), O, or N-R₁ with the proviso that no S-S, S-O or O-O bond formation can occur to form a saturated ring;
R₁, R₂, R₆ and R₇ are as defined in claim 1;
R₄ i s H, optionally substituted C1-C6 alkyl, OH (provided both R4 are not OH), C1-C6 alkoxy, -S-C1-C6 alkyl, COOR₆, -NR₆R₇, -CONR₆R₇ ; or R₄R₄ may together be =O or R₄R₄ together with the carbon to which they are attached may form a spiro system of five to eight members with or without the presence of heteroatoms selected N, O, S(O)n (where n =0 to 2), N-R₁;
and t = 1 to 3.

22. A process according to claim 12 wherein the tricyclic heteroaryl group is 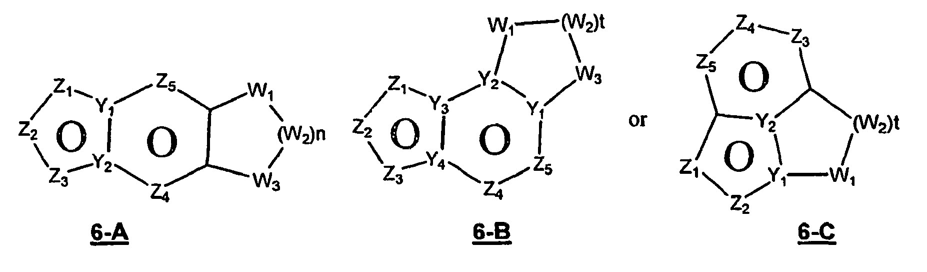
wherein Z₁. Z₂, Z₃, Z₄ and Z₅ are independently CR₂, N, O, S or N-R₁ provided one of Z₁ -
Z₅ is a carbon atom to which the remainder of the molecule is attached;
Y₁, and Y₂ are independently C or N;
W₁, W₂ and W₃ are independently CR₄R₄ , S(O)r ( r = 0 -2), O, or N-R₁ with the proviso that no S-S, S-O or O-O bond formation can occur to form a saturated ring;
R₁, R₂, R₆ and R₇ are as defined in claim 1;
R₄ is H, optionally substituted C1-C6 alkyl, OH (provided both R4 are not OH), C1-C6 alkoxy, -S-C1-C6 alkyl, COOR₆, -NR₆R₇, -CONR₆R₇ ; or R₄R₄ may together be =O or R₄R₄ together with the carbon to which they are attached may form a spiro system of five to eight members with or without the presence of heteroatoms selected N, O, S(O)n (where n =0 to 2), N-R₁;
and t = 1 to 3.

23. A process according to claim 12 wherein the tricyclic heteroaryl group is 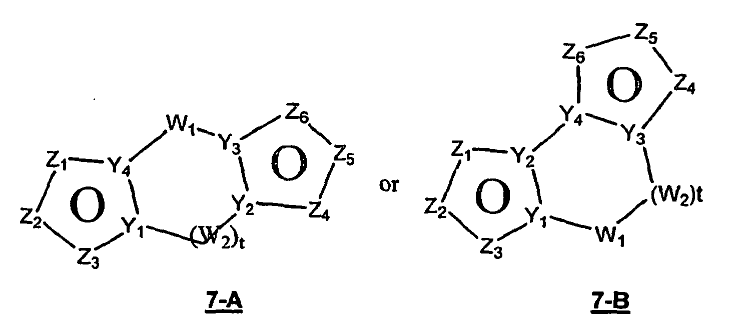
wherein Z₁, Z₂, Z₃, Z₄, Z₅ and Z₆ are independently CR₂ , N, O, S, and N-R₁; provided one
of Z₁ - Z₆ is a carbon atom to which the remainder of the molecule is attached; Y₁, Y₂, Y₃ and Y₄ are independently C or N;
W₁ and W₂ are independently CR₄R₄ , S(O)r ( r = 0 -2) , O, N-R₁ with the proviso that no S-S, S-O or O-O bond formation can occur to form a saturated ring;
R₁, R₂, R₆ and R₇ are as defined in claim 1;
R₄ is H, optionally substituted C1-C6 alkyl, OH (provided both R4 are not OH), C1-C6 alkoxy, -S-C1-C6 alkyl, COOR₆, -NR₆R₇, -CONR₆R₇ ; or R₄R₄ may together be =O or R₄R₄ together with the carbon to which they are attached may form a spiro system of five to eight members with or without the presence of heteroatoms selected N, O, S(O)n (where n =0 to 2), N-R₁;
and t = 1 to 3.

24. The process according to claim 12 wherein the tricyclic heteroaryl group is 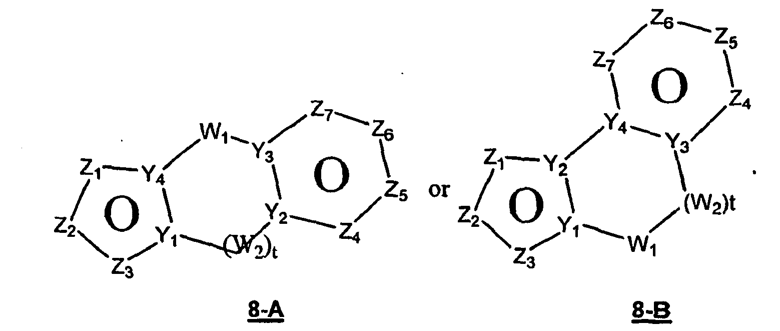
wherein Z₁, Z₂, Z₃, Z₄, Z₅, Z₆ and Z₇ are indepdently CR₂ , N, O, S or N-R₁ provided one of the Z₁ - Z₇ is a carbon atom to which the remainder of the molecule is attached;
Y₁,Y₂, Y₃ and Y₄ are independently C or N;
W₁ and W₂ are independently CR₄R₄ , S(O)r ( r = 0 -2) , O, or N-R₁ with the proviso that no S-S, S-O or O-O bond formation can occur to form a saturated ring;
R₁, R₂, R₆ and R₇ are as defined in claim 1;
R₄ is H, optionally substituted C1-C6 alkyl, OH (provided both R4 are not OH), C1-C6 alkoxy, -S-C1-C6 alkyl, COOR₆, -NR₆R₇, -CONR₆R₇ ; or R₄R₄ may together be =O or R₄R₄ together with the carbon to which they are attached may form a spiro system of five to eight members with or without the presence of heteroatoms selected N, O, S(O)n (where n =0 to 2), N-R₁;
and t = 0-3.

25. A process according to claim 12 wherein the tricyclic heteroaryl group is 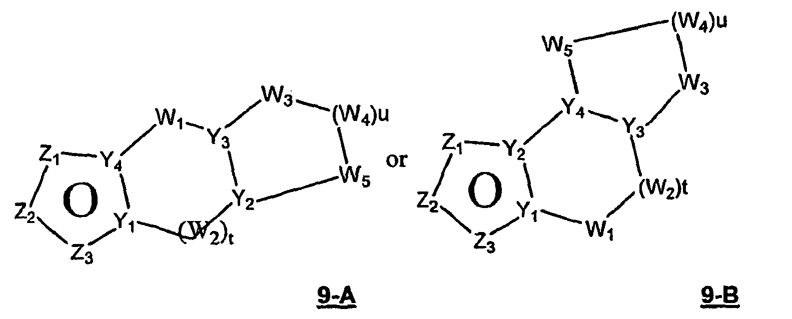
wherein Z, , Z₂ and Z₃ are independently CR₂, N, O, S or N-R₁ provided one of Z₁ - Z₃ is a carbon atom to which the remainder of the molecule is attached;
Y₁ and Y₄ are independently C or N;
Y₂ and Y₃ are independently CH or N;
W₁. W₂, W₃, W₄ and W₅ are independently CR₄R₄, S(O)r ( r = 0 -2), O, or N-R₁ with the proviso that no S-S, S-O or O-O bond formation can occur to form a saturated ring;
R₁, R₂, R₆ and R₇ are as defined in claim 1;
R₄ is H, optionally substituted C1-C6 alkyl, OH (provided both R4 are not OH), C1-C6 alkoxy, -S-C1-C6 alkyl, COOR₆, -NR₆R₇, -CONR₆R₇ ; or R₄R₄ may together be =O or R₄R₄ together with the carbon to which they are attached may form a spiro system of five to eight members with or without the presence of heteroatoms selected N, O, S(O)n (where n =0 to 2), N-R₁;
t = 0 to 2; and
u = 1 to 3.

26. A process according to claim 12 wherein the tricyclic heteroaryl group is 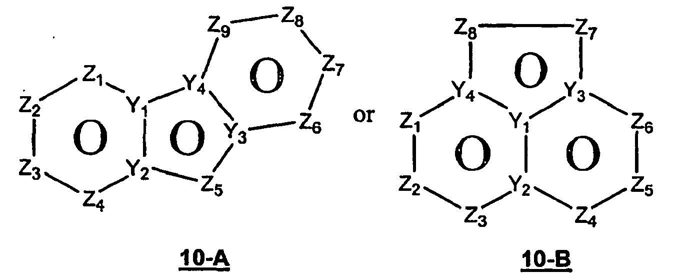 wherein Z₁, Z_{2.} Z₃, Z₄, Z₅ , Z₆, Z₇, Z₈ and Z₉ are independently CR₂, N, O, S or N-R₁ provided one of the Z₁ - Z₉ is a carbon atom to which the remainder of the molecule is attached; R₁, R₂, R₆ and R₇ are as defined in claim 1; and Y₁, Y₂, Y₃ and Y₄ are independently C or N.

27. A process according to claim 12 wherein the tricyclic heteroaryl group is 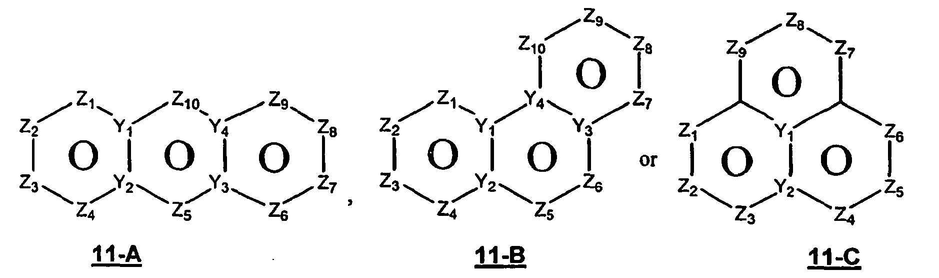
wherein Z₁, Z₂. Z₃, Z₄, Z₅, Z₆, Z₇, Z₈, Z₉ and Z₁₀ are independently CR₂, N, O, S or N-R₁ provided one of Z₁ - Z₁₀ is a carbon atom to which the remainder of the molecule is attached;
R₁. R₂, R₆ and R₇ are as defined in claim 1; and
Y₁, Y₂, Y₃ and Y₄ are independently C or N.

28. A process according to claim 12 wherein the tricyclic heteroaryl group is 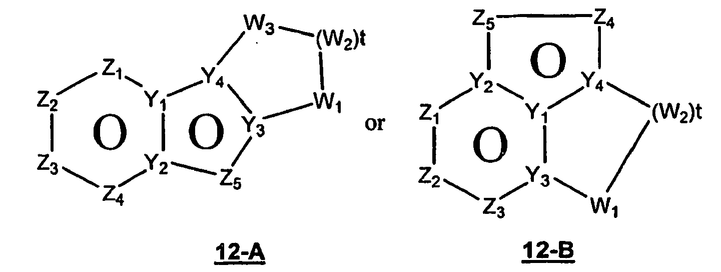 wherein
Z₁, Z₂, Z₃, Z₄ and Z₅ are independently CR₂ , N, O, S or N-R₁ provided that one of Z₁ - Z₅ is a carbon atom to which the remainder of the molecule is attached;
Y₁, Y₂, Y₃ and Y₄ are independently C or N;
W₁, W₂, W₃ are independently CR₄R₄ O, N-R₁, or S=(O), (r = 0-2) with the proviso that no S-S, S-O or O-O bond formation can occur to form a saturated ring;
R₁, R₂, R₆ and R₇ are as defined in claim 1;
R₄ is H, optionally substituted C1-C6 alkyl, OH (provided both R4 are not OH), C1-C6 alkoxy, -S-C1-C6 alkyl, COOR₆, -NR₆R₇, -CONR₆R₇ ; or R₄R₄ may together be =O or R₄R₄ together with the carbon to which they are attached may form a spiro system of five to eight members with or without the presence of heteroatoms selected N, O, S(O)n (where n =0 to 2), N-R₁;
and t = 1-4.

29. A process according to claim 12 wherein the tricyclic heteroaryl group is 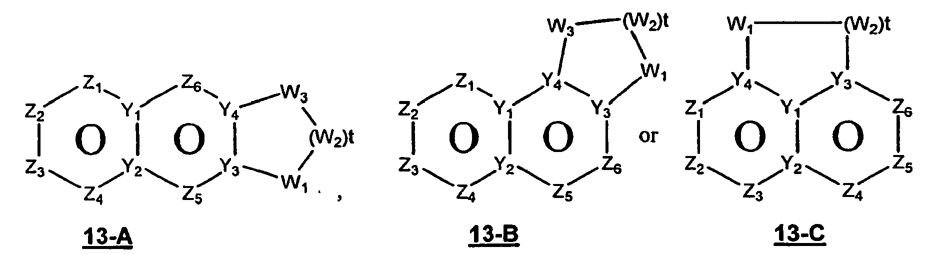
wherein Z₁ , Z₂, Z₃, Z₄, Z₅ and Z₆ are independently CR₂ , N, O, S or N-R₁ provided one of Z₁ - Z₆ is a carbon atom to which the remainder of the molecule is attached;
Y₁, Y₂ , Y₃ and Y₄ are independently C or N;
W₁, W₂ and W₃ are independently CR₄R₄, S(O)r ( r = 0 -2) , O, or N-R₁ with the proviso that no S-S, S-O or O-O bond formation can occur to form a saturated ring;
R₁, R₂, R₆ and R₇ are as defined in claim 1;
R₄ is H, optionally substituted C1-C6 alkyl, OH (provided both R4 are not OH), C1-C6 alkoxy, -S-C1-C6 alkyl, COOR₆, -NR₆R₇, -CONR₆R₇ ; or R₄R₄ may together be =O or R₄R₄ together with the carbon to which they are attached may form a spiro system of five to eight members with or without the presence of heteroatoms selected N, O, S(O)n (where n =0 to 2), N-R₁;
and t = 1 to 3.

30. A process according to claim 12 wherein the tricyclic heteroaryl group is 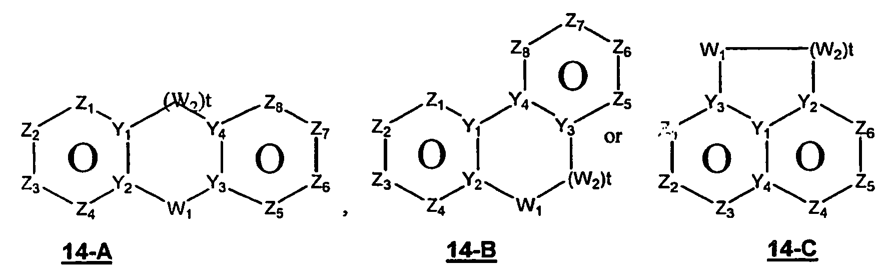
wherein Z₁, Z₂, Z₃, Z₄, Z₅, Z₆, Z₇ and Z₈ are independently CR₂, N, O, S or N-R₁ provided one of Z₁ - Z₈ is a carbon atom to which the remainder of the molecule is attached;
Y₁. Y₂, Y₃ and Y₄ are independently C or N;
W₁, and W₂ are independently CR₄R₄ , S(O)r ( r = 0 -2) , O, or N-R₁ with the proviso that no S-S, S-O or O-O bond formation can occur to form a saturated ring;
R₁, R₂, R₆ and R₇ are as defined in claim 1;
R₄ is H, optionally substituted C1-C6 alkyl, OH (provided both R4 are not OH), C1-C6 alkoxy, -S-C1-C6 alkyl, COOR₆, -NR₆R₇, -CONR₆R₇ ; or R₄R₄ may together be =O or R₄R₄ together with the carbon to which they are attached may form a spiro system of five to eight members with or without the presence of heteroatoms selected N, O, S(O)n (where n =0 to 2), N-R₁;
and t = 1 to 2.

31. A process according to claim 12 wherein the tricyclic heteroaryl group is 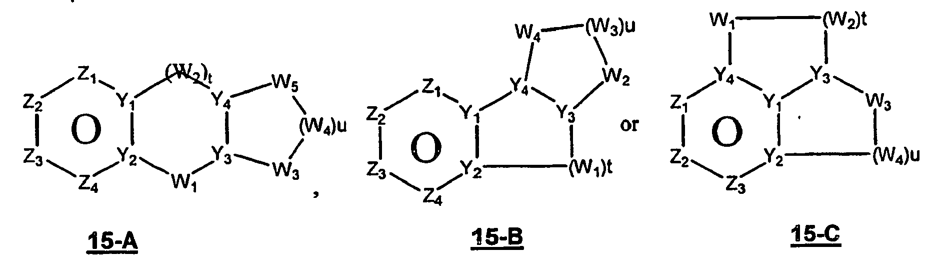
wherein Z₁, Z₂. Z₃ and Z₄ are independently CR₂, N, O, S or N-R₁ provided one of Z₁ - Z₄ is a carbon atom to which the remainder of the molecule is attached;
Y₁, Y₂, Y₃ and Y₄ are independently C or N;
W₁, W₂, W₃, W₄ and W₅ are independently CR₄R₄ , S(O)r ( r = 0 -2) , O, or N-R₁ with the proviso that no S-S, S-O or O-O bond formation can occur to form a saturated ring;
R₁, R₂, R₆ and R₇ are as defined in claim 1;
R₄ is H, optionally substituted C1-C6 alkyl, OH (provided both R4 are not OH), C1-C6 alkoxy, -S-C1-C6 alkyl, COOR₆, -NR₆R₇, -CONR₆R₇ ; or R₄R₄ may together be =O or R₄R₄ together with the carbon to which they are attached may form a spiro system of five to eight members with or without the presence of heteroatoms selected N, O, S(O)n (where n =0 to 2), N-R₁;
t = 1 to 3; and
u = 1 to 3.

32. The 6-bromo-penem derivative of structure **16** 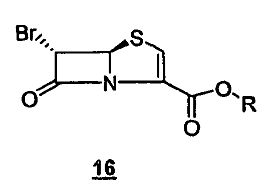 wherein R is p-nitrobenzyl.

33. A process for the preparation of 4-nitrobenzyl (5R,6S)-6-bromopenem-3-carboxylate as claimed in claim 32 which comprises the following steps:
(A) (i) reacting 6-aminopenicillanic acid with hydrobromic acid in an organic solvent and water to form the 6-bromo derivative **21** 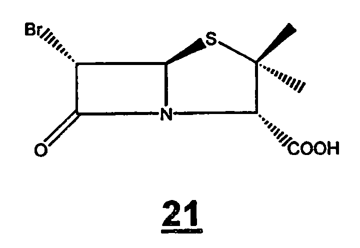 and (ii) converting the 6-bromopenicillanic acid **21** derivative to the p-nitrobenzyl 6-brompenicillanate **22** 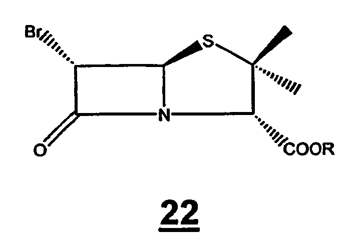 whrein R is p-nitrobenzyl, using 4-nitrobenzylbromide in the presence of base in an organic solvent;
(B) oxidizing the 4-nitrobenzyl 6-bromopenicillanate **22** to form 4-nitrobenzyl 6-bromopenicillanate 1-oxide **23** 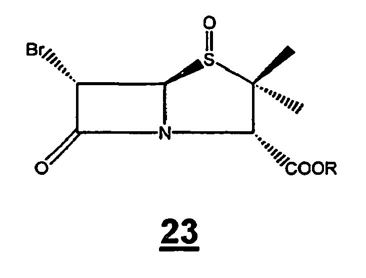
(C) refluxing the 4-nitrobenzyl 6-bromopenicillanate 1-oxide **23** with 2-mercaptobenzothiazole in an aromatic solvent to form 4-nitrobenzyl(2R)-2-[(3S,4R)-4-(benzothiazol-2-yldithio)-3-bromo-2-oxoazetidine-1-yl]-3-methylbut-3-enoate **24** ;
(D) dissolving the 4-nitrobenzyl(2R)-2-[(3S,4R)-4-(benzothiazol-2-yldithio)-3-bromo-2-oxoazetidine-1-yl]-3-methylbut-3-enoate **24** in an organic solvent and reacting with an organic tertiary base to form 4-nitrobenzyl-2-[(3S,4R)-4-(benzothiazol-2-yldithio)-3-bromo-2-oxoazetidine-1-yl]-3-methylbut-2-enoate **25**
(E) converting the 4-nitrobenzyl-2-[(3S,4R)-4-(benzothiazol-2-yldithio)-3-bromo-2-oxoazetidine-1-yl]-3-methylbut-2-enoate **25** to 4-nitrobenzyl 2-[(3S,4R)-3-bromo-4-formylthio-2-oxoazetidin-1-yl]-3-methylbut-2-enoate **26** by reacting in an aromatic organic solvent in the presence of an organic acid, acetic anhydride/ organic tertiary base and trialkyl or triaryl phosphine at from about -10° C to about -30° C;
(F) said 4-nitrobenzyl 2-[(3S,4R)-3-bromo-4-formylthio-2-oxoazetidin-1-yl]-3-methylbut-2-enoate **26** being taken up in an organic solvent at -70°C to -90° C and ozonized oxygen being passed through it for 3 to 4 hrs followed by intramolecular cyclization using a phosphite reagent to form 4-nitrobenzyl (5R,6S)-6-bromopenem-3-carboxylate **16.**

34. A process according to claim 1 for the preparation of compound of formula **I** 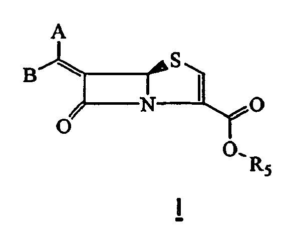 as defined in claim 1 in which the 4-nitrobenzyl (5R,6S)-6-bromopenem-3-carboxylate **16** used in step (a) is prepared according to claim 33.

35. A process according to claim 33 or claim 34 wherein the 6-aminopenicillanic acid is dissolved in methanol or THF.

36. A process according to any one of claims 33 to 35 wherein step (A)(i) is performed in the presence of 48% w/w hydrobromic acid and sodium or potassium nitrite solution.

37. A process according to any one of claims 33 to 36 wherein step (A)(i) is performed at a temperature from about -10° C to about-30°C.

38. A process according to any one of claims 33 to 37 wherein the base in step (A)(ii) is sodium or potassium carbonate and the organic solvent is THF or DMF.

39. A process according to any one of claims 33 to 38 wherein the aromatic solvent in step (C) is toluene or xylene.

40. A process according to any one of claims 33 to 39 comprising the sequential conversion of compound **23** to **26** wherein there is no isolation of the intermediates.

41. A process according to claim 40 wherein the 4-nitrobenzyl 6-bromopenicillanate 1-oxide **23** is reacted with mercaptobenzothiazole in refluxing aromatic organic solvent and is treated with triethylamine at about 0 to -20° C to form a reaction mixture; said reaction mixture is charged with an organic acid and an anhydride, an organic tertiary base and a trialkyl or triaryl phosphate sequentially at about -10° C to -40° C.

## Patentansprüche

1. Verfahren zur Herstellung von Verbindungen der Formel **I** 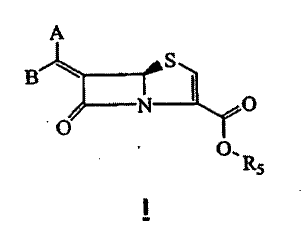 wobei:
eines von A und B Wasserstoff bedeutet und das andere Folgendes ist: ein gegebenenfalls mit einem oder zwei R₂ substituiertes Aryl, ein gegebenenfalls mit einem oder zwei R₂ substituiertes Heteroaryl, ein gegebenenfalls mit einem oder zwei R₂ substituiertes kondensiertes bicyclisches Heteroaryl, ein gegebenenfalls mit einem oder zwei R₂ substituiertes kondensiertes tricyclisches Heteroaryl, ein gegebenenfalls mit einem oder zwei R₂ substituiertes Cycloalkyl, ein gegebenenfalls mit einem oder zwei R₂ substituiertes Alkyl, ein gegebenenfalls mit einem oder zwei R₂ substituiertes Alkenyl, ein gegebenenfalls mit einem oder zwei R₂ substituiertes Alkinyl, ein gegebenenfalls mit einem oder zwei R₂ substituiertes gesättigtes oder teilweise gesättigtes Heteroaryl; und wobei irgendeiner der ein NH-Ringatom enthaltenden Heteroarylreste gegebenenfalls an dem Stickstoff durch R₁ substituiert sein kann;
R₅ H, C₁-C₆-Alkyl, C₅-C₆-Cycloalkyl oder CHR₃OCO-C₁-C₆-Alkyl oder ein Salz davon ist;
R₁ Folgendes ist: H, gegebenenfalls substituiertes Alkyl, gegebenenfalls substituiertes Aryl, gegebenenfalls substituiertes Heteroaryl oder gegebenenfalls substituierte mono- oder bicyclische gesättigte Heterocyclen, gegebenenfalls substituiertes Cycloalkyl, gegebenenfalls substituiertes Alkenyl, gegebenenfalls substituiertes Alkinyl unter der Voraussetzung, dass weder die Doppelbindung noch die Dreifachbindung an dem Kohlenstoffatom vorhanden sein sollten, welches direkt mit N verbunden ist; gegebenenfalls substituiertes Perfluoralkyl, gegebenenfalls substituiertes -S(O)p-Alkyl oder -S(O)p-Aryl, wobei p 0-2 ist, gegebenenfalls substituiertes -C=O-Heteroaryl, gegebenenfalls substituiertes -C=O-Aryl, gegebenenfalls substituiertes -C=O-Alkyl, gegebenenfalls substituiertes - C=O-Cycloalkyl, gegebenenfalls substituierte mono- oder bicyclische gesättigte -C=O-Heterocyclen, gegebenenfalls substituiertes C₁-C₆-Alkylaryl, gegebenenfalls substituiertes C₁-C₆-Alkylheteroaryl, gegebenenfalls substituiertes Aryl-C₁-C₆-Alkyl., gegebenenfalls substituiertes Heteroaryl-C₁-C₆-Alkyl, gegebenenfalls substituierte mono- oder bicyclische gesättigte C₁-C₆-Alkyl-Heterocyclen, gegebenenfalls substituiertes Arylalkenyl mit 8 bis 16 Kohlenstoffatomen, -CONR₆R₇, -SO₂NR₆R₇, gegebenenfalls substituiertes Arylalkyloxyalkyl, gegebenenfalls substituiertes -Alkyl-O-Alkyl-Aryl, gegebenenfalls substituiertes -Alkyl-O-Alkyl-Heteroaryl, gegebenenfalls substituiertes Aryloxyalkyl, gegebenenfalls substituiertes Heteroaryloxyalkyl, gegebenenfalls substituiertes Aryloxyaryl, gegebenenfalls substituiertes Aryloxyheteroaryl, gegebenenfalls substituiertes C₁-C₆-Alkylaryloxyaryl, gegebenenfalls substituiertes C₁-C₆-Alkylaryloxyheteroaryl, gegebenenfalls substituierte Alkylaryloxyalkylamine, gegebenenfalls substituiertes Alkoxycarbonyl, gegebenenfalls substituiertes Aryloxycarbonyl oder gegebenenfalls substituiertes Heteroaryloxycarbonyl;
R₂ Folgendes ist: Wasserstoff, gegebenenfalls substituiertes C₁-C₆-Alkyl, gegebenenfalls substituiertes C₂-C₆-Alkenyl, gegebenenfalls substituiertes C₂-C₆-Alkinyl, Halogen, Cyano, N-R₆R₇, gegebenenfalls substituiertes C₁-C₆-Alkoxy, Hydroxy; gegebenenfalls substituiertes Aryl, gegebenenfalls substituiertes Heteroaryl, COOR₆, gegebenenfalls substituierte Alkylaryloxyalkylamine, gegebenenfalls substituiertes Aryloxy, gegebenenfalls substituiertes Heteroaryloxy, gegebenenfalls substituiertes C₃-C₆-Alkenyloxy, gegebenenfalls substituiertes C₃-C₆-Alkinyloxy, C₁-C₆-Alkylamino-C₁-C₆-Alkoxy, Alkylendioxy, gegebenenfalls substituiertes Aryloxy-C₁-C₆-Alkylamin, C₁-C₆-Perfluoralkyl, gegebenenfalls substituiertes S(O)_{q}-C₁-C₆-Alkyl, gegebenenfalls substituiertes S(O)q-Aryl, wobei q 0, 1 oder 2 ist, CONR₆R₇, Guanidino oder cyclisches Guanidino, gegebenenfalls substituiertes Alkylaryl, gegebenenfalls substituiertes Arylalkyl, gegebenenfalls substituiertes C₁-C₆-Alkylheteroaryl, gegebenenfalls substituiertes Heteroaryl-C₁-C₆-Alkyl, gegebenenfalls substituierte mono- oder bicyclische gesättigte C₁-C₆-Alkyl-Heterocyclen, gegebenenfalls substituiertes Arylalkenyl mit 8 bis 16 Kohlenstoffatomen, SO₂NR₆R₇, gegebenenfalls substituiertes Arylalkyloxyalkyl, gegebenenfalls substituiertes Aryloxyalkyl, gegebenenfalls substituiertes Heteroaryloxyalkyl, gegebenenfalls substituiertes Aryloxyaryl, gegebenenfalls substituiertes Aryloxyheteroaryl, gegebenenfalls substituiertes Heteroaryloxyaryl, gegebenenfalls substituiertes C₁-C₆-Alkylaryloxyaryl, gegebenenfalls substituiertes C₁-C₆-Alkylaryloxyheteroaryl, gegebenenfalls substituiertes Aryloxyalkyl, gegebenenfalls substituiertes Heteroaryloxyalkyl oder gegebenenfalls substituiertes Alkylaryloxyalkylamin;
R₃ Wasserstoff, C₁-C₆-Alkyl, C₅-C₆-Cycloalkyl, gegebenenfalls substituiertes Aryl, gegebenenfalls substituiertes Heteroaryl ist;
R₆ und R₇ unabhängig voneinander Folgendes sind: H, gegebenenfalls substituiertes C₁-C₆-Alkyl, gegebenenfalls substituiertes Aryl, gegebenenfalls substituiertes Heteroaryl, gegebenenfalls substituiertes C₁-C₆-Alkylaryl, gegebenenfalls substituiertes Arylalkyl, gegebenenfalls substituiertes Heteroarylalkyl, gegebenenfalls substituiertes C₁-C₆-Alkylheteroaryl, R₆ und R₇ zusammen sein können, um ein 3-7-gliedriges gesättigtes Ringsystem zu bilden, das gegebenenfalls ein oder zwei Heteroatome aufweist wie beispielsweise N-R₁, O, S=(O)ₙ, wobei n = 0-2;
wobei das Verfahren Folgendes umfasst:
(a) Kondensieren eines in geeigneter Weise substituierten Aldehyds 17
**A'-CHO** **17**
wobei A' das wie vorstehend definierte A ist, wenn B Wasserstoff ist, oder das wie vorstehend definierte B ist, wenn A Wasserstoff ist,
mit einem 6-Brom-Penemderivat der Struktur **16** 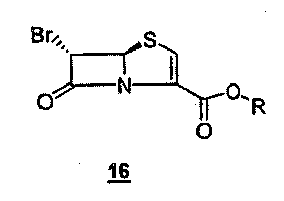 wobei R p-Nitrobenzyl ist,
in Gegenwart einer Lewis-Säure und einer milden Base bei niedriger Temperatur, um ein Aldol-Zwischenprodukt **18** zu bilden 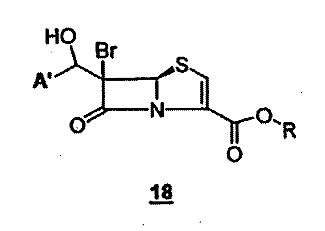 wobei A' und R wie vorstehend definiert sind;
(b) Reagieren des Zwischenprodukts **18** mit einem Säurechlorid oder -anhydrid der Formel (R₈)Cl oder (R₈)₂O oder mit einem Tetrahalogenmethan der Formel C(X₁)₄ und Triphenylphosphin, wobei R₈ Alkyl-SO₂, Aryl-SO₂, Alkyl-CO oder Aryl-CO ist; X₁ Br, I oder Cl ist;
um eine Zwischenverbindung **19** zu bilden 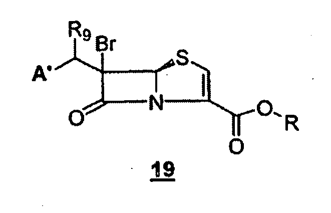 wobei R₉ X₁ oder OR₈ ist, wobei R₈, X₁, A' und R wie vorstehend definiert sind; und
(c) Umsetzen der Zwischenverbindung **19** zu der gewünschten Verbindung der Formel !, wobei R₅ Wasserstoff ist, durch ein Verfahren der reduktiven Eliminierung; und gewünschtenfalls Umsetzen zu einem pharmazeutisch annehmbaren Salz oder zu einem Ester, wobei R₅ C₁-C₆-Alkyl, C₅-C₆-Cycloalkyl oder CHR₃OCO-C₁-C₆-Alkyl ist.

2. Verfahren nach Anspruch 1, wobei die Lewis-Säure ein wasserfreies Magnesiumhalogenid ist.

3. Verfahren nach Anspruch 2, wobei die Lewis-Säure wasserfreies MgBr₂ ist.

4. Verfahren nach irgendeinem der Ansprüche 1 bis 3, wobei die milde Base Triethylamin, DMAP oder Diisopropylethylamin ist.

5. Verfahren nach irgendeinem der Ansprüche 1 bis 4, wobei die niedrige Temperatur ungefähr -20°C bis ungefähr -40°C beträgt.

6. Verfahren nach irgendeinem der Ansprüche 1 bis 5, wobei die Zwischenverbindung **19** ein Acetat, Triflat oder Tosylat ist.

7. Verfahren nach irgendeinem der Ansprüche 1 bis 6, wobei die Zwischenverbindung **19** nicht isoliert wird.

8. Verfahren nach irgendeinem der Ansprüche 1 bis 7, wobei Schritt (c) bei einer milden Temperatur durchgeführt wird.

9. Verfahren nach Anspruch 8, wobei die milde Temperatur ungefähr 20°C bis 35°C beträgt.

10. Verfahren nach irgendeinem der Ansprüche 1 bis 9, wobei das Verfahren der reduktiven Eliminierung unter Verwendung von aktiviertem Zink und einem Phosphatpuffer bei einem pH-Wert von ungefähr 6,5 bis 8,0 oder durch Hydrieren über einem Katalysator durchgeführt wird.

11. Verfahren nach Anspruch 10, wobei der Katalysator Palladium auf Holzkohle ist.

12. Verfahren nach irgendeinem der Ansprüche 1 bis 11, wobei A oder B eine kondensierte tricyclische Heteroarylgruppe ist.

13. Verfahren nach irgendeinem der Ansprüche 1 bis 11, wobei A oder B eine kondensierte bicyclische Heteroarylgruppe ist.

14. Verfahren nach Anspruch 13, wobei die kondensierte bicyclische Heteroarylgruppe die Strukturformel 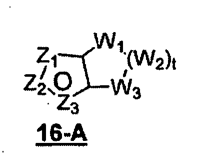 hat,
wobei Z₁, Z₂ und Z₃ unabhängig voneinander CR₂, N, O, S oder N-R₁ sind, vorausgesetzt, dass eines von Z₁, Z₂ oder Z₃ Kohlenstoff ist und wie in Formel **I** dargestellt an den übrigen Teil des Moleküls gebunden ist;
W₁, W₂ und W₃ unabhängig voneinander CR₄R₄, S, SO, SO₂, O, N-R₁, C=O sind; unter der Voraussetzung, dass keine Bildung einer S-S- oder O-O- oder S-O-Bindung zum Bilden des gesättigten Ringsystems auftreten kann;
t = 1 bis 4;
R₁, R₂, R₆ und R₇ wie in Anspruch 1 definiert sind; und
R₄ Folgendes ist: H, gegebenenfalls substituiertes C₁-C₆-Alkyl, eines von R₄ OH ist, C₁-C₆-Alkoxy, -S-C₁-C₆-Alkyl, COOR₆, -NR₆R₇, -CONR₆R₇; oder R₄R₄ zusammen =O sein können oder R₄R₄ zusammen mit dem Kohlenstoff, an welchen sie gebunden sind, ein Spirosystem mit fünf bis acht Gliedern mit oder ohne Vorhandensein von Heteroatomen bilden können, die aus N, O, S=(O)ₙ (wobei n = 0 bis 2), N-R₁ ausgewählt sind.

15. Verfahren nach Anspruch 13, wobei die kondensierte bicyclische Heteroarylgruppe die Strukturformel 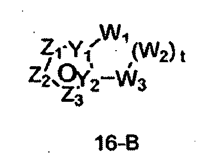 hat,
wobei
Z₁, Z₂ und Z₃ unabhängig voneinander CR₂, N, O, S oder N-R₁ sind, vorausgesetzt, dass eines von Z₁ - Z₃ Kohlenstoff ist und an den übrigen Teil des Moleküls gebunden ist;
W₁, W₂ und W₃ unabhängig voneinander CR₄R₄, S, SO, SO₂, O oder N-R₁ sind;
t = 1 bis 4;
Y₁ und Y₂ unabhängig voneinander N oder C sind; unter der Voraussetzung, dass, wenn der aromatische Ringabschnitt der bicyclischen Heteroarylgruppe Imidazol ist, der nichtaromatische Ringabschnitt keinen dem Brückenkopf-Kohlenstoff benachbarten S enthalten kann;
R₁ und R₂ wie in Anspruch 1 definiert sind;
R₄ Folgendes ist: H, gegebenenfalls substituiertes C₁-C₆-Alkyl, eines von R₄ OH ist, C₁-C₆-Alkoxy, -S-C₁-C₆-Alkyl, COOR₆, -NR₆R₇, -CONR₆R₇; oder R₄R₄ zusammen =O sein können oder R₄R₄ zusammen mit dem Kohlenstoff, an welchen sie gebunden sind, ein Spirosystem mit fünf bis acht Gliedern mit oder ohne Vorhandensein von Heteroatomen bilden können, die aus N, O, S=(O)n (wobei n = 0 bis 2) und N-R₁ ausgewählt sind; und
R₆ und R₇ unabhängig voneinander Folgendes sind: H, gegebenenfalls substituiertes C₁-C₆-Alkyl, gegebenenfalls substituiertes Aryl, gegebenenfalls substituiertes Heteroaryl, gegebenenfalls substituiertes C₁-C₆-Alkylaryl, gegebenenfalls substituiertes Arylalkyl, gegebenenfalls substituiertes Heteroarylalkyl, gegebenenfalls substituiertes C₁-C₆-Alkylheteroaryl, oder R₆ und R₇ zusammen sein können, um ein 3-7-gliedriges gesättigtes Ringsystem zu bilden, das gegebenenfalls ein oder zwei aus N, O oder S ausgewählte Heteroatome aufweist.

16. Verfahren nach Anspruch 13, wobei die kondensierte bicyclische Heteroarylgruppe 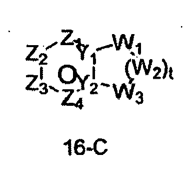 ist,
wobei
Z₁, Z₂, Z₃ und Z₄ unabhängig voneinander CR₂ oder N sind, vorausgesetzt, dass eines von Z₁ - Z₄ Kohlenstoff ist und an den übrigen Teil des Moleküls gebunden ist;
W₁, W₂ und W₃ unabhängig voneinander CR₄R₄, S, SO, SO₂, O oder N-R₁ sind; unter der Voraussetzung, dass keine Bildung einer S-S- oder O-O- oder S-O-Bindung zum Bilden des gesättigten Ringsystems auftreten kann;
t = 1 bis 4;
Y₁ und Y₂ unabhängig voneinander C oder N sind;
R₁, R₂, R₄, R₆ und R₇ wie in Anspruch 13 definiert sind.

17. Verfahren nach Anspruch 12, wobei die kondensierte tricyclische Heteroarylgruppe die Formel 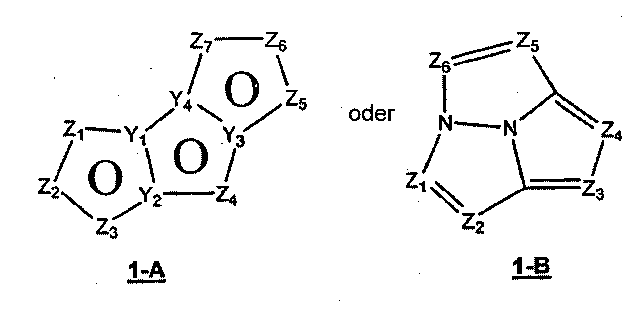 hat,
wobei Z₁, Z₂, Z₃, Z₄, Z₅, Z₆ und Z₇ unabhängig voneinander CR₂, N, O, S oder N-R₁ sind, vorausgesetzt, dass eines von Z₁ - Z₇ ein Kohlenstoffatom ist, an welches der übrige Teil des Moleküls gebunden ist;
R₁, R₂, R₆ und R₇ wie in Anspruch 1 definiert sind;
und Y₁, Y₂, Y₃ und Y₄ unabhängig voneinander C oder N sein können.

18. Verfahren nach Anspruch 12, wobei die tricyclische Heteroarylgruppe 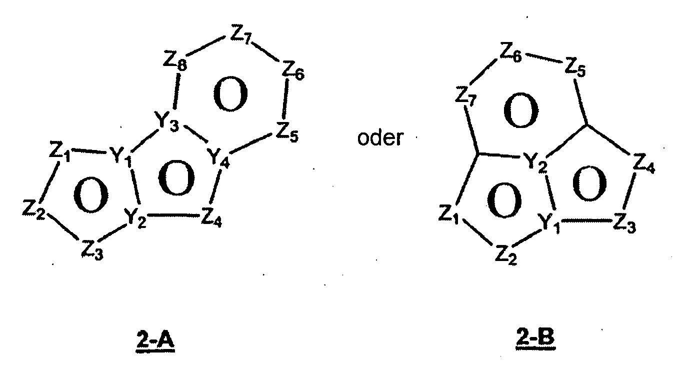 ist,
wobei Z₁, Z₂, Z₃, Z₄, Zₛ, Z₆, Z₇ und Z₈ unabhängig voneinander CR₂, N, O, S oder N-R₁ sind, vorausgesetzt, dass eines von Z₁ - Z₈ ein Kohlenstoffatom ist, an welches der übrige Teil des Moleküls gebunden ist;
R₁, R₂, R₆ und R₇ wie in Anspruch 1 definiert sind;
und Y₁, Y₂, Y₃ und Y₄ unabhängig voneinander C oder N sind.

19. Verfahren nach Anspruch 12, wobei die tricyclische Heteroarylgruppe 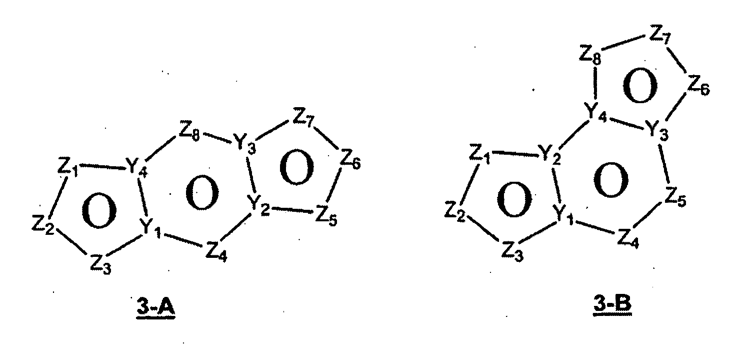 ist,
wobei Z₁, Z₂, Z₃, Z₄, Z₅, Z₆, Z₇ und Z₈ unabhängig voneinander CR₂, N, O, S oder N-R₁ sind, vorausgesetzt, dass eines von Z₁ - Z₈ ein Kohlenstoffatom ist, an welches der übrige Teil des Moleküls gebunden ist;
R₁, R₂, .R₆ und R₇ wie in Anspruch 1 definiert sind;
und Y₁, Y₂, Y₃ und Y₄ C oder N sein können.

20. Verfahren nach Anspruch 12, wobei die tricyclische Heteroarylgruppe 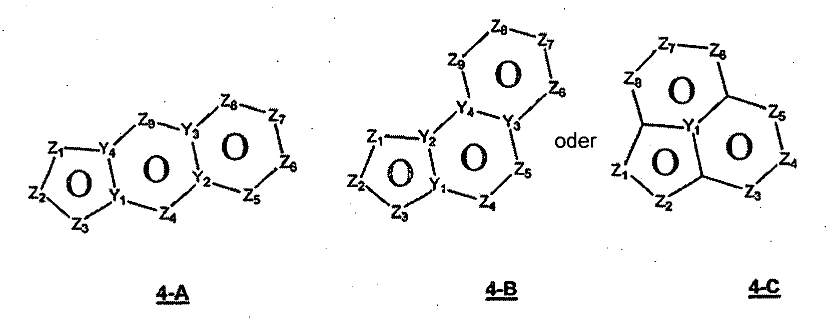 ist,
wobei Z₁, Z₂, Z₃, Z₄, Z₅, Z₆, Z₇, Z₈ und Z₉ unabhängig voneinander CR₂, N, O, S oder N-R₁ sind, vorausgesetzt, dass eines von Z₁ - Z₉ ein Kohlenstoffatom ist, an welches der übrige Teil des Moleküls gebunden ist;
R₁, R₂, R₆ und R₇ wie in Anspruch 1 definiert sind; und
Y₁, Y₂, Y₃ und Y₄ unabhängig voneinander C oder N sind.

21. Verfahren nach Anspruch 12, wobei die tricyclische Heteroarylgruppe 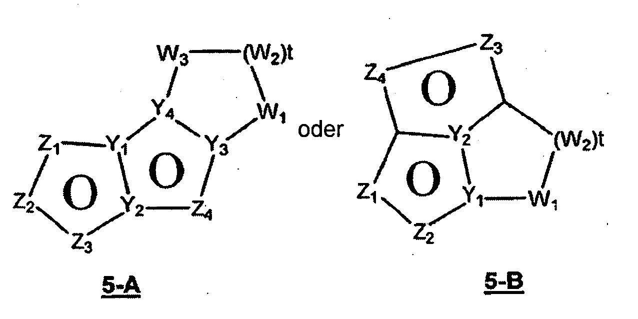 ist,
wobei Z₁, Z₂, Z₃ und Z₄ unabhängig voneinander CR₂, N, O, S oder N-R₁ sind, vorausgesetzt, dass eines von Z₁ - Z₄ ein Kohlenstoffatom ist, an welches der übrige Teil des Moleküls gebunden ist;
Y₁, Y₂, Y₃ und Y₄ unabhängig voneinander C oder N sind;
W₁, W₂ und W₃ unabhängig voneinander CR₄R₄, S(O)r (r = 0-2), O oder N-R₁ sind, unter der Voraussetzung, dass keine Bildung einer S₋S-, S-O- oder O-O-Bindung zum Bilden eines gesättigten Rings auftreten kann;
R₁, R₂, R₆ und R₇ wie in Anspruch 1 definiert sind;
R₄ Folgendes ist: H, gegebenenfalls substituiertes C₁-C₆-Alkyl, OH (vorausgesetzt, dass nicht beide R₄ OH sind), C₁-C₆-Alkoxy, -S-C₁-C₆-Alkyl, COOR₆, -NR₆R₇, -CONR₆R₇; oder R₄R₄ zusammen =O sein können oder R₄R₄ zusammen mit dem Kohlenstoff, an welchen sie gebunden sind, ein Spirosystem mit fünf bis acht Gliedern mit oder ohne Vorhandensein von Heteroatomen bilden können, die aus N, O, S(O)n (wobei n = 0 bis 2), N-R₁ ausgewählt sind;
und t = 1 bis 3.

22. Verfahren nach Anspruch 12, wobei die tricyclische Heteroarylgruppe 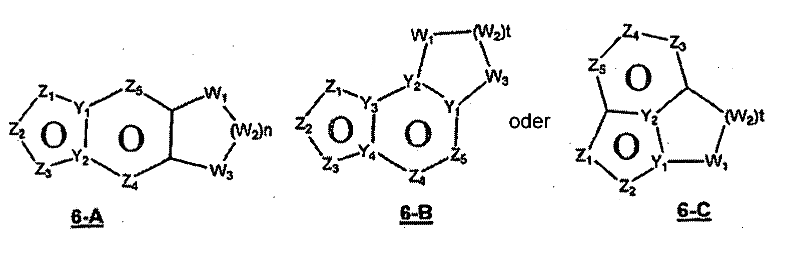 ist,
wobei Z₁, Z₂, Z₃, Z₄ und Z₅ unabhängig voneinander CR₂, N, O, S oder N-R₁ sind, vorausgesetzt, dass eines von Z₁ - Z₅ ein Kohlenstoffatom ist, an welches der übrige Teil des Moleküls gebunden ist;
Y₁ und Y₂ unabhängig voneinander C oder N sind;
W₁, W₂ und W₃ unabhängig voneinander CR₄R₄, S(O)r (r = 0-2), O oder N-R₁ sind, unter der Voraussetzung, dass keine Bildung einer S-S-, S-O- oder O-O-Bindung zum Bilden eines gesättigten Rings auftreten kann;
R₁, R₂, R₆ und R₇ wie in Anspruch 1 definiert sind;
R₄ Folgendes ist: H, gegebenenfalls substituiertes C₁-C₆-Alkyl, OH (vorausgesetzt, dass nicht beide R₄ OH sind), C₁-C₆-Alkoxy, -S-C₁-C₆-Alkyl, COOR₆, -NR₆R₇, -CONR₆R₇; oder R₄R₄ zusammen =O sein können oder R₄R₄ zusammen mit dem Kohlenstoff, an welchen sie gebunden sind, ein Spirosystem mit fünf bis acht Gliedern mit oder ohne Vorhandensein von Heteroatomen bilden können, die aus N, O, S(O)n (wobei n = 0 bis 2), N-R₁ ausgewählt sind;
und t = 1 bis 3.

23. Verfahren nach Anspruch 12, wobei die tricyclische Heteroarylgruppe 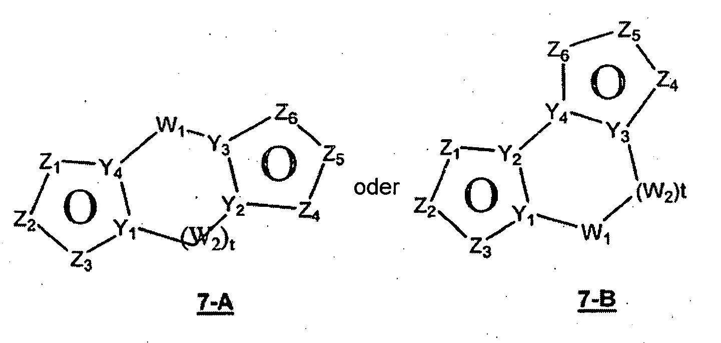 ist,
wobei Z₁, Z₂, Z₃, Z₄, Z₅ und Z₆ unabhängig voneinander CR₂, N, O, S und N-R₁ sind; vorausgesetzt, dass eines von Z₁ - Z₆ ein Kohlenstoffatom ist, an welches der übrige Teil des Moleküls gebunden ist;
Y₁, Y₂, Y₃ und Y₄ unabhängig voneinander C oder N sind;
W₁ und W₂ unabhängig voneinander CR₄R₄, S(O)r (r = 0-2), O, N-R₁ sind, unter der Voraussetzung, dass keine Bildung einer S-S-, S-O- oder O-O-Bindung zum Bilden eines gesättigten Rings auftreten kann;
R₁, R₂, R₆ und R₇ wie in Anspruch 1 definiert sind;
R₄ Folgendes ist: H, gegebenenfalls substituiertes C₁-C₆-Alkyl, OH (vorausgesetzt, dass nicht beide R₄ OH sind), C₁-C₆-Alkoxy, -S-C₁-C₆-Alkyl, COOR₆, -NR₆R₇, -CONR₆R₇; oder R₄R₄ zusammen =O sein können oder R₄R₄ zusammen mit dem Kohlenstoff, an welchen sie gebunden sind, ein Spirosystem mit fünf bis acht Gliedern mit oder ohne Vorhandensein von Heteroatomen bilden können, die aus N, O, S(O)n (wobei n = 0 bis 2), N-R₁ ausgewählt sind;
und t = 1 bis 3.

24. Verfahren nach Anspruch 12, wobei die tricyclische Heteroarylgruppe 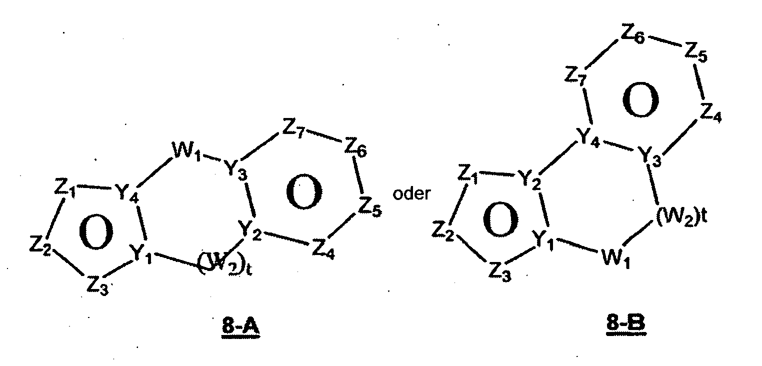 ist,
wobei Z₁ Z₂, Z₃, Z₄, Z₅, Z₆ und Z₇ unabhängig voneinander CR₂, N, O, S oder N-R₁ sind, vorausgesetzt, dass eines von Z₁ - Z₇ ein Kohlenstoffatom ist, an welches der übrige Teil des Moleküls gebunden ist;
Y₁, Y₂, Y₃ und Y₄ unabhängig voneinander C oder N sind;
W₁ und W₂ unabhängig voneinander CR₄R₄, S(O)r (r = 0-2), O oder N-R₁ sind, unter der Voraussetzung, dass keine Bildung einer S-S-, S-O- oder O-O-Bindung zum Bilden eines gesättigten Rings auftreten kann;
R₁, R₂, R₆ und R₇ wie in Anspruch 1 definiert sind;
R₄ Folgendes ist: H, gegebenenfalls substituiertes C₁-C₆-Alkyl, OH (vorausgesetzt, dass nicht beide R₄ OH sind), C₁-C₆-Alkoxy, -S-C₁-C₆-Alkyl, COOR₆, -NR₆R₇, -CONR₆R_{7;} oder R₄R₄ zusammen =O sein können oder R₄R₄ zusammen mit dem Kohlenstoff, an welchen sie gebunden sind, ein Spirosystem mit fünf bis acht Gliedern mit oder ohne Vorhandensein von Heteroatomen bilden können, die aus N, O, S(O)n (wobei n = 0 bis 2), N-R₁ ausgewählt sind;
und t = 0-3.

25. Verfahren nach Anspruch 12, wobei die tricyclische Heteroarylgruppe 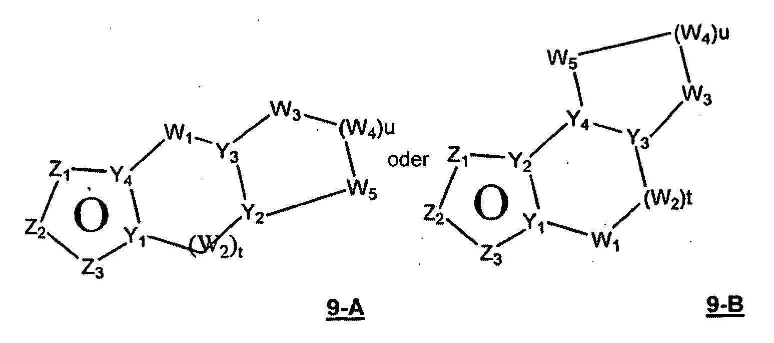 ist,
wobei Z₁, Z₂ und Z₃ unabhängig voneinander CR₂, N, O, S oder N-R₁ sind, vorausgesetzt, dass eines von Z₁ - Z₃ ein Kohlenstoffatom ist, an welches der übrige Teil des Moleküls gebunden ist;
Y₁ und Y₄ unabhängig voneinander C oder N sind;
Y₂ und Y₃ unabhängig voneinander CH oder N sind;
W₁, W₂, W₃, W₄ und W₅ unabhängig voneinander CR₄R₄, S(O)r (r = 0-2), O oder N-R₁ sind, unter der Voraussetzung, dass keine Bildung einer S-S-, S-O- oder O-O-Bindung zum Bilden eines gesättigten Rings auftreten kann;
R₁, R₂, R₆ und R₇ wie in Anspruch 1 definiert sind;
R₄ Folgendes ist: H, gegebenenfalls substituiertes C₁-C₆-Alkyl, OH (vorausgesetzt, dass nicht beide R₄ OH sind), C₁-C₆-Alkoxy, -S-C₁-C₆-Alkyl, COOR₆, -NR₆R₇, -CONR₆R₇; oder R₄R₄ zusammen =O sein können oder R₄R₄ zusammen mit dem Kohlenstoff, an welchen sie gebunden sind, ein Spirosystem mit fünf bis acht Gliedern mit oder ohne Vorhandensein von Heteroatomen bilden können, die aus N, O, S(O)n (wobei n = 0 bis 2), N-R₁ ausgewählt sind;
t = 0 bis 2; und
u = 1 bis 3.

26. Verfahren nach Anspruch 12, wobei die tricyclische Heteroarylgruppe 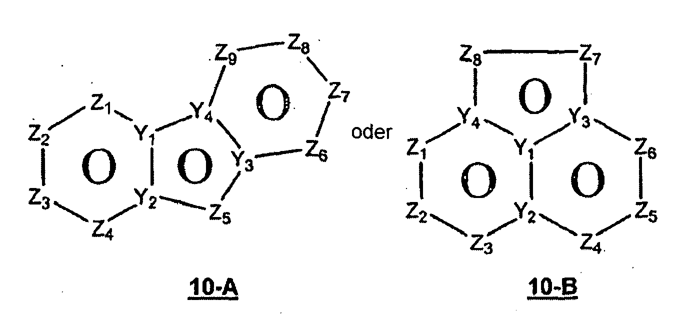 ist,
wobei Z₁, Z₂, Z₃, Z₄, Z₅, Z₆, Z₇, Z₈ und Z₉ unabhängig voneinander CR₂, N, O, S oder N-R₁ sind, vorausgesetzt, dass eines von Z₁ - Z₉ ein Kohlenstoffatom ist, an welches der übrige Teil des Moleküls gebunden ist;
R₁, R₂, R₆ und R₇ wie in Anspruch 1 definiert sind; und Y₁, Y₂, Y₃ und Y₄ unabhängig voneinander C oder N sind.

27. Verfahren nach Anspruch 12, wobei die tricyclische Heteroarylgruppe 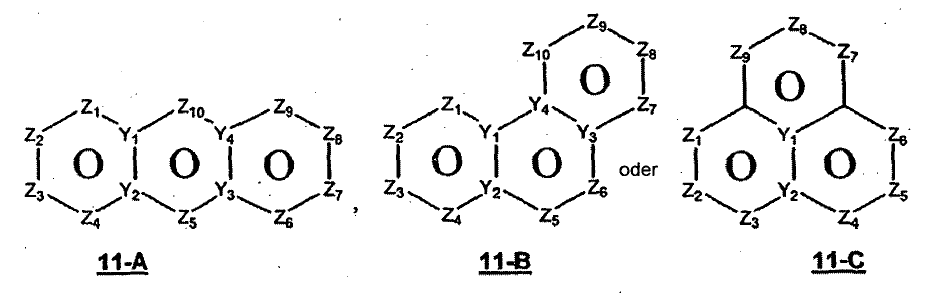 ist,
wobei Z₁, Z₂, Z₃, Z₄, Z₅, Z₆, Z₇, Z₈, Z₉ und Z₁₀ unabhängig voneinander CR₂, N, O, S oder N-R₁ sind, vorausgesetzt, dass eines von Z₁ - Z₁₀ ein Kohlenstoffatom ist, an welches der übrige Teil des Moleküls gebunden ist;
R₁, R₂, R₆ und R₇ wie in Anspruch 1 definiert sind; und
Y₁, Y₂, Y₃ und Y₄ unabhängig voneinander C oder N sind.

28. Verfahren nach Anspruch 12, wobei die tricyclische Heteroarylgruppe 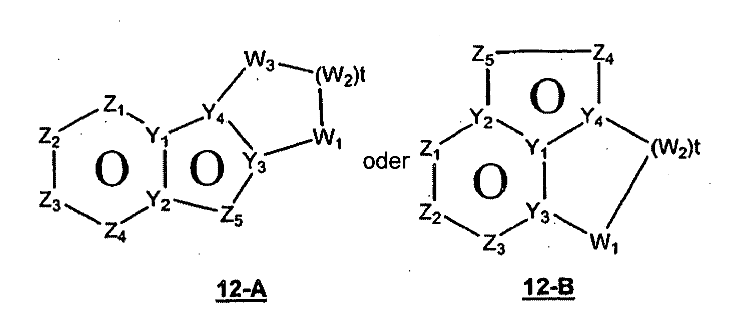 ist,
wobei Z₁ Z₂, Z₃, Z₄ und Z₅ unabhängig voneinander CR₂, N, O, S oder N-R₁ sind, vorausgesetzt, dass eines von Z₁ - Z₅ ein Kohlenstoffatom ist, an welches der übrige Teil des Moleküls gebunden ist;
Y₁, Y₂, Y₃ und Y₄ unabhängig voneinander C oder N sind;
W₁, W₂, W₃ unabhängig voneinander CR₄R₄, O, N-R, oder S(O), (r = 0-2) sind, unter der Voraussetzung, dass keine Bildung einer S-S-, S-O- oder O-O-Bindung zum Bilden eines gesättigten Rings auftreten kann;
R₁, R₂, R₆ und R₇ wie in Anspruch 1 definiert sind;
R₄ Folgendes ist: H, gegebenenfalls substituiertes C₁-C₆-Alkyl, OH (vorausgesetzt, dass nicht beide R₄ OH sind), C₁-C₆-Alkoxy, -S-C₁-C₆-Alkyl, COOR₆, -NR₆R₇, -CONR₆R₇; oder R₄R₄ zusammen =O sein können oder R₄R₄ zusammen mit dem Kohlenstoff, an welchen sie gebunden sind, ein Spirosystem mit fünf bis acht Gliedern mit oder ohne Vorhandensein von Heteroatomen bilden können, die aus N, O, S(O)n (wobei n = 0 bis 2), N-R₁ ausgewählt sind;
und t = 1-4.

29. Verfahren nach Anspruch 12, wobei die tricyclische Heteroarylgruppe 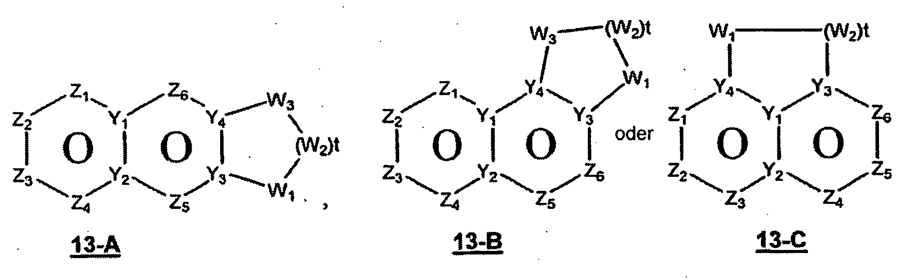 ist,
wobei Z₁, Z₂, Z₃, Z₄, Z₅ und Z₆ unabhängig voneinander CR₂, N, O, S oder N-R₁ sind, vorausgesetzt, dass eines von Z₁ - Z₆ ein Kohlenstoffatom ist, an welches der übrige Teil des Moleküls gebunden ist;
Y₁, Y₂, Y₃ und Y₄ unabhängig voneinander C oder N sind;
W₁, W₂ und W₃ unabhängig voneinander CR₄R₄, S(O)r (r = 0-2), O oder N-R₁ sind, unter der Voraussetzung, dass keine Bildung einer S-S-, S-O- oder O-O-Bindung zum Bilden eines gesättigten Rings auftreten kann;
R₁, R₂, R₆ und R₇ wie in Anspruch 1 definiert sind;
R₄ Folgendes ist: H, gegebenenfalls substituiertes C₁-C₆-Alkyl, OH (vorausgesetzt, dass nicht beide R₄ OH sind), C₁-C₆-Alkoxy, -S-C₁-C₆-Alkyl, COOR₆, -NR₆R₇, -CONR₆R₇; oder R₄R₄ zusammen =O sein können oder R₄R₄ zusammen mit dem Kohlenstoff, an welchen sie gebunden sind, ein Spirosystem mit fünf bis acht Gliedern mit oder ohne Vorhandensein von Heteroatomen bilden können, die aus N, O, S(O)n (wobei n = 0 bis 2), N-R₁ ausgewählt sind;
und t = 1 bis 3.

30. Verfahren nach Anspruch 12, wobei die tricyclische Heteroarylgruppe 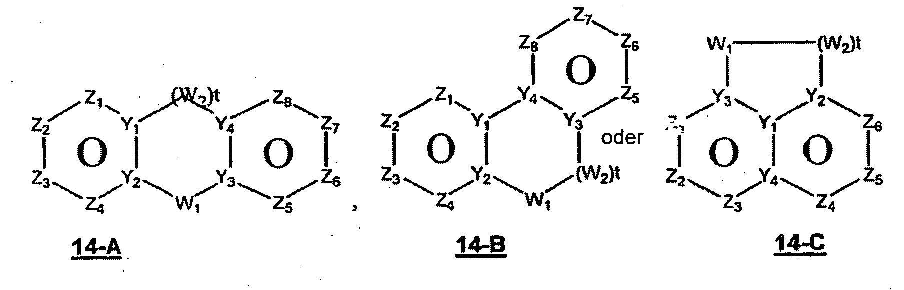 ist,
wobei Z₁, Z₂, Z₃, Z₄, Z₅, 7₆, Z₇ und Z₈ unabhängig voneinander CR₂, N, O, S oder N-R₁ sind, vorausgesetzt, dass eines von Z₁ - Z₈ ein Kohlenstoffatom ist, an welches der übrige Teil des Moleküls gebunden ist;
Y₁, Y₂, Y₃ und Y₄ unabhängig voneinander C oder N sind;
W₁ und W₂ unabhängig voneinander CR₄R₄, S(O)r (r = 0-2), O oder N-R₁ sind, unter der Voraussetzung, dass keine Bildung einer S-S-, S-O- oder O-O-Bindung zum Bilden eines gesättigten Rings auftreten kann;
R₁, R₂, R₆ und R₇ wie in Anspruch 1 definiert sind;
R₄ Folgendes ist: H, gegebenenfalls substituiertes C₁-C₆-Alkyl, OH (vorausgesetzt, dass nicht beide R₄ OH sind), C₁-C₆-Alkoxy, -S-C₁-C₆-Alkyl, COOR₆, -NR₆R₇, -CONR₆R₇; oder R₄R₄ zusammen =O sein können oder R₄R₄ zusammen mit dem Kohlenstoff, an welchen sie gebunden sind, ein Spirosystem mit fünf bis acht Gliedern mit oder ohne Vorhandensein von Heteroatomen bilden können, die aus N, O, S(O)n (wobei n = 0 bis 2), N-R₁ ausgewählt sind;
und t = 1 bis 2.

31. Verfahren nach Anspruch 12, wobei die tricyclische Heteroarylgruppe 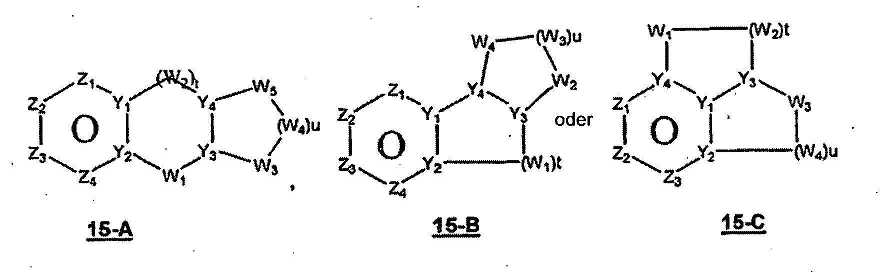 ist,
wobei Z₁, Z₂, Z₃ und Z₄ unabhängig voneinander CR₂, N, O, S oder N-R₁ sind, vorausgesetzt, dass eines von Z₁ - Z₄ ein Kohlenstoffatom ist, an welches der übrige Teil des Moleküls gebunden ist;
Y₁, Y₂, Y₃ und Y₄ unabhängig voneinander C oder N sind;
W₁, W₂, W₃, W₄ und W₅ unabhängig voneinander CR₄R₄, S(O)r (r = 0-2), O oder N-R₁ sind, unter der Voraussetzung, dass keine Bildung einer S-S-, S-O- oder O-O-Bindung zum Bilden eines gesättigten Rings auftreten kann;
R₁, R₂, R₆ und R₇ wie in Anspruch 1 definiert sind;
R₄ Folgendes ist: H, gegebenenfalls substituiertes C₁-C₆-Alkyl, OH (vorausgesetzt, dass nicht beide R₄ OH sind), C₁-C₆-Alkoxy, -S-C₁-C₆-Alkyl, COOR₆, -NR₆R₇, -CONR₆R₇; oder R₄R₄ zusammen =O sein können oder R₄R₄ zusammen mit dem Kohlenstoff, an welchen sie gebunden sind, ein Spirosystem mit fünf bis acht Gliedern mit oder ohne Vorhandensein von Heteroatomen bilden können, die aus N, O, S(O)n (wobei n = 0 bis 2), N-R₁ ausgewählt sind;
t = 1 bis 3; und
u = 1 bis 3.

32. 6-Brompenem-Derivat der Struktur 16 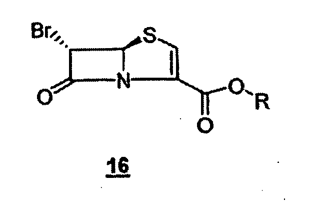 wobei R p-Nitrobenzyl ist.

33. Verfahren zur Herstellung des 4-Nitrobenzyl-(5R,65)-6-brompenem-3-carboxylats nach Anspruch 32, welches folgende Schritte umfasst:
(A) (i) Reagieren von 6-Aminopenicillansäure mit Bromwasserstoffsäure in einem organischen Lösungsmittel und Wasser zum Bilden des 6-Bromderivats **21** 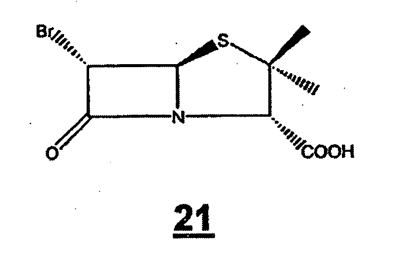 und (ii) Umsetzen des Derivats 6-Brompenicillansäure **21** zu dem p-Nitrobenzyl-6-brompenicillanat **22** 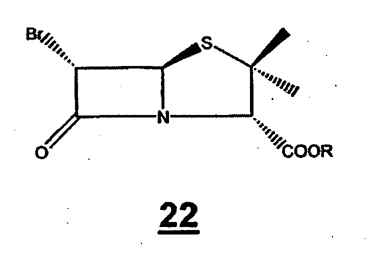 wobei R p-Nitrobenzyl ist, unter Verwendung von 4-Nitrobenzylbromid in Gegenwart einer Base in einem organischen Lösungsmittel;
(B) Oxidieren des 4-Nitrobenzyl-6-brompenicillanats **22,** um 4-Nitrobenzyl-6-brompenicillanat-1-oxid **23** zu bilden 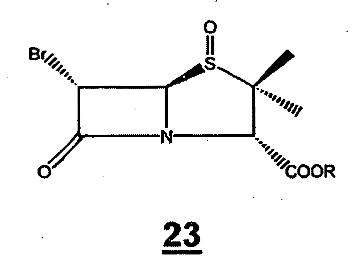
(C) Rückflusserhitzen des 4-Nitrobenzyl-6-brompenicillanat-1-oxids **23** mit 2-Mercaptobenzothiazol in einem aromatischen Lösungsmittel, um 4-Nitrobenzyl-(2R)-2-[(3S,4R)-4-(benzothiazol-2-yldithio)-3-brom-2-oxoazetidin-1-yl]-3-methylbut-3-enoat **24** zu bilden;
(D) Lösen des 4-Nitrobenzyl-(2R)-2-[(3S,4R)-4-(benzothiazol-2-yldithio)-3-brom-2-oxoazetidin-1-yl]-3-methylbut-3-enoats **24** in einem organischen Lösungsmittel und Reagieren mit einer organischen tertiären Base, um 4-Nitrobenzyl-2-[(3S,4R)-4-(benzothiazol-2-yldithio)-3-brom-2-oxoazetidin-1-yl]-3-methylbut-2-enoat **25** zu bilden;
(E) Umsetzen des 4-Nitrobenzyl-2-[(3S,4R)-4-(benzothiazol-2-yldithio)-3-brom-2-oxoazetidin-1-yl]-3-methylbut-2-enoats **25** zu 4-Nitrobenzyl-2-[(3S,4R)-3-brom-4-formylthio-2-oxoazetidin-1-yl]-3-methylbut-2-enoat **26** durch Reagieren in einem aromatischen organischen Lösungsmittel in Gegenwart von einer organischen Säure, Essigsäureanhydrid / einer organischen tertiären Base und Trialkyl- oder Triarylphosphin bei ungefähr -10°C bis ungefähr -30°C;
(F) wobei das 4-Nitrobenzyl-2-[(3S,4R)-3-brom-4-formylthio-2-oxoazetidin-1-yl]-3-methylbut-2-enoat **26** in einem organischen Lösungsmittel bei -70°C bis -90°C aufgenommen wird und ozonisierter Sauerstoff 3 bis 4 Stunden durch es hindurchgeleitet wird und anschließend eine intramolekulare Cyclisierung mit einem Phosphitreagens erfolgt, um 4-Nitrobenzyl-(5R,6S)-6-brompenem-3-carboxylat **16** zu bilden.

34. Verfahren nach Anspruch 1 zur Herstellung einer wie in Anspruch 1 definierten Verbindung der Formel **I**, 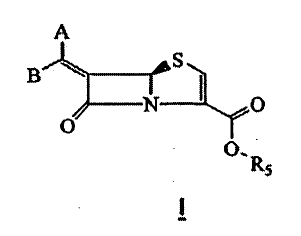 in welchem das in Schritt (a) verwendete 4-Nitrobenzyl-(5R,6S)-6-brompenem-3-carboxylat **16** nach Anspruch 33 hergestellt wird.

35. Verfahren nach Anspruch 33 oder Anspruch 34, wobei die 6-Aminopenicillansäure in Methanol oder THF gelöst wird.

36. Verfahren nach irgendeinem der Ansprüche 33 bis 35, wobei Schritt (A) (i) in Gegenwart von 48 Gew.-% Bromwasserstoffsäure und Natrium- oder Kaliumnitrit-Lösung durchgeführt wird.

37. Verfahren nach irgendeinem der Ansprüche 33 bis 36, wobei Schritt (A) (i) bei einer Temperatur von ungefähr -10°C bis ungefähr -30°C durchgeführt wird.

38. Verfahren nach irgendeinem der Ansprüche 33 bis 37, wobei die Base in Schritt (A) (ii) Natrium- oder Kaliumcarbonat ist und das organische Lösungsmittel THF oder DMF ist.

39. Verfahren nach irgendeinem der Ansprüche 33 bis 38, wobei das aromatische Lösungsmittel in Schritt (C) Toluol oder Xylol ist.

40. Verfahren nach irgendeinem der Ansprüche 33 bis 39, umfassend die sequentielle Umsetzung der Verbindung **23** zu **26,** wobei keine Isolierung der Zwischenprodukte erfolgt.

41. Verfahren nach Anspruch 40, wobei das 4-Nitrobenzyl-6-brompenicillanat-1-oxid **23** in einem unter Rückfluss erhitzten aromatischen organischen Lösungsmittel mit Mercaptobenzothiazol zur Reaktion gebracht und bei ungefähr 0 bis -20°C mit Triethylamin behandelt wird, um ein Reaktionsgemisch zu bilden; wobei das Reaktionsgemisch nacheinander mit einer organischen Säure und einem Anhydrid, einer organischen tertiären Base und einem Trialkyl- oder Triarylphosphat bei ungefähr -10°C bis -40°C versetzt wird.

## Revendications

1. Procédé pour la préparation de composés répondant à la formule **I** 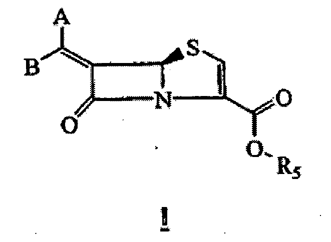 dans laquelle
un des radicaux A et B désigne un atome d'hydrogène, l'autre représentant un groupe aryle facultativement substitué avec un ou deux radicaux R₂, un groupe hétéroaryle facultativement substitué avec un ou deux radicaux R₂, un groupe hétéroaryle bicyclique condensé facultativement substitué avec un ou deux radicaux R₂, un groupe hétéroaryle tricyclique condensé facultativement substitué avec un ou deux radicaux R₂, un groupe cycloalkyle facultativement substitué avec un ou deux radicaux R₂, un groupe alkyle facultativement substitué avec un ou deux radicaux R₂, un groupe alcényle facultativement substitué avec un ou deux radicaux R₂, un groupe alcynyle facultativement substitué avec un ou deux radicaux R₂, un groupe hétéroaryle saturé ou partiellement saturé facultativement substitué avec un ou deux radicaux R₂, et dans laquelle l'une quelconque desdites fractions hétéroaryle contenant un atome nucléaire NH peut être facultativement substituée sur ledit atome d'azote par un radical R₁ ;
R₅ représente un atome d'hydrogène, un groupe alkyle en C₁-C₆, un groupe cycloalkyle en C₅-C₆, ou un groupe CHRₛOCOalkyte en C₁-C₆ ou un de leurs sels ;
R₁ représente un atome d'hydrogène, un groupe alkyle facultativement substitué, un groupe aryle facultativement substitué, un groupe hétéroaryle facultativement substitué ou des hétérocycles monocycliques ou bicycliques saturés facultativement substitués, un groupe cycloalkyle facultativement substitué, un groupe alcényle facultativement substitué, un groupe alcynyle facultativement substitué, avec cette réserve que ni la liaison double, ni la liaison triple ne peut être présente sur l'atome de carbone qui est directement lié à N ; un groupe perfluoroalkyle facultativement substitué, un groupe -S(O)_{P}-alkyle ou -S(O)_{P}-aryle facultativement substitué dans lequel p est égal à 0-2, un groupe -C=Ohétéroaryle facultativement substitué, un groupe -C=Oaryle facultativement substitué, un groupe -C=Oalkyle facultativement substitué, un groupe -C=Ocycloalkyle facultativement substitué, des -C=Ohétérocydes monocycliques ou bicycliques saturés facultativement substitués, un groupe alkyl(en C₁-C₆)aryle facultativement substitué, un groupe alkyl(en C₁-C₆)hétéroaryle facultativement substitué, un groupe arylalkyle en C₁-C₆ facultativement substitué, un groupe hétéroarylalkyle en C₁-C₆ facultativement substitué, des groupes alkyl(en C₁-C₆)hétérocycles monocycliques ou bicycliques saturés facultativement substitués, un groupe arylalcényle facultativement substitué contenant de 8 à 16 atomes de carbone, un groupe-CONR₆R₇, un groupe -SO₂NR₆R₇, un groupe arylalkyloxyalkyle facultativement substitué, un groupe alkyl-O-alkyl-aryle facultativement substitué, un groupe alkyl-O-alkyl-hétéroaryle facultativement substitué, un groupe aryloxyalkyle facultativement substitué, un groupe hétéroaryloxyalkyle facultativement substitué, un groupe aryloxyaryle facultativement substitué, un groupe aryloxyhétéroaryle facultativement substitué, un groupe alkyl(en C₁-C₆)aryloxyaryle facultativement substitué, un groupe alkyl(en C₁-C₆)aryloxyhétéroaryle facultativement substitué, un groupe alkylaryloxyalkylamine facultativement substitué, un groupe alcoxycarbonyle facultativement substitué, un groupe aryloxycarbonyle facultativement substitué, ou un groupe hétéroaryloxycarbonyle facultativement substitué ;
R₂ représente un atome d'hydrogène, un groupe alkyle en C₁-C₆ facultativement substitué, un groupe alcényle en C₂-C₆ facultativement substitué, un groupe alcynyle en C₂-C₆ facultativement substitué, un atome d'halogène, un groupe cyano, un groupe N-R₆R₇, un groupe alcoxy en C₁-C₆ facultativement substitué, un groupe hydroxyle ; un groupe aryle facultativement substitué, un groupe hétéroaryle facultativement substitué, un groupe COOR₆, un groupe alkylaryloxyalkylamine facultativement substitué, un groupe aryloxy facultativement substitué, un groupe hétéroaryloxy facultativement substitué, un groupe alcényl(en C₃-C₆)oxy facultativement substitué, un groupe alcynyl(en C₃-C₆)oxy facultativement substitué, un groupe alkyl(en C₁-C₆)aminoalcoxy en C₁-C₆, un groupe alkylènedioxy, un groupe aryloxyalkyl(en C₁-C₆)amine facultativement substitué, un groupe perfluoroalkyle en C₁-C₆, un groupe S(O)_{q}-alkyle en C₁-C₆ facultativement substitué, un groupe S(O)_{q}-aryle facultativement substitué dans lequel q est égal à 0, 1 ou 2, un groupe CONR₆R₇, un groupe guanidino ou un groupe guanidino cyclique, un groupe alkylaryle facultativement substitué, un groupe arylalkyle facultativement substitué, un groupe alkyl(en C₁-C₆)hétéroaryle facultativement substitué, un groupe hétéroarylalkyle en C₁-C₆ facultativement substitué, des groupes alkyl(en C₁-C₆)hétérocycles monocycliques ou bicycliques saturés facultativement substitués, un groupe arylalcényle facultativement substitué contenant de 8 à 16 atomes de carbone, un groupe SO₂NR₆R₇, un groupe arylalkyloxyalkyle facultativement substitué, un groupe aryloxyalkyle facultativement substitué, un groupe hétéroaryloxyalkyle facultativement substitué, un groupe aryloxyaryle facultativement substitué, un groupe aryloxyhétéroaryle facultativement substitué, un groupe hétéroaryloxyaryle facultativement substitué, un groupe alkyl(en C₁-C₆)aryloxyaryle facultativement substitué, un groupe alkyl(en C₁-C₆)aryloxyhétéroaryle facultativement substitué, un groupe aryloxyalkyle facultativement substitué, un groupe hétéroaryloxyalkyle facultativement substitué, ou un groupe alkylaryloxyalkylamine facultativement substitué ;
R₃ représente un atome d'hydrogène, un groupe alkyle en C₁-C₆, un groupe cycloalkyle en C₅-C₆, un groupe aryle facultativement substitué, un groupe hétéroaryle facultativement substitué ;
R₆ et R₇ représentent, de manière indépendante, un atome d'hydrogène, un groupe alkyle en C₁-C₆ facultativement substitué, un groupe aryle facultativement substitué, un groupe hétéroaryle facultativement substitué, un groupe alkyl(en C₁-C₆)aryle facultativement substitué, un groupe arylalkyle facultativement substitué, un groupe hétéroarylalkyle facultativement substitué, un groupe alkyl(en C₁-C₆)hétéroaryle facultativement substitué, R₆ et R₇ pouvant former ensemble un système cyclique saturé à 3-7 membres contenant, de manière facultative, un ou deux hétéroatomes tels qu'un groupe N-R₁, un atome d'oxygène, un groupe S=(O)ₙ où n = 0-2 ;
ledit procédé comprenant :
(a) la condensation d'un aldéhyde **17** substitué de manière appropriée
**A'-CHO** **17**
dans lequel A' représente le radical A tel qu'il a été défini ci-dessus lorsque B représente un atome d'hydrogène, ou bien le radical B tel qu'il a été défini ci-dessus lorsque A représente un atome d'hydrogène,
avec un dérivé de 6-bromo-pénème de structure 16 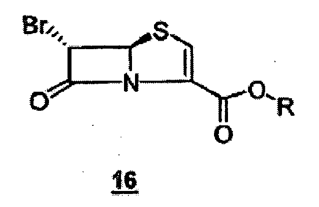 dans laquelle R représente un groupe p-nitrobenzyle,
en présence d'un acide de Lewis et d'une base douce, à basse température pour obtenir le produit intermédiaire aldol **18** 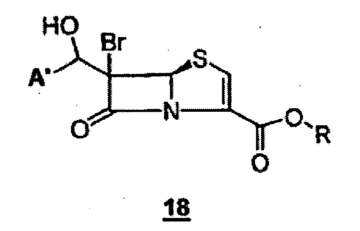 dans lequel A' et R sont tels que défini ci-dessus ;
(b) la mise en réaction du produit intermédiaire **18** avec un chlorure d'acide ou avec un anhydride d'acide répondant à la formule : (R₈)Cl ou (R₈)₂O, ou bien avec un tétrahalogénométhane répondant à la formule : C(X₁)₄, et avec de la triphénylphosphine, R₈ représentant un groupe alkylSO₂, un groupe aryISO₂, un groupe alkylCO, ou un groupe arylCO, X₁ représentant un atome de brome, un atome d'iode ou un atome de chlore pour obtenir le composé intermédiaire 19 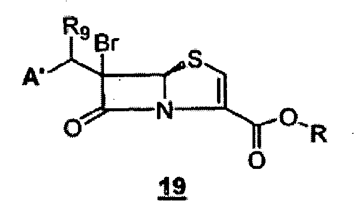 dans lequel R₉ représente un groupe X₁ ou un groupe OR₈, R₈, X₁, A' et R étant tels que définis ci-dessus ; et
(c) la conversion du composé intermédiaire **19** pour obtenir le composé désiré répondant à la formule dans lequel R₅ représente un atome d'hydrogène, via un procédé d'élimination par réduction ; et si on le souhaite, la conversion en un sel pharmaceutiquement acceptable ou en un ester dans lequel R₅ représente un groupe alkyle en C₁-C₆, un groupe cycloalkyle en C₅-C₆, ou un groupe CHR₃OCOalkyle en C₁-C₆.

2. Procédé selon la revendication 1, dans lequel l'acide de Lewis est un halogénure de magnésium anhydre.

3. Procédé selon la revendication 2, dans lequel l'acide Lewis est du MgBr₂ anhydre.

4. Procédé selon l'une quelconque des revendications 1 à 3, dans lequel la base douce est la triéthylamine, la DMAP ou la diisopropyléthylamine.

5. Procédé selon l'une quelconque des revendications 1 à 4, dans lequel la basse température s'élève d'environ -20 °C à environ -40 °C.

6. Procédé selon l'une quelconque des revendications 1 à 5, dans lequel le composé intermédiaire 19 est un acétate, un triflate ou un tosylate.

7. Procédé selon l'une quelconque des revendications 1 à 6; dans lequel le composé intermédiaire 19 n'est pas isolé.

8. Procédé selon l'une quelconque des revendications 1 à 7, dans lequel l'étape (c) est mise en oeuvre à une température douce.

9. Procédé selon la revendication 8, dans lequel la température douce s'élève d'environ 20 °C à 35 °C.

10. Procédé selon l'une quelconque des revendications 1 à 9, dans lequel le processus d'élimination par réduction est mis en oeuvre en utilisant du zinc activé et un tampon de phosphate à un pH d'environ 6,5 à 8,0 ou bien par hydrogénation par-dessus un catalyseur.

11. Procédé selon la revendication 10, dans lequel le catalyseur est du charbon palladié.

12. Procédé selon l'une quelconque des revendications 1 à 11, dans lequel A ou B représente un groupe hétéroaryle tricyclique condensé.

13. Procédé selon l'une quelconque des revendications 1 à 11, dans lequel A ou B représente un groupe hétéroaryle bicyclique condensé.

14. Procédé selon la revendication 13, dans lequel le groupe hétéroaryle bicyclique condensé répond à la formule développée 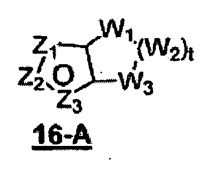
dans laquelle Z₁, Z₂, et Z₃ représentent, de manière indépendante, un groupe CR₂, un atome d'azote, un atome d'oxygène, un atome de soufre ou un groupe N-R₁, à condition qu'un des radicaux Z₁, Z₂, ou Z₃ représente un atome de carbone et soit lié au reste de la molécule, comme représenté dans la formule **I** ;
W₁, W₂ et W₃ représentent, de manière indépendante, un groupe CR₄R₄, un atome de soufre, un groupe SO, un groupe SO₂, un atome d'oxygène, un groupe N-R₁, un groupe C=O ; avec cette réserve qu'aucune formation de liaison S-S ou O-O ou S-O ne peut avoir lieu pour former le système cyclique saturé ;
t=1 à 4;
R₁, R₂, R₆ et R₇ sont tels que définis à la revendication 1; et
R₄ représente un atome d'hydrogène, un groupe alkyle en C₁-C₆ facultativement substitué, un des radicaux R₄ représentant un groupe OH, un groupe alcoxy en C₁-C₆, un groupe -S-alkyle en C₁-C₆, un groupe COOR₆, un groupe -NR₆R₇, un groupe -CONR₆R₇ ou bien R₄R₄ peuvent représenter ensemble un groupe =O ou bien R₄R₄, ensemble avec l'atome de carbone auquel ils sont fixés peuvent former un système spiro de cinq à huit membres en présence ou en l'absence d'hétéroatomes choisis parmi un atome d'azote, un atome d'oxygène, un groupe S=(O)ₙ (où n = 0 à 2), un groupe N-R₁.

15. Procédé selon la revendication 13, dans lequel le groupe hétéroaryle bicyclique condensé répond à la formule développée 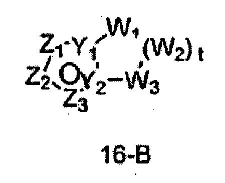 dans laquelle
Z₁, Z₂ et Z₃ représentent, de manière indépendante, un groupe CR₂, un atome d'azote, un atome d'oxygène, un atome de soufre ou un groupe N-R₁, à condition qu'un des radicaux Z₁ à Z₃ représente un atome de carbone et soit lié au reste de la molécule ;
W₁, W₂ et W₃ représentent, de manière indépendante, un groupe CR₄R₄, un atome de soufre, un groupe SO, un groupe SO₂, un atome d'oxygène, ou un groupe N-R₁ ;
t = 1 à 4;
Y₁ et Y₂ représentent, de manière indépendante, un atome d'azote ou un atome de carbone ; avec cette réserve que, si la portion nucléaire aromatique du groupe hétéroaryle bicyclique représente un groupe imidazole, la portion nucléaire non aromatique ne peut contenir un atome de soufre en position adjacente à l'atome de carbone en tête de pont ;
R₁ et R₂ sont tels que définis à la revendication 1;
R4 représente un atome d'hydrogène, un groupe alkyle en C₁-C₆ facultativement substitué, un des radicaux R₄ représentant un groupe OH, un groupe alcoxy en C₁-C₆, un groupe -S-alkyle en C₁-C₆, un groupe COOR₆, un groupe -NR₆R₇, un groupe -CONR₆R₇ ; ou bien R₄R₄ peuvent représenter ensemble un groupe =O ou bien R₄R₄, ensemble avec l'atome de carbone auquel ils sont fixés peuvent former un système spiro de cinq à huit membres en présence ou en l'absence d'hétéroatomes choisis parmi un atome d'azote, un atome d'oxygène, un groupe S=(O)ₙ (où n = 0 à 2), et un groupe N-R₁ ; et
R₆ et R₇ représentent, de manière indépendante, un atome d'hydrogène, un groupe alkyle en C₁-C₆ facultativement substitué; un groupe aryle facultativement substitué, un groupe hétéroaryle facultativement substitué, un groupe alkyl(en C₁-C₆)aryle facultativement substitué, un groupe arylalkyle facultativement substitué, un groupe hétéroarylalkyle, facultativement substitué, un groupe alkyl(en C₁-C₆)hétéroaryle facultativement substitué, ou bien R₆ et R₇ peuvent former ensemble un système cyclique saturé de 3 à 7 membres contenant, de manière facultative, un ou deux hétéroatomes choisis parmi un atome d'azote, un atome d'oxygène, ou un atome de soufre.

16. Procédé selon la revendication 13, dans lequel le groupe hétéroaryle bicyclique condensé représente un groupe 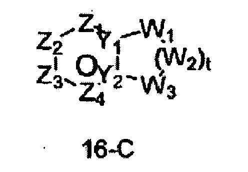 dans lequel
Z₁, Z₂, Z₃ et Z₄ représentent, de manière indépendante, un groupe CR₂ ou un atome d'azote, à condition qu'un des radicaux Z₁ à Z₄ représente un atome de carbone et soit lié au reste de la molécule ;
W₁, W₂ et W₃ représentent, de manière indépendante, un groupe CR₄R₄, un atome de soufre, un groupe SO, un groupe SO₂, un atome d'oxygène, ou un groupe N-R₁ ; avec cette réserve qu'aucune formation de liaison S-S ou O-O ou S-O ne peut avoir lieu pour former le système cyclique saturé ;
t = 1 à 4;
Y₁ et Y₂ représentent, de manière indépendante, un atome de carbone ou un atome d'azote ;
R₁, R₂, R₄, R₆ et R₇ sont tels que définis à la revendication 13.

17. Procédé selon la revendication 12, dans lequel le groupe hétéroaryle tricyclique condensé répond à la formule 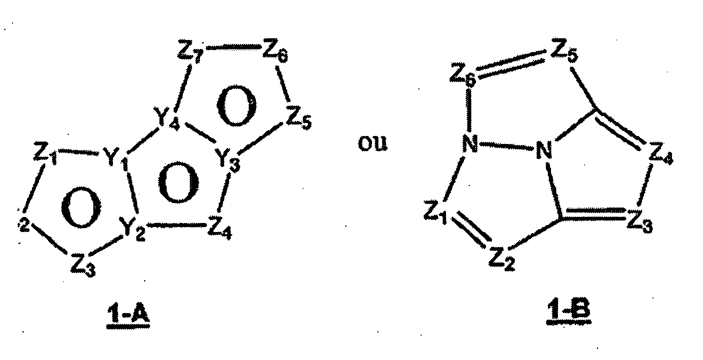
dans laquelle Z₁, Z₂, Z₃, Z₄, Z₅, Z₆ et Z₇ représentent, de manière indépendante, un groupe CR₂, un atome d'azote, un atome d'oxygène, un atome de soufre ou un groupe N-R₁, à condition qu'un des radicaux Z₁ à Z₇ représente un atome de carbone auquel vient se fixer le reste de la molécule ;
R₁, R₂, R₆ et R₇ sont tels que définis à la revendication 1 ;
et Y₁, Y₂, Y₃ et Y₄ peuvent représenter, de manière indépendante, un atome de carbone ou un atome d'azote.

18. Procédé selon la revendication 12, dans lequel le groupe hétéroaryle tricyclique représente un groupe : 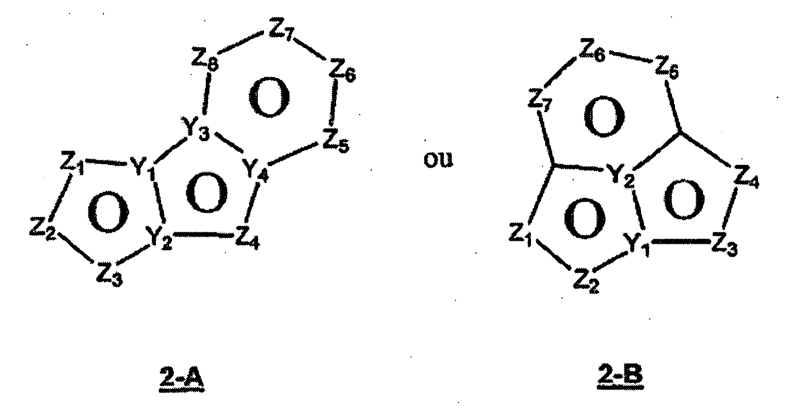
dans lequel Z₁, Z₂, Z₃, Z₄, Z₅, Z₆, Z₇ et Z₈ représentent, de manière indépendante, un groupe CR₂, un atome d'azote, un atome d'oxygène, un atome de soufre ou un groupe N-R₁, à condition qu'un des radicaux Z₁ à Z₈ représente un atome de carbone auquel vient se fixer le reste de la molécule ;
R₁, R₂, R₆ et R₇ sont tels que définis à la revendication 1 ;
et Y₁, Y₂, Y₃ et Y₄ représentent, de manière indépendante, un atome de carbone ou un atome d'azote.

19. Procédé selon la revendication 12, dans lequel le groupe hétéroaryle tricyclique représente un groupé : 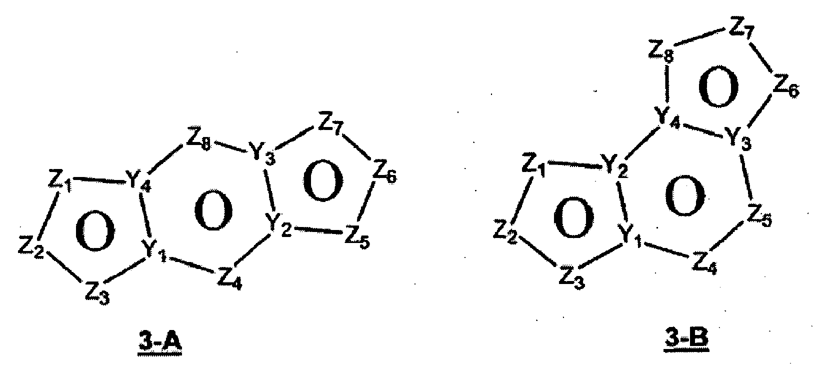
dans lequel Z₁, Z₂, Z₃, Z₄, Z₅, Z₆, Z₇ et Z₈ représentent, de manière indépendante, un groupe CR₂, un atome d'azote, un atome d'oxygène, un atome de soufre ou un groupe N-R₁, à condition qu'un des radicaux Z₁ à Z₈ représente un atome de carbone auquel vient se fixer le reste de la molécule ;
R₁, R₂, R₆ et R₇ sont tels que définis à la revendication 1 ;
et Y₁, Y₂, Y₃ et Y₄ peuvent représenter un atome de carbone ou un atome d'azote.

20. Procédé selon la revendication 12, dans lequel le groupe hétéroaryle tricyclique représente un groupe : 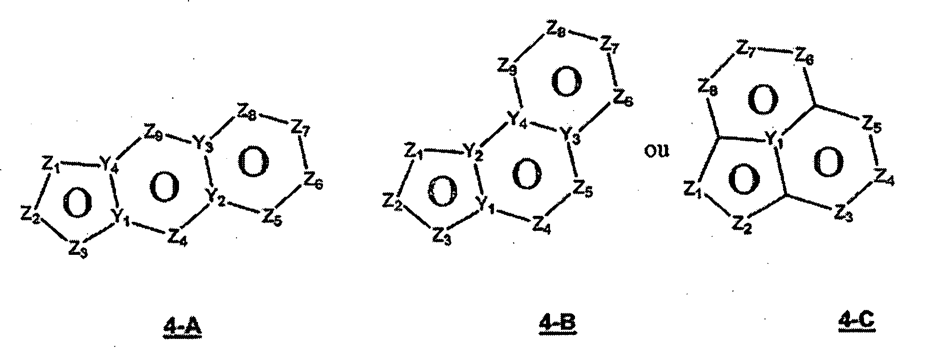
dans lequel Z₁, Z₂, Z₃, Z₄, Z₅, Z₆, Z₇, Z₈ et Z₉ représentent, de manière indépendante, un groupe CR₂, un atome d'azote, un atome d'oxygène, un atome de soufre ou un groupe N-R₁, à condition qu'un des radicaux Z₁ à Z₉ représente un atome de carbone auquel vient se fixer le reste de la molécule ;
R₁, R₂, R₆ et R₇ sont tels que définis à la revendication 1 ;
et Y₁, Y₂, Y₃ et Y₄ représentent, de manière indépendante, un atome de carbone ou un atome d'azote.

21. Procédé selon la revendication 12, dans lequel le groupe hétéroaryle tricyclique représente un groupe : 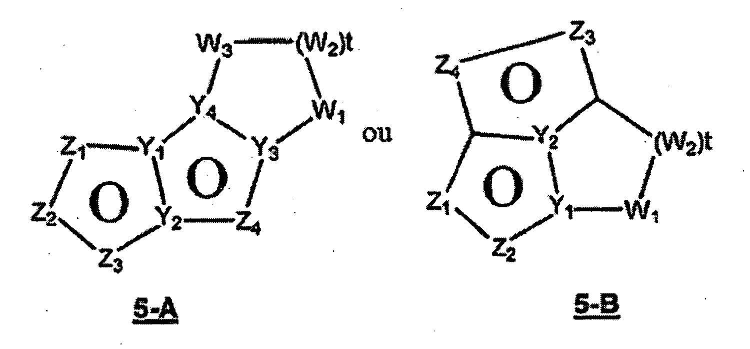
dans lequel Z₁, Z₂, Z₃ et Z₄ représentent, de manière indépendante, un groupe CR₂, un atome d'azote, un atome d'oxygène, un atome de soufre ou un groupe N-R₁, à condition qu'un des radicaux Z₁ à Z₄ représente un atome de carbone auquel vient se fixer le reste de la molécule ;
Y₁, Y₂, Y₃ et Y₄ représentent, de manière indépendante, un atome de carbone ou un atome d'azote ;
W₁, W₂ et W₃ représentent, de manière indépendante, un groupe CR₄R₄, un groupe S(O)r (r = 0-2), un atome d'oxygène ou un groupe N-R₁ ; avec cette réserve qu'aucune formation de liaison S-S, S-O ou O-O ne peut avoir lieu pour former un noyau saturé ;
R₁, R₂, R₆ et R₇ sont tels que définis à la revendication 1 ;
R₄ représente un atome d'hydrogène, un groupe alkyle en C₁-C₆ facultativement substitué, un groupe OH (à condition que les deux radicaux R₄ ne représentent pas un groupe OH), un groupe alcoxy en C₁-C₆, un groupe -S-alkyle en C₁-C₆, un groupe COOR₆, un groupe -NR₆R₇, un groupe -CONR₆R₇ ;
ou bien R₄R₄ peuvent représenter ensemble un groupe =O ou bien R₄R₄, ensemble avec l'atome de carbone auquel ils sont fixés peuvent former un système spiro de cinq à huit membres en présence ou en l'absence d'hétéroatomes choisis parmi un atome d'azote, un atome d'oxygène, un groupe S(O)ₙ (où n = 0 à 2), un groupe N-R₁ ;
ett=1à3.

22. Procédé selon la revendication 12, dans lequel le groupe hétéroaryle tricyclique représente un groupe : 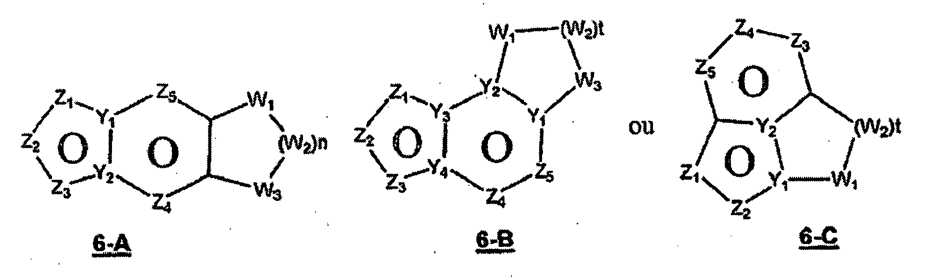
dans lequel Z₁, Z₂, Z₃, Z₄ et Z₅ représentent, de manière indépendante, un groupe CR₂, un atome d'azote, un atome d'oxygène, un atome de soufre ou un groupe N-R₁, à condition qu'un des radicaux Z₁ à Z₅ représente un atome de carbone auquel vient se fixer le reste de la molécule ;
Y₁ et Y₂ représentent, de manière indépendante, un atome de carbone ou un atome d'azote ;
W₁, W₂ et W₃ représentent, de manière indépendante, un groupe CR₄R₄, un groupe S(O)r (r = 0-2), un atome d'oxygène ou un groupe N-R₁ ; avec cette réserve qu'aucune formation de liaison S-S, S-O ou O-O ne peut avoir lieu pour former un noyau saturé ;
R₁, R₂, R₆ et R₇ sont tels que définis à la revendication 1 ;
R₄ représente un atome d'hydrogène, un groupe alkyle en C₁-C₆ facultativement substitué, un groupe OH (à condition que les deux radicaux R₄ ne représentent pas un groupe OH), un groupe alcoxy en C₁-C₆, un groupe -S-alkyle en C₁-C₆, un groupe COOR₆, un groupe -NR₆R₇, un groupe -CONR₆R₇ ;
ou bien R₄R₄ peuvent représenter ensemble un groupe =O ou bien R₄R₄, ensemble avec l'atome de carbone auquel ils sont fixés peuvent former un système spiro de cinq à huit membres en présence ou en l'absence d'hétéroatomes choisis parmi un atome d'azote, un atome d'oxygène, un groupe S(O)ₙ (où n = 0 à 2), un groupe N-R₁ ;
ett=1à3.

23. Procédé selon la revendication 12, dans lequel le groupe hétéroaryle tricyclique représente un groupe : 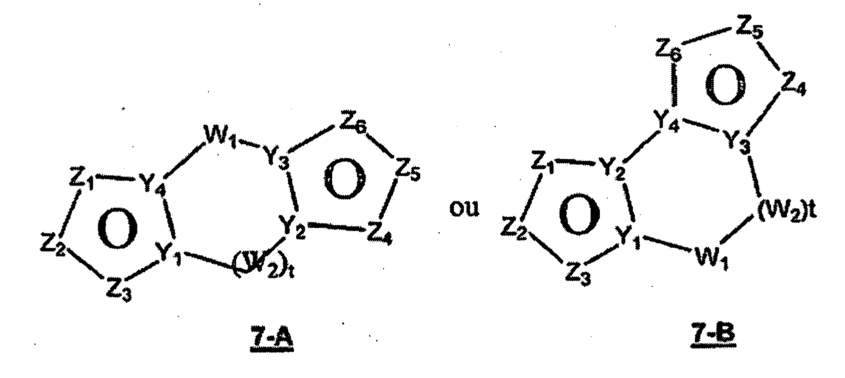
dans lequel Z₁, Z₂, Z₃, Z₄, Z₅ et Z₆ représentent, de manière indépendante, un groupe CR₂, un atome d'azote, un atome d'oxygène, un atome de soufre et un groupe N-R₁, à condition qu'un des radicaux Z₁ à Z₆ représente un atome de carbone auquel vient se fixer le reste de la molécule ;
Y₁, Y₂, Y₃ et Y₄ représentent, de manière indépendante, un atome de carbone ou un atome d'azote ;
W₁ et W₂ représentent, de manière indépendante, un groupe CR₄R₄, un groupe S(O)r (r = 0-2), un atome d'oxygène, un groupe N-R₁ ; avec cette réserve qu'aucune formation de liaison S-S, S-O ou O-O ne peut avoir lieu pour former un noyau saturé ;
R₁, R₂, R₆ et R₇ sont tels que définis à la revendication 1 ;
R₄ représente un atome d'hydrogène, un groupe alkyle en C₁-C₆ facultativement substitué, un groupe OH (à condition que les deux radicaux R₄ ne représentent pas un groupe OH), un groupe alcoxy en C₁-C₆, un groupe -S-alkyle en C₁-C₆, un groupe COOR₆, un groupe -NR₆R₇, un groupe -CONR₆R₇ ;
ou bien R₄R₄ peuvent représenter ensemble un groupe =O ou bien R₄R₄, ensemble avec l'atome de carbone auquel ils sont fixés peuvent former un système spiro de cinq à huit membres en présence ou en l'absence d'hétéroatomes choisis parmi un atome d'azote, un atome d'oxygène, un groupe S(O)ₙ (où n = 0 à 2), un groupe N-R₁ ;
et t = 1 à 3.

24. Procédé selon la revendication 12, dans lequel le groupe hétéroaryle tricyclique représente un groupe : 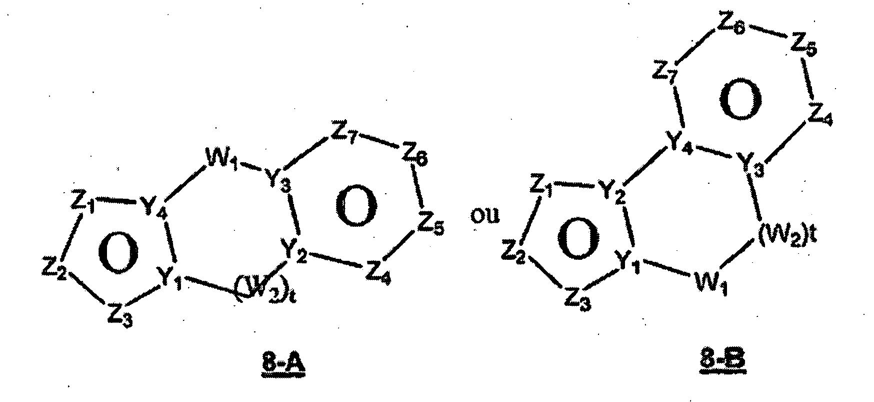
dans lequel Z₁, Z₂, Z₃, Z₄, Z₅, Z₆ et Z₇ représentent, de manière indépendante, un groupe CR₂, un atome d'azote, un atome d'oxygène, un atome de soufre ou un groupe N-R₁, à condition qu'un des radicaux Z₁ à Z₇ représente un atome de carbone auquel vient se fixer le reste de la molécule ;
Y₁, Y₂, Y₃ et Y₄ représentent, de manière indépendante, un atome de carbone ou un atome d'azote ;
W₁ et W₂ représentent, de manière indépendante, un groupe CR₄R₄, un groupe S(O)r (r = 0-2), un atome d'oxygène ou un groupe N-R₁ ; avec cette réserve qu'aucune formation de liaison S-S, S-O ou O-O ne peut avoir lieu pour former un noyau saturé ;
R₁, R₂, R₆ et R₇ sont tels que définis à la revendication 1 ;
R₄ représente un atome d'hydrogène, un groupe alkyle en C₁-C₆ facultativement substitué, un groupe OH (à condition que les deux radicaux R₄ ne représentent pas un groupe OH), un groupe alcoxy en C₁-C₆, un groupe -S-alkyle en C₁-C₆, un groupe COOR₆, un groupe -NR₆R₇, un groupe -CONR₆R₇ ;
ou bien R₄R₄ peuvent représenter ensemble un groupe =O ou bien R₄R₄, ensemble avec l'atome de carbone auquel ils sont fixés peuvent former un système spiro de cinq à huit membres en présence ou en l'absence d'hétéroatomes choisis parmi un atome d'azote, un atome d'oxygène, un groupe S(O)ₙ (où n **=** 0 à 2), un groupe N-R₁ ;
et t = à 3.

25. Procédé selon la revendication 12, dans lequel le groupe hétéroaryle tricyclique représente un groupe : 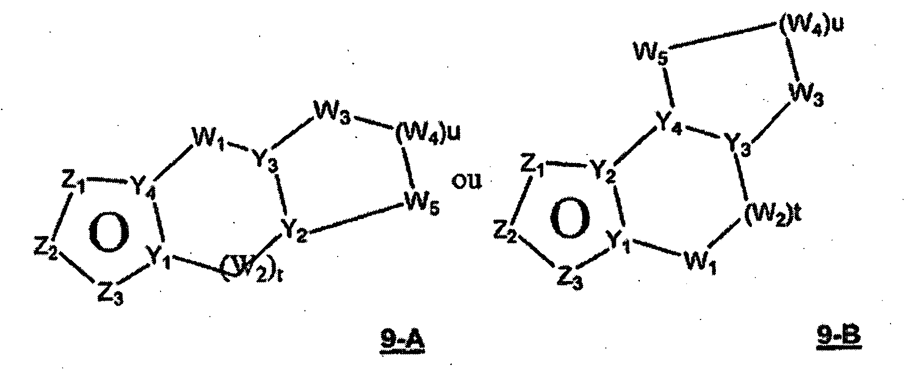
dans lequel Z₁, Z₂ et Z₃ représentent, de manière indépendante, un groupe CR₂, un atome d'azote, un atome d'oxygène, un atome de soufre ou un groupe N-R₁, à condition qu'un des radicaux Z₁ à Z₃ représente un atome de carbone auquel vient se fixer le reste de la molécule ;
Y₁ et Y₄ représentent, de manière indépendante, un atome de carbone ou un atome d'azote ;
Y₂ et Y₃ représentent, de manière indépendante, un groupe CH ou un atome d'azote ;
W₁, W₂, W₃, W₄ et W₅ représentent, de manière indépendante, un groupe CR₄R₄, un groupe S(O)r (r = 0-2), un atome d'oxygène ou un groupe N-R₁ ;
avec cette réserve qu'aucune formation de liaison S-S, S-O ou O-O ne peut avoir lieu pour former un noyau saturé ;
R₁, R₂, R₆ et R₇ sont tels que définis à la revendication 1 ;
R₄ représente un atome d'hydrogène, un groupe alkyle en C₁-C₆ facultativement substitué, un groupe OH (à condition que les deux radicaux R₄ ne représentent pas un groupe OH), un groupe alcoxy en C₁-C₆, un groupe -S-alkyle en C₁-C₆, un groupe COOR₆, un groupe -NR₆R₇, un groupe -CONR₆R₇;
ou bien R₄R₄ peuvent représenter ensemble un groupe =O ou bien R₄R₄, ensemble avec l'atome de carbone auquel ils sont fixés peuvent former un système spiro de cinq à huit membres en présence ou en l'absence d'hétéroatomes choisis parmi un atome d'azote, un atome d'oxygène, un groupe S(O)ₙ (où n = 0 à 2), un groupe N-R₁;
t=0à2;et
u = 1 à 3.

26. Procédé selon la revendication 12, dans lequel le groupe hétéroaryle tricyclique représente un groupe : 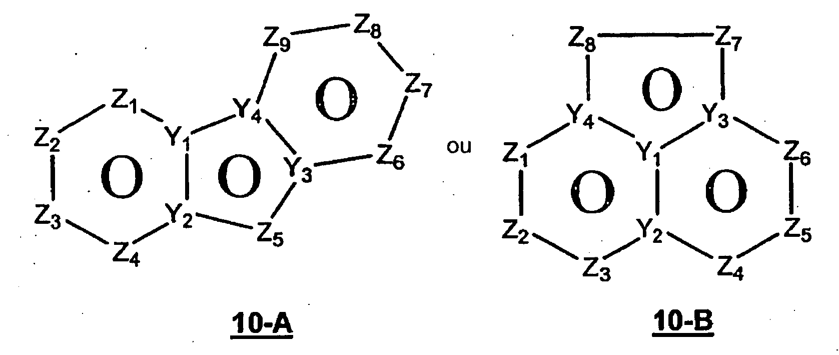
dans lequel Z₁, Z₂, Z₃, Z₄, Z₅, Z₆, Z₇, Z₈ et Z₉ représentent, de manière indépendante, un groupe CR₂, un atome d'azote, un atome d'oxygène, un atome de soufre ou un groupe N-R₁, à condition qu'un des radicaux Z₁ à Z₉ représente un atome de carbone auquel vient se fixer le reste de la molécule ;
R₁, R₂, R₆ et R₇ sont tels que définis à la revendication 1 ; et
Y₁, Y₂, Y₃ et Y₄ représentent, de manière indépendante, un atome de carbone ou un atome d'azote.

27. Procédé selon la revendication 12, dans lequel le groupe hétéroaryle tricyclique représente un groupe : 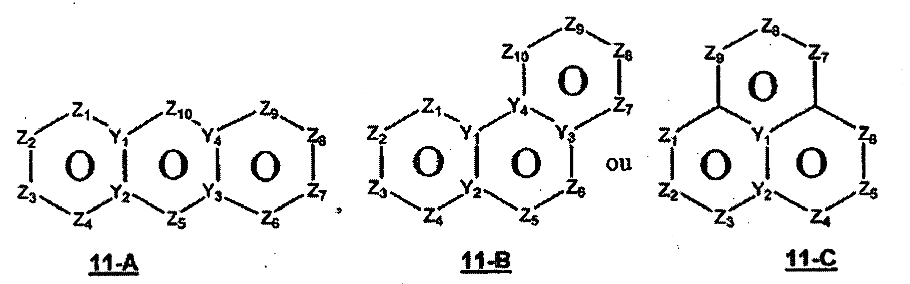
dans lequel Z₁, Z₂, Z₃, Z₄, Z₅, Z₆, Z₇, Z₈, Z₉ et Z₁₀ représentent, de manière indépendante, un groupe CR₂, un atome d'azote, un atome d'oxygène, un atome de soufre ou un groupe N-R₁, à condition qu'un des radicaux Z₁ à Z₁₀ représente un atome de carbone auquel vient se fixer le reste de la molécule ;
R₁, R₂, R₆ et R₇ sont tels que définis à la revendication 1 ; et
Y₁, Y₂, Y₃ et Y₄ représentent, de manière indépendante, un atome de carbone ou un atome d'azote.

28. Procédé selon la revendication 12, dans lequel le groupe hétéroaryle tricyclique représente un groupe : 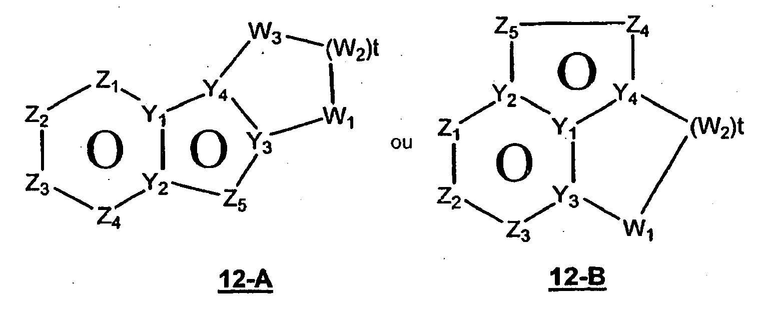
dans lequel Z₁, Z₂, Z₃ Z₄ et Z₅ représentent, de manière indépendante, un groupe CR₂, un atome d'azote, un atome d'oxygène, un atome de soufre ou un groupe N-R₁, à condition qu'un des radicaux Z₁ à Z₅ représente un atome de carbone auquel vient se fixer le reste de la molécule ;
Y₁, Y₂, Y₃ et Y₄ représentent, de manière indépendante, un atome de carbone ou un atome d'azote ;
W₁, W₂, W₃ représentent, de manière indépendante, un groupe CR₄R₄, un atome d'oxygène, un groupe N-R₁ ou un groupe S(O)r (r = 0-2), avec cette réserve qu'aucune formation de liaison S-S, S-O ou O-O ne peut avoir lieu pour former un noyau saturé ;
R₁, R₂, R₆ et R₇ sont tels que définis à la revendication 1;
R₄ représente un atome d'hydrogène, un groupe alkyle en C₁-C₆ facultativement substitué, un groupe OH (à condition que les deux radicaux R₄ ne représentent pas un groupe OH), un groupe alcoxy en C₁-C₆, un groupe -S-alkyle en C₁-C₆, un groupe COOR₆, un groupe -NR₆R₇, un groupe -CONR₆R₇;
ou bien R₄R₄ peuvent représenter ensemble un groupe =O ou bien R₄R₄, ensemble avec l'atome de carbone auquel ils sont fixés peuvent former un système spiro de cinq à huit membres en présence ou en l'absence d'hétéroatomes choisis parmi un atome d'azote, un atome d'oxygène, un groupe S(O)ₙ (où n = 0 à 2), un groupe N-R₁;
et t=1 à4.

29. Procédé selon la revendication 12, dans lequel le groupe hétéroaryle tricyclique représente un groupe : 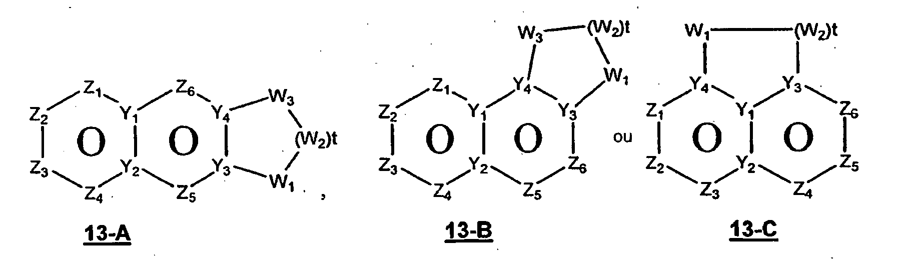
dans lequel Z₁, Z₂, Z₃ Z₄, Z₅ et Z₆ représentent, de manière indépendante, un groupe CR₂, un atome d'azote, un atome d'oxygène, un atome de soufre ou un groupe N-R₁, à condition qu'un des radicaux Z₁ à Z₆ représente un atome de carbone auquel vient se fixer le reste de la molécule ;
Y₁, Y₂, Y₃ et Y₄ représentent, de manière indépendante, un atome de carbone ou un atome d'azote ;
W₁, W₂ et W₃ représentent, de manière indépendante, un groupe CR₄R₄, un groupe S(O)r (r = 0-2), un atome d'oxygène ou un groupe N-R₁; avec cette réserve qu'aucune formation de liaison S-S, S-O ou O-O ne peut avoir lieu pour former un noyau saturé ;
R₁, R₂, R₆ et R₇ sont tels que définis à la revendication 1 ;
R₄ représente un atome d'hydrogène, un groupe alkyle en C₁-C₆ facultativement substitué, un groupe OH (à condition que les deux radicaux R₄ ne représentent pas un groupe OH), un groupe alcoxy en C₁-C₆, un groupe -S-alkyle en C₁-C₆, un groupe COOR₆, un groupe -NR₆R₇, un groupe -CONR₆R₇;
ou bien R₄R₄ peuvent représenter ensemble un groupe =O ou bien R₄R₄, ensemble avec l'atome de carbone auquel ils sont fixés peuvent former un système spiro de cinq à huit membres en présence ou en l'absence d'hétéroatomes choisis parmi un atome d'azote, un atome d'oxygène, un groupe S(O)ₙ (où n = 0 à 2), un groupe N-R₁ ;
et t = 1 à 3.

30. Procédé selon la revendication 12, dans lequel le groupe hétéroaryle tricyclique représente un groupe : 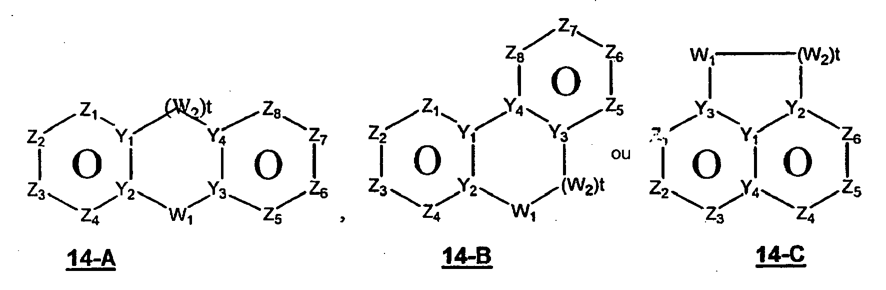
dans lequel Z₁, Z₂, Z₃ Z₄, Z₅, Z₆, Z₇ et Z₈ représentent, de manière indépendante, un groupe CR₂, un atome d'azote, un atome d'oxygène, un atome de soufre ou un groupe N-R₁, à condition qu'un des radicaux Z₁ à Z₈ représente un atome de carbone auquel vient se fixer le reste de la molécule ;
Y₁, Y₂, Y₃ et Y₄ représentent, de manière indépendante, un atome de carbone ou un atome d'azote ;
W₁ et W₂ représentent, de manière indépendante, un groupe CR₄R₄, un groupe S(O)r (r = 0-2), un atome d'oxygène ou un groupe N-R₁; avec cette réserve qu'aucune formation de liaison S-S, S-O ou O-O ne peut avoir lieu pour former un noyau saturé ;
R₁, R₂, R₆ et R₇ sont tels que définis à la revendication 1 ;
R₄ représente un atome d'hydrogène, un groupe alkyle en C₁-C₆ facultativement substitué, un groupe OH (à condition que les deux radicaux R₄ ne représentent pas un groupe OH), un groupe alcoxy en C₁-C₆, un groupe -S-alkyle en C₁-C₆, un groupe COOR₆, un groupe -NR₆R₇, un groupe -CONR₆R₇ ;
ou bien R₄R₄ peuvent représenter ensemble un groupe =O ou bien R₄R₄, ensemble avec l'atome de carbone auquel ils sont fixés peuvent former un système spiro de cinq à huit membres en présence ou en l'absence d'hétéroatomes choisis parmi un atome d'azote, un atome d'oxygène, un groupe S(O)ₙ (où n = 0 à 2), un groupe N-R₁;
et t=1à2.

31. Procédé selon la revendication 12, dans lequel le groupe hétéroaryle tricyclique représente un groupe : 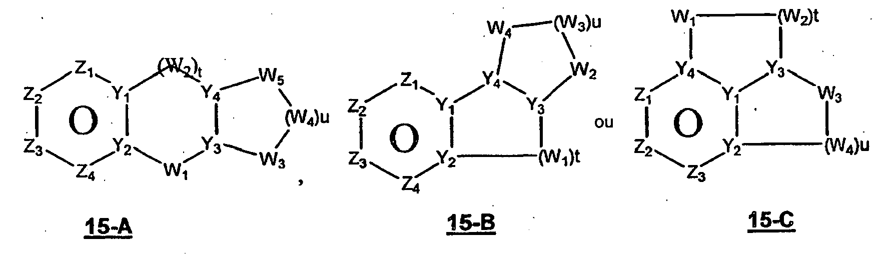
dans lequel Z₁, Z₂, Z₃ et Z₄ représentent, de manière indépendante, un groupe CR₂, un atome d'azote, un atome d'oxygène, un atome de soufre ou un groupe N-R₁, à condition qu'un des radicaux Z₁ à Z₄ représente un atome de carbone auquel vient se fixer le reste de la molécule ;
V₁, Y₂, Y₃ et Y₄ représentent, de manière indépendante, un atome de carbone ou un atome d'azote ;
W₁, W₂, W₃, W₄ et W₅ représentent, de manière indépendante, un groupe CR₄R₄, un groupe S(O)r (r = 0-2), un atome d'oxygène ou un groupe N-R₁;
avec cette réserve qu'aucune formation de liaison S-S, S-O ou O-O ne peut avoir lieu pour former un noyau saturé ;
R₁, R₂, R₆ et R₇ sont tels que définis à la revendication 1 ;
R₄ représente un atome d'hydrogène, un groupe alkyle en C₁-C₆ facultativement substitué, un groupe OH (à condition que les deux radicaux R₄ ne représentent pas un groupe OH), un groupe alcoxy en C₁-C₆, un groupe -S-alkyle en C₁-C₆, un groupe COOR₆, un groupe -NR₆R₇, un groupe -CONR₆R₇ ;
ou bien R₄R₄ peuvent représenter ensemble un groupe =O ou bien R₄R₄, ensemble avec l'atome de carbone auquel ils sont fixés peuvent former un système spiro de cinq à huit membres en présence ou en l'absence d'hétéroatomes choisis parmi un atome d'azote, un atome d'oxygène, un groupe S(O)ₙ (où n = 0 à 2), un groupe N-R₁;
t = 1 à 3 ; et
u = 1 à 3.

32. Dérivé de 6-bromo-pénème de structure **16** 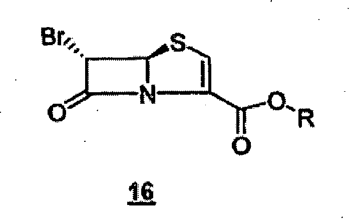 dans lequel R représente un groupe p-nitrobenzyle.

33. Procédé pour la préparation du (5R,6S)-6-bromopénème-3-carboxylate de 4-nitrobenzyle selon la revendication 32, qui comprend les étapes suivantes :
(A) (i) la mise en réaction de l'acide 6-aminopénicillanique avec de l'acide bromhydrique dans un solvant organique et de l'eau pour obtenir le dérivé 6-bromo 21 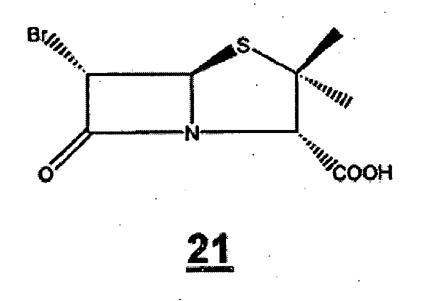 et (ii) la conversion du dérivé de l'acide 6-bromopénicillanique 21 pour obtenir le 6-bromopénicillanate de p-nitrobenzyle 22 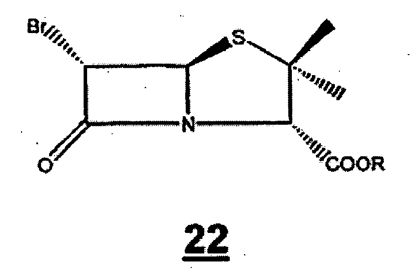 dans lequel R représente un groupe p-nitrobenzyle, en utilisant du bromure de 4-nitrobenzyle en présence d'une base dans un solvant organique ;
(B) l'oxydation du 6-bromopénicillanate de 4-nitrobenzyle **22** pour obtenir le 1-oxyde du 6-bromopénicillanate de 4-nitrobenzyle **23** 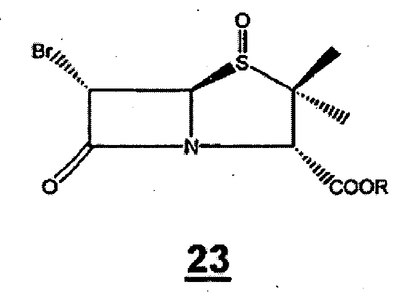
(C) le chauffage à reflux du 1-oxyde du 6-bromopénicillanate de 4-nitrobenzyle **23** avec du 2-mercaptobenzothiazole dans un solvant organique pour obtenir le (2R)-2-[(3S,4R)-4(benzothiazol-2-yldithio)-3-bromo-2-oxoazétidin-1-yl]-3-méthylbut-3-énoate de 4-nitrobenzyle **24** ;
(D) la dissolution du (2R)-2-[(3S,4R)-4(benzothiazol-2-yldithio)-3-bromo-2-oxoazétidin-1-yl]-3-méthylbut-3-énoate de 4-nitrobenzyle **24** dans un solvant organique et la mise en réaction de la solution avec une base tertiaire organique pour obtenir le 2-[(3S,4R)-4-(benzothiazol-2-yldithio)-3-bromo-2-oxoazétidin-1-yl]-3-méthylbut-2-énoate de 4-nitrobenzyle **25** ;
(E) la conversion du 2-[(3S,4R)-4-(benzothiazol-2-yldithio)-3-bromo-2-oxoazétidin-1-yl]-3-méthylbut-2-énoate de 4-nitrobenzyle **25** en 2-[(3S,4R)-3-bromo-4-formylthio-2-oxoazétidin-1-yl]-3-méthylbut-2-énoate de 4-nitrobenzyle 26 par mise en réaction dans un solvant organique aromatique en présence d'un acide organique, d'un anhydride acétique/d'une base tertiaire organique et de trialkyl- ou triaryl-phosphine à une température d'environ -10 °C à environ -30 °C ;
(F) ledit 2-[(3S,4R)-3-bromo-4-formylthio-2-oxoazétidin-1-yl]-3-méthylbut-2-énoate de 4-nitrobenzyle **26** étant repris dans un solvant organique à une température de -70 °C à -90 °C pour y faire passer de l'oxygène ozonizé pendant un laps de temps de 3 à 4 heures, avant de procéder à une cyclisation intramoléculaire en utilisant un réactif de phosphite pour obtenir le (5R,6S)-6-bromopénème-3-carboxylate de 4-nitrobenzyle **16.**

34. Procédé selon la revendication 1, pour la préparation du composé répondant la formule **I** 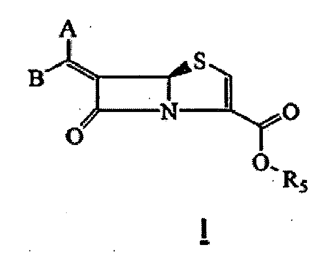 comme défini à la revendication 1, dans lequel on prépare le (5R,6S)-6-bromopénème-3-carboxylate de 4-nitrobenzyle **16** que l'on utilise à l'étape (a) conformément à la revendication 33.

35. Procédé selon la revendication 33 ou 34, dans lequel on dissout l'acide 6-aminopénicillanique dans du méthanol ou dans du THF.

36. Procédé selon l'une quelconque des revendications 33 à 35, dans lequel on met en oeuvre l'étape (A)(i) en présence d'acide bromhydrique à 48 % en poids/poids et en présence d'une solution de nitrite de sodium ou de potassium.

37. Procédé selon l'une quelconque des revendications 33 à 36, dans lequel on met en oeuvre l'étape (A)(i) à une température d'environ -10 °C à environ -30 °C.

38. Procédé selon l'une quelconque des revendications 33 à 37, dans lequel la base à l'étape (A)(ii) représente le carbonate de sodium ou de potassium et le solvant organique est le THF ou le DMF.

39. Procédé selon l'une quelconque des revendications 33 à 38, dans lequel le solvant aromatique à l'étape (C) représente le toluène ou le xylène.

40. Procédé selon l'une quelconque des revendications 33 à 39, comprenant la conversion séquentielle des composés **23** à **26**, en l'absence d'une isolation des produits intermédiaires.

41. Procédé selon la revendication 40, dans lequel on fait réagir le 1-oxyde de 6-bromopénicillanate de 4-nitrobenzyle **23** avec du mercaptobenzothiazole dans un solvant organique aromatique chauffé à reflux et on traite avec de la triéthylamine à une température d'environ 0 à -20 °C pour obtenir un mélange réactionnel, ledit mélange réactionnel étant chargé avec un acide organique et avec un anhydride, avec une base tertiaire organique et avec un phosphate de trialkyle ou de triaryle de manière séquentielle à une température d'environ -10 °C à -40 °C.
